# FASCICULE DE BREVET EUROPEEN

(11) **EP 0 984 960 B1**
(45) Date de publication et mention de la délivrance du brevet: **05.02.2003**
(21) Numéro de dépôt: 98924383.7
(22) Date de dépôt: 06.05.1998
(51) Int. Cl.: C07D 403/12, C07D 403/14, A61K 31/41, C07D 249/14

(54) **N-TRIAZOLYL-2-INDOLECARBOXAMIDES ET LEUR UTILISATION COMME AGONISTES DE CCK-A**
N-TRIAZOLYL-2-INDOLCARBONSÄUREAMIDE UND IHRE VERWENDUNG ALS CCK-A AGONISTEN
N-TRIAZOLYL-2-INDOLECARBOXAMIDES AND THEIR USE AS CCK-A AGONISTS

(30) Priorité: 13.05.1997 FR 9705850
(43) Date de publication de la demande: 15.03.2000
(73) Titulaire: SANOFI-SYNTHELABO, 75013 Paris (FR)
(72) Inventeur: BIGNON, Eric, F-31120 Pinsaguel (FR); BRAS, Jean-Pierre, F-31500 Toulouse (FR); DE COINTET, Paul, F-31000 Toulouse (FR); DESPEYROUX, Pierre, F-31860 Labarthe sur Leze (FR); FREHEL, Daniel, F-31160 Estadens (FR); GULLY, Danielle, F-31160 Muret (FR)
(74) Mandataire: Thouret-Lemaitre, Elisabeth
(86) Numéro de dépôt international: FR9800905
(87) Numéro de publication internationale: WO98051686

(56) Documents cités:
- EP-A- 0 611 766
- EP-A- 0 620 221

## Description

La présente invention concerne des dérivés du triazole, un procédé pour leur préparation et les médicaments les contenant.

Plus particulièrement, la présente invention a pour objet des composés non peptidiques affins pour les récepteurs de la cholécystokinine (CCK).

La CCK est un peptide qui, en réponse à une ingestion d'aliment, est sécrétée au niveau périphérique et participe à la régulation de nombreux processus digestifs (Crawley JN. *et al*., Peptides, 1994, *15* (4), 731-735).

La CCK a été identifiée par la suite dans le cerveau, et pourrait être le neuropeptide le plus abondant agissant comme neuromodulateur des fonctions cérébrales par stimulation des récepteurs de type CCK-B (Crawley J.N. *et al*., Peptides, 1994, *15* (4), 731-735). Dans le système nerveux central, la CCK interagit avec la transmission neuronale médiée par la dopamine (Crawley J.N. *et al*., ISIS Atlas of Sci., Pharmac, 1988, 84-90). Elle intervient également dans des mécanismes impliquant l'acétylcholine, le gaba (acide 4-aminobutyrique), la sérotonine, les opioïdes, la somatostatine, la substance P et dans les canaux ioniques.

Son administration provoque des modifications physiologiques : ptose palpébrale, hypothermie, hyperglycémie, catalepsie; et des modifications comportementales : hypolocomotricité, diminution de l'exploration, analgésie, modification de la faculté d'apprentissage, modification du comportement sexuel et satiété.

Le CCK exerce son activité biologique par l'intermédiaire d'au moins deux types de récepteurs : les récepteurs CCK-A localisés principalement en périphérie, et les récepteurs CCK-B présents essentiellement dans le cortex cérébral. Les récepteurs CCK-A de type périphérique sont aussi présents dans certaines zones du système nerveux central incluant l'area postrema, le noyau de tractus solitaire et le noyau interpédonculaire (Moran T.H. *et al*., Brain Research, 1986, *362*, 175-179 ; Hill D.R. *et al*., J. Neurosci, 1990, *10*, 1070-1081 ; avec cependant des différences d'espèce (Hill D.R. *et al*., J. Neurosci, 1990, *10*, 1070-1081) ; Mailleux P. *et al*., Neurosci Lett., 1990, *117*, 243-247 ; Barrett R.W. *et al*., Mol. Pharmacol., 1989, *36*, 285-290 ; Mercer J.G. *et al*., Neurosci Lett., 1992, *137*, 229-231 ; Moran T.H. *et al*., Trends in Pharmacol. Sci., 1991, *12*, 232-236).

A la périphérie, par l'intermédiaire des récepteurs CCK-A (Moran T.H. *et al*., Brain Research, 1986, *362,* 175-179), la CCK retarde la vidange gastrique, module la motilité intestinale, stimule la contraction vésiculaire, augmente la sécrétion biliaire, contrôle la sécrétion pancréatique (McHugh P.R. *et al*., Fed. Proc., 1986, *45*, 1384-1390 ; Pendleton R.G. *étal.,* J. Pharmacol. Exp. Ther., 1987, *241*, 110-116).

La CCK pourrait agir dans certains cas sur la pression artérielle et influencer les sytèmes immunitaires.

Le rôle de la CCK dans le signal de satiété est supporté par le fait que les concentrations plasmatiques de CCK, dépendantes de la composition des repas (fortes concentrations de protéines ou de lipides) sont, après les repas, supérieures à celles observées avant les repas (Izzo R.S. *et al*., Regul. Pept., 1984, *9*, 21-34 ; Pfeiffer A. *et al*., Eur. J. Clin. Invest., 1993, *23,* 57-62 ; Lieverse R.J. Gut, 1994, *35*, 501). Chez les boulimiques, il y a une diminution de la sécrétion de la CCK induite par un repas, (Geraciotti T.D. Jr. *et al*., N. Engl. J. Med., 1988, *319*, 683-688 ; Devlin M.J. *et al*., Am. J. Clin. Nutr., 1997, *65*, 114-120) et une baisse des concentrations de CCK dans le liquide cérébrospinal (Lydiard R.B. *et al*., Am. J. Psychiatry, 1993, *150*, 1099-1101). Dans les lymphocytes T, un compartiment cellulaire pouvant refléter les sécrétions neuronales centrales, les concentrations basales de CCK sont significativement inférieures chez les patients atteints de Bulimia nervosa (Brambilla F. *et al*., Psychiatry Research, 1995, *37*, 51-56).

Les traitements (par exemple par la L-phénylalanine, ou les inhibiteurs de trypsine) qui augmentent la sécrétion de la CCK endogène provoquent une réduction de la prise alimentaire dans plusieurs espèces dont l'homme (Hill A.J. *et al*., Physiol. Behav. 1990, *48*, 241-246 ; Ballinger A.B. *et al*., Metabolism 1994, *43*, 735-738). De même, l'administration de CCK exogène réduit la prise de nourriture dans de nombreuses espèces dont l'homme (Crawley J .N. *et al*. Peptides 1994, *15*, 731-755).

L'inhibition de la prise de nourriture par la CCK est médiée par le récepteur CCK-A. En effet, le devazepide, un antagoniste sélectif des récepteurs CCK-A, inhibe l'effet anorexigène de la CCK alors que les agonistes sélectifs de ces récepteurs inhibent la prise de nourriture (Asin K.E. *et al*., Pharmacol. Biochem. Behav. 1992, *42*, 699-704 ;

Elliott R.L. *et al*., J. Med. Chem. 1994, 37, 309-313 ; Elliott R.L. *et al*., J. Med. Chem. 1994, *37*, 1562-1568). De plus, les rats OLEFT, n'exprimant pas le récepteur CCK-A sont insensibles à l'effet anorexigène de la CCK (Miyasaka K. *et al*., 1994, *180*, 143-146).

Basé sur ces évidences du rôle clé de la CCK dans le signal de satiété périphérique, l'utilité d'agonistes et d'antagonistes de la CCK comme médicament dans le traitement de certains troubles du comportement alimentaire, de l'obésité et du diabète est indiscutable. Un agoniste des récepteurs de la CCK peut aussi être utilisé en thérapeutique dans le traitement des troubles du comportement émotionnel, sexuel et amnésique (Itoh S. *et al*., Drug. Develop. Res., 1990, *21*, 257-276), de la schizophrénie, des psychoses (Crawley J.N. *et al*., Isis Atlas of Sci., Pharmac., 1988, 84-90 et Crawley J.N., Trends in Pharmacol. Sci., 1991, *12*, 232-265), de la maladie de Parkinson (Bednar I. *et al*., Biogenic amine, 1996, *12*(4), 275-284), des dyskinésies tardives (Nishikawa T. *et al*., Prog. Neuropsychopharmacol. Biol. Psych., 1988, *12*, 803-812: Kampen J.V. *et al*., Eur. J. Pharmacol., 1996, *298*, 7-15) et de divers troubles de la sphère gastrointestinale (Drugs of the Future, 1992, *17*(3), 197-206).

Des agonistes du récepteur CCK-A de la CCK sont décrits dans la littérature. Par exemple, certains produits ayant de telles propriétés sont décrits dans EP383690 et WO90/06937, WO95/28419, WO96/11701 ou encore WO96/11940

La plupart des agonistes CCK-A décrits à ce jour sont de nature peptidique. Ainsi, le FPL 14294 dérivé de la CCK-7 est un puissant agoniste CCK-A non sélectif *vis-à-vis* des récepteurs CCK-B. Il possède une puissante activité inhibitrice de la prise de nourriture chez le rat et chez le chien après administration intranasale (Simmons R.D. *et al*., Pharmacol. Biochem. Behav., 1994, *47* (3), 701-708 ; Kaiser E.F. *et al*., Faseb, 1991, 5, A864). De même, il a été montré que le A-71623, un tétrapeptide agoniste sélectif des récepteurs CCK-A, est efficace dans des modèles d'anorexie sur une période de 11 jours et entraîne une réduction significative de la prise de poids par rapport au contrôle chez les rongeurs et les singes cynomologues (Asin K.E. *et al*., Pharmacol. Biochem. Behav., 1992, *42*, 699-704). De la même façon, des analogues structuraux du A 71623, possédant une bonne efficacité et sélectivité pour les récepteurs CCK-A sont dotés d'une puissante activité anorexigène chez le rat (Elliott R.L. *et al*., J. Med. Chem., 1994, *37*, 309-313 ; Elliott R.L. *et al*., J. Med. Chem., 1994, *37*, 1562-1568). Le GW 7854 (Hirst G.C. *et al*., J. Med. Chem., 1996, *39*, 5236-5245), une benzodiazépine-1,5, est un agoniste des récepteurs CCK-A *in vitro*. Cette molécule est également active par voie orale sur la contraction de la vésicule biliaire chez la souris et sur la prise de nourriture chez le rat.

On a maintenant trouvé, de façon surprenante, qu'une série de dérivés de triazole possède une activité agoniste partielle ou totale des récepteurs CCK-A.

Les composés selon l'invention ont fait l'objet d'études systématiques pour caractériser :
- leur potentialité pour déplacer [¹²⁵I]-CCK de ses sites de liaison présents sur des membranes pancréatiques de rat (récepteur CCK-A) ou de cellules 3T3 exprimant le récepteur recombinant CCK-A humain ;
- leur affinité *vis-à-vis* du récepteur CCK-B présent sur des membranes de cortex de cobaye, certains des composés étant des ligands sélectifs ou non des récepteurs CCK-A ;
- leur propriété agoniste des récepteurs CCK-A à travers leur capacité à induire *in vitro* une mobilisation du calcium intracellulaire dans les cellules 3T3 exprimant le récepteur CCK-A humain.

Les dérivés du triazole, selon la présente invention, sont des agonistes CCK-A puisqu'ils sont capables de stimuler partiellement, ou totalement comme la CCK, la mobilisation du calcium intracellulaire dans une lignée cellulaire exprimant le récepteur recombinant CCK-A humain. Ils sont, de manière surprenante, beaucoup plus puissants que les dérivés : du thiazole décrits dans les demandes de brevets EP518731, EP611766, du thiadiazole décrits dans la demande de brevet EP620221, des benzodiazépines décrits dans le brevet EP667344.

En effet, ces dérivés thiazole, thiadazole et benzodiazépine sont incapables d'induire cette mobilisation du calcium intracellulaire médiée par le récepteur CCK-A.

Les dérivés du triazole selon l'invention sont également beaucoup plus puissants que ces dérivés thiazole, thiadiazole ou benzodiazépine par leur capacité à bloquer *in vivo,* par la voie intrapéritonéale, la vidange gastrique chez la souris.

Ainsi, les propriétés agonistes CCK-A ont été étudiées *in vivo*, en évaluant leur capacité à bloquer la vidange gastrique chez la souris ou à provoquer, toujours *in vivo,* la vidange de la vésicule biliaire chez la souris.

Certains dérivés possèdent également une activité antagoniste des récepteurs CCK-B.

Ainsi, la présente invention concerne des composés de formule : 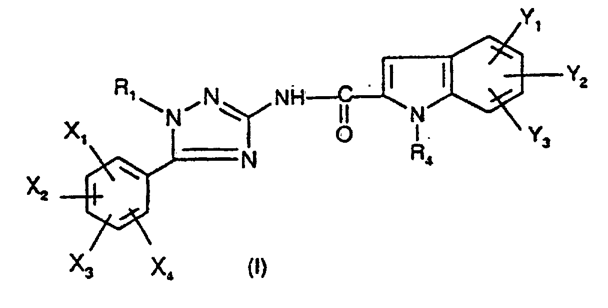 dans laquelle :
- R₁ représente un (C₂-C₆)alkyle ; un groupe -(CH₂)ₘ-G avec m variant de 0 à 5 et G représentant un groupe hydrocarboné mono-ou polycyclique non aromatique en C₃-C₁₃ éventuellement substitué par un ou plusieurs (C₁-C₃)alkyle ; un phényl(C₁-C₃)alkyle dans lequel le groupe phényle est éventuellement substitué une ou plusieurs fois par un halogène, par un (C₁-C₃)alkyle ou par un (C₁-C₃)alcoxy ; un groupe -(CH₂)ₙNR₂R₃ dans lequel n représente un nombre entier de 1 à 6 et R₂ et R₃ identiques ou différents représentent un (C₁-C₃)alkyle ou constituent avec l'atome d'azote auquel ils sont liés un groupe morpholino, pipéridino, pyrolidinyle ou pipérazinyle ;
- X₁,X₂,X₃ ou X₄ représentent chacun indépendamment un atome d'hydrogène, d'halogène, un (C₁-C₆)alkyle, un (C₁-C₃)alcoxy ou un trifluorométhyle; étant entendu qu'un seul parmi X₁, X₂, X₃ et X₄ représente éventuellement un atome d'hydrogène ;
- R₄ représente l'hydrogène, un groupe -(CH₂)ₙCOOR₅ dans lequel n est tel que défini précédemment et R₅ représente un atome d'hydrogène, un (C₁-C₆)alkyle ou un (C₆-C₁₀)aryl-(C₁-C₆)alkyle ; un (C₁-C₆)alkyle ; un groupe -(CH₂)ₙOR₅ ou un groupe -(CH₂)ₙNR₂R₃ dans lesquels n, R₂, R₃ et R₅ sont tels que définis précédemment ; un groupe -(CH₂)ₙ- tétrazolyle dans lequel n est tel que défini précédemment,
   ou R₄ représente l'un de ces groupes sous la forme de sel d'un métal alcalin ou alcalino-terreux;
- Y₁, Y₂ et Y₃ représentent indépendamment un hydrogène, un halogène, un (C₁-C₃)alkyle, un (C₁-C₃)alcoxy, un nitro, cyano, (C₁-C₆)acylamino, carbamoyle, trifluorométhyle, un groupe COOR₆ dans lequel R₆ représente l'hydrogène, ou (C₁-C₃)alkyle;
ou un de leurs sels ou solvates.

Selon la présente invention, par "(C₁-C₆)alkyle" ou "(C₂-C₆)alkyle", on entend un alkyle droit ou ramifié ayant 1 à 6 atomes de carbone ou respectivement 2 à 6 atomes de carbone.

Le radical alkoxy désigne un radical alkyloxy dans lequel alkyle est tel que défini ci-dessus.

Le radical acyle désigne un radical alkylcarbonyle dans lequel alkyle est tel que défini ci-dessus. (C₁-C₆)acylamino est un radical alkylcarbonylamino en C₁-C₆.

Les groupes hydrocarbonés non aromatiques en C₃-C₁₃ comprennent les radicaux mono- ou poly-cycliques, condensés ou pontés, saturés ou insaturés, éventuellement terpéniques. Ces radicaux sont éventuellement mono- ou poly- substitués par un (C₁-C₃)alkyle. Les radicaux monocycliques incluent les cycloalkyles par exemple les cyclopropyle, cyclobutyle, cyclopentyle, cyclohexyle, cycloheptyle, cyclooctyle, cyclododécyle. Les radicaux polycycliques incluent par exemple le norbornane, l'adamantane, l'hexahydroindane, le norbornène, le dihydrophénalène, le bicyclo [2.2.1]heptane, le bicyclo [3.3.1]nonane ; le tricyclo [5.2.1.0^{2,6}]décane.

Selon la présente invention, par halogène on entend un atome choisi parmi le fluor, le chlore, le brome ou l'iode, de préférence le fluor ou le chlore.

Des exemples de groupes aryle sont phényle et naphtyle.

Les cations de métaux alcalins ou alcalino-terreux sont de préférence choisis parmi sodium, potassium ou calcium.

Lorsqu'un composé selon l'invention présente un ou des carbones asymétriques, les isomères optiques de ce composé font partie intégrante de l'invention.

Lorsqu'un composé selon l'invention présente une stéréoisomérie par exemple de type axial-équatorial, l'invention comprend tous les stéréoisomères de ce composé.

Les sels des composés de formule (I) selon la présente invention comprennent ceux avec des acides minéraux ou organiques qui permettent une séparation ou une cristallisation convenable des composés de formule (I), tels que l'acide picrique, l'acide oxalique ou un acide optiquement actif, par exemple un acide tartrique, un acide dibenzoyltartrique, un acide mandélique ou un acide camphosulfonique, et ceux qui forment des sels physiologiquement acceptables, tels que le chlorhydrate, le bromhydrate, le sulfate, l'hydrogénosulfate, le dihydrogénophosphate, le maléate, le fumarate, le 2-naphtalènesulfonate, le paratoluènesulfonate.

Les sels des composés de formule (I) comprennent également des sels avec des bases organiques ou minérales, par exemple les sels des métaux alcalins ou alcalino-terreux, comme les sels de sodium, de potassium, de calcium, les sels de sodium et de potassium étant préférés, ou avec une amine, telle que le trométamol, ou bien les sels d'arginine, de lysine, ou de toute amine physiologiquement acceptable.

Les groupes fonctionnels éventuellement présents dans la molécule des composés de formule (I) et dans les intermédiaires réactionnels peuvent être protégés, soit sous forme permanente soit sous forme temporaire, par des groupes protecteurs qui assurent une synthèse univoque des composés attendus.

Par groupe protecteur temporaire des aminés, alcools ou des acides carboxyliques on entend les groupes protecteurs tels que ceux décrits dans Protective Groups in Organic Synthesis, Greene T.W. et Wuts P.G.M., ed. John Wiley et Sons, 1991 et dans Protecting Groups, Kocienski P.J., 1994, Georg Thieme Verlag.

Les composés (I) peuvent comporter des groupes précurseurs d'autres fonctions qui sont générées ultérieurement en une ou plusieurs autres étapes.

Les composés de formule (I) dans lesquels R₁ représente un cyclohexyl(C₁-C₃)-alkyle sont des composés préférés.

Egalement préférés sont les composés de formule (I), dans lesquels le phényle en position 5 du triazole est trisubstitué de préférence par un méthoxy en position 2 et 6, et par un méthyle en position 4.

Et encore plus préférés sont les composés de formule (I) dans lesquels le phényle en position 5 du triazole est trisubstitué de préférence par un méthoxy en position 2 et 5 et par un méthyle ou un chlore en position 4.

De façon particulière, on préfère les composés de formule : 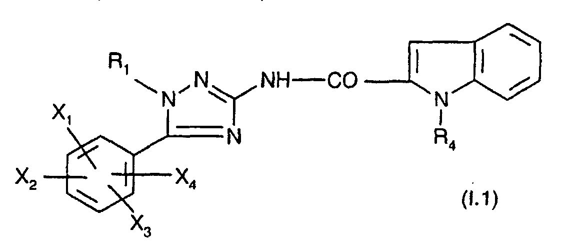 dans laquelle R₁, R₄, X₁, X₂, X₃ et X₄ sont tels que définis pour (I); un de leurs sels ou solvates.

Parmi ces composés, on préfère ceux dans lesquels 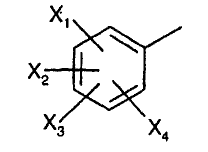 représente 2,6-diméthoxy-4-méthylphényle.

Plus particulièrement préférés sont les composés de formule : 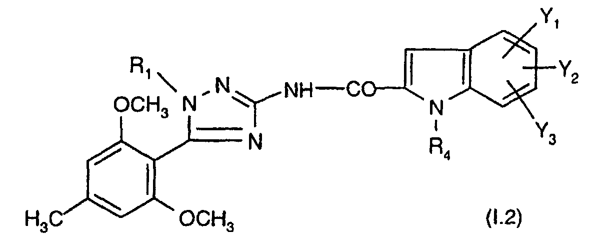 dans laquelle R₁ et R₄ sont tels que définis pour (I); un de leurs sels ou solvates.

On préfère tout particulièrement les composés de formule : 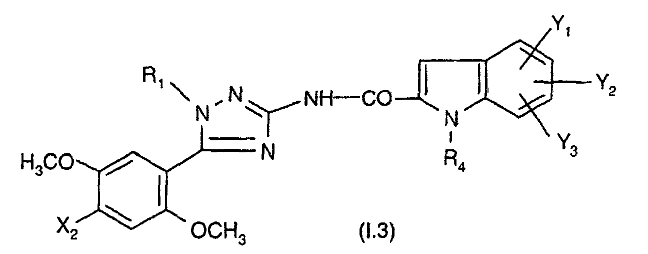 dans laquelle R₁, R₄, Y₁, Y₂ et Y₃ sont tels que définis pour (I), et X₂ représente un méthyle ou un atome de chlore ; un de leurs sels ou solvates.

La présente invention a également pour objet un procédé pour la préparation des composés de formule (I), comprenant la réaction d'un aminotriazole de formule : 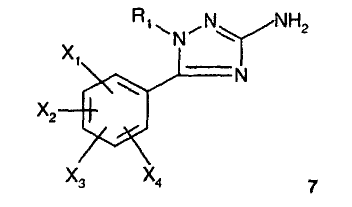 dans lequel R₁, X₁, X₂, X₃ et X₄ sont tels que définis pour (I)
- soit avec un dérivé acide indole carboxylique de formule 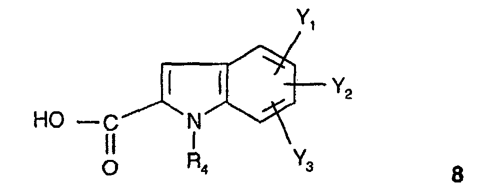 dans laquelle R₄, Y₁, Y₂ et Y₃ sont tels que définis ci-dessus pour (I);
- soit avec un dérivé acide indole carboxylique de formule : 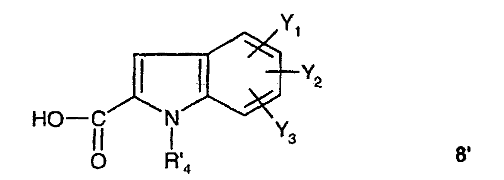 dans laquelle Y₁,Y₂,Y₃ sont tels que définis ci-dessus pour (I) et R'₄ est un groupe précurseur de R₄, auquel cas on forme le composé de formule: 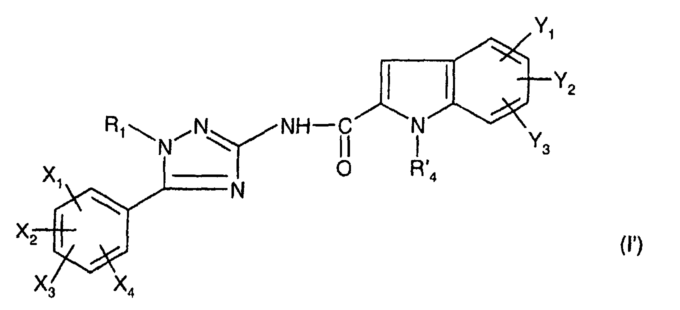 dans laquelle R₁, X₁, X₂, X₃, X₄, Y₁, Y₂ et Y₃ sont tels que définis pour (I) et R'₄ est un groupe précurseur de R₄, R₄ étant tel que défini pour (I) ;
pour obtenir les composés de formule (I), un de leurs sels ou solvates.

Les composés intermédiaires (I') conduisent aux composés de formule (I) par transformation du groupe R'₄ en R₄, laquelle est mise en oeuvre de façon connue en soi selon des procédés conventionnels de la chimie organique.

Les aminotriazoles de formule 7 constituent des intermédiaires clés, nouveaux, utiles pour la préparation des composés (I) et forment un objet de l'invention.

Les produits de départ sont disponibles commercialement ou préparés selon les méthodes ci-après.

Le schéma 1 suivant illustre une voie de synthèse des composés de formule **7**.

Le schéma 2 suivant illustre la préparation des composés de formule (I) à partir des aminotriazoles de formule **7**.

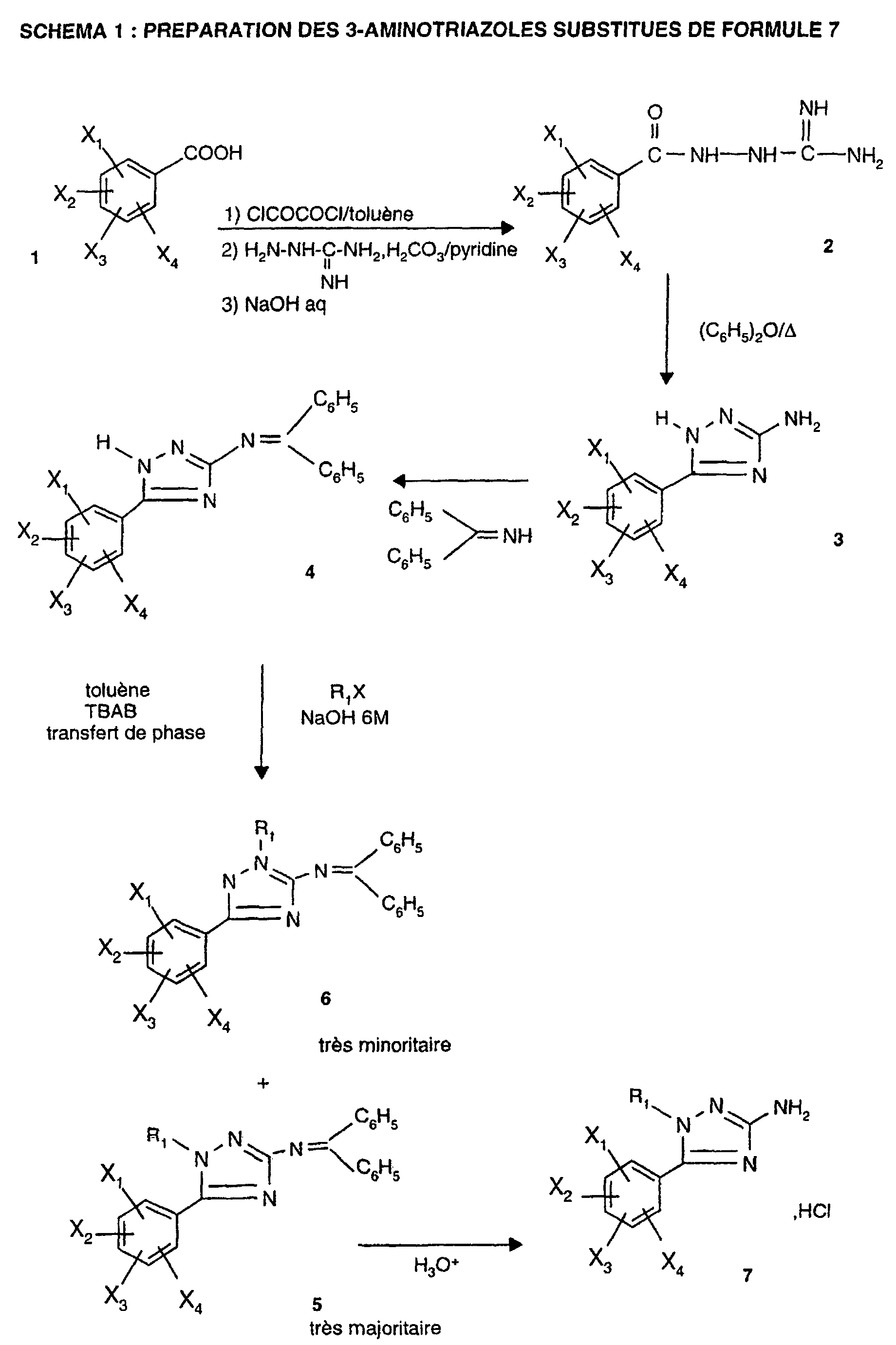

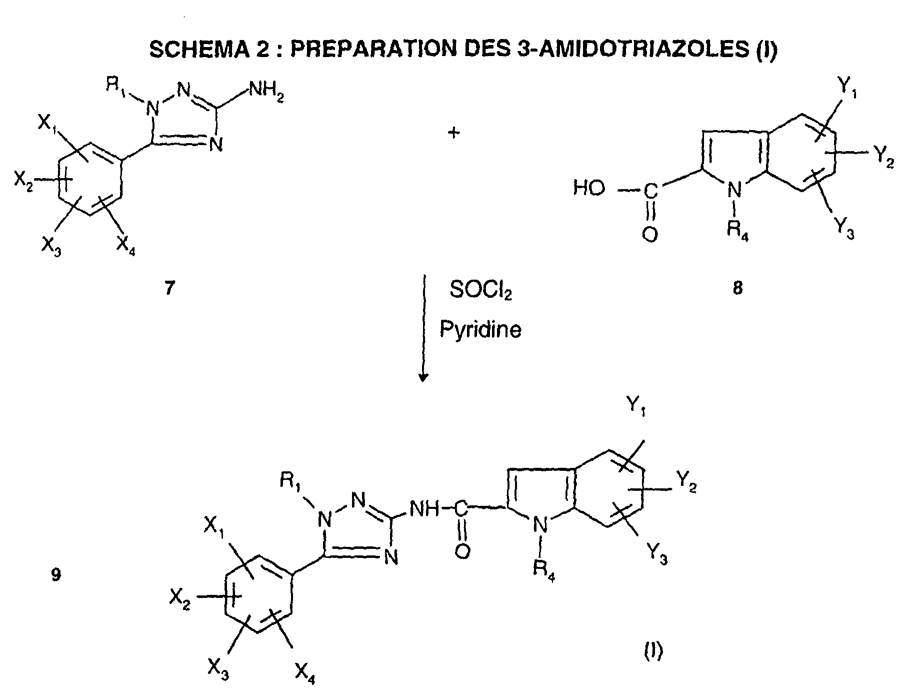

Lorsque R₄ = -(CH₂)ₙCOOH, les composés (I) sont obtenus à partir des esters correspondants, eux-mêmes obtenus à partir du SCHEMA 2.

Lorsque R₄ = -(CH₂)ₙ-tétrazolyle, les composés (I) sont obtenus à partir des nitriles correspondants de formule : 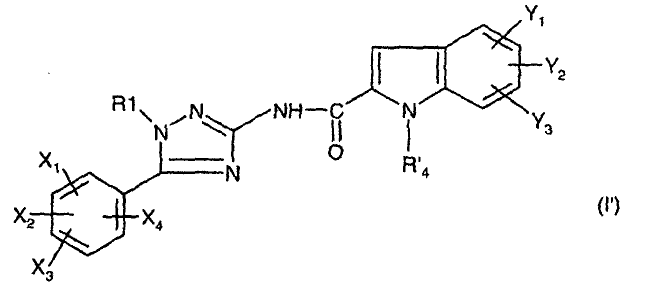 dans laquelle R'₄ = ―(CH₂)ₙ―C≡N, par action de l'azidotriméthylsilane en présence d'oxyde de dibutylétain selon le procédé décrit dans J.Org. Chem. 1993, *58*, 4139-4141.

Les composés de formule (l') sont obtenus selon le SCHEMA 2, à partir des composés 7 et 8' de formule : 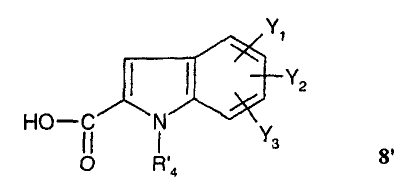 dans laquelle R'₄ = ―(CH₂)ₙ―C≡N

Les acides benzoïques substitués 1 sont disponibles commercialement ou préparés selon une adaptation des procédés décrits dans la littérature, par exempte :
1) par lithiation régiosélective de benzènes substitués, suivie de carboxylation du dérivé lithié avec CO₂ selon le SCHEMA 3 : 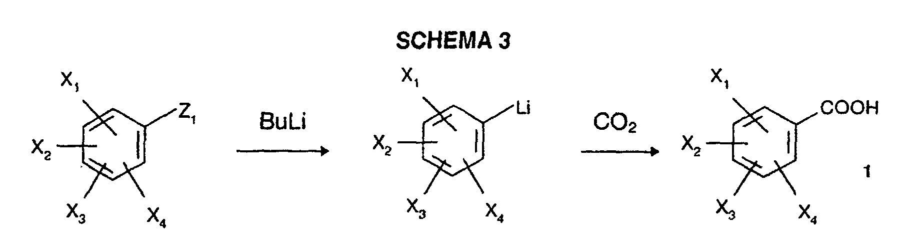 avec Z₁ = Br ou H suivant la nature et/ou la position des substituants X₁, X₂, X₃, X₄ selon : N.S. Narasimhan *et al*., Indian J. Chem., 1973, *11*, 1192 ; R.C. Gambie *et al*., Austr. J. Chem., 1991, *44*, 1465 ; T. de Paulis *et al*., J. Med. Chem., 1986, *29*, 61; ou bien
2) par formylation régiosélective de benzènes substitués, suivie de l'oxydation du benzaldéhyde substitué par KMnO₄, selon le SCHEMA 4 : 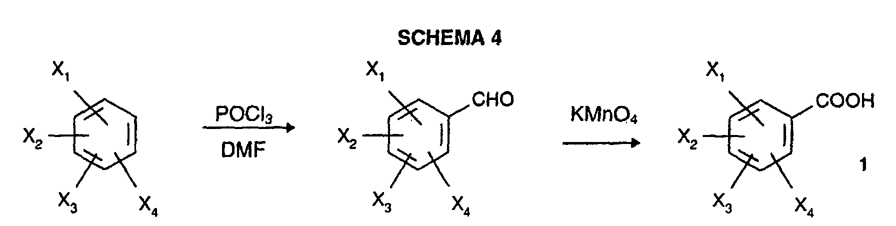 selon la méthode décrite par S.B. Matin *et al*., J. Med. Chem., 1974, 17; 877; ou bien
3) par oxydation haloforme selon R. Levine *et al*., J. Am. Chem. Soc., 1959, *72*, 1642 de méthylcétones aromatiques, obtenues par acylation de Friedel-Crafts, de benzènes substitués (C.A. Bartram *et al*., J. Chem. Soc., 1963, 4691) ou par réarrangement de Fries d'acyloxybenzènes substitués selon S.E. Cremer *et al*., J. Org. Chem., 1961, *26*, 3653, selon les SCHEMAS 5 et 6 ci-après :

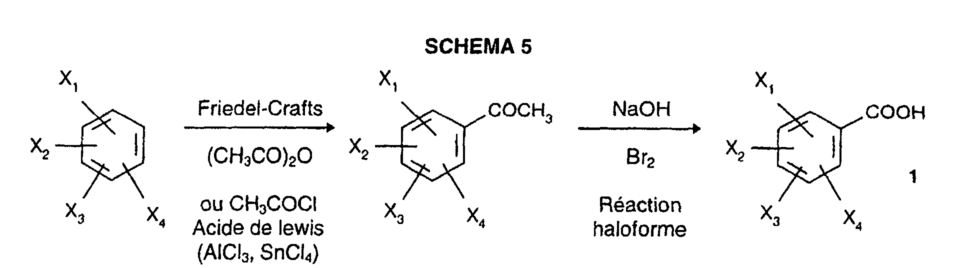

Les acides substitués en position 2 par un méthoxy peuvent être préparés à partir d'un dérivé phénol substitué par réaction de l'anhydride acétique dans la pyridine, suivie d'une réaction de Fries en présence de chlorure d'aluminium pour conduire à l'hydroxy acétophénone, sur laquelle on fait réagir l'iodure de méthyle en milieu alcalin pour obtenir finalement, par une réaction haloforme, l'acide **1'** attendu selon le SCHEMA 6 ci-après :

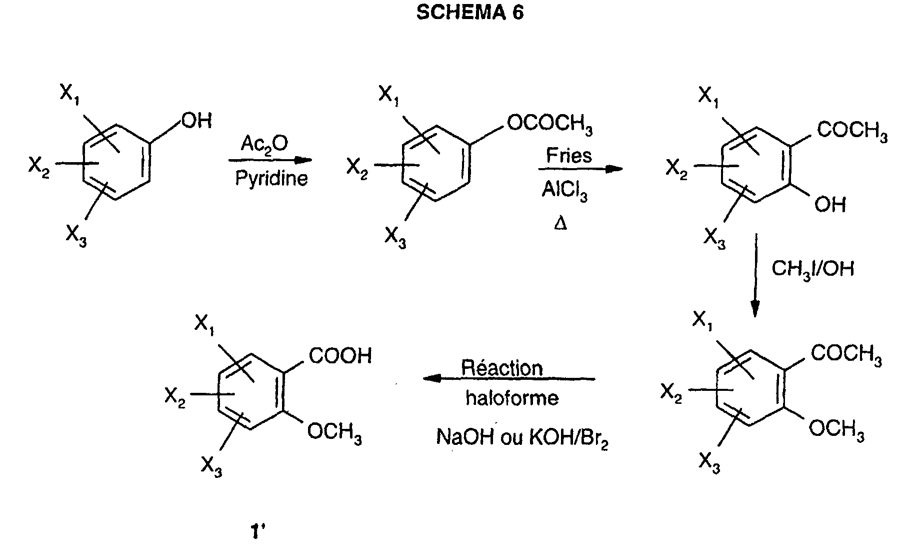

La benzamidoguanidine **2** est obtenue par acylation d'hydrogénocarbonate d'aminoguanidine par le chlorure de l'acide benzoïque obtenu à partir de l'acide benzoïque **1** par des procédés classiques (SOCl₂, chlorure d'oxalyle dans un solvant inerte), selon une adaptation du procédé décrit par E. Hoggarth, J. Chem. Soc., 1950, 612. Elle peut également être obtenue selon la variante décrite dans cette même publication selon le SCHEMA 7 suivant :

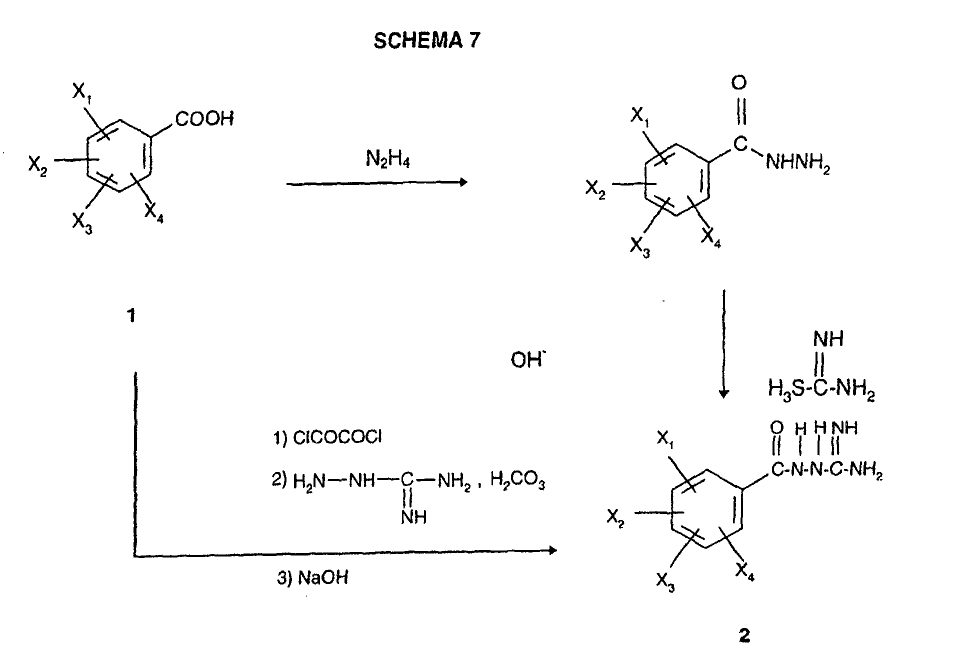

La cyclisation thermique de la benzamidoguanidine **2** dans un solvant à haut point d'ébullition, tel que le diphényléther, conduit à l'aryl-5-amino-3-triazole **3** selon une adaptation du procédé décrit par E. Hoggarth, J. Chem. Soc., 1950, 612.

La protection de la fonction amino primaire du triazole **3** sous forme de diphénylimine conduit au triazole N-protégé **4**, selon une adaptation d'un procédé décrit par M.J. O'Donnell *et al*., J. Org. Chem., 1982, *47*, 2663.

On peut également obtenir le composé **4** selon une variante qui consiste à traiter le triazole **3**, préalablement transformé en chlorhydrate **3'** par la diphénylimine, selon le SCHEMA 8 ci-après :

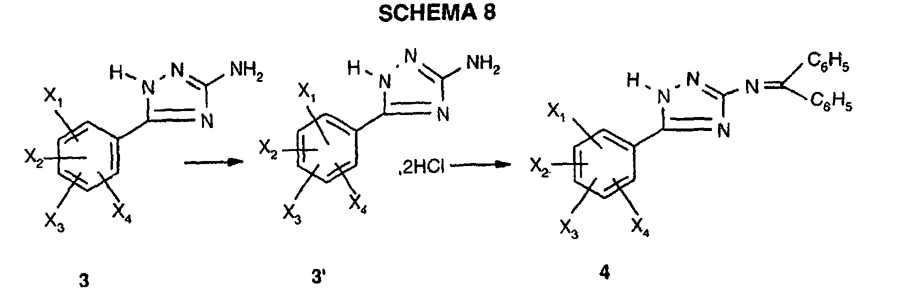

La N-alkylation du diphényliminotriazole 4, par un halogènure d'alkyle R₁X, sous des conditions de transfert de phase (base forte en solution aqueuse concentrée, en présence d'un cosolvant organique non miscible et d'un catalyseur ammonium quaternaire) conduit très majoritairement au triazole 5, accompagné du triazole très minoritaire 6. Les bases fortes utilisées peuvent être des solutions aqueuses de NaOH ou KOH à des concentrations de 6 M à 12 M. Le cosolvant peut être le toluène, le benzène et l'ammonium quaternaire choisi parmi tous les sels d'ammonium quaternaires et, plus particulièrement, le TBAB (bromure de tétrabutylammonium).
a) La N-alkylation du diphényliminotriazole 4 peut s'opérer dans un milieu non aqueux (diméthylformamide, tétrahydrofurane par exemple) en présence d'une base forte telle que K₂CO₃ ou NaH.
b) On peut également utiliser une variante telle que celle décrite par E. Akerblom, Acta Chem. Scand., 1965, *19*, 1142, laquelle met en oeuvre un agent alkylant dans un alcool tel que l'éthanol en présence d'une base forte solide telle que KOH ou NaOH.

Le triazole 5 est très facilement séparé de son isomère 6 par chromatographie sur colonne de silice ou flash-chromatographie, selon la nature du groupement R₁.

Le clivage du produit 5, obtenu après séparation de son isomère minoritaire, s'opère en milieu aqueux acide tel que HCI 1N, suivant une adaptation du procédé décrit par J. Yaozhong *et al*., Tetrahedron, 1988, *44*, 5343 ou M.J. O'Donnell *et al.,* J. Org. Chem., 1982, *47*, 2663. Il permet d'obtenir les amino-3-triazole N-alkylés en position 1 de formule 7.

Les indoles carboxyliques de formule 8 ont été préparés selon des procédés décrits dans le brevet n°EP 611766 selon le SCHEMA 9 ci-après :

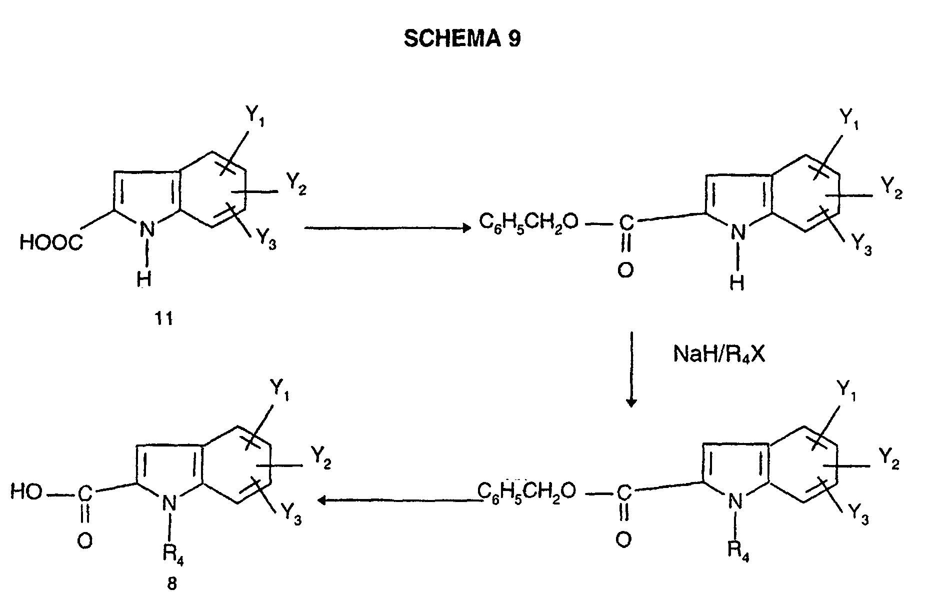

Les indoles carboxyliques 8' dans lesquelles R'₄ = ―(CH₂)ₙ―C≡N
ont été préparés selon un procédé analogue présenté dans le SCHÉMA 9 bis ci-après :

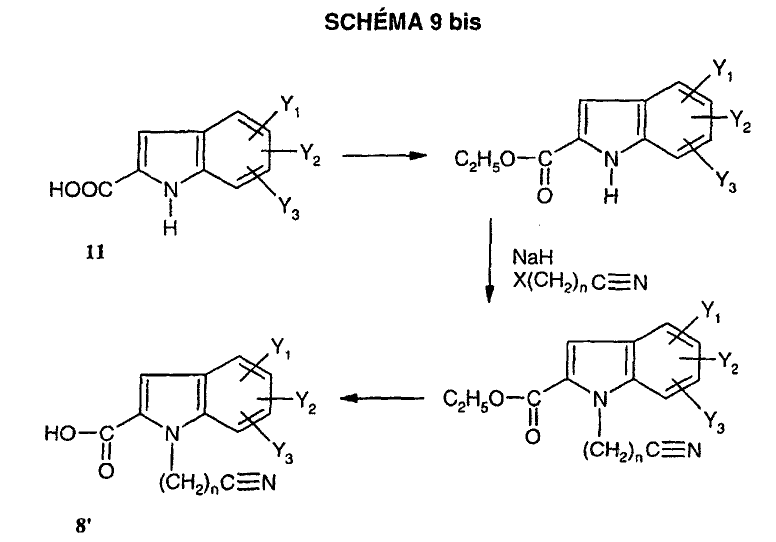

Les indoles 11 sont commerciaux ou préparés selon une adaptation des procédés décrits dans la littérature, par exemple selon L. Henn *et al*., J. Chem. Soc. Perkin Trans. I, 1984, 2189 selon le SCHEMA 10 ci-après : 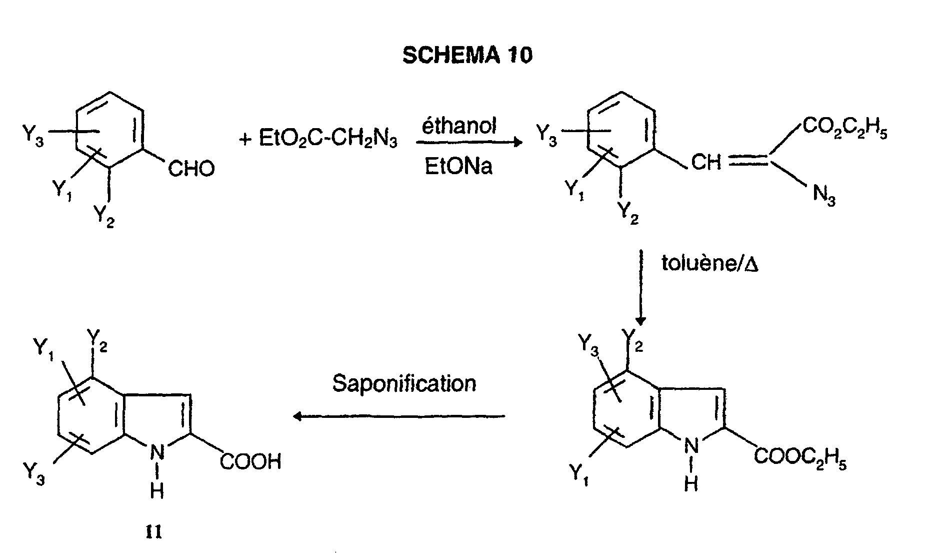 ou encore, par exemple, selon la synthèse de Fischer (V. Prelog *et al*., Helv. Chim. Acta., 1948, *31*, 1178) selon le SCHEMA 11 ci-après : 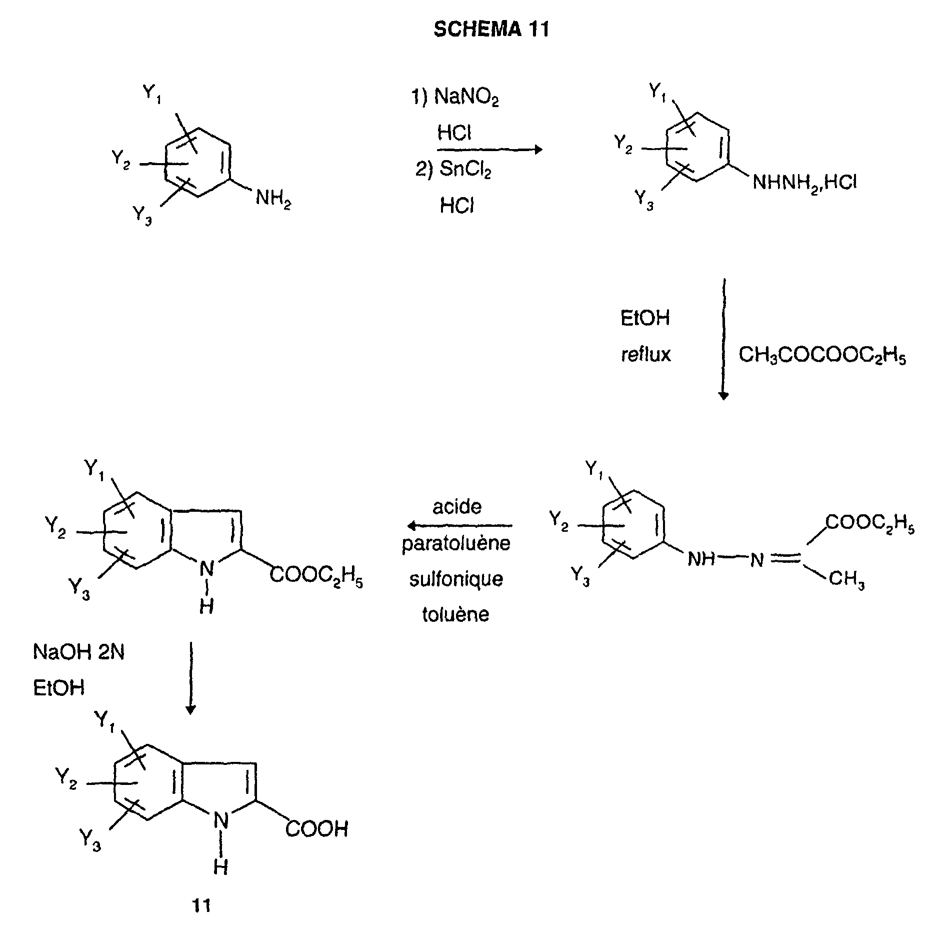 ou encore selon la synthèse de Japp-Klingemann (H. Ishii *et al*., J. Chem. Soc. Perkin. Trans. 1, 1989, 2407) selon le SCHEMA 12 ci-après :

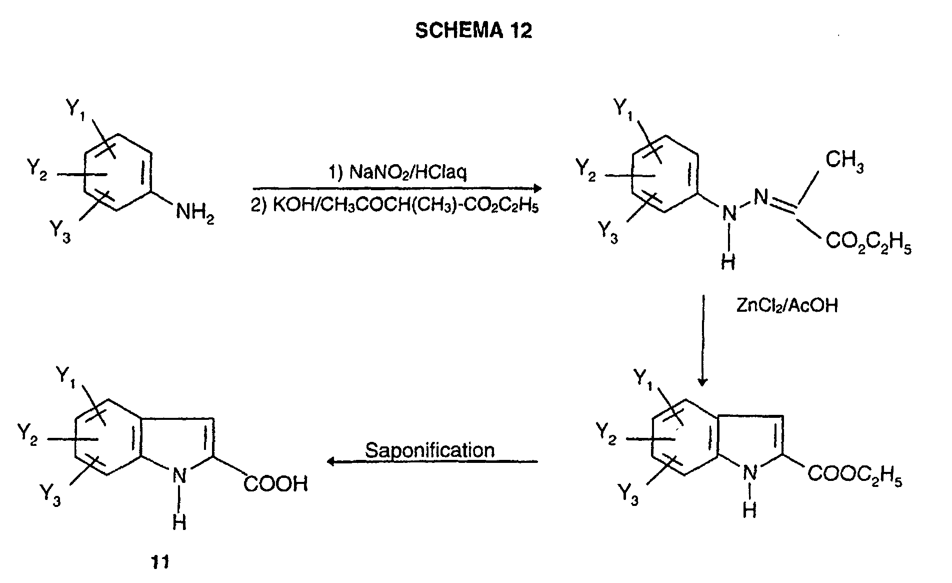

Les composés de formule **(I)** ci-dessus comprennent également ceux dans lesquels un ou plusieurs atomes d'hydrogène, de carbone ou d'halogène, notamment de chlore ou de fluor ont été remplacés par leur isotope radioactif par exemple le tritium ou le carbone-14. De tels composés marqués sont utiles dans des travaux de recherche, de métabolisme ou de pharmacocinétique, dans des essais biochimiques en tant que ligand de récepteurs.

Les composés de formule (I) ont fait l'objet d'études de liaison *in vitro* aux récepteurs CCK-A et CCK-B, en utilisant la méthode décrite dans Europ. J. Pharmacol., 1993, *232*, 13-19.

L'activité agoniste des composés vis-à-vis des récepteurs CCK-A a été évaluée *in vitro* dans les cellules 3T3 exprimant le récepteur CCK-A humain, par la mesure de la mobilisation du calcium intracellulaire ([Ca⁺⁺]ᵢ), selon une technique dérivée de celle de Lignon MF *et al*., Eur. J. Pharmacol., 1993, *245*, 241-245. La concentration en calcium [Ca⁺⁺]ᵢ est évaluée avec le Fura-2 par la méthode de la double longueur d'onde d'excitation. Le rapport de la fluorescence émise à deux longueurs d'onde donne la concentration en [Ca⁺⁺]ᵢ après un étalonnage (Grynkiewiez G. *et al*., J. Biol. Chem., 1985, *260*, 3440-3450).

Les composés selon l'invention stimulent le [Ca⁺⁺]ᵢ partiellement, ou totalement comme la CCK, et se comportent donc comme des agonistes du récepteur CCK-A.

Une étude de l'effet agoniste des composés sur la vidange gastrique a été réalisée comme suit. Les souris femelles Swiss albino CD1 (20-25g) sont mises à jeun solide pendant 18 heures. Le jour de l'expérience, les produits (en suspension dans une solution de carboxyméthylcellulose à 1 % ou de méthylcellulose à 0,6 %) ou le véhicule correspondant sont administrés par voie intrapéritonéale, 30 minutes avant l'administration d'un repas de charbon (0,3 ml par souris d'une suspension dans l'eau de 10 % de poudre de charbon, 5 % de gomme arabique et 1 % de carboxyméthylcellulose) ou par voie orale une heure avant. Les souris sont sacrifiées 5 minutes plus tard par dislocation cervicale, et la vidange gastrique est définie comme la présence de charbon dans l'intestin au-delà du sphincter pylorique (Europ. J. Pharmacol., 1993, *232*, 13-19). Les composés de formule (I) bloquent la vidange gastrique partiellement ou complètement comme la CCK elle-même, et se comportent donc comme des agonistes des récepteurs CCK. Certains d'entre eux ont des DE₅₀ (dose efficace induisant 50 % de l'effet de la CCK) inférieures à 0,1 mg/kg par la voie intrapéritonéale.

Une étude de l'effet agoniste des composés sur la contraction de la vésicule biliaire a été réalisée comme suit. Les souris femelles Swiss albino CD1 (20-25 g) sont mises à jeun solide pendant 24 heures. Le jour de l'expérience, les produits (en suspension dans une solution de carboxyméthylcellulose à 1 % ou de méthylcellulose à 0,6 %) ou le véhicule correspodant sont administrés par voie orale. Les souris sont sacrifiées par dislocation cervicale une heure après l'administration des produits, et les vésicules biliaires sont prélevées et pesées. Les résultats sont exprimés en mg/kg de poids corporel (Europ. J. Pharmacol., 1993, *232*, 13-19). Les composés de formule (I) contractent la vésicule biliaire partiellement ou totalement comme la CCK elle-même, et se comportent donc comme des agonistes des récepteurs CCK. Certains d'entre eux ont des DE₅₀ (dose efficace induisant 50 % de la diminution du poids des vésicules observées avec la CCK) inférieures à 0,1 mg/kg par la voie orale.

Par conséquent, les composés de formule (I) sont utilisés, en tant qu'agonistes des récepteurs de la CCK de type A, pour la préparation de médicaments destinés à combattre les maladies dont le traitement nécessite une stimulation par agonisme total ou partiel des récepteurs CCK A de la cholécystokinine. Plus particulièrement, les composés de formule (I) sont utilisés pour la fabrication de médicaments destinés au traitement de certains troubles de la sphère gastrointestinale (prévention des calculs biliaires, syndrome du colon irritable) du comportement alimentaire, de l'obésité, et des pathologies associées telles que le diabète et l'hypertension. Les composés (I) induisent un état de satiété, et sont ainsi utilisés pour traiter les désordres alimentaires, pour réguler l'appétit et réduire la prise alimentaire, traiter la boulimie, l'obésité et provoquer une perte de poids. Les composés **(I)** sont également utiles dans des troubles du comportement émotionnel, sexuel et mnésique, des psychoses et, notamment, la schizophrénie, de la maladie de Parkinson, de la dyskinésie tardive. Ils peuvent aussi servir le traitement des troubles de l'appétence, c'est-à-dire pour réguler les désirs de consommation, en particulier la consommation de sucres, de carbohydrates, d'alcool ou de drogues et plus généralement d'ingrédients appétissants.

Les composés de formule **(I)** sont peu toxiques ; leur toxicité est compatible avec leur utilisation comme médicament pour le traitement des troubles et des maladies ci-dessus. Aucun signe de toxicité n'est observé avec ces composés aux doses pharmacologiquement actives, et leur toxicité est donc compatible avec leur utilisation médicale comme médicaments.

La présente invention a donc également pour objet des compositions pharmaceutiques contenant une dose efficace d'un composé selon l'invention ou d'un sel pharmaceutiquement acceptable de celui-ci, et des excipients convenables.

Lesdits excipients sont choisis selon la forme pharmaceutique et le mode d'administration souhaité.

Dans les compositions pharmaceutiques de la présente invention pour l'administration orale, sublinguale, sous-cutanée, intramusculaire, intra-veineuse, topique, intratrachéale, intranasale, transdermique, rectale ou intraocculaire, les principes actifs de formule (I) ci-dessus, ou leurs sels éventuels, peuvent être administrés sous formes unitaires d'administration, en mélange avec des supports pharmaceutiques classiques, aux animaux et aux êtres humains pour la prophylaxie ou le traitement des troubles ou des maladies ci-dessus. Les formes unitaires d'administration appropriées comprennent les formes par voie orale telles que les comprimés, les gélules, les poudres, les granules et les solutions ou suspensions orales, les formes d'administration sublinguale, buccale, intratrachéale, intranasale, les formes d'administration sous-cutanée, intramusculaire ou intraveineuse et les formes d'administration rectale. Pour l'application topique, on peut utiliser les composés selon l'invention dans des crèmes, pommades, lotions ou collyres.

Afin d'obtenir l'effet prophylactique ou thérapeutique désiré, la dose de principe actif peut varier entre 0,01 et 50 mg par kg de poids du corps et par jour.

Chaque dose unitaire peut contenir de 0,5 à 1000 mg, de préférence de 1 à 500 mg, d'ingrédients actifs en combinaison avec un support pharmaceutique. Cette dose unitaire peut être administrée 1 à 5 fois par jour de façon à administrer un dosage journalier de 0,5 à 5000 mg, de préférence de 1 à 2500 mg.

Lorsqu'on prépare une composition solide sous forme de comprimés, on mélange l'ingrédient actif principal avec un véhicule pharmaceutique, tel que la gélatine, l'amidon, le lactose, le stéarate de magnésium, le talc, la gomme arabique ou analogues. On peut enrober les comprimés de saccharose, d'un dérivé cellulosique, ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir ou pour l'administration sous forme de gouttes peut contenir l'ingrédient actif conjointement avec un édulcorant, acalorique de préférence, du méthylparaben et du propylparaben comme antiseptique, ainsi qu'un agent donnant du goût et un colorant approprié. Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols. Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs, ou bien avec des matrices telles qu'un polymère ou une cyclodextrine (patch, formes à libération prolongée).

Les compositions selon l'invention peuvent être utilisés dans le traitement ou la prévention de différentes affections où la CCK est d'un intérêt thérapeutique.

Les compositions de la présente invention peuvent contenir, à côté des produits de formule (I) ci-dessus ou de leurs sels pharmaceutiquement acceptables, d'autres principes actifs qui peuvent être utiles dans le traitement des troubles ou maladies indiquées ci-dessus.

De façon avantageuse les compositions de la présente invention contiennent un produit de formule (I.1), (1.2) ou (1.3) ci-dessus ou un de ses sels, solvates ou hydrates pharmaceutiquement acceptables.

### PREPARATION DES INTERMEDIAIRES DE SYNTHESE

### A. PREPARATION DES ACIDES 1 (VARIANTES)

### Acide 2,5-diméthoxy-4-méthylbenzoïque (Composé A.1)

### a) 2,5-Diméthoxy-4-méthylbenzaldéhyde

Après 40 minutes d'agitation à température ambiante d'un mélange de 8,5 ml de N-méthylformanilide (0,068 mole), et de 6,3 ml de trichlorure d'oxyde de phosphore (0,068 mole), on introduit 17,8 g de 2,5-diméthoxytoluène (0,117 mole). On chauffe le mélange réactionnel pendant 6 heures à 50°C puis, après avoir ramené la température à 20°C, on hydrolyse par 100 ml de solution aqueuse à 10 % d'acétate de sodium, on extrait deux fois à l'éther diéthylique et on concentre. Le résidu est repris par une solution aqueuse d'hydrogénosulfite de sodium, et extrait deux fois à l'éther diéthylique. La phase aqueuse est alcalinisée (pH = 12) pour fournir des cristaux blancs ; F = 83°C ; Rendement = 67 %.

### b) Acide 2,5-diméthoxy-4-méthylbenzoique

On chauffe à 75°C 23,86 g (0,132 mole) de 2,5-diméthoxy-4-méthylbenzaldéhyde en solution dans 500 ml d'eau, et on introduit 29,3 g (0,185 mole) de permanganate de potassium en solution dans 500 ml d'eau. On abandonne le mélange réactionnel pendant 2 heures à 75°C, puis on filtre à chaud l'insoluble après avoir ajusté le pH à 10 avec une solution à 10 % d'hydroxyde de sodium, et on le rince trois fois avec 80 ml d'eau chaude. Le filtrat est refroidi, le précipité formé est filtré, séché sous vide à 40°C pour fournir des cristaux blancs ; F = 120°C ; Rendement = 71 %.

### Acide 2,5-diméthoxy-4-chlorobenzoïque (Composé A.2)

### a) (2,5-Diméthoxy-4-chlorophényl)méthylcétone

A 2 litres de tétrachlorure de carbone, on additionne à température ambiante, 162,5 g de trichlorure d'aluminium (1,2 mole) puis à 0°C, goutte à goutte, 82 ml de chlorure d'acétyle (1,2 mole), puis 200 g de 1,4-diméthoxy-2-chlorobenzène (1,2 mole). On abandonne le mélange réactionnel pendant 3 heures et demie à 0°C, puis on l'hydrolyse par 700 ml d'eau. La phase organique est lavée avec une solution d'hydroxyde de sodium 2 M, séchée sur sulfate de sodium anhydre et concentrée. Le résidu semi-cristallin est repris à l'éther de pétrole, filtré, séché, pour fournir des cristaux blancs ; F = 96°C ; Rendement = 70 %.

### b) Acide 2,5-diméthoxy-4-chlorobenzoïque

A 800 ml d'eau, on additionne 278 g d'hydroxyde de potassium (4,96 moles), puis, à 5°C, 84 ml de brome (1,6 mole) goutte à goutte. On laisse le mélange réactionnel pendant une heure à température ambiante. La solution aqueuse d'hypobromite de sodium obtenue est additionnée à 107 g de (2,5-diméthoxy-4-chlorophényl)méthylcétone (0,494 mole) en solution dans 1,5 litre de 1,4-dioxane. Après une heure à 20°C, on chauffe le mélange réactionnel pendant une heure à reflux. Lorsque la réaction est terminée, on introduit 100 ml d'une solution aqueuse d'hydrogénosulfite de sodium, puis on évapore le solvant. Le résidu est acidifié par une solution d'acide chlorhydrique 6 N, puis on extrait deux fois à l'acétate d'éthyle. La phase organique est séchée sur sulfate de sodium anhydre, et concentrée. Le résidu est concrété dans l'éther diisopropylique, pour fournir des cristaux blancs ; F = 160°C ; Rendement = 91 %.

### Acide 2,6-diméthoxy-4-méthylphénylbenzoïque (Composé A.3)

On dissout 231,6 g (1,5 moles) de 3,5-diméthoxytoluène dans 1 litre d'éther diéthylique, puis on ajoute goutte à goutte sous azote à température ambiante, 1 litre d'une solution 1,6 N de butyllithium (1,6 mole) dans l'hexane. On abandonne le mélange réactionnel pendant 18 heures à température ambiante puis, après refroidissement à -30°C, on ajoute 1 litre d'éther diéthylique et on fait barboter du gaz carbonique pendant une heure, tout en maintenant la température à -30°C. On reprend le mélange réactionnel par 6 litres d'une solution d'hydroxyde de sodium 2 M, on décante la phase aqueuse, et on l'acidifie avec une solution d'acide chlorhydrique 6 N. On filtre le précipité formé, le rince à l'eau et sèche sous vide à 40°C pour obtenir des cristaux blancs ; F = 187°C ; Rendement = 88 %.

### B. PREPARATION DES INDOLES SUBSTITUES ET LEURS VARIANTES

### Préparation du 5-méthyl-1-H-2 indole carboxylate d'éthyle (Composé B.1)

### 1ère méthode : (méthode de Japp-Klingemann):

A -5°C on additionne 7,2 g (0,104 mole) de nitrite de sodium en solution dans 40 ml d'eau, sur un mélange de 10,7 g (0,1 mole) de 4-méthylaniline, 74 ml d'acide chlorhydrique 12 N et de 140 ml d'eau. On agite le mélange réactionnel pendant 15 minutes à -5°C, et on neutralise par addition de 8,1 g d'acétate de sodium. Dans un tricol, on introduit 12,33 g (0,085 mole) d'α-méthylacétoacétate d'éthyle, 80 ml d'éthanol, puis à 0°C 4,8 g (0,085 mole) d'hydroxyde de potassium en solution dans 20 ml d'eau et 100 g de glace. A ce mélange réactionnel, on ajoute goutte à goutte à 0°C la solution de diazonium préparée précédemment, et on abandonne pendant 18 heures à 0°C. La phase aqueuse est extraite 4 fois par 50 ml d'acétate d'éthyle, les phases organiques sont rassemblées et séchées sur sulfate de sodium anhydre. Le résidu est repris par 100 ml de toluène et 16,3 g (0,085 mole) d'acide paratoluènesulfonique monohydraté. On chauffe alors lentement à 110°C et maintient cette température pendant 5 heures. Après refroidissement, puis addition d'une solution saturée de carbonate de sodium, l'insoluble est écarté par filtration, la phase organique est décantée, séchée sur sulfate de sodium anhydre et concentrée. Le résidu est chromatographié sur colonne de gel de silice, éluant : dichlorométhane/cyclohexane 30/70 (v/v) pour fournir des cristaux beiges ; F = 94°C ; Rendement = 25%.

### Préparation du 4-méthyl-1H-2-indolecarboxylate d'éthyle (Composé B2)

### 2ème méthode :

### - Etape 1 : préparation de l'azide

9,3 g (0,405 mole) de sodium sont additionnés, par portions, à 200 ml d'éthanol. A cette solution d'éthylate dans l'éthanol, on introduit goutte à goutte à -20°C, 16,2 g (0,135 mole) d'ortho-tolualdéhyde en solution, dans 52,2 g (0,405 mole) d'azidoacétate d'éthyle. Après 2 heures à -10°C, on verse le mélange réactionnel sur 400 ml d'eau, et filtre le précipité formé. On sèche 18 heures à 40°C sous vide pour obtenir des cristaux blancs ; F = 55°C ; Rendement = 78 %.

### - Etape 2 : cyclisation de l'azide

19,5 g (0,0844 mole) de l'azide préparé selon l'étape 1 sont additionnés, par portions, à 100 ml de xylène chauffés à 140°C. L'addition terminée, on abandonne le mélange réactionnel pendant 1 heure à 140°C. On concentre le xylène et le résidu est repris par de l'éther isopropylique, filtré, séché 18 heures sous vide à 40°C, pour fournir des cristaux blancs ; F = 141°C ; Rendement = 62 %.

### Préparation de l'acide 5-éthyl-1H-2-indolcarboxylique (selon la méthode de Fischer) - (Composé B.3)

### 3ème méthode :

### - Etape 1 : Chlorhydrate de 4-éthylphénylhydrazine

A 24,2 g (0,2 mole) de 4-éthylaniline, on additionne 150 ml d'eau et 160 ml d'acide chlorhydrique 12 N. On refroidit le mélange à 0°C, puis on introduit goutte à goutte 14 g (0,2 mole) de nitrite de sodium en solution dans 140 ml d'eau. Après 1 heure à 0°C, on ajoute au mélange réactionnel à -10°C, 112 g (0,496 mole) de dihydrate de chlorure stanneux en solution dans 90 ml d'acide chlorhydrique 12 N. Après 1 heure 30 à -10°C, on filtre le mélange réactionnel pour obtenir un solide brun ; F = 198°C ; Rendement = 95 %.

### - Etape 2 : 2-[2-(4-Ethylphényl)hydrazono]propanoate d'éthyle

A 34,5 g (0,2 mole) de chlorhydrate de 4-éthylphénylhydrazine préparé précédemment en suspension dans 500 ml d'éthanol, on additionne 23 ml (0,2 mole) de pyruvate d'éthyle, et on chauffe le mélange réactionnel durant 3 heures 30 à reflux. On ramène ensuite la température à 20°C, et évapore l'éthanol. Le résidu solide est lavé au pentane, séché à 40°C sous vide pour obtenir un liquide incolore ; Rendement = 94 %.

### - Etape 3 : 5-Ethyl-1H-2-indolecarboxylate d'éthyle

A 44 g (0,188 mole) d'hydrazone préparée précédemment en suspension dans 300 ml de toluène, on additionne durant 7 heures, par portions, à reflux, 19 g (0,1 mole) d'acide paratoluènesulfonique monohydraté. On ramène la température à 20°C, on sépare un insoluble par filtration et le rince au toluène. Le filtrat est lavé avec une solution aqueuse saturée en carbonate de potassium; on décante, sèche sur sulfate de sodium anhydre et concentre. Le résidu est purifié par chromatographie sur colonne de gel de silice, éluant : dichlorométhane/cyclohexane 5/5 (v/v) pour obtenir des cristaux beiges ; F = 94°C ; Rendement = 51 %.

### - Etape 4 : Acide 5-éthyl-1H-2-indolecarboxylique

A 150 ml de 1,4-dioxanne, on additionne 15,8 g (0,073 mole) de 5-éthyl-2-indolecarboxylate d'éthyle préparé selon l'étape 3, puis 45 ml d'une solution 2 M d'hydroxyde de sodium (0,09 mole). On abandonne le mélange réactionnel pendant 48 heures à température ambiante. Après évaporation du 1,4-dioxanne, le résidu est repris par une solution d'acide chlorhydrique 6 N, le précipité formé est filtré, séché sous vide à 60°C pour fournir l'acide 5-éthyl-1H-2-indolcarboxylique sous forme de cristaux blancs ; F = 184 °C ; Rendement = 92 %.

### PREPARATION DES ACIDES 1H-2-INDOLECARBOXYLIQUES N-ALKYLES

### Acide 5-éthyl-1-(méthoxycarbonylméthyl)-1H-2-indolecarboxylique - (Composé B.4)

### Etape 1 : 5-Ethyl-1H-2-indolecarboxylate de benzyle

A 70 ml de diméthylformamide, on additionne successivement 12,7 g (0,067 mole) d'acide 5-éthyl-1*H*-2-indolecarboxylique, 10 ml de 1,8-diazabicyclo[5.4.0]undéc-7-ène (0,067 mole). On laisse le mélange réactionnel pendant 40 minutes à 0°C, puis on introduit goutte à goutte 10,6 ml de bromure de benzyle (0,089 mole). Après 18 heures de réaction à température ambiante, le mélange réactionnel est versé sur 300 ml d'eau, le précipité formé est filtré, rincé à l'eau, puis séché pendant 18 heures à 50°C sous vide pour fournir des cristaux jaunes ; F = 99°C ; Rendement = 90 %.

### Etape 2 : 5-Ethyl-1-(méthoxycarbonylméthyl)-1H-2-indolecarboxylate de benzyle

A 1,5 g (0,031 mole) d'hydrure de sodium à 50 % en suspension dans l'huile, on additionne 75 ml de diméthylformamide puis, par portions, 7,9 g (0,0283 mole) de 5-éthyl-1 *H*-2-indolecarboxylate de benzyle préparé selon l'étape 1. Après 40 minutes à 0°C, on introduit goutte à goutte 3,5 ml (0,0315 mole) de bromoacétate de méthyle, et abandonne le mélange réactionnel pendant 2 heures à 20°C. On additionne 300 ml d'acétate d'éthyle, et lave avec 2x300 ml d'eau puis on décante, sèche la phase organique sur sulfate de sodium anhydre et concentre. On obtient 9,5 g d'huile incolore ; Rendement = 95 %.

### Etape 3 : Acide 5-éthyl-1-(méthoxycarbonylméthyl)-1H-2-indolecarboxylique

A 9,5 g (0,0269 mole) de 5-éthyl-1-(méthoxycarbonylméthyl)-1 H-2-indolecarboxylate de benzyle préparé selon l'étape 2 en solution dans 150 ml d'éthanol, on additionne 2,5 g de Pd/C à 10 %, puis 40 ml de cyclohexène (0,395 mole). On chauffe le mélange réactionnel pendant 2 heures à 70°C, puis ramène la température à 20°C. Le mélange réactionnel est filtré sur talc et le filtrat est évaporé à sec. Le résidu est séché pendant 18 heures à 40°C sous vide, pour fournir des cristaux beiges ; F = 181°C ; Rendement = 90 %.

En procédant selon les PREPARATIONS ci-dessus à partir des intermédiaires de synthèse appropriés, on synthétise les *Composés B5 à B70* décrits dans le TABLEAU I ci-après.

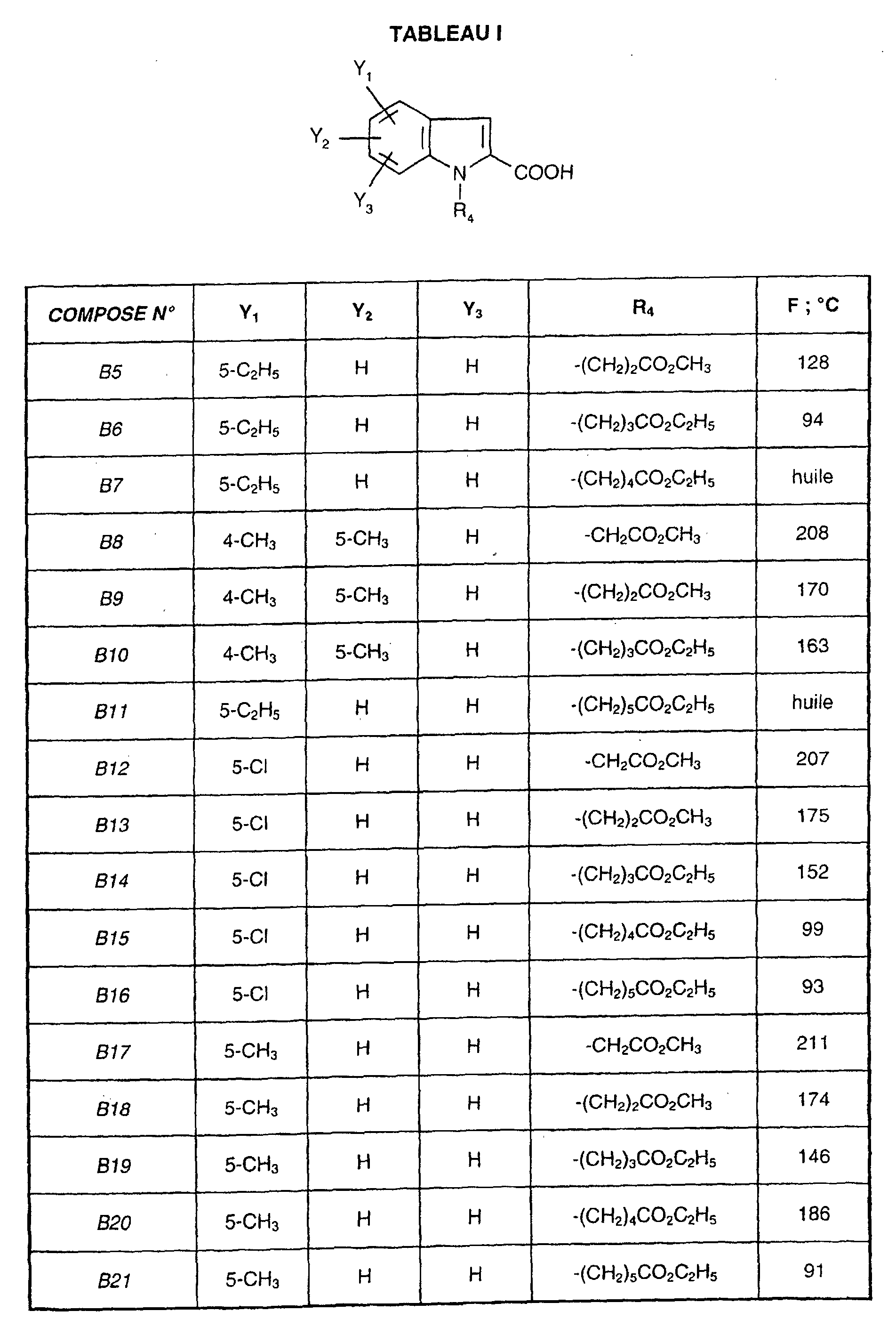

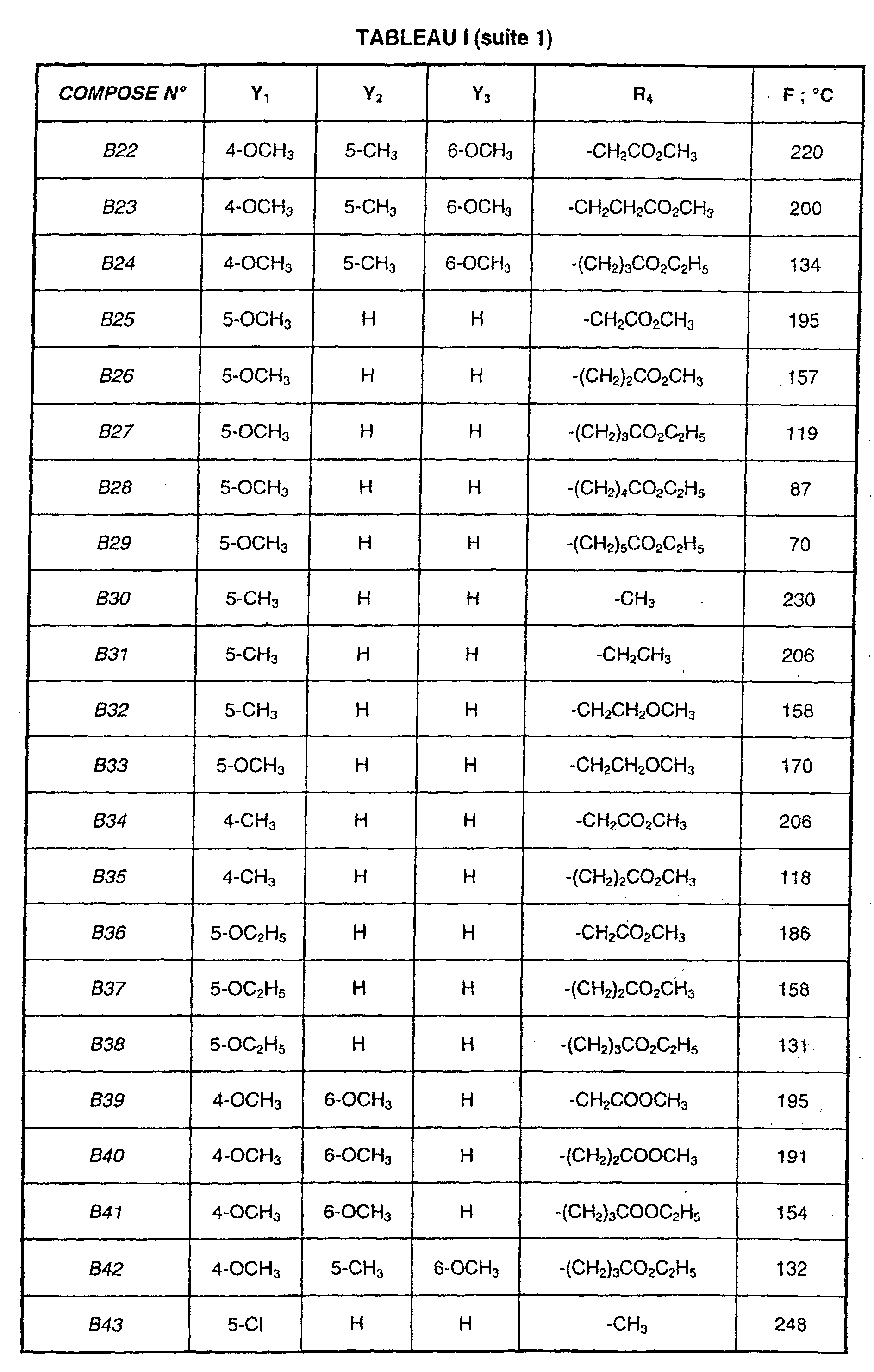

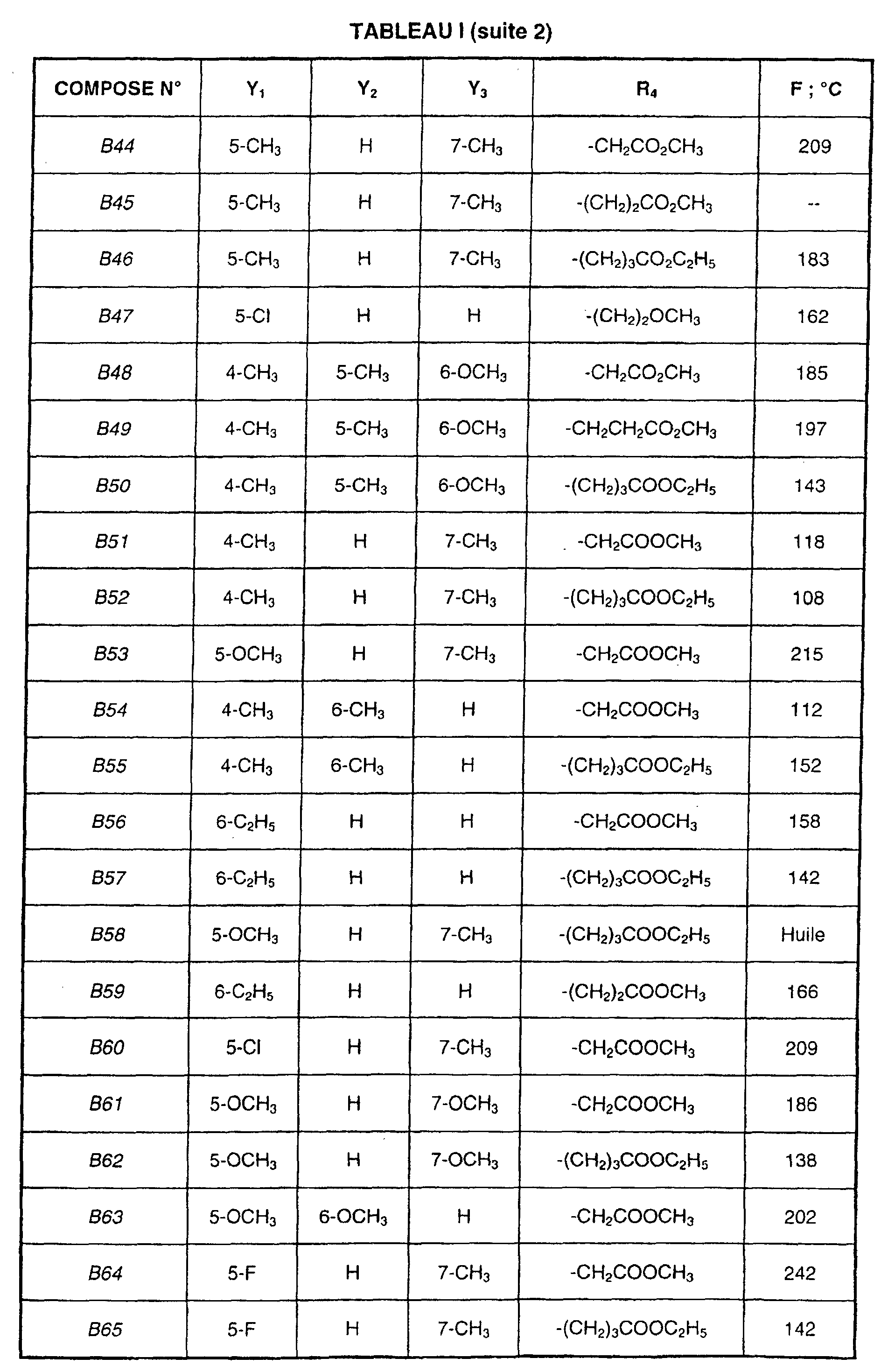

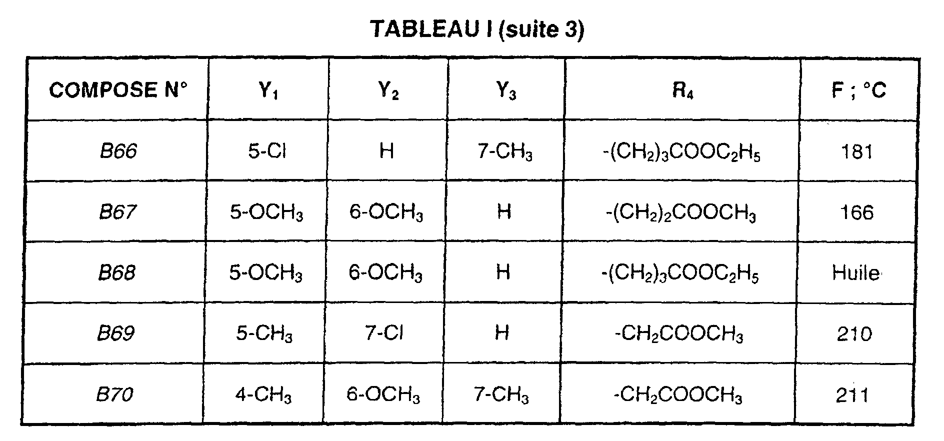

### Acide 4,5-diméthyl-1-(3-cyanopropyl)-1H-2-indolecarboxylique (Composé B71)

### Etape 1 : 4,5-Diméthyl-1-(3-cyanopropyl)-1H-2-indolecarboxylate d'éthyle

A 1,92 g (0,040 mole) d'hydrure de sodium à 50 % en suspension dans l'huile, on additionne 75 ml de diméthylformamide puis, par portions, 7,9 g (0,0363 mole) de 4,5-diméthyl-*1H*-2-indolecarboxylate d'éthyle. Après 40 minutes d'agitation à 0°C, on introduit goutte à goutte 4,0 ml (0,040 mole) de 4-bromobutyronitrile, et maintient le mélange réactionnel pendant 2 heures à 20°C. On additionne 300 ml d'acétate d'éthyle, et lave avec 2 fois 300 ml d'eau puis on décante, sèche la phase organique sur sulfate de sodium anhydre et concentre. On obtient 9,8 g d'huile incolore ;
Rendement = 95 %.

### Etape 2 : Acide 4,5-Diméthyl-1-(3-cyanopropyl)-1H-2-indolecarboxylique

A 150 ml de 1,4-dioxane, on additionne 9,8 g (0,0345 mole) de 4,5 -diméthyl-1-(3-cyanopropyl)-*1H*-2-indolecarboxylate d'éthyle, puis 25 ml d'une solution 2M d'hydroxyde de sodium (0,05 mole). On maintient le mélange réactionnel pendant 48 heures à température ambiante. Après évaporation du 1,4-dioxane, le résidu est repris par une solution d'acide chlorhydrique 6M, le précipité formé est filtré, séché sous pression réduite à 60°C pour fournir l'acide 4,5-diméthyl-1-(3-cyanopropyl)-*1H*-2-indolecarboxylique sous forme de cristaux blancs ; F = 175°C,
Rendement = 92 %.

De la même manière, on prépare les composés B72 à B75 présentés dans le TABLEAU I bis ci-après.

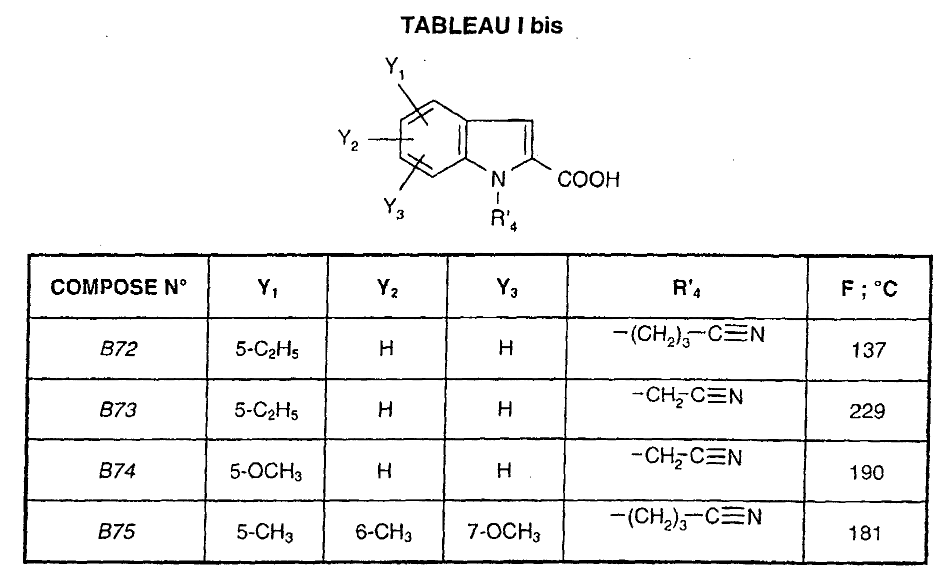

### C. PREPARATION DES DERIVES BENZAMIDOGUANIDINE

### Préparation de la 2,6-diméthoxy-4-méthylbenzamidoguanidine (Composé C.1)

A 353 g (1,8 mole) d'acide 2,6-diméthoxy-4-méthylbenzoïque en suspension dans 1,5 litre de toluène, on additionne 1 ml de diméthylformamide puis, goutte à goutte, 190 ml de chlorure d'oxalyle (2,16 moles). Le mélange réactionnel est abandonné pendant deux heures à température ambiante, puis évaporé à sec. Le résidu cristallin est ajouté par portions à une suspension de 293,8 g d'hydrogénocarbonate d'aminoguanidine (2,16 moles) dans 2,5 litres de pyridine à ± 5°C, et abandonné pendant 18 heures à 20°C. Le mélange réactionnel est évaporé à sec, puis le résidu est repris par 1 litre d'une solution d'hydroxyde de sodium 2 M. On filtre le précipité et on le rince avec un minimum d'eau, puis on sèche sous vide à 60°C pour obtenir un résidu cristallin ; F = 222°C ; Rendement = 81 %.

### D. PREPARATION DES DERIVES 3-AMINOTRIAZOLES

### 3-Amino-5-(2,6-diméthoxy-4-méthylphényl)-1,2,4-triazole (Composé D.1)

A 230 g (0,91 mole) de 2,6-diméthoxy-4-méthylbenzamidoguanidine, on additionne 2 litres de diphényléther, puis on chauffe le mélange réactionnel pendant 5 minutes à 220°C. On ramène la température à 80°C, puis on filtre le précipité, le rince à l'éther diisopropylique, et sèche sous vide à 60°C pour obtenir des cristaux ;
F = 286°C ; Rendement = 93 %.

En procédant selon cette PREPARATION, et en utilisant les produits de départ appropriés, on synthétise de la même façon les *Composés D2 à D11* décrits dans le TABLEAU II ci-après.

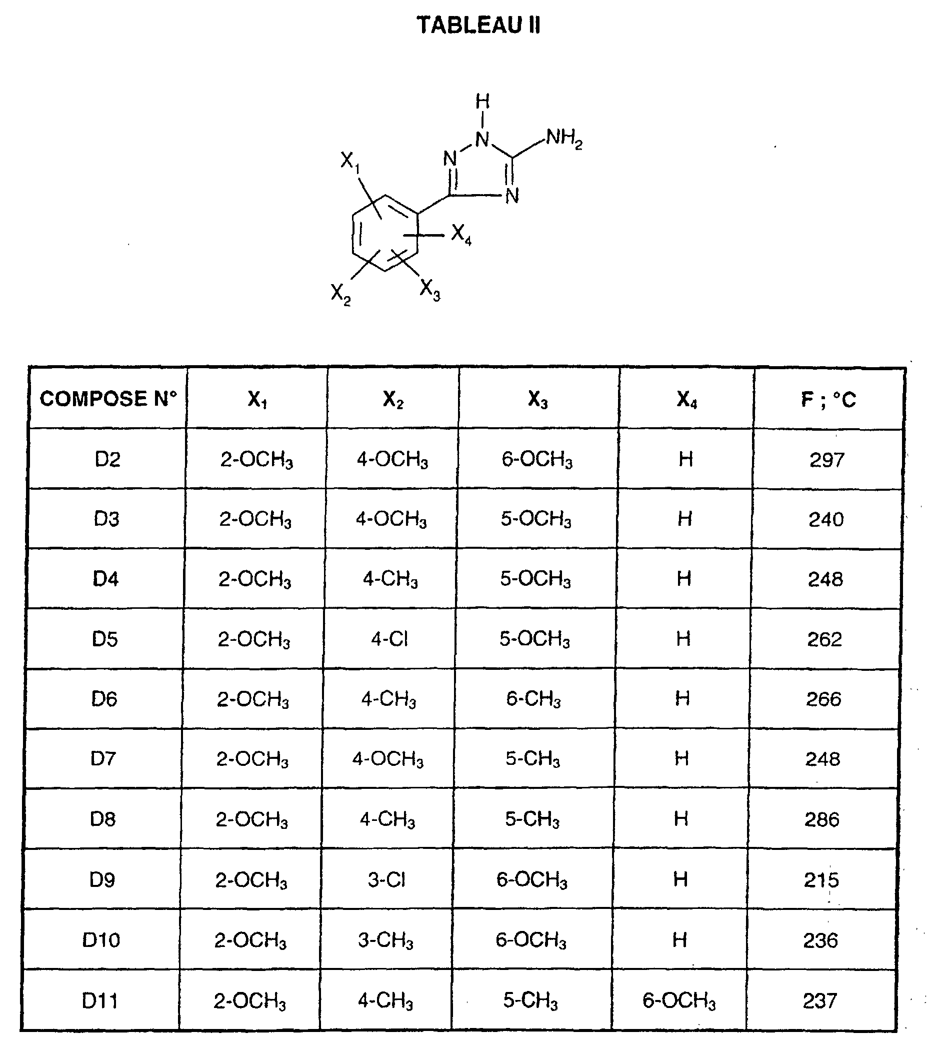

### E. PREPARATION DES DERIVES DIPHENYLIMINO

### Préparation de la N-[3-(2,6-diméthoxy-4-méthylphényl)-1H-1,2,4-triazol-5-yl]-N-diphénylméthylèneamine (Composé E.1)

On chauffe à 140°C, 105 g (0,45 mole) de 3-amino-5-(2,6-diméthoxy-4-méthylphényl)-1,2,4-triazole en suspension dans 200 ml de xylène et 150 g (0,9 mole) de benzophénoneimine, pendant 48 heures, sous courant d'argon. On ramène la température à 80°C, puis on verse le mélange réactionnel dans 4 litres d'éther isopropylique, on filtre le précipité formé, le rince à l'éther diisopropylique, et le sèche pendant 18 heures à 50°C ; F = 126°C ; Rendement = 90 %.

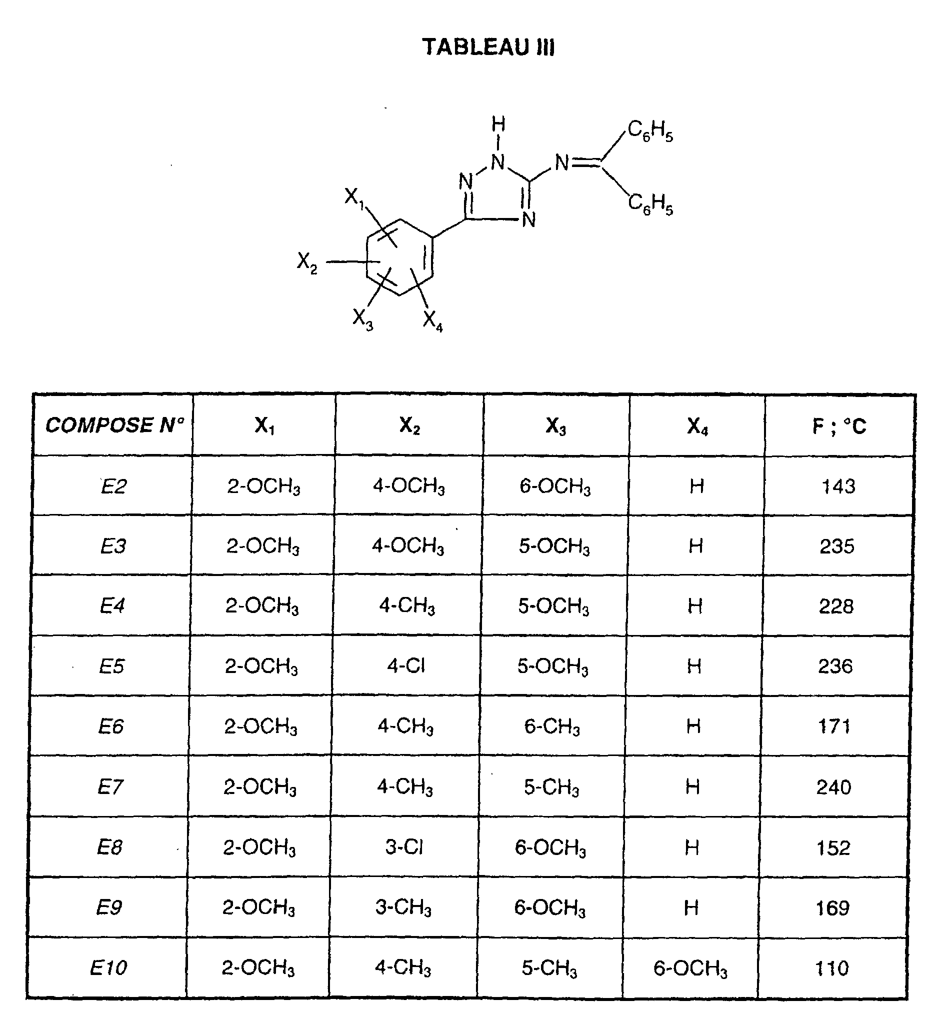

### F. PREPARATION DES 3-AMINO TRIAZOLE-1-SUBSTITUES

### Préparation de 1-(2-cyclohexyléthyl)-5-(2,6-diméthoxy-4-méthylphényl)-1H-1,2,4-triazol-3-amine (Composé F.1)

### a) N-alkylation du triazole

A 400 ml de toluène, on additionne successivement 300 ml d'une solution aqueuse 6 N d'hydroxyde de sodium, 24 g (0,06 mole) de N-[3-(2,6-diméthoxy-4-méthylphényl)-1*H*-1,2,4-triazol-5-yl]-N-diphénylméthylèneamine et 2,7 g de bromure de tétrabutylammonium. Au mélange réactionnel chauffé à 70°C, on ajoute goutte à goutte 17 g (0,09 mole) de 2-bromoéthylcyclohexane. On maintient la réaction pendant deux heures à 80°C. On décante la phase organique, sèche sur sulfate de sodium anhydre, et évapore à sec. Le résidu est chromatographié sur colonne de gel de silice, éluant : toluène/acétate d'éthyl 90/10 (v/v). On obtient 21,4 g d'huile incolore ; Rendement = 70 %.

### b) Hydrolyse de la fonction diphénylimine

A 10,3 g (0,02 mole) de N-[1-(2-cyclohexyléthyl)-5-(2,6-diméthoxy-4-méthylphényl)-1*H*-1,2,4-triazol-3-yl]-N-diphénylméthylèneamine en solution dans 200 ml de méthanol, on additionne 100 ml d'une solution 1 N d'acide chlorhydrique. On abandonne le mélange réactionnel pendant 18 heures à température ambiante, puis on évapore à sec. Le résidu huileux est concrétisé dans l'éther diiéthylique, le précipité obtenu est filtré et séché sous vide à 40°C ; F = 136°C (chlorhydrate) ; Rendement = 90 %.

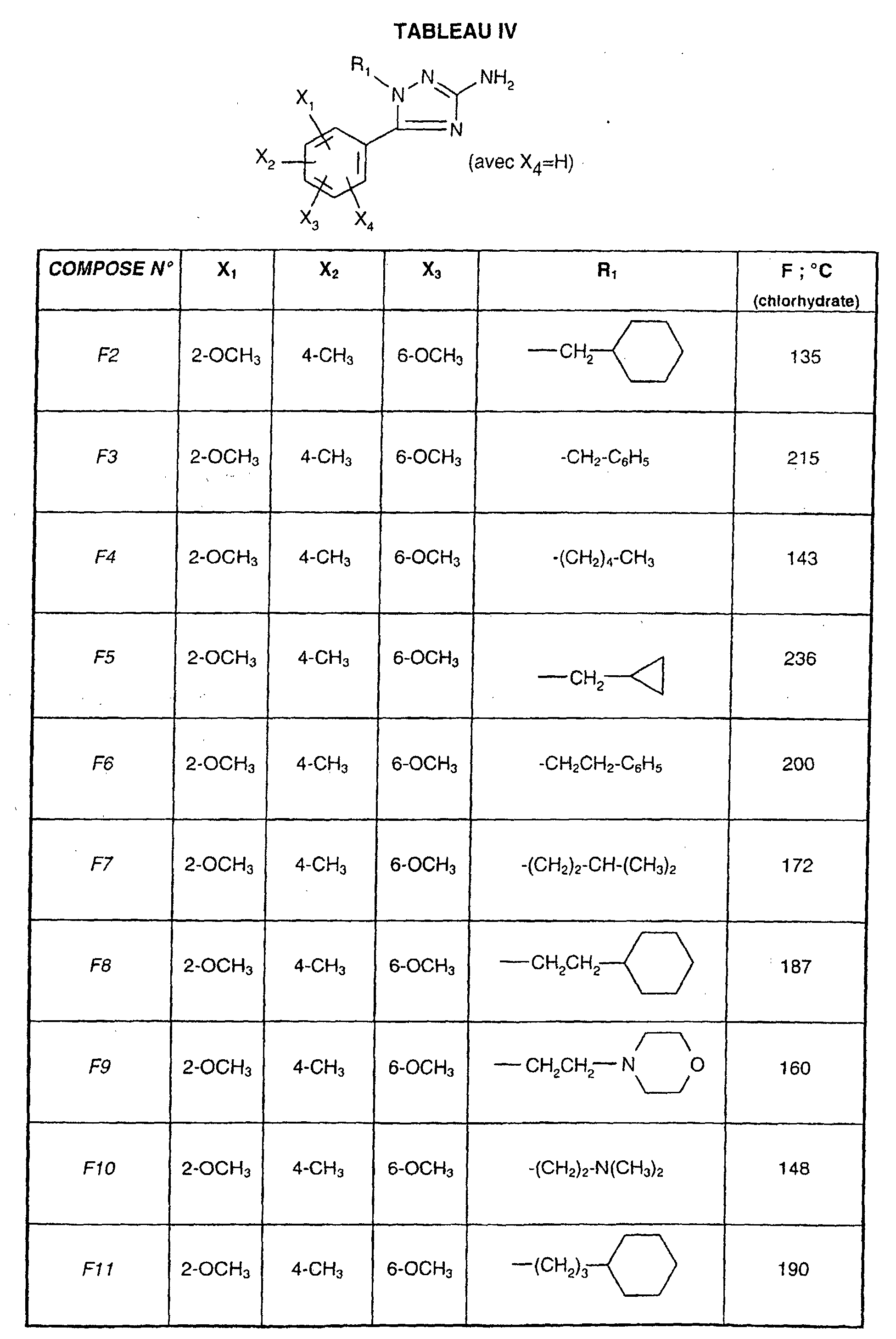

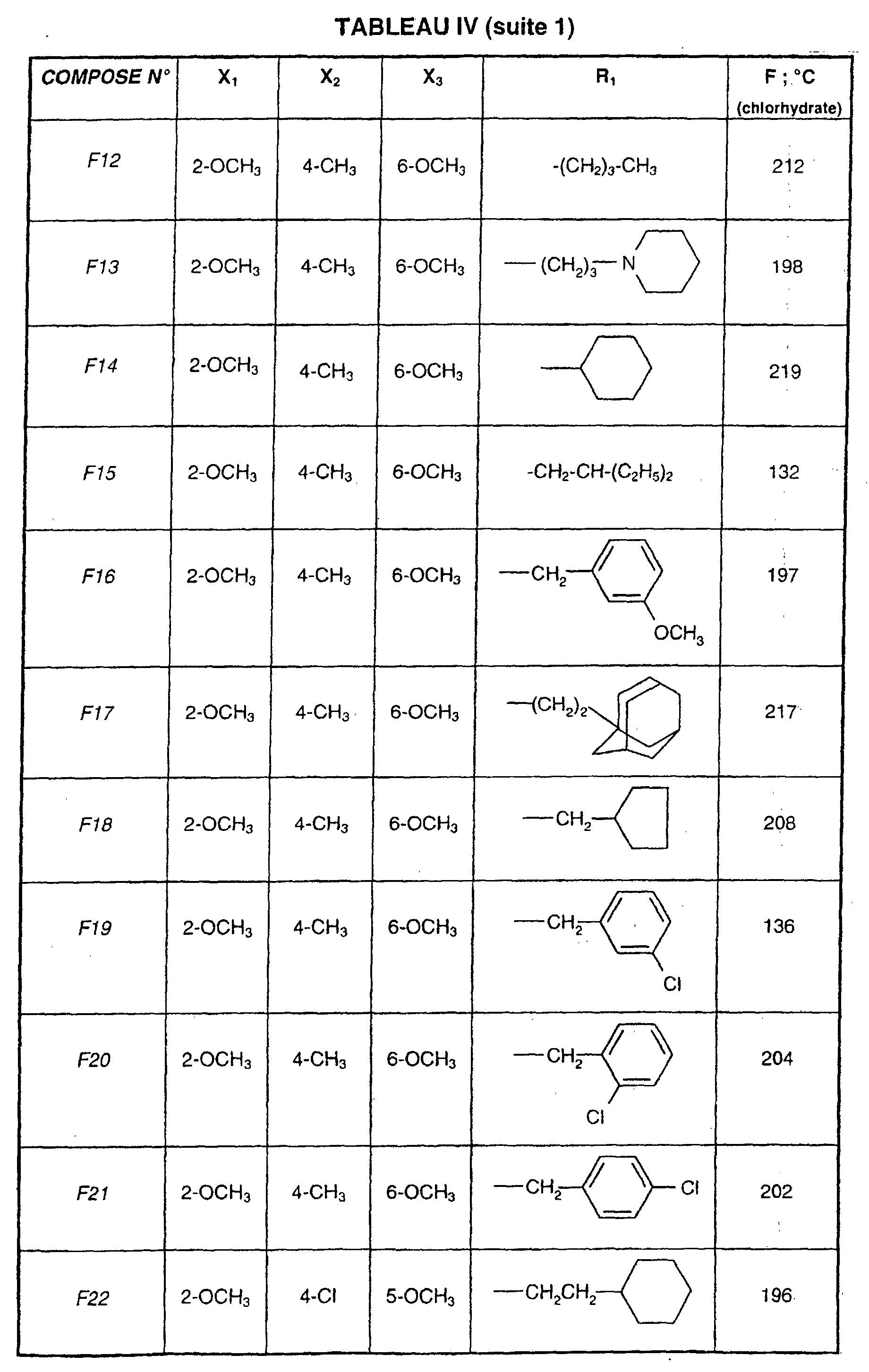

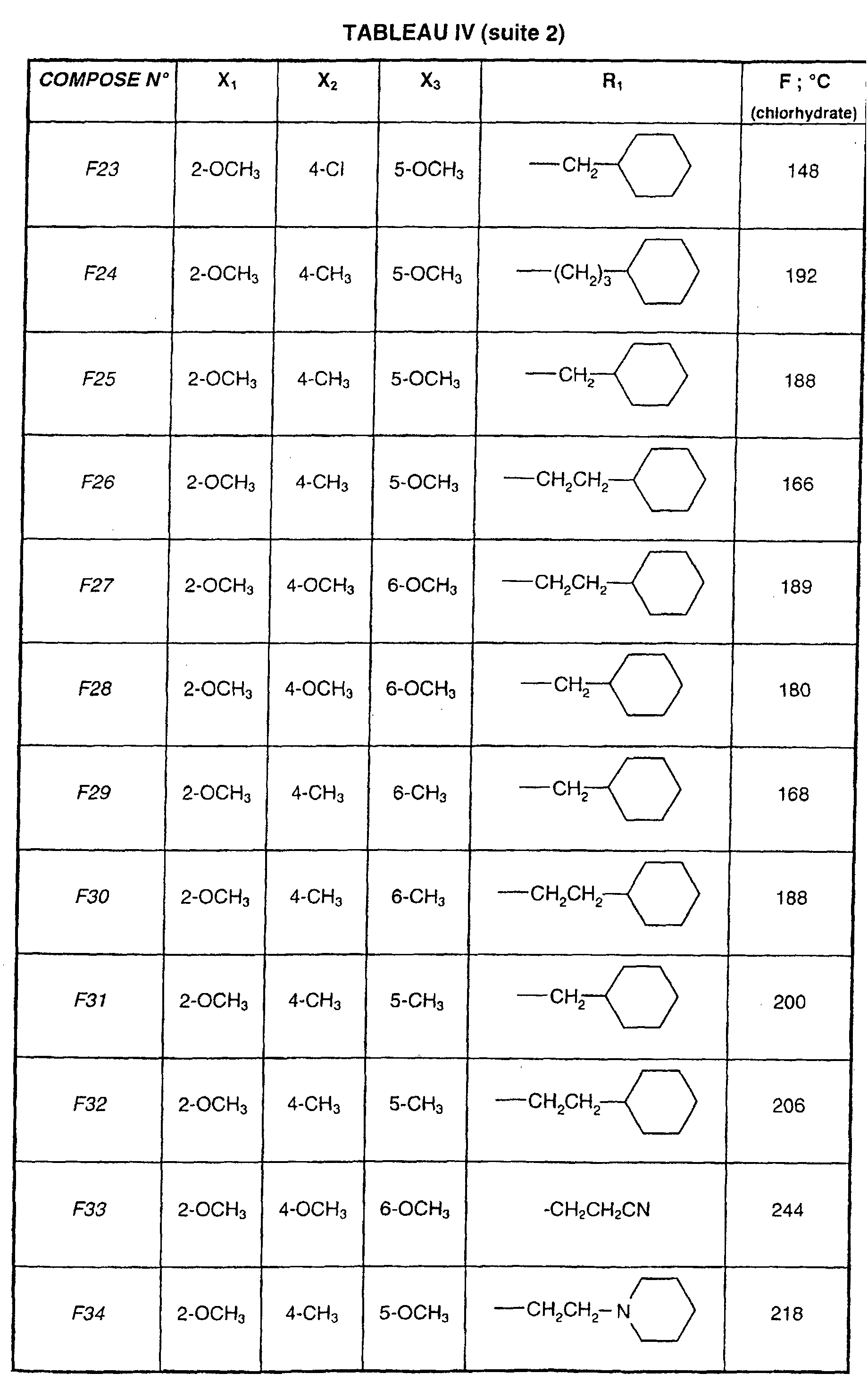

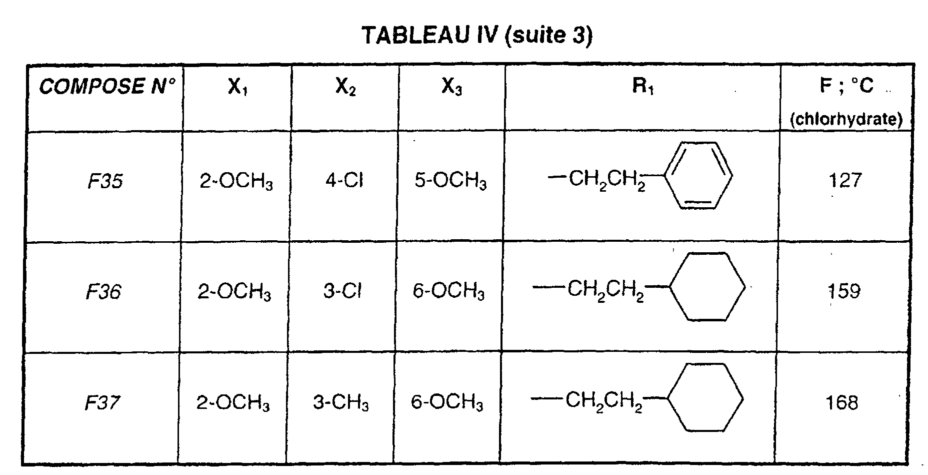

De la même manière, on prépare à partir du composé E10, le **1-(2-cyclohexyléthyl)-5-(2,6-diméthoxy-4,5-diméthylphényl)-1*H*-1,2,4-triazol-3-amine (Composé F38) ;** F = 180°C.

### G. PREPARATION DES DERIVES AMIDOTRIAZOLES AVEC INDOLES NON N-SUBSTITUES

**Synthèse du N-[1-(2-chlorobenzyl)-5-(2,6-diméthoxy-4-méthylphényl)-1*H*-1,2,4-triazol-3-yl]-5-chloro-1*H*-2-indolecarboxamide *(Composé G.1)*** A une solution de 1 ml de pyridine (0,013 mole) dans 30 ml de chlorure de méthylène, on additionne à 0°C 0,2 ml de chlorure de thionyle (0,0028 mole). Après 15 minutes à 0°C, on introduit 500 mg (0,0025 mole) d'acide 5-chloroindole carboxylique, et on abandonne le mélange réactionnel pendant 30 minutes à 0°C. Au chlorure d'acyle formé, on additionne 0,91 g (0,0028 mole) de chlorhydrate de 1-[(2-chlorophényl)méthyl]-5-(2,6-diméthoxy-4-méthylphényl)-1 H-1,2,4-triazol-3-amine, et laisse pendant 18 heures à 20°C.

Le mélange réactionnel est lavé avec une solution 1M d'hydroxyde de sodium. La phase organique est séchée sur sulfate de sodium anhydre et évaporée à sec. Le résidu est chromatographié sur gel de silice, éluant : dichlorométhane/méthanol 95/5 (v/v) pour fournir 0,980 g de cristaux ; F = 262°C ; Rendement = 73 %.

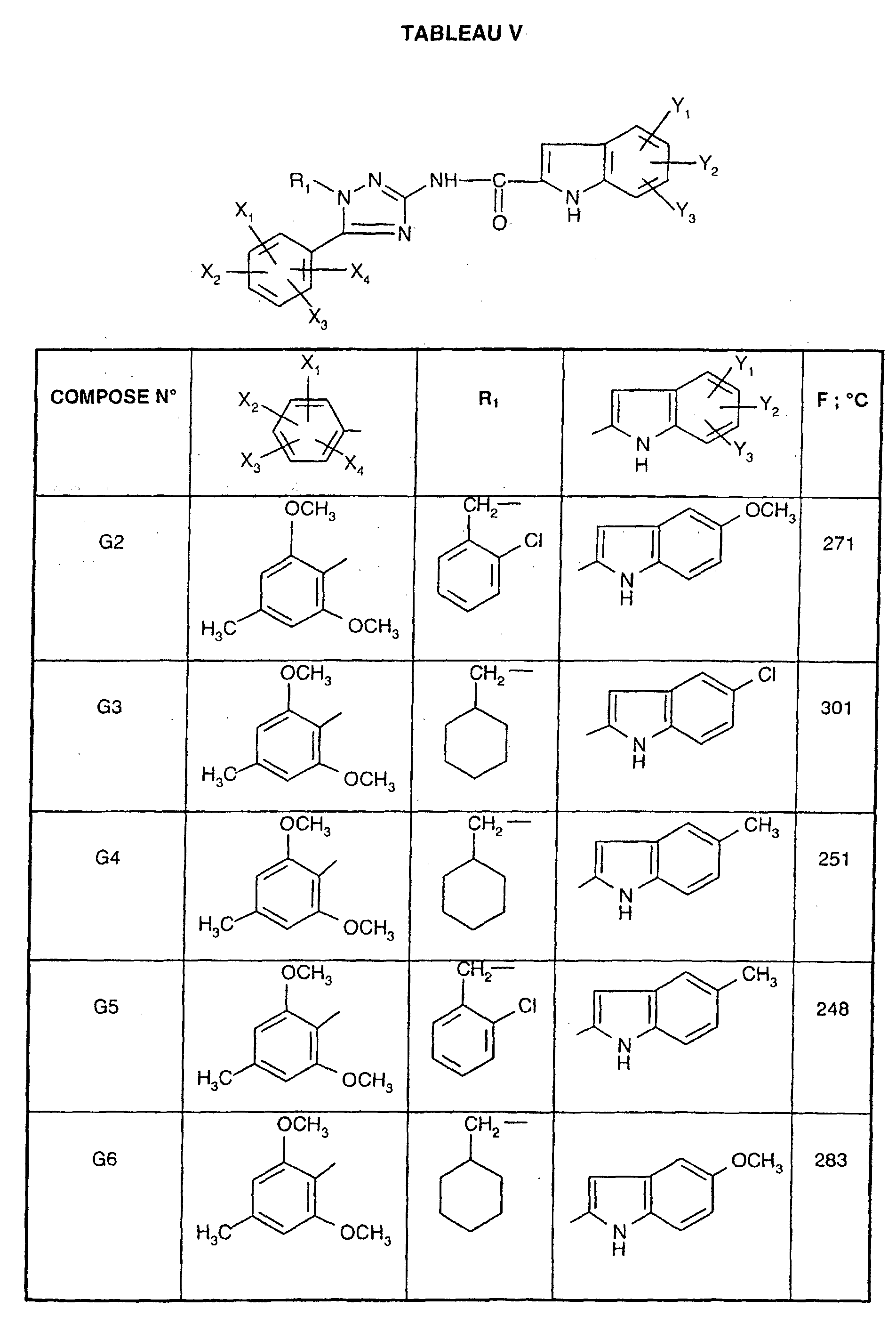

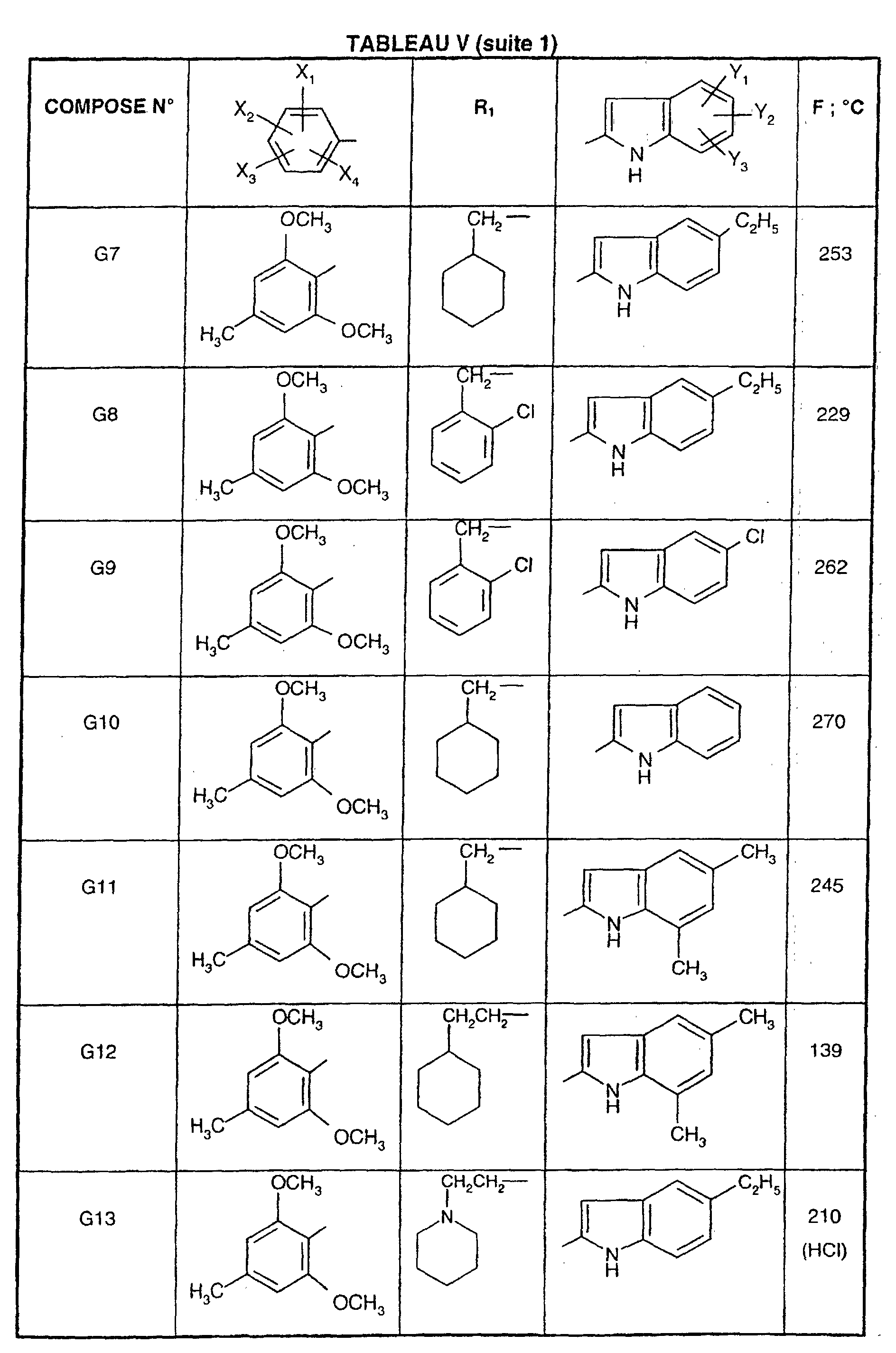

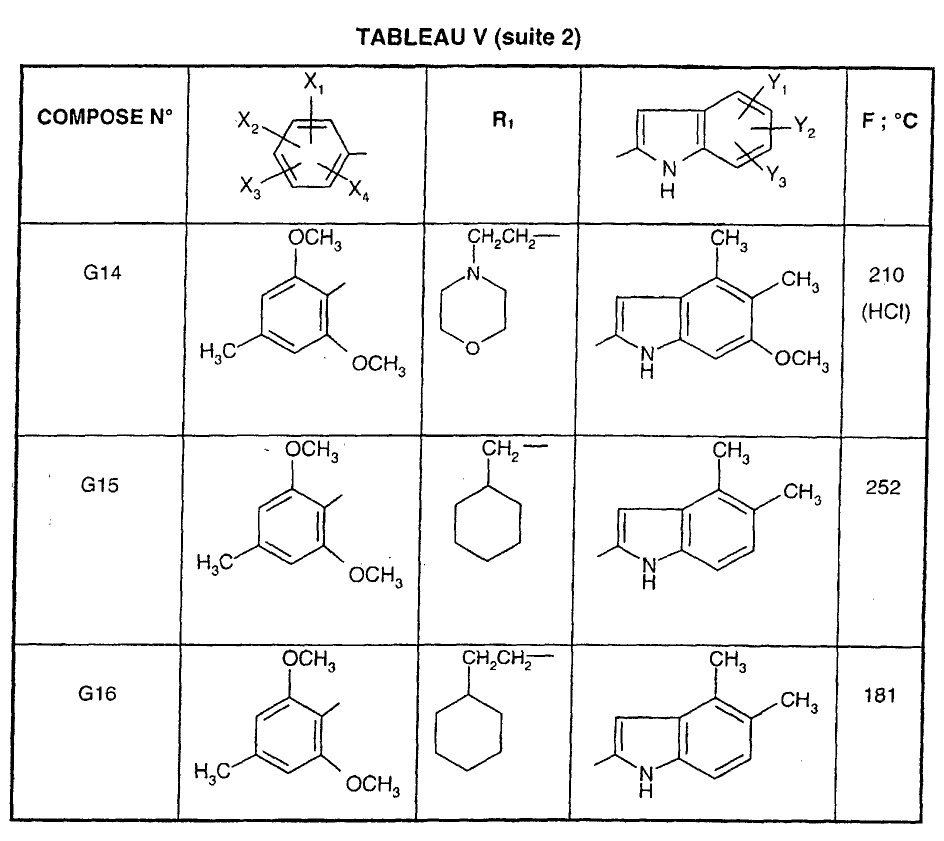

### H. PREPARATION DES DERIVES AMINOTRIAZOLES AVEC INDOLES N-SUBSTITUES

### EXEMPLE 1

### 2-[2-({[1-(2-cyclohexyléthyl)-5-(2,6-diméthoxy-4-méthylphényl)-1H-1,2,4-triazol -3-yl]amino)carbonyl)-5-éthyl-1H-indol-1-yl]acétate de méthyle

A 15 ml de dichlorométhane, on additionne successivement 1 ml de pyridine (0,013 mole) et 0,21 ml de chlorure de thionyle (0,00029 mole). Après 15 minutes à 0°C, on introduit 0,627 g de l'acide 5-éthyl-1-méthoxycarbonylméthyl-1*H*-2-indolecarboxylique (0,0024 mole), puis 0,9 g de chlorhydrate de 1-(2-cyclohexyléthyl)-5-(2,6-diméthoxy-4-méthylphényl)-1*H*-1,2,4-triazol-3-amine. On abandonne le mélange réactionnel pendant 18 heures à température ambiante, puis on fait un lavage acide, puis un lavage basique. La phase organique est séchée sur sulfate de sodium anhydre et concentrée. Le résidu huileux est chromatographié sur gel de silice, éluant : dichlorométhane/méthanol 98,5/1,5 (v/v) pour fournir une poudre blanche ; F = 191°C ;
Rendement = 87 %.

### EXEMPLE 2

### Acide 2-[2-({[1-(2-cyclohexyléthyl)-5-(2,6-diméthoxy-4-méthylphényl)-1H-1,2,4-triazol-3-yl]amino}carbonyl)-5-éthyl-1H-indol-1-yl]acétique

A 530 mg (0,0009 mole) de 2-[2-({[1-(2-cyclohexyléthyl)-5-(2,6-diméthoxy-4-méthylphényl)-1*H*-1,2,4-triazol-3-yl]amino}carbonyl)-5-éthyl-1*H*-indol-1-yl]acétate de méthyle préparé selon l'EXEMPLE 1 en solution dans 50 ml de méthanol, on additionne 1,8 ml (0,0018 mole) d'une solution 1N d'hydroxyde de sodium. Après 18 heures à température ambiante, on évapore le mélange réactionnel à sec. Le résidu est repris par de l'acétate d'éthyle, et une solution d'acide chlorhydrique 0,5 N. On décante et sèche la phase organique sur sulfate de sodium anhydre et concentre. Le résidu est purifié par chromatographie sur colonne de gel de silice, éluant : dichlorométhane/méthanol 92/8 (v/v) pour fournir des cristaux blancs ; F = 198°C ;
Rendement = 91 %.

En procédant selon les EXEMPLES 1 et 2 ci-dessus, on prépare de la même façon, à partir des intermédiaires appropriés, les EXEMPLES 3 à 511 décrits dans les TABLEAUX VI et VII ci-après.

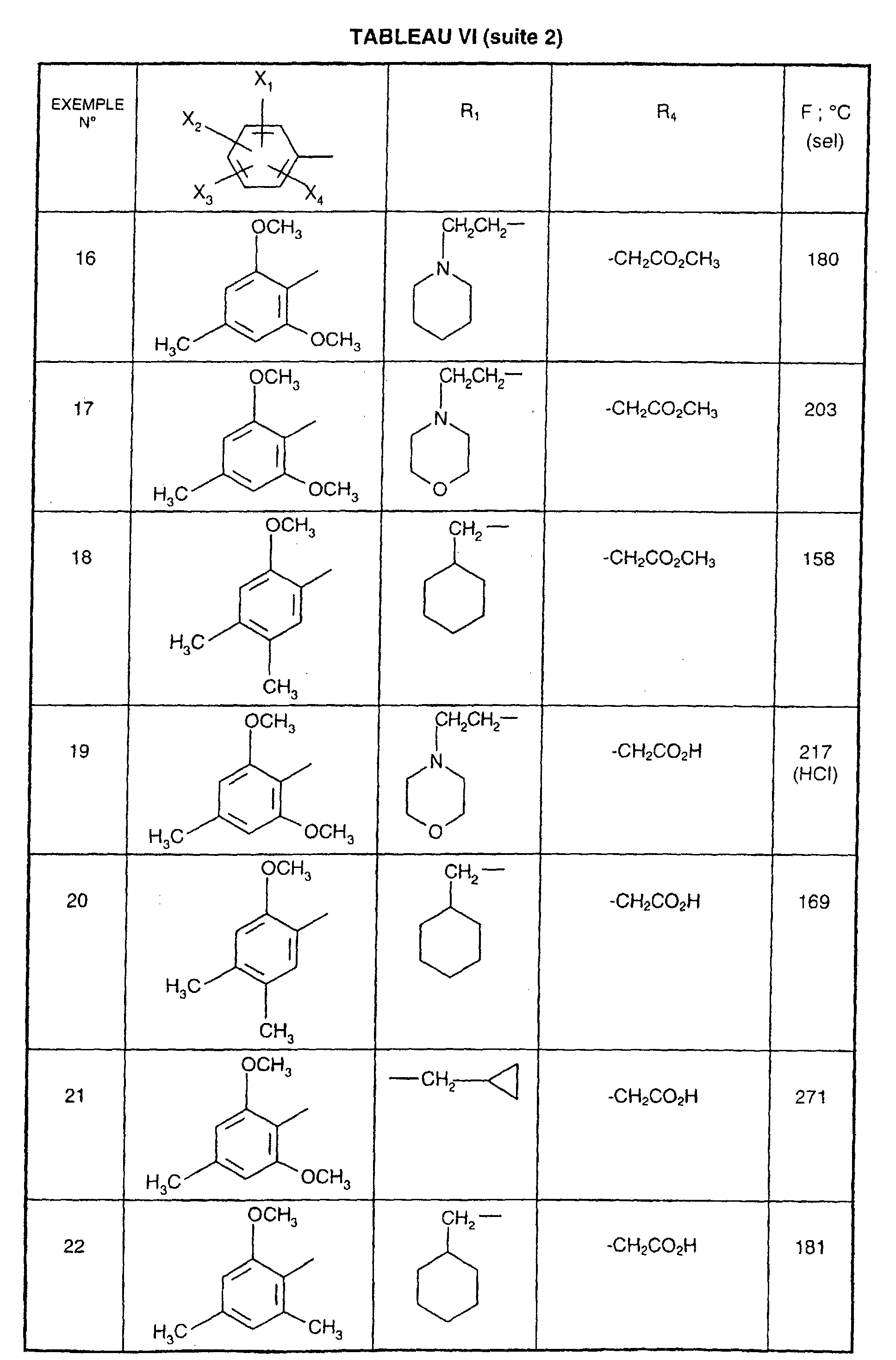

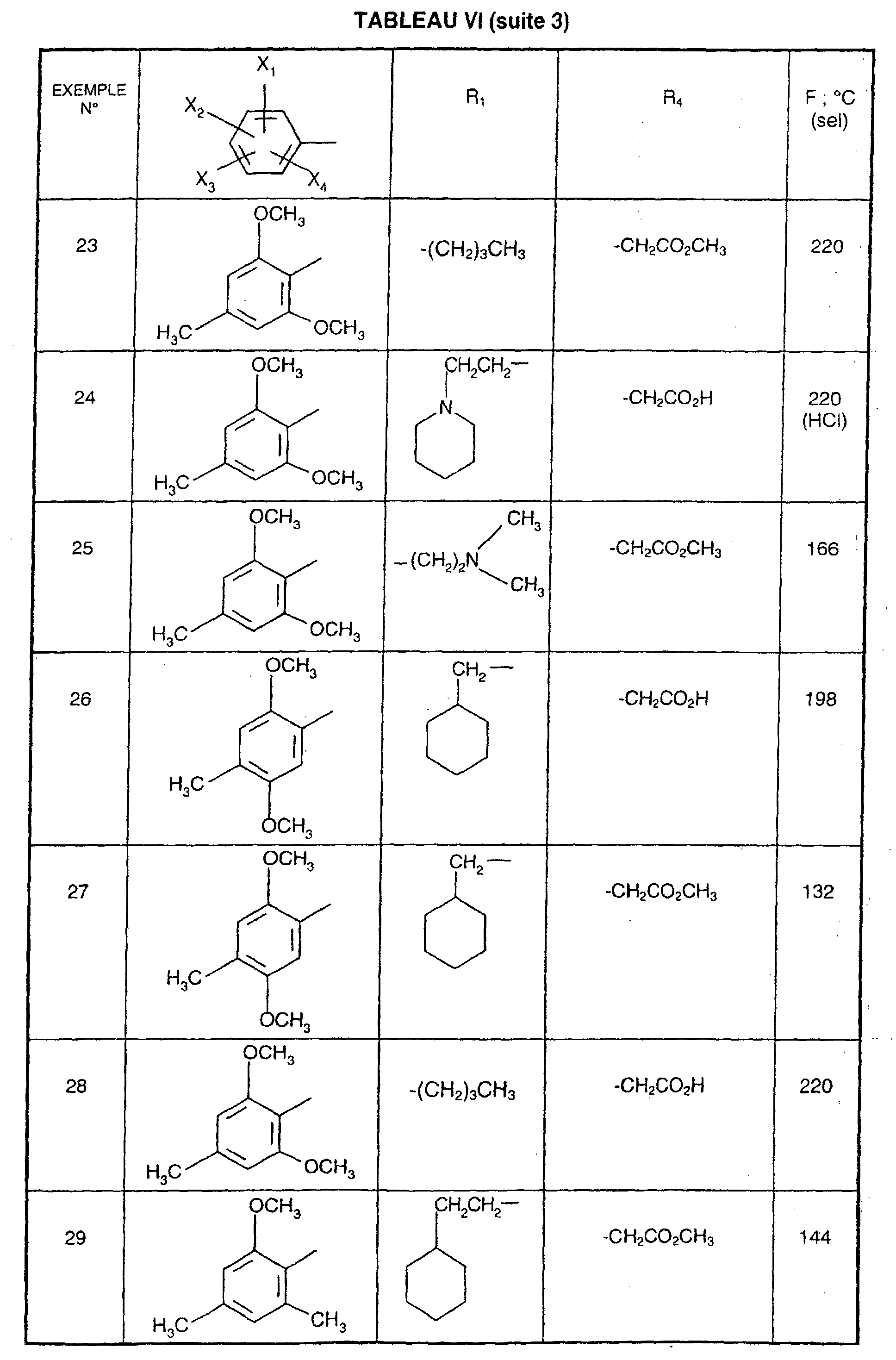

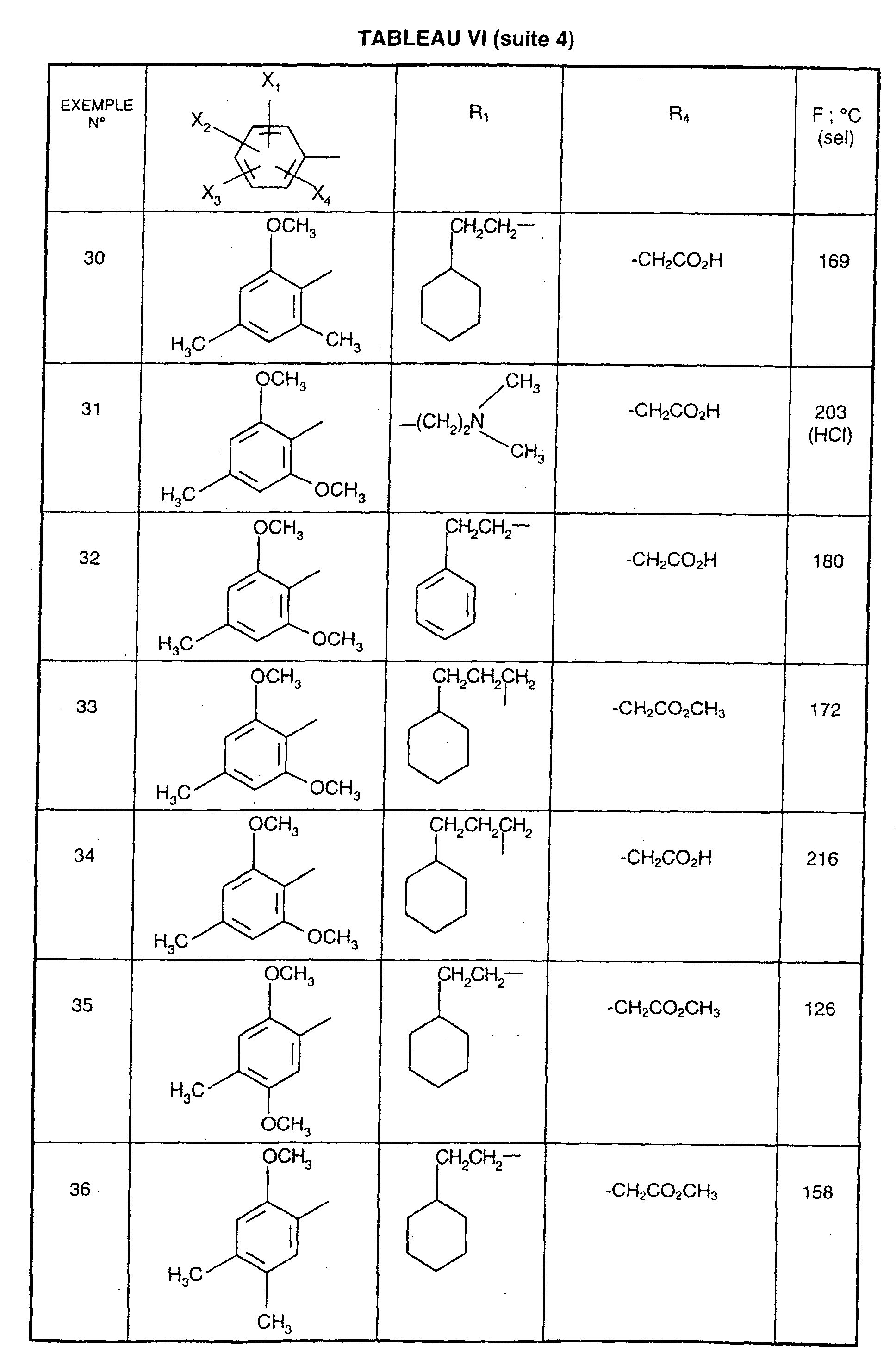

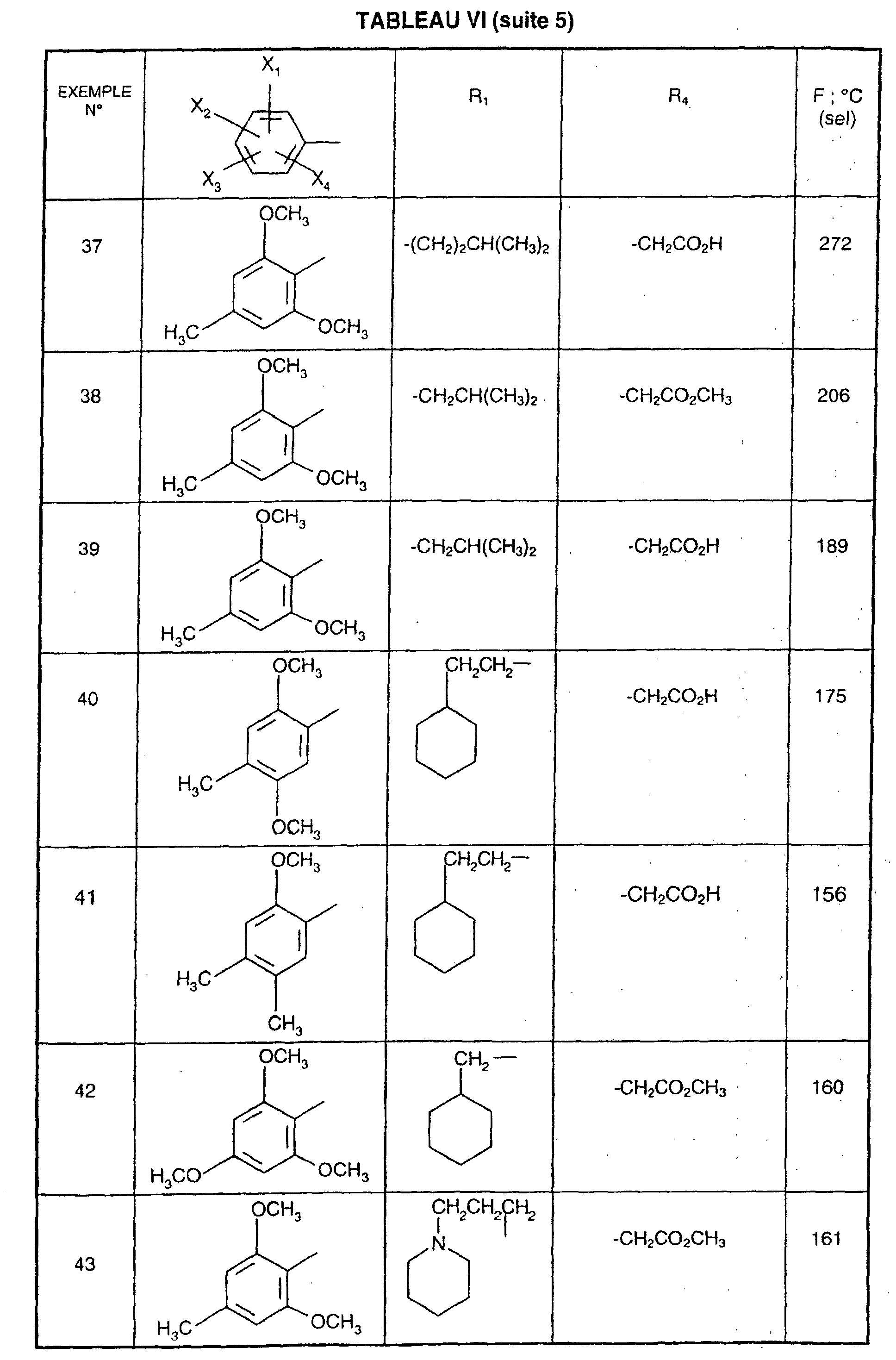

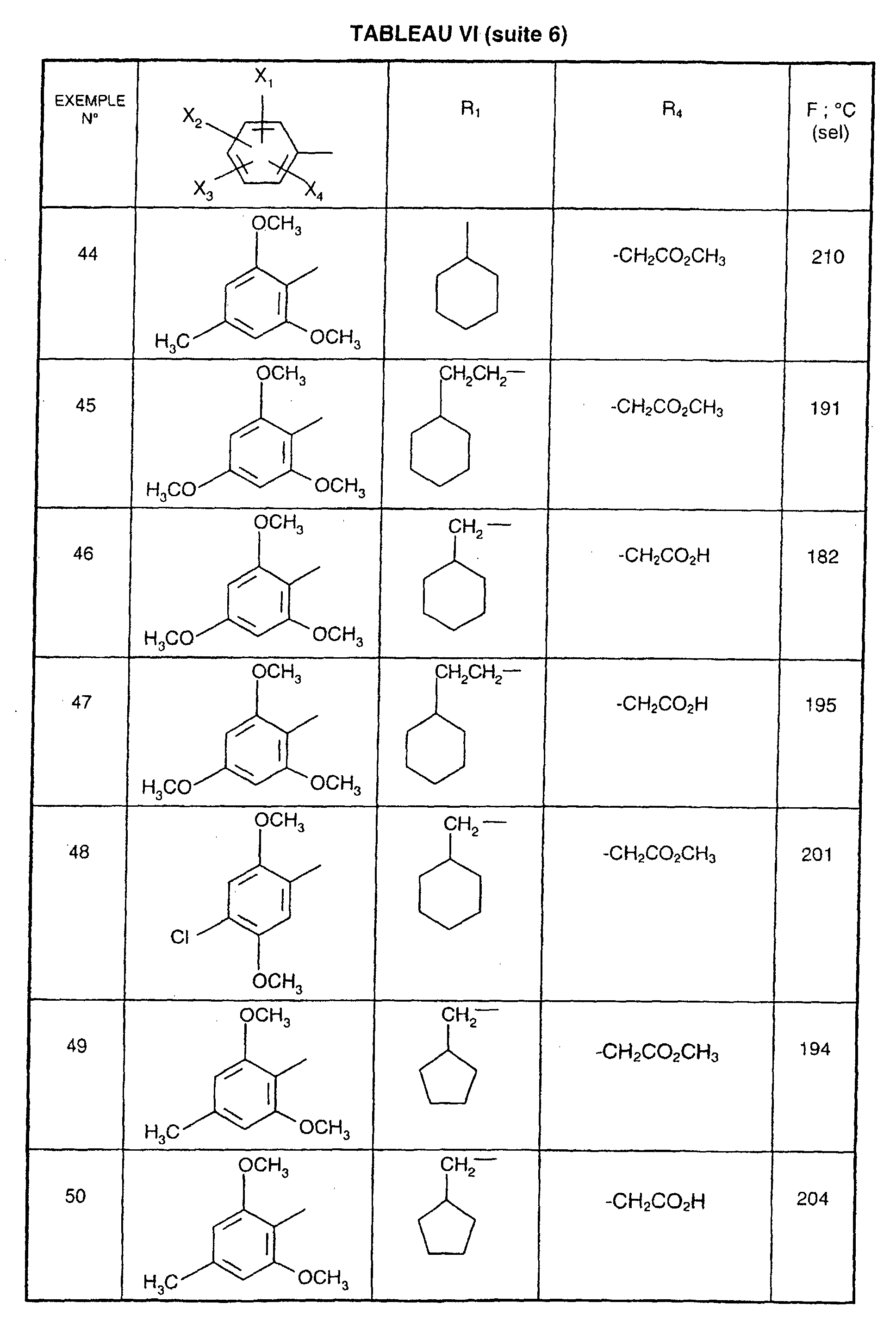

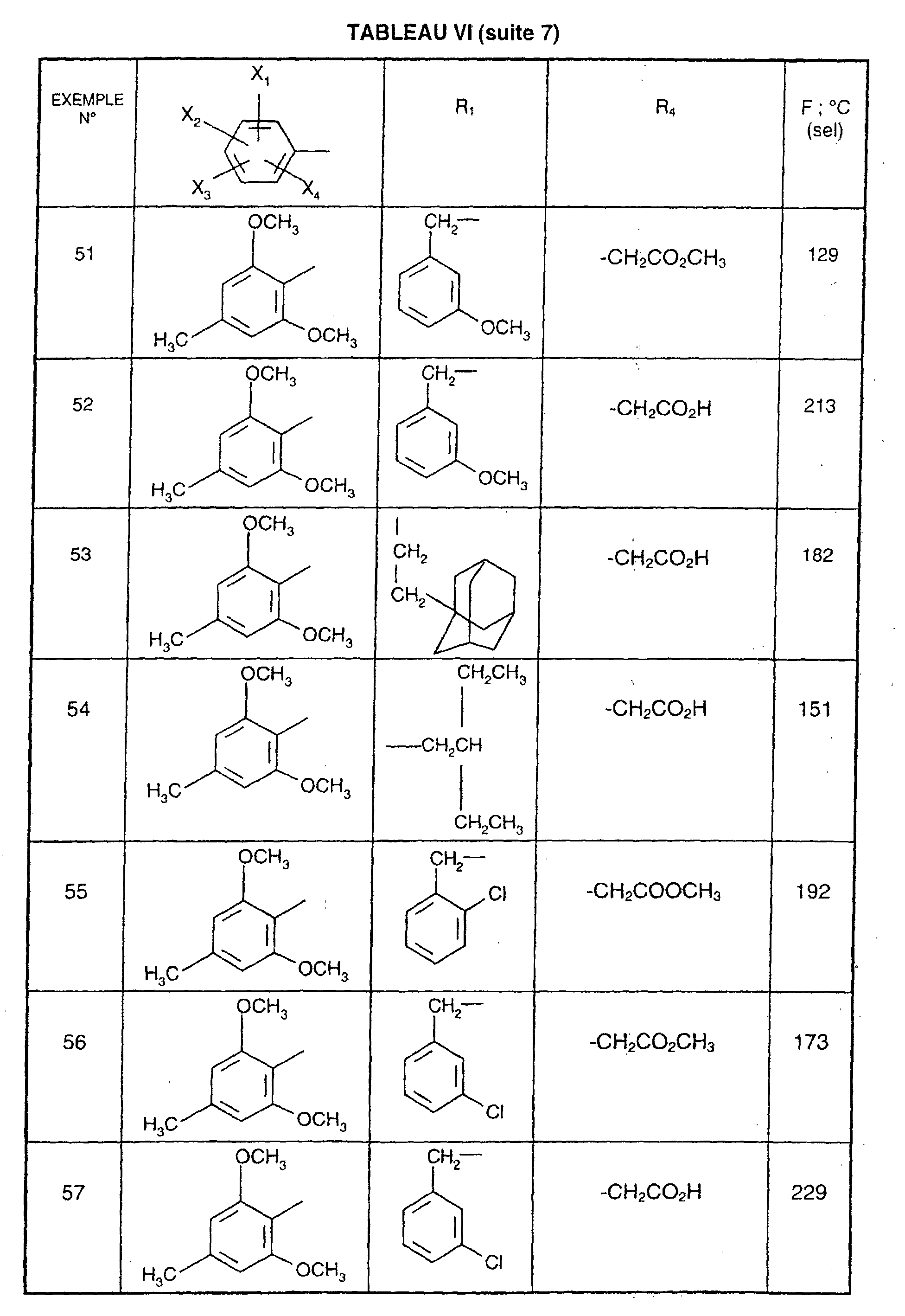

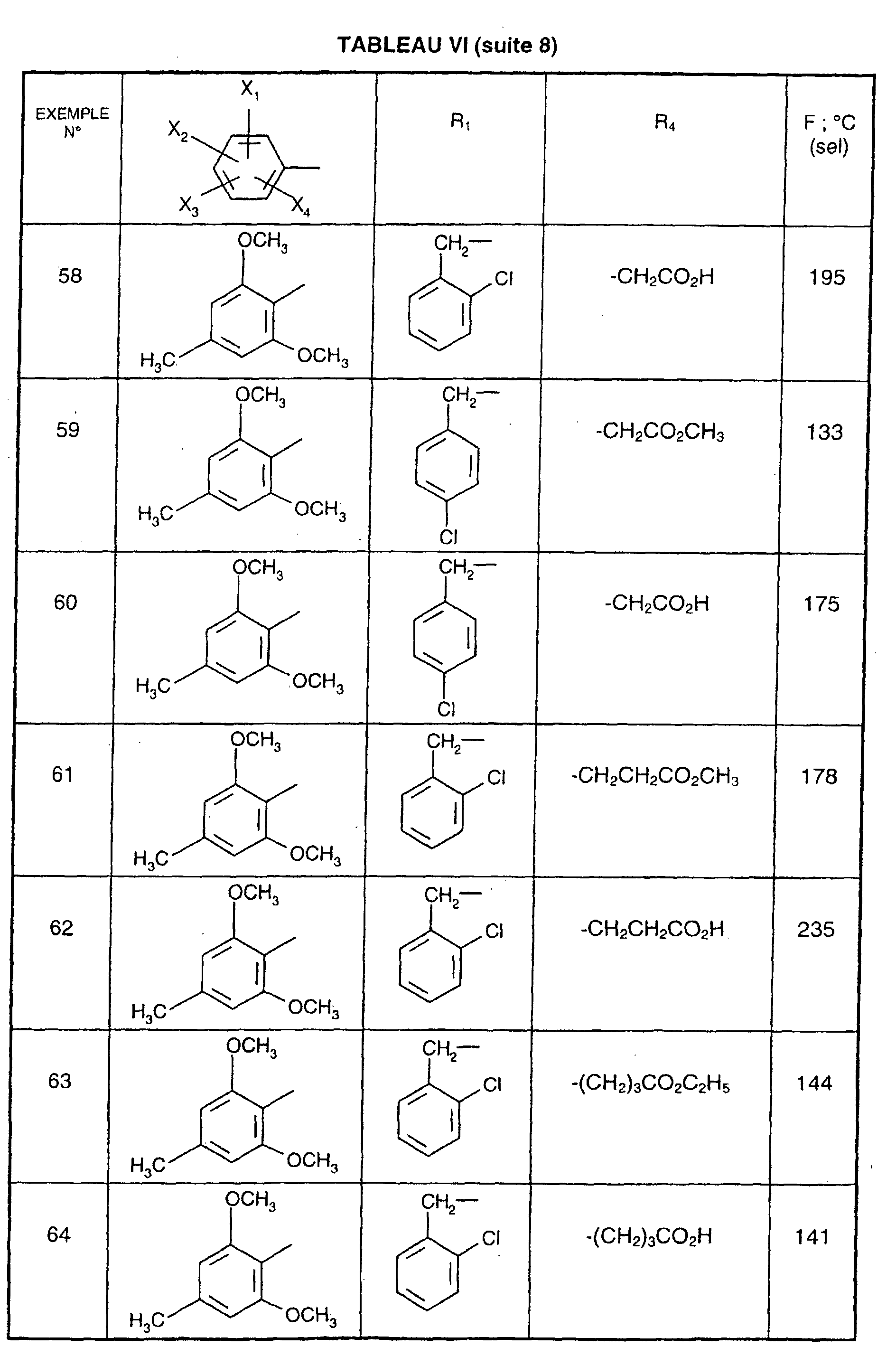

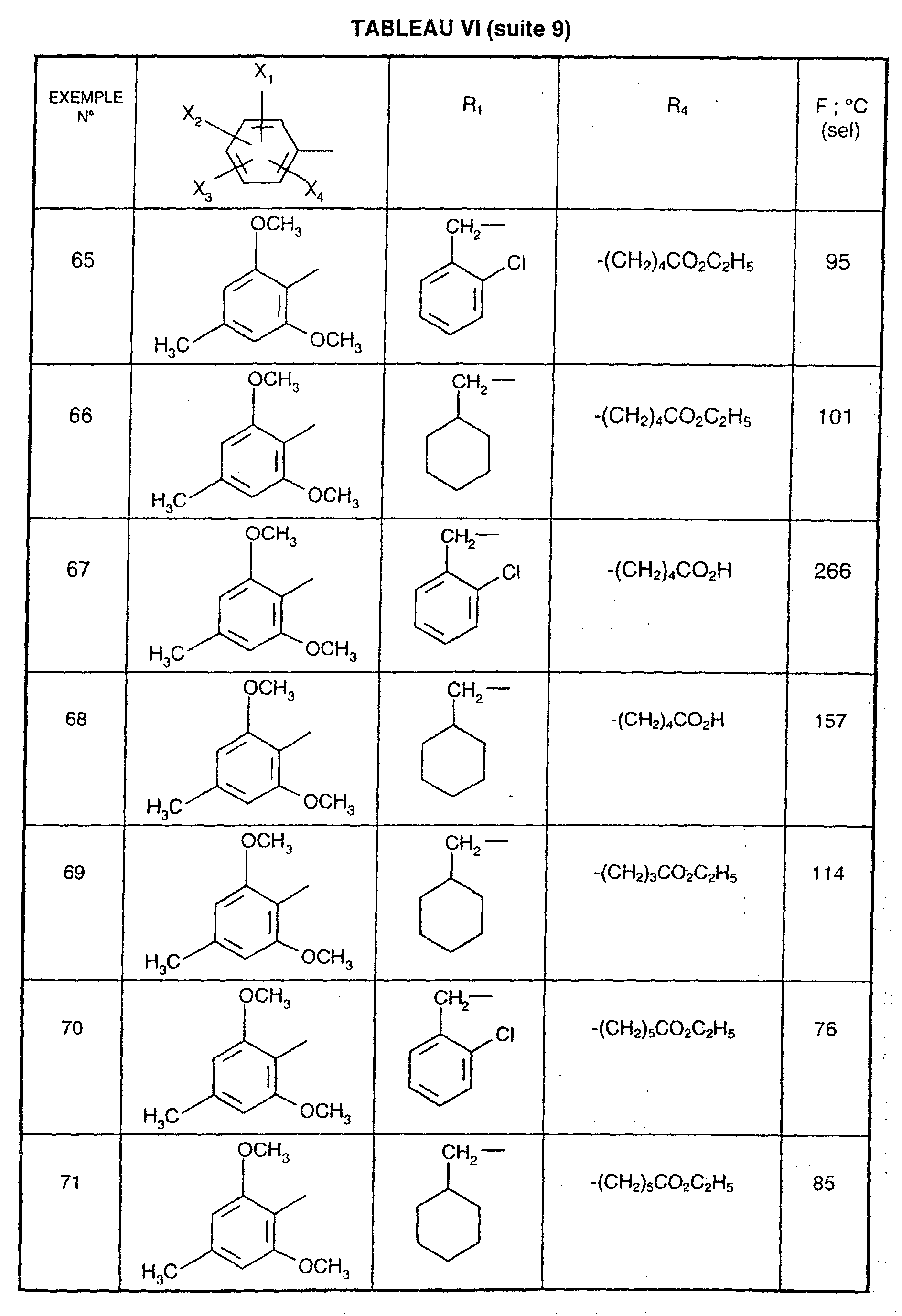

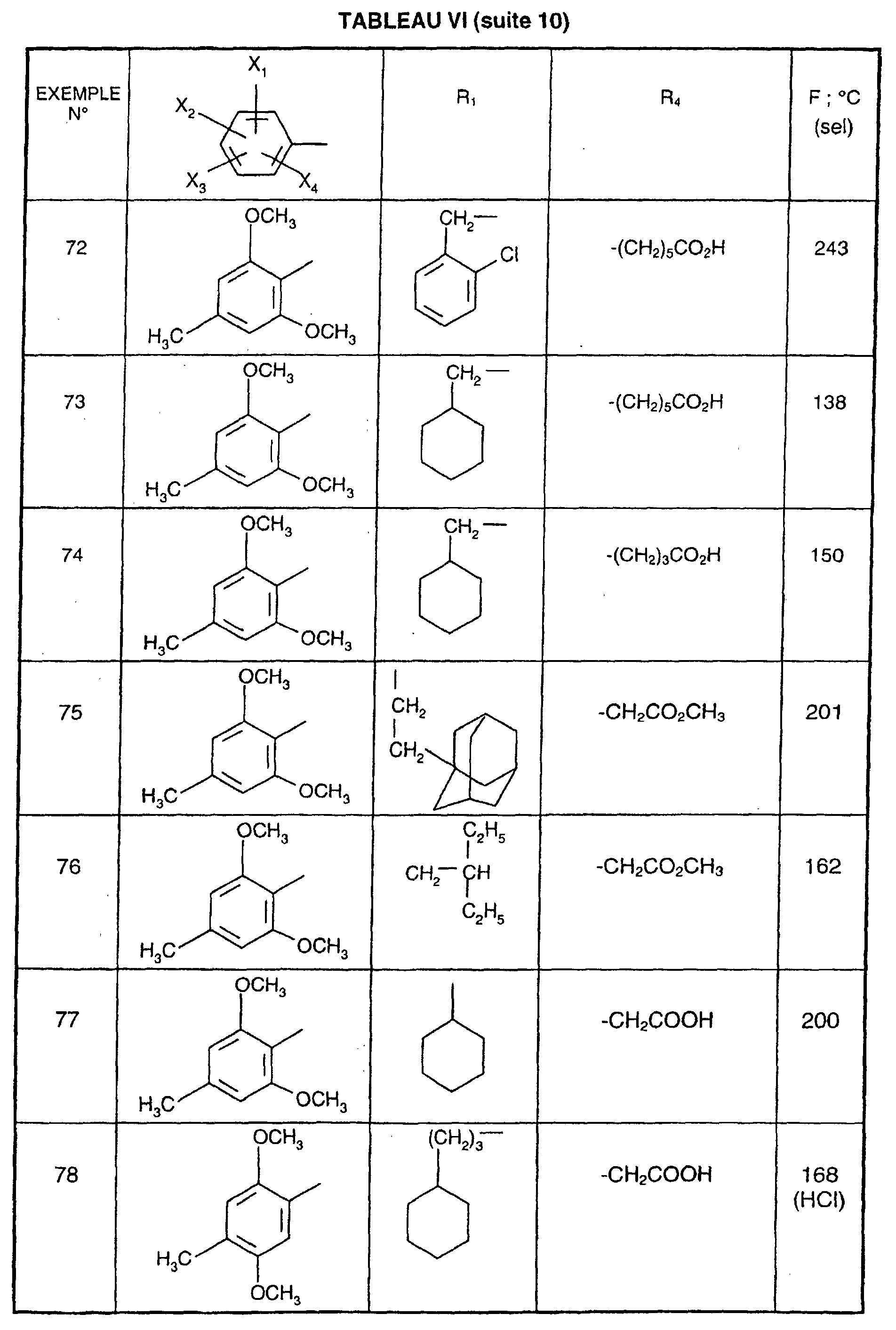

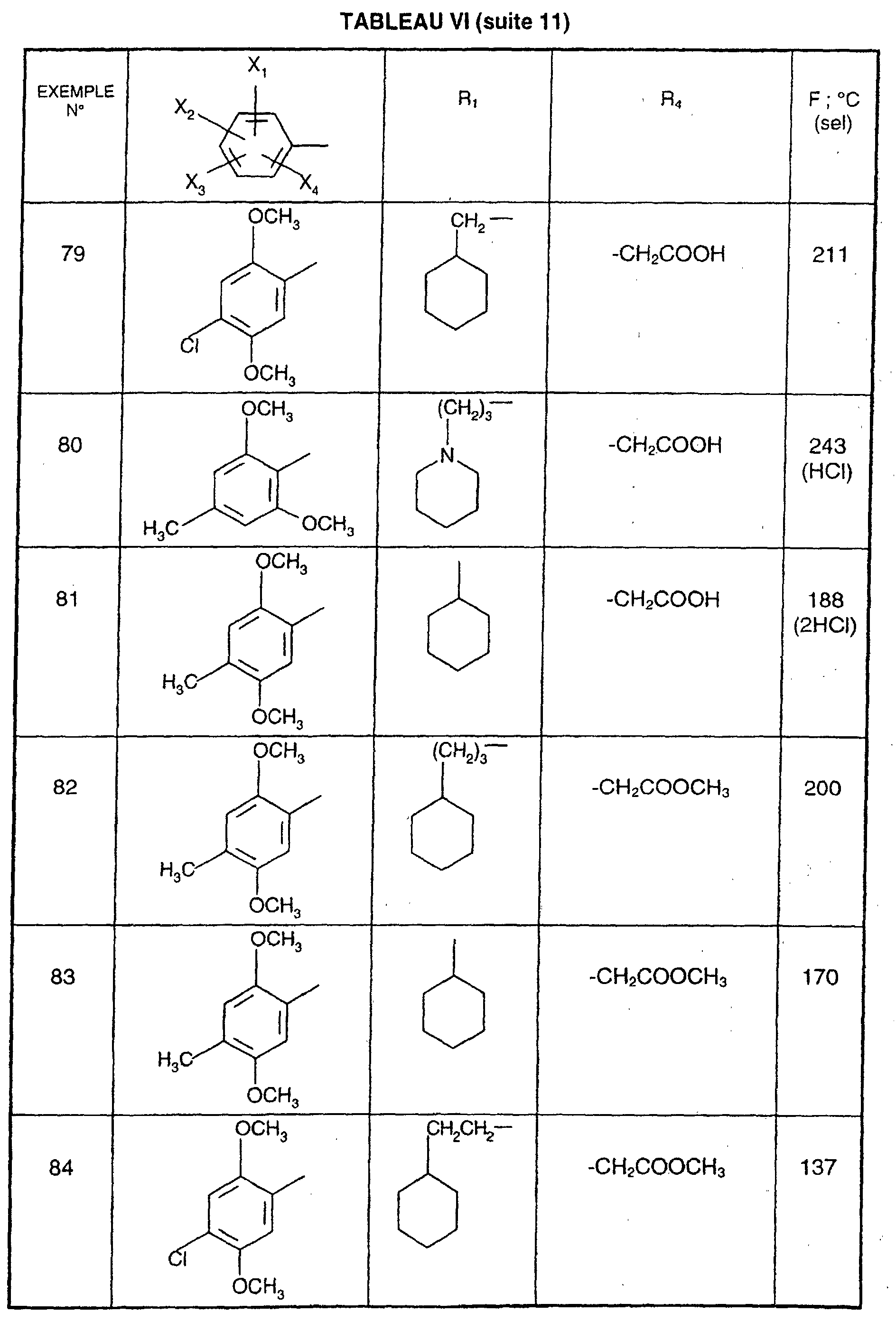

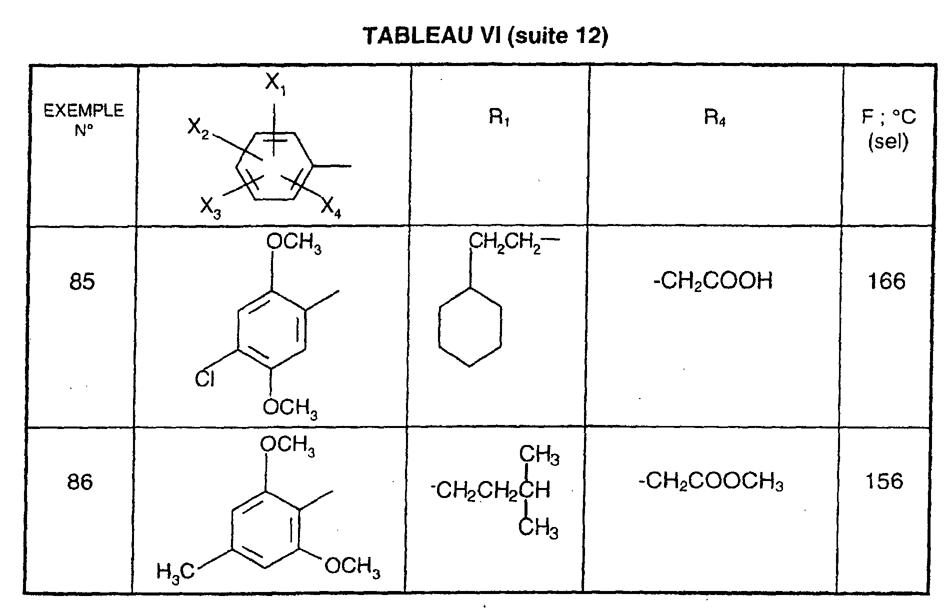

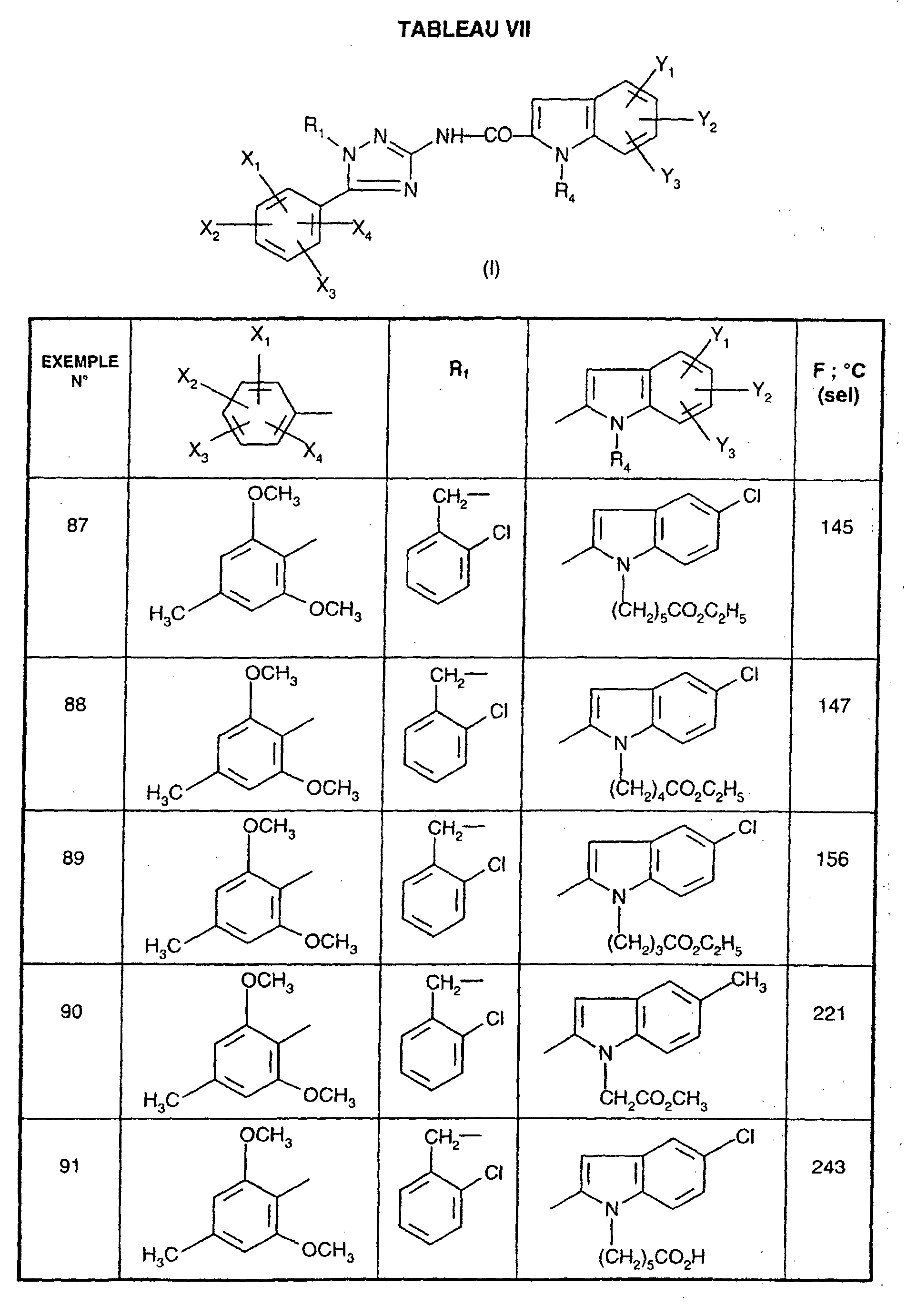

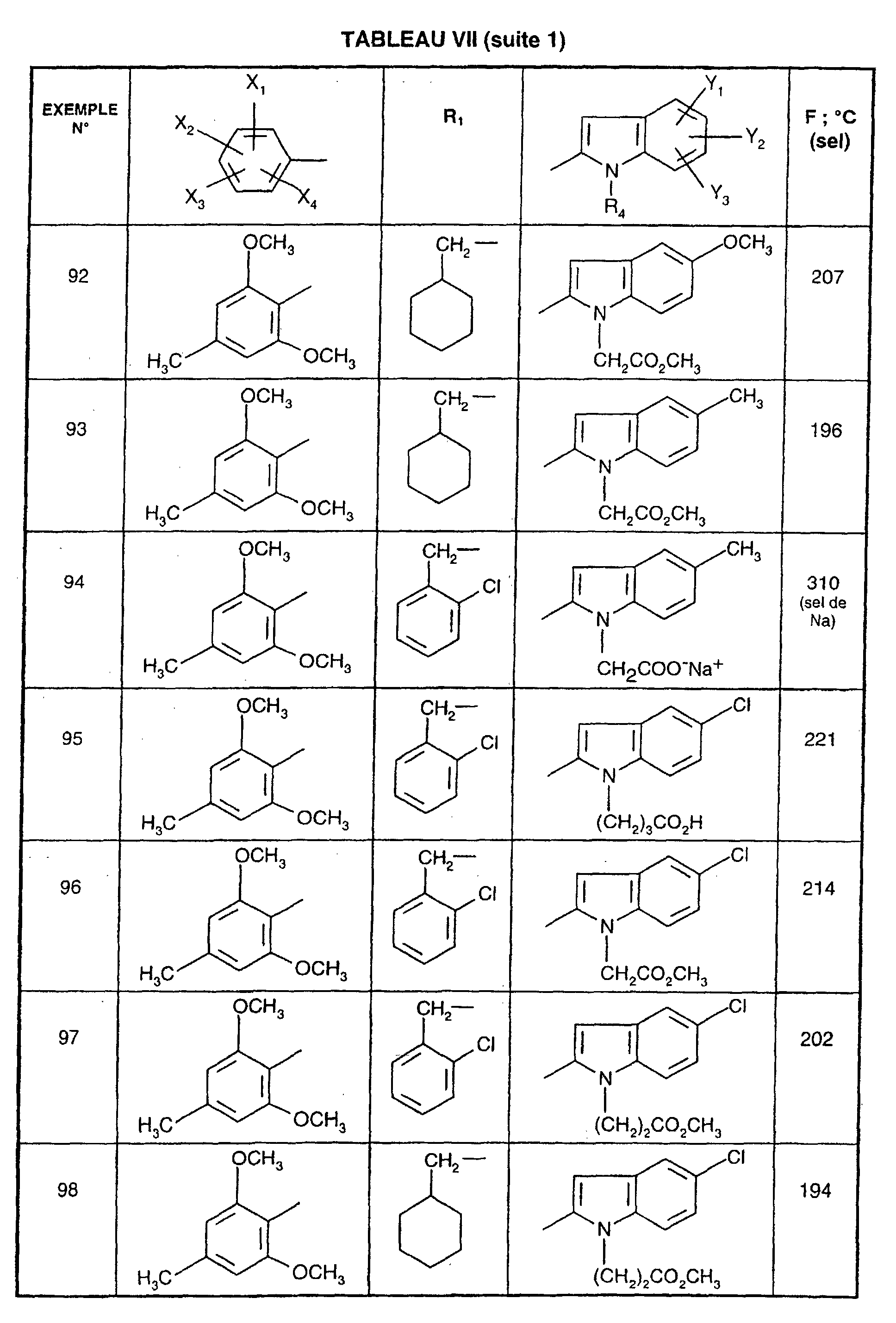

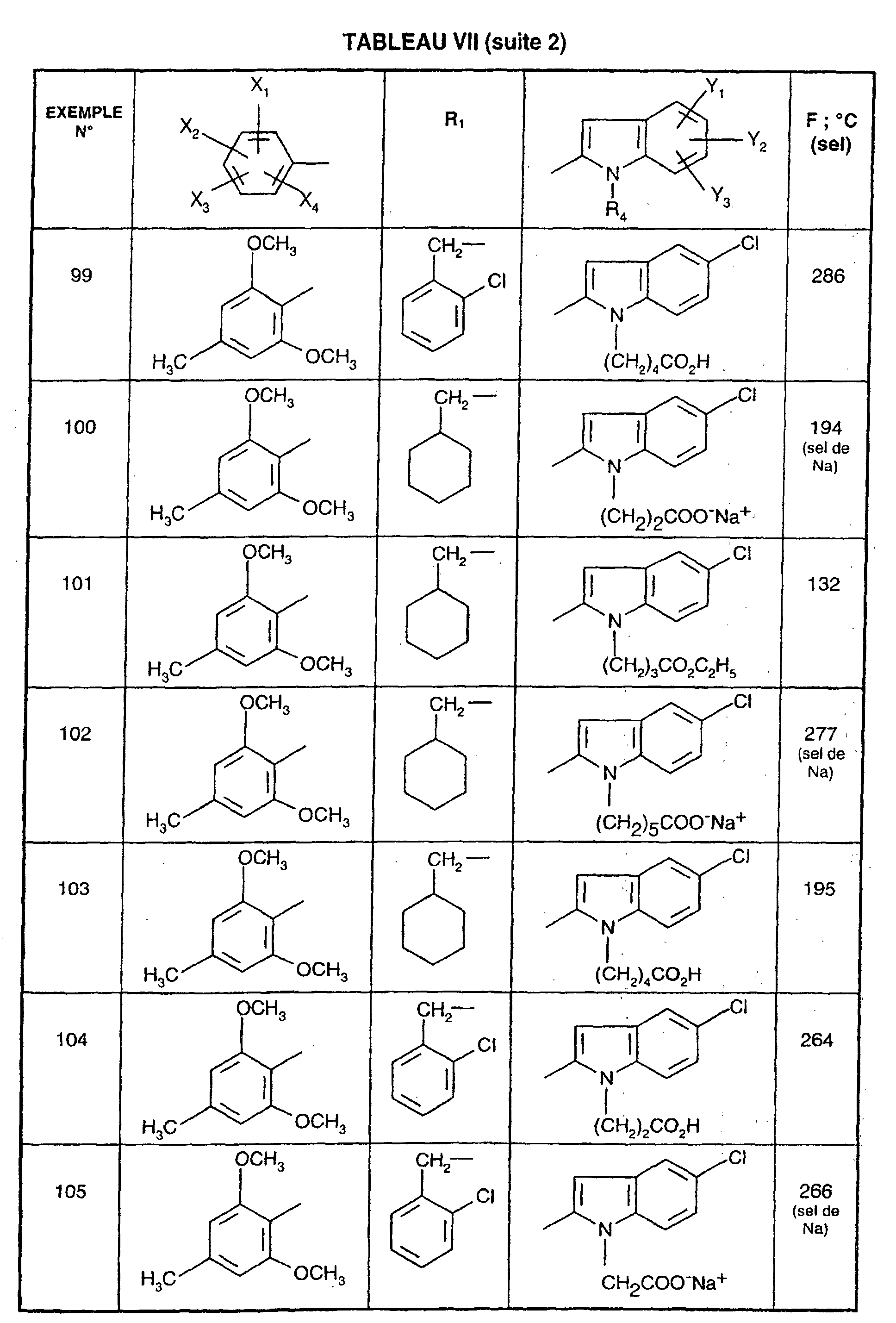

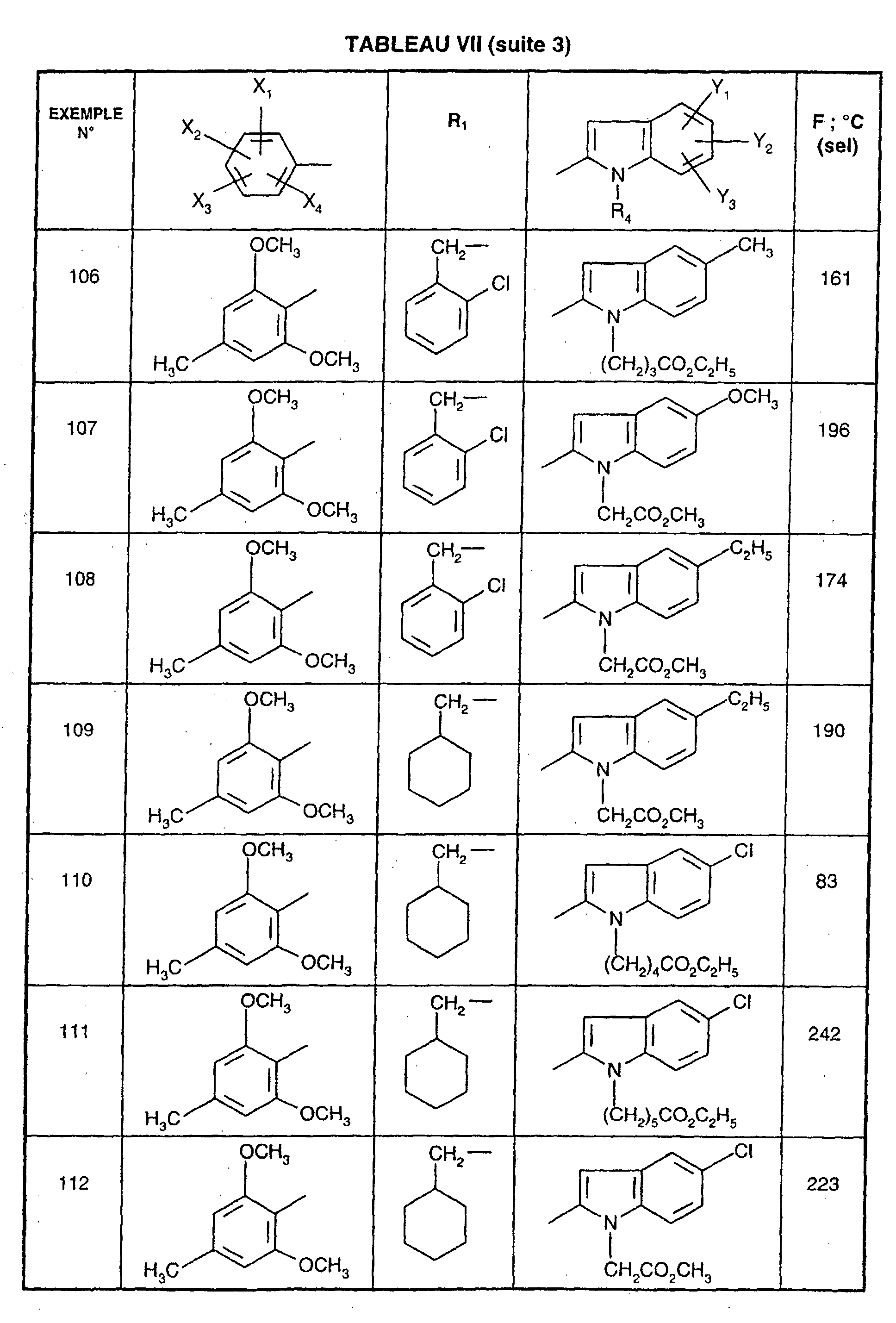

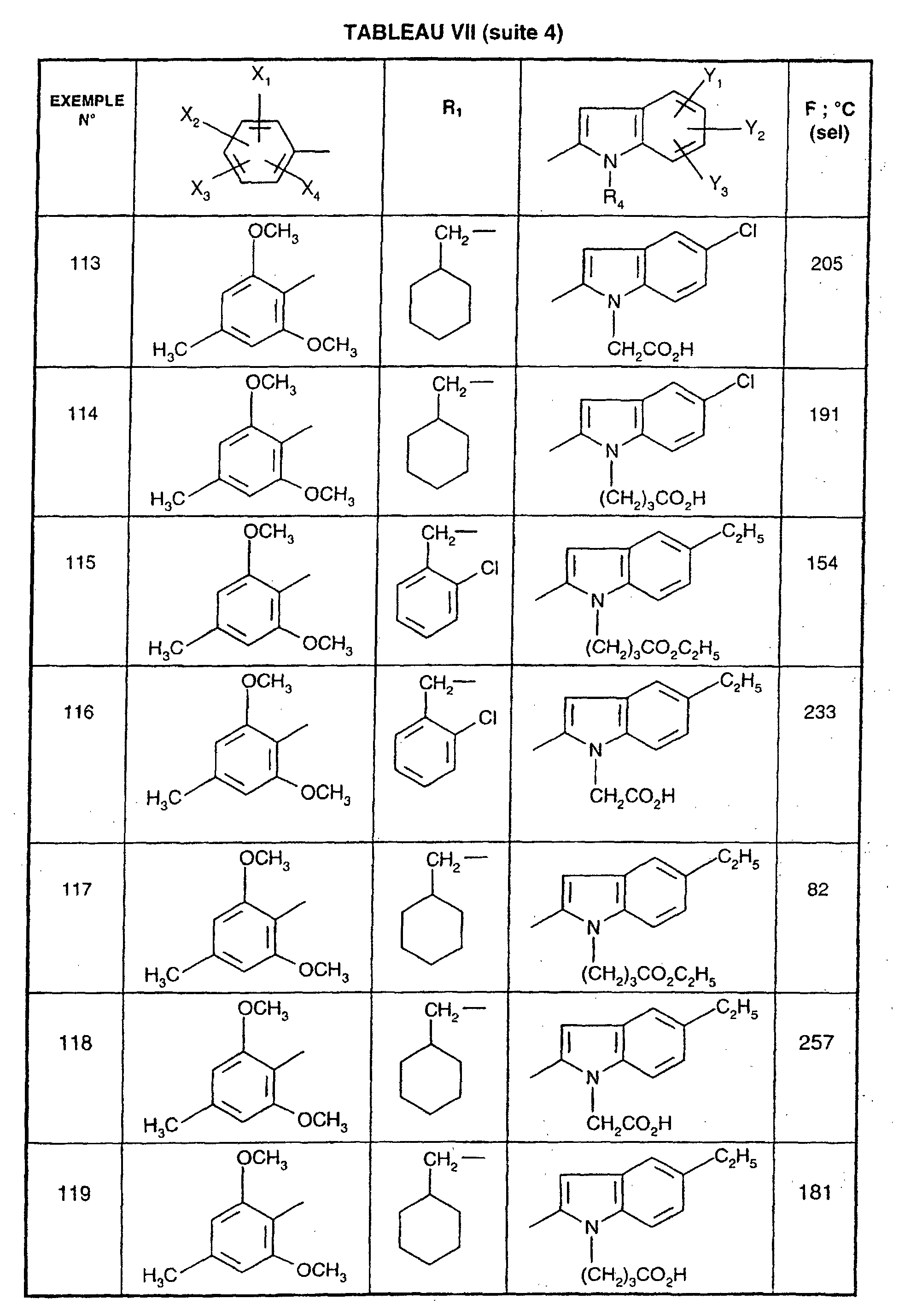

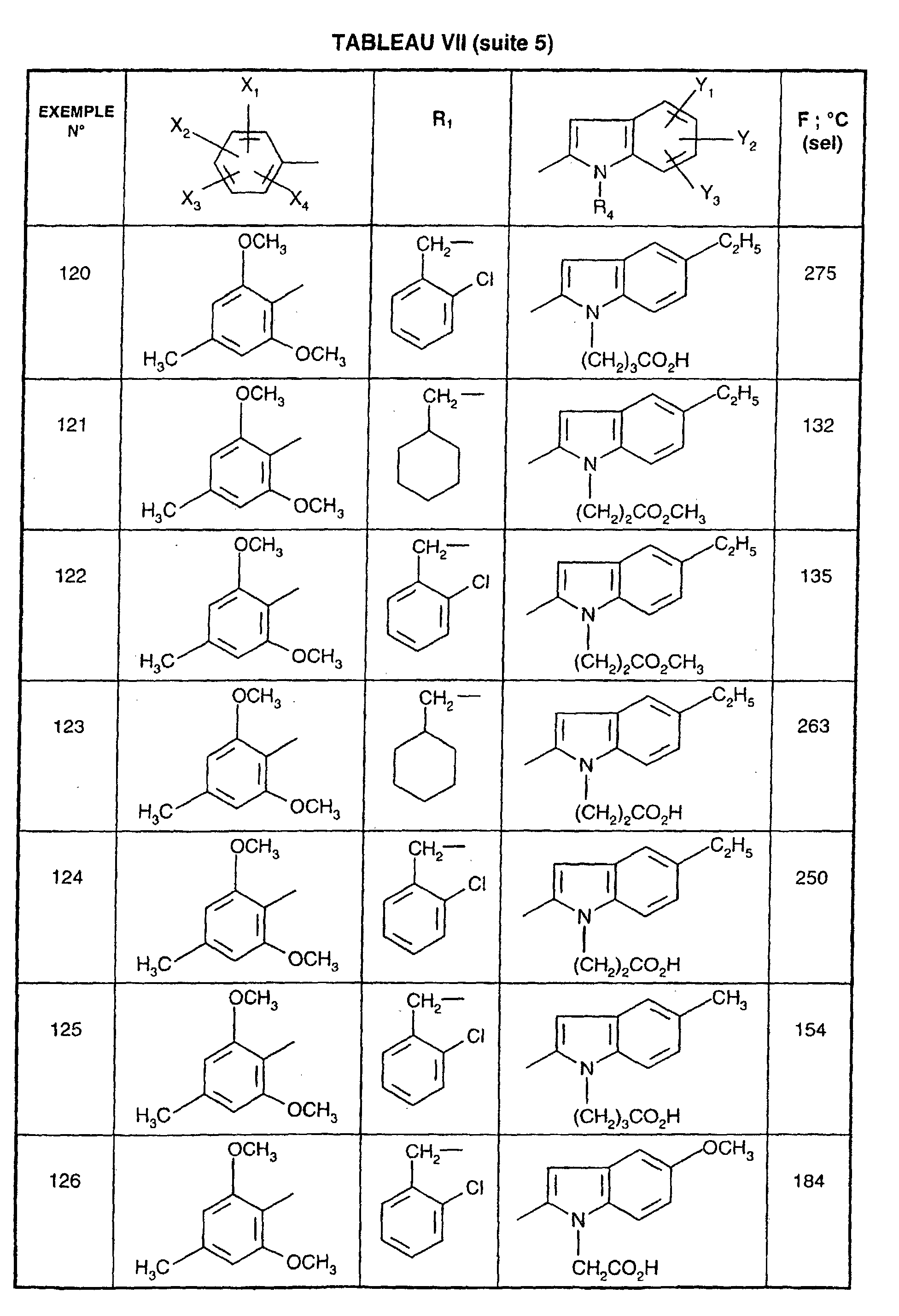

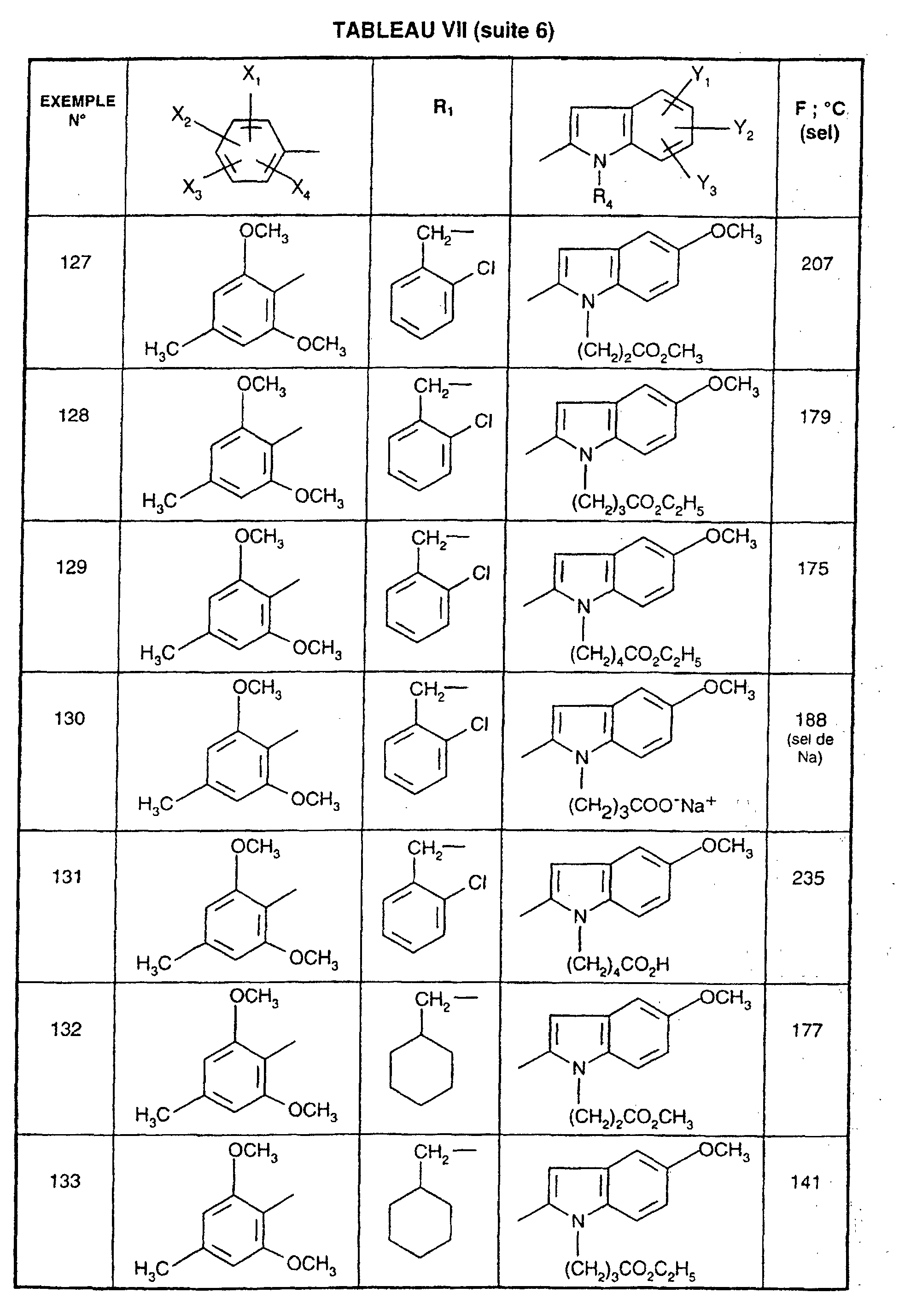

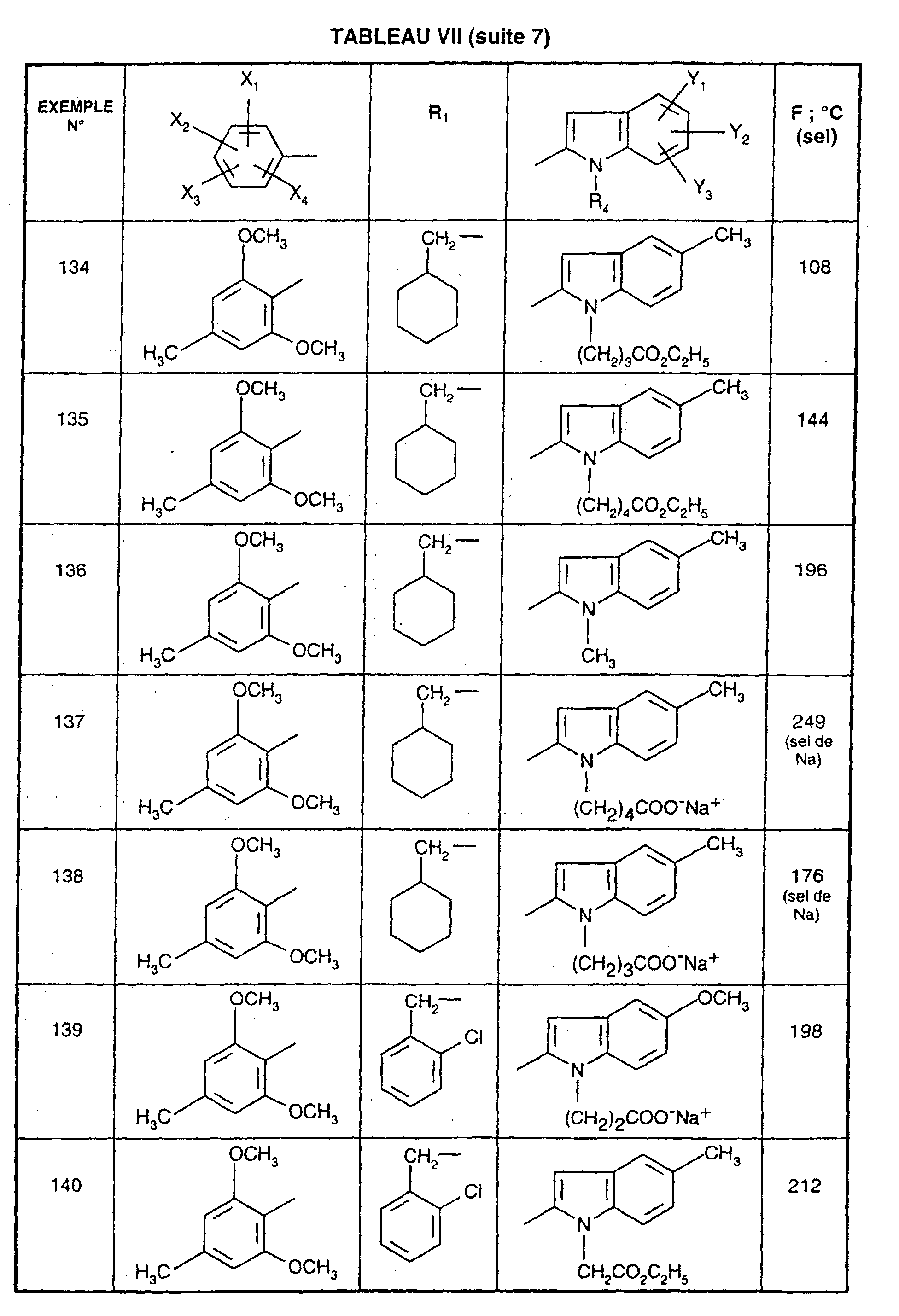

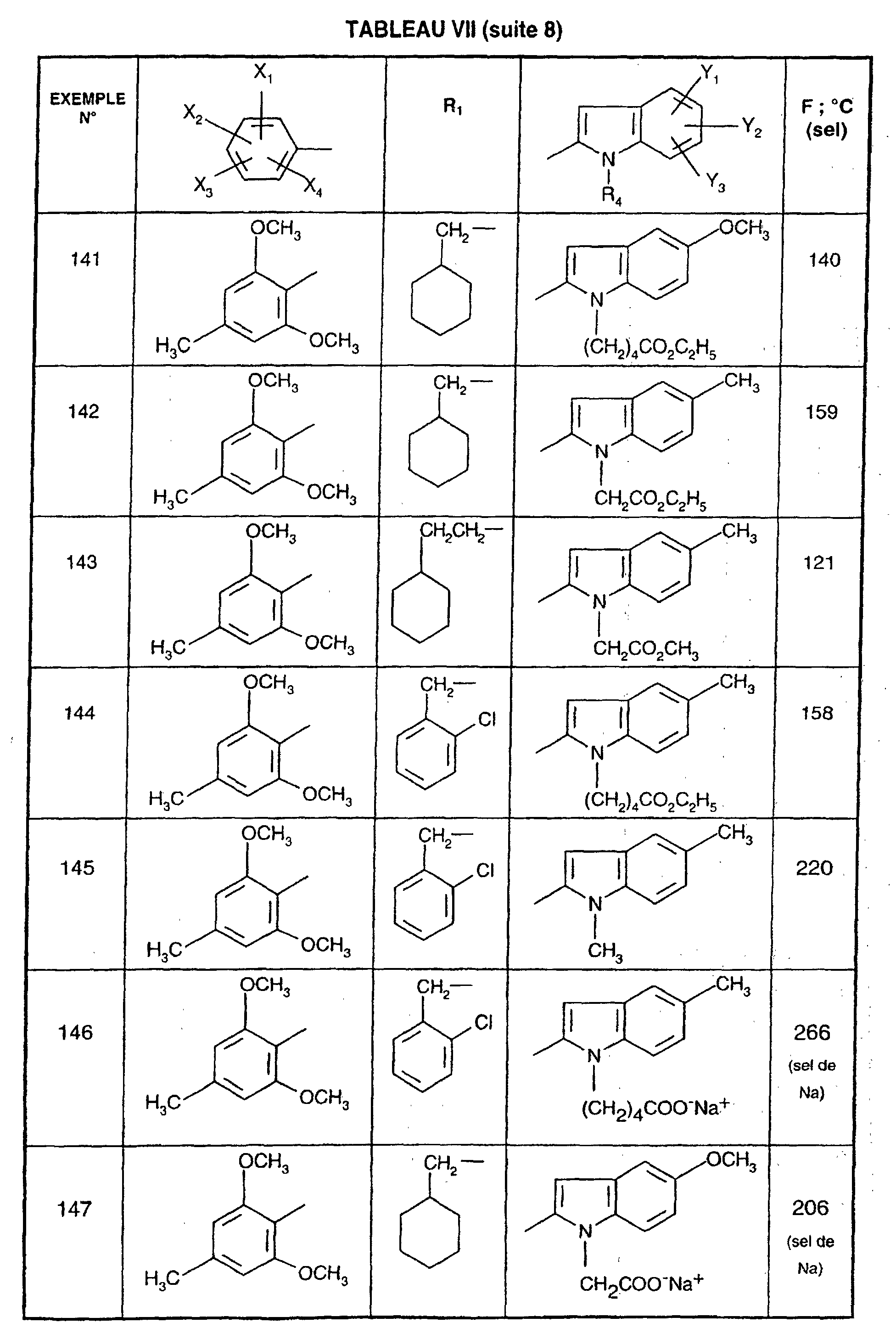

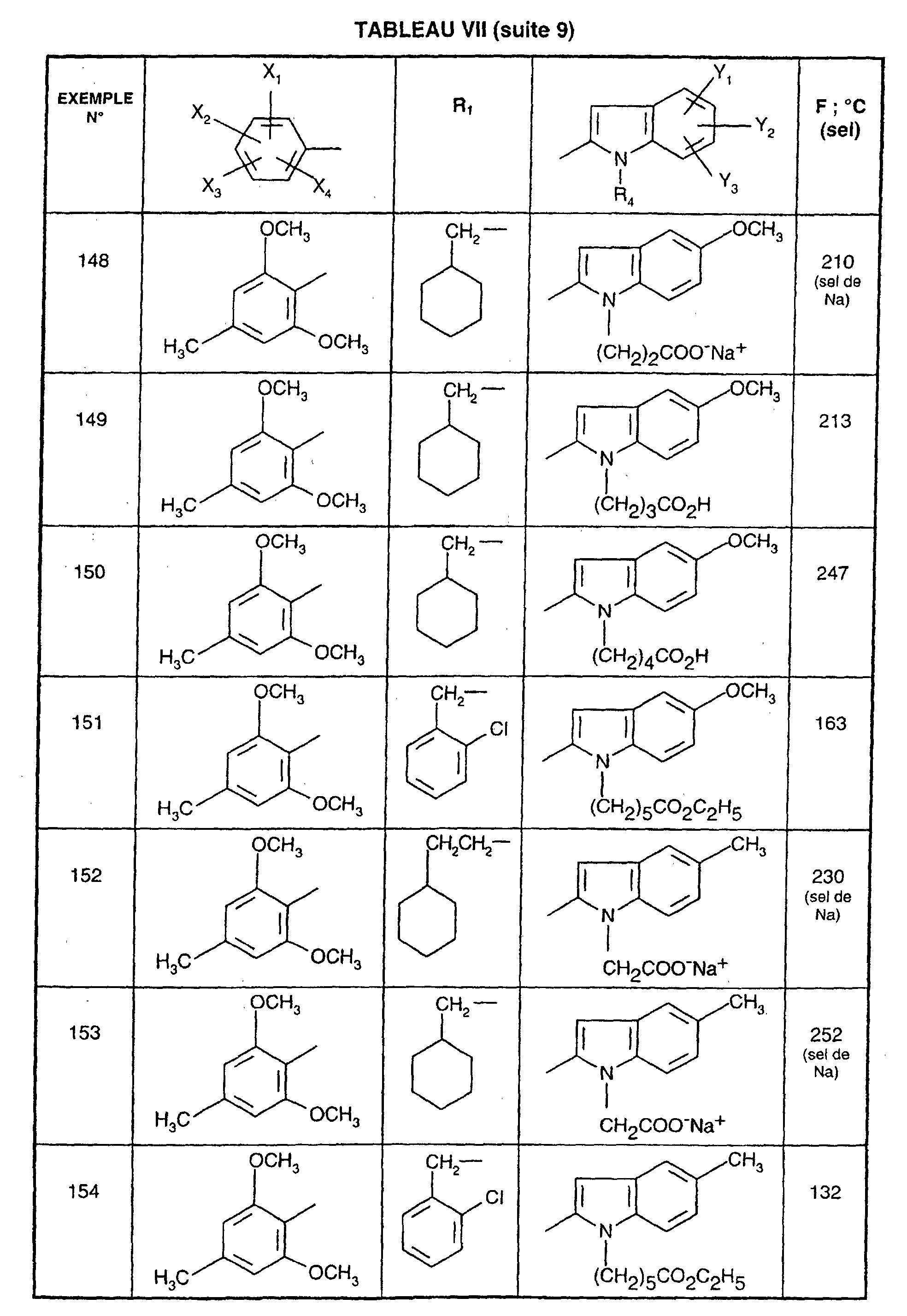

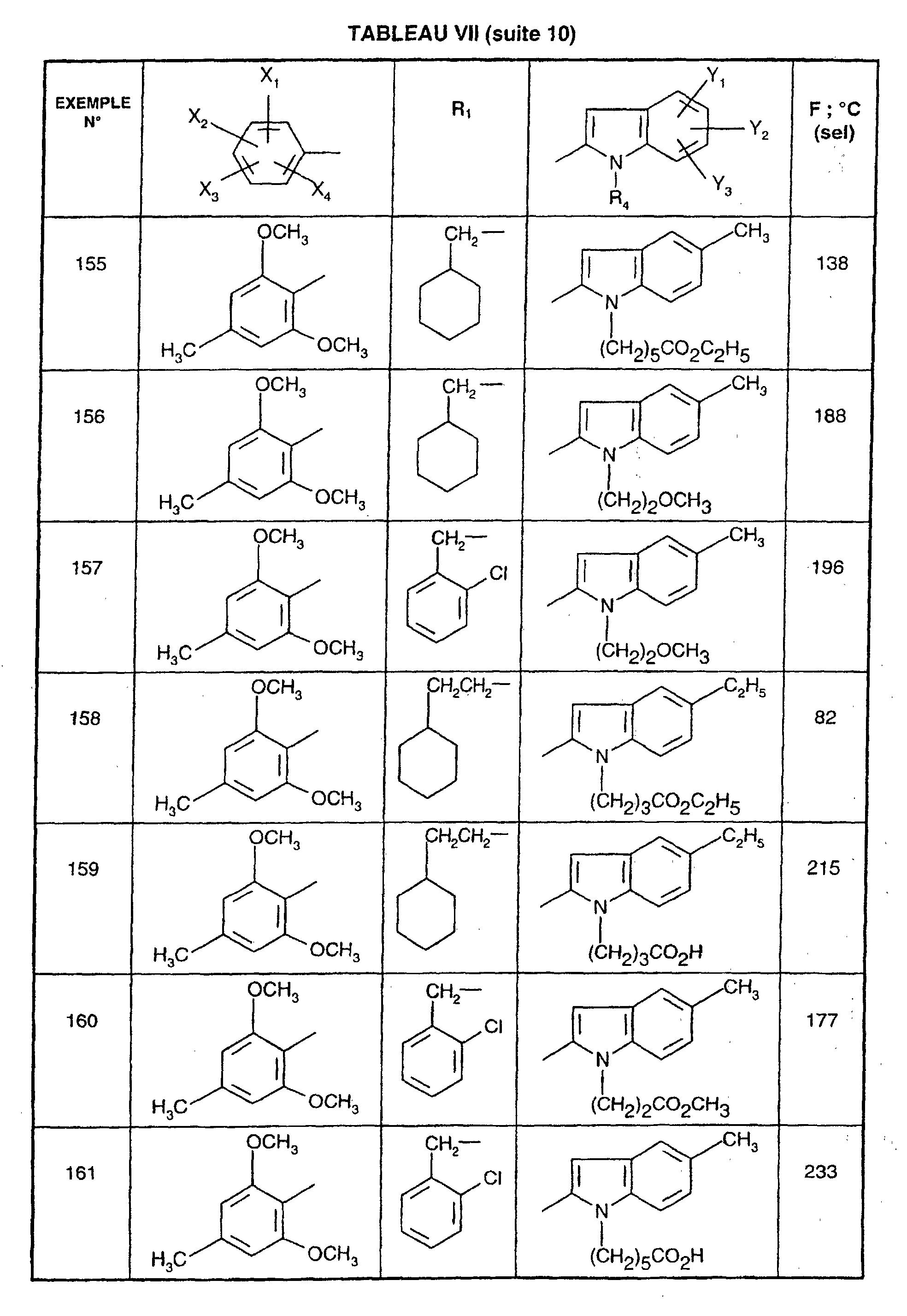

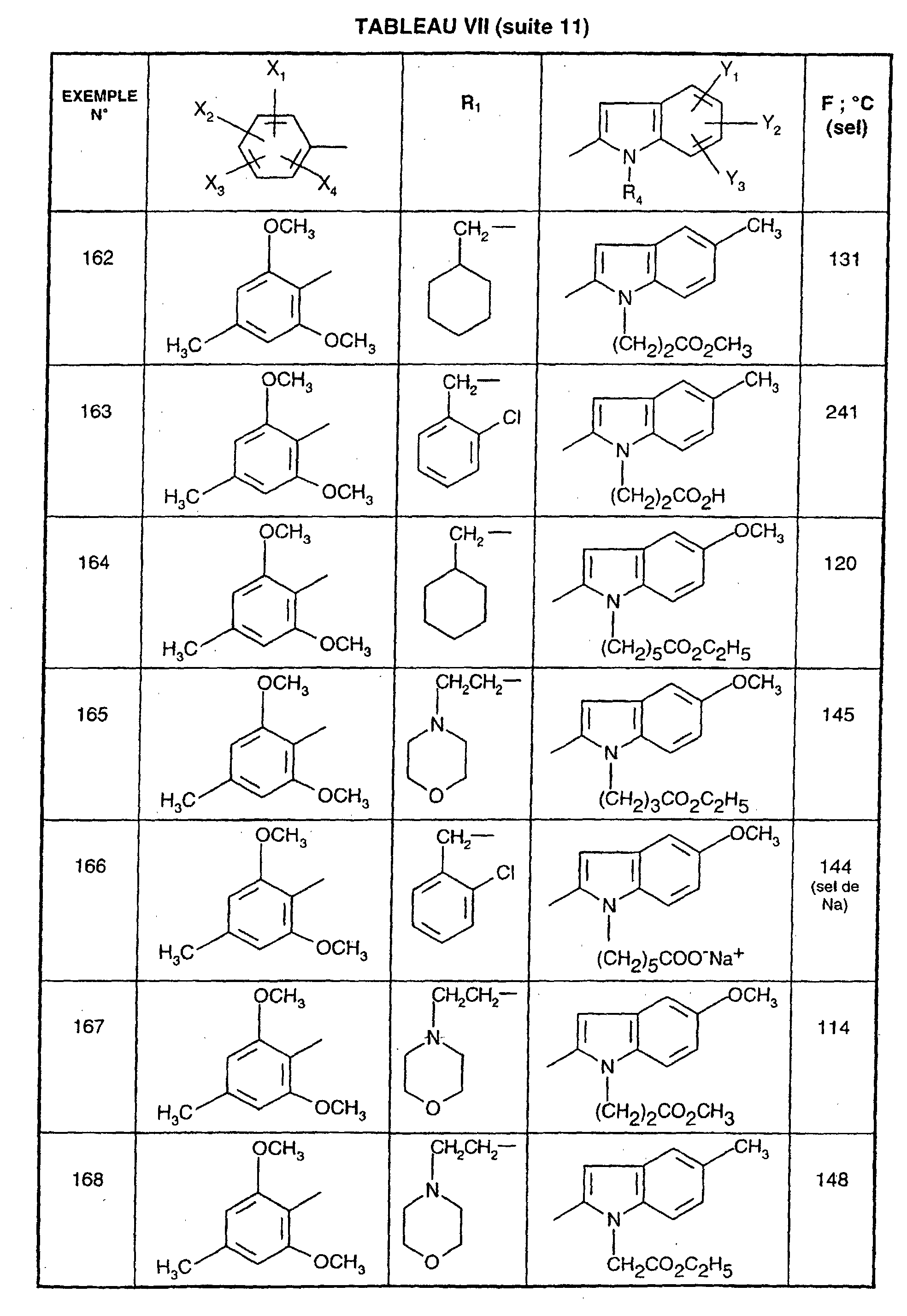

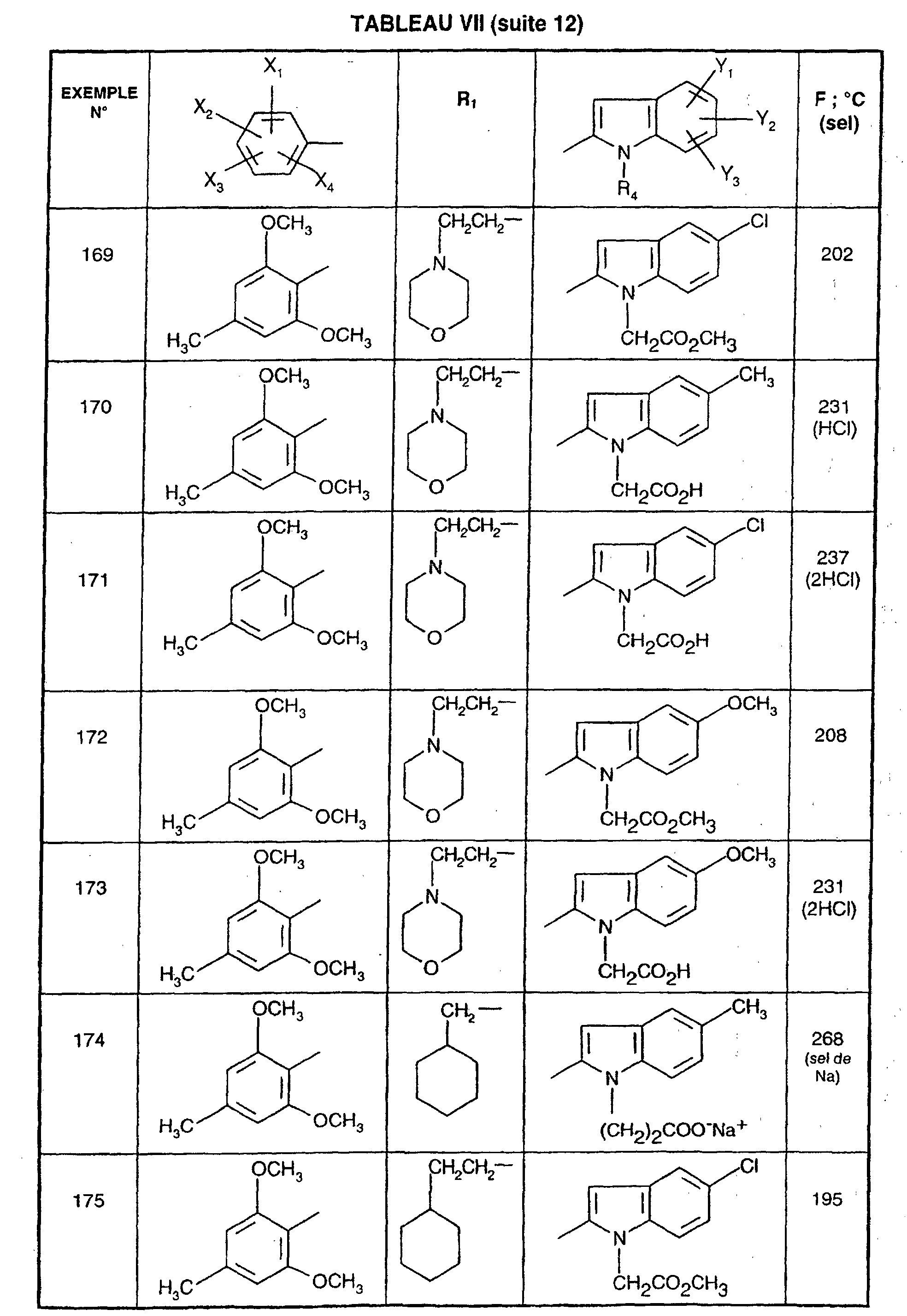

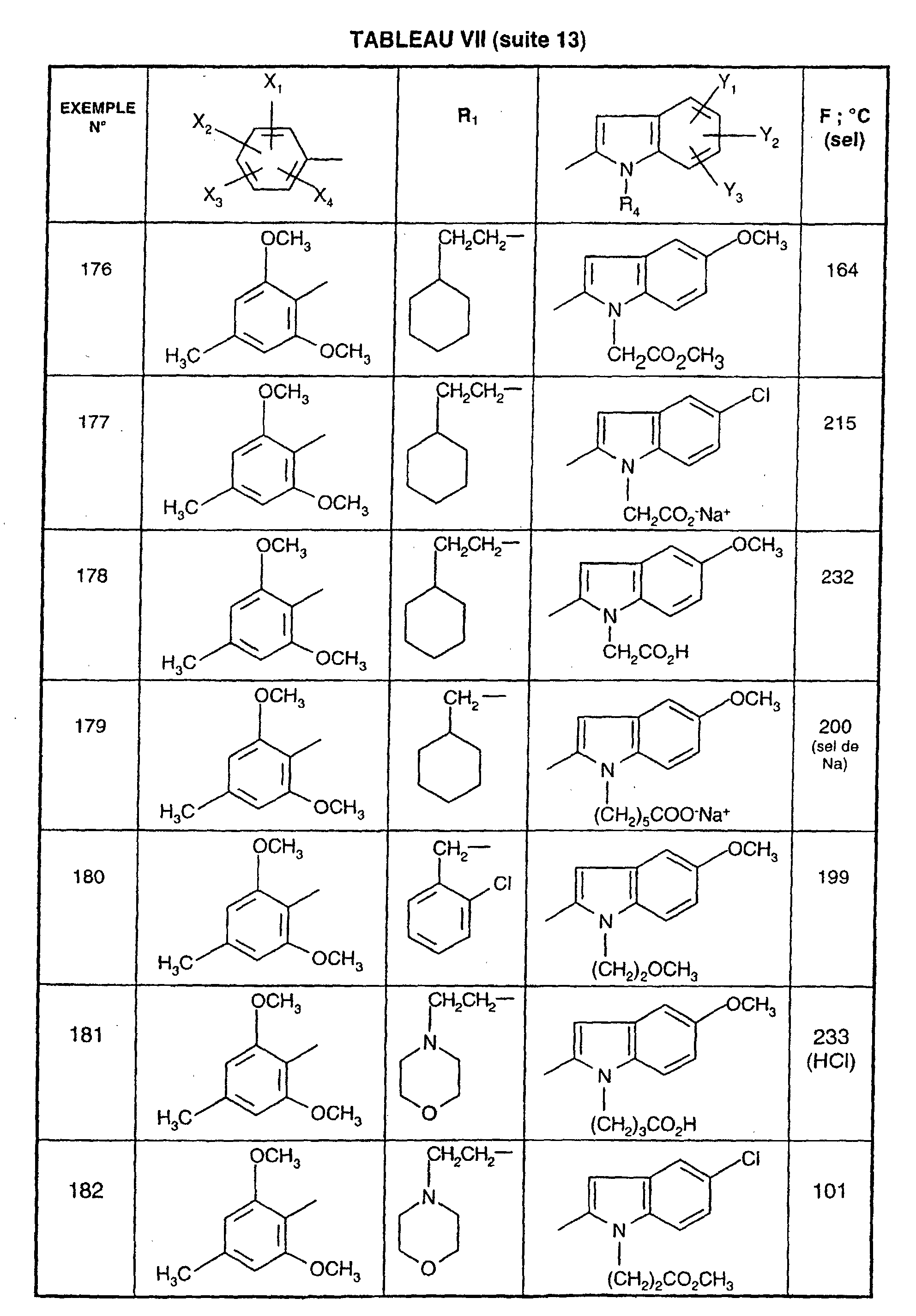

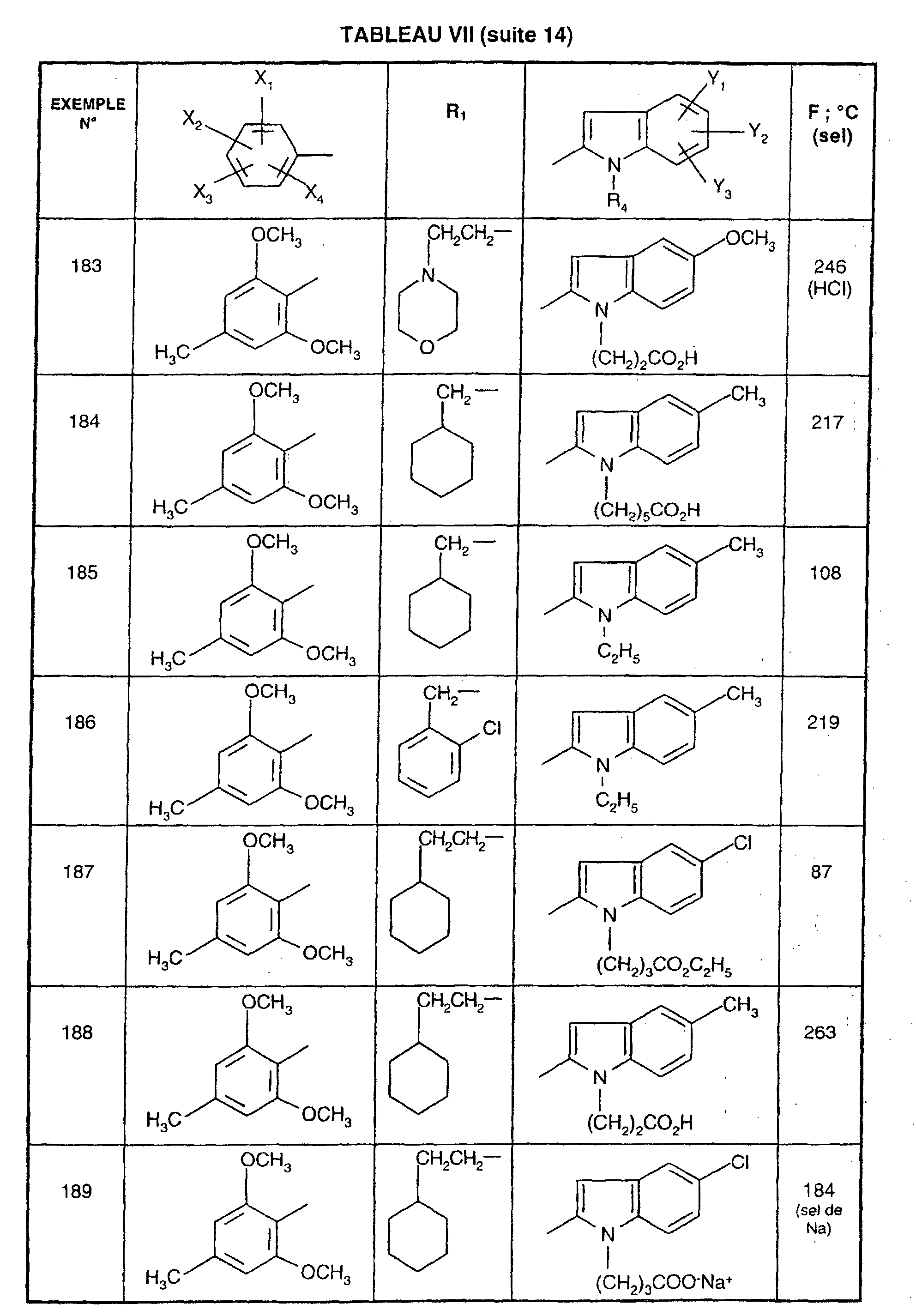

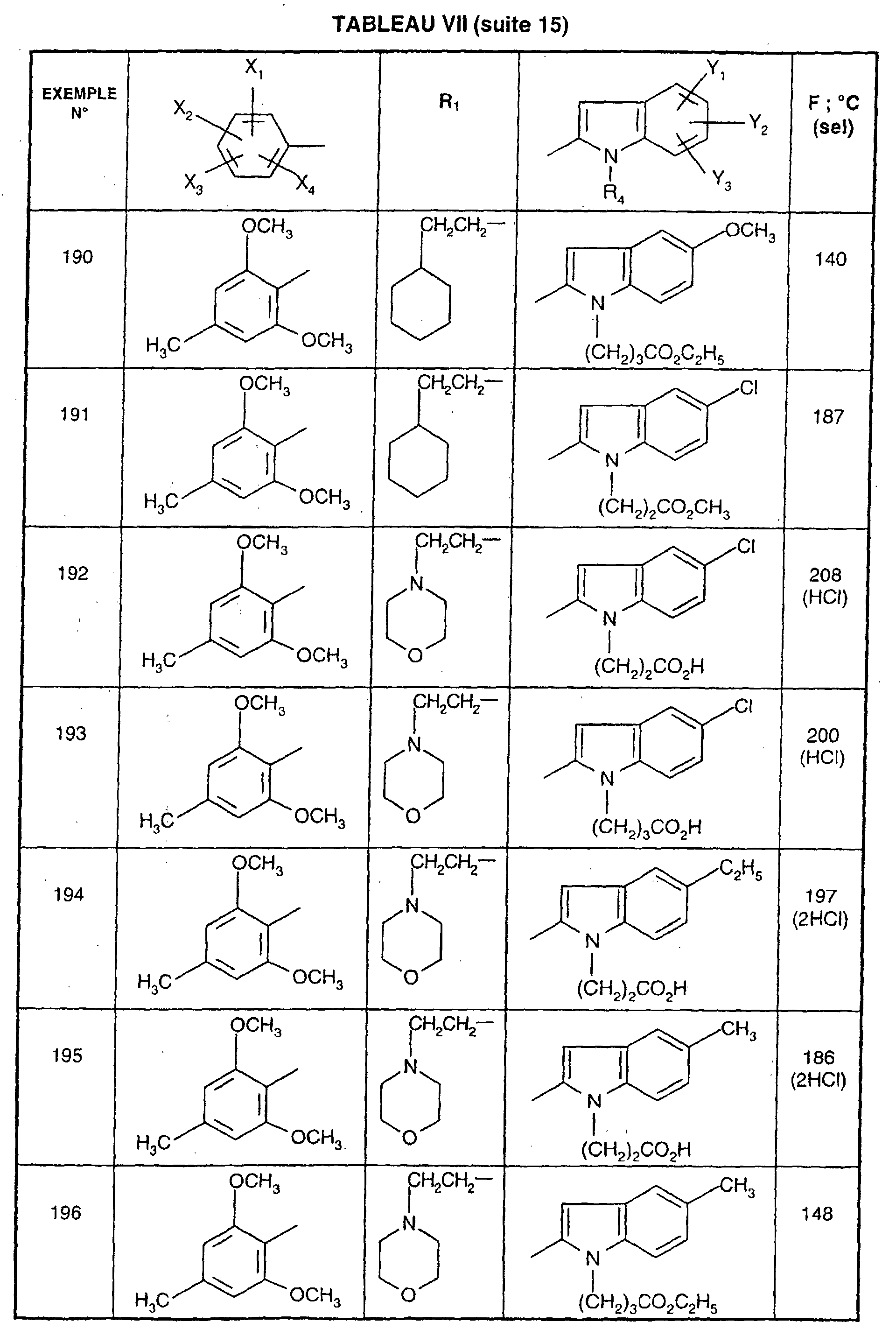

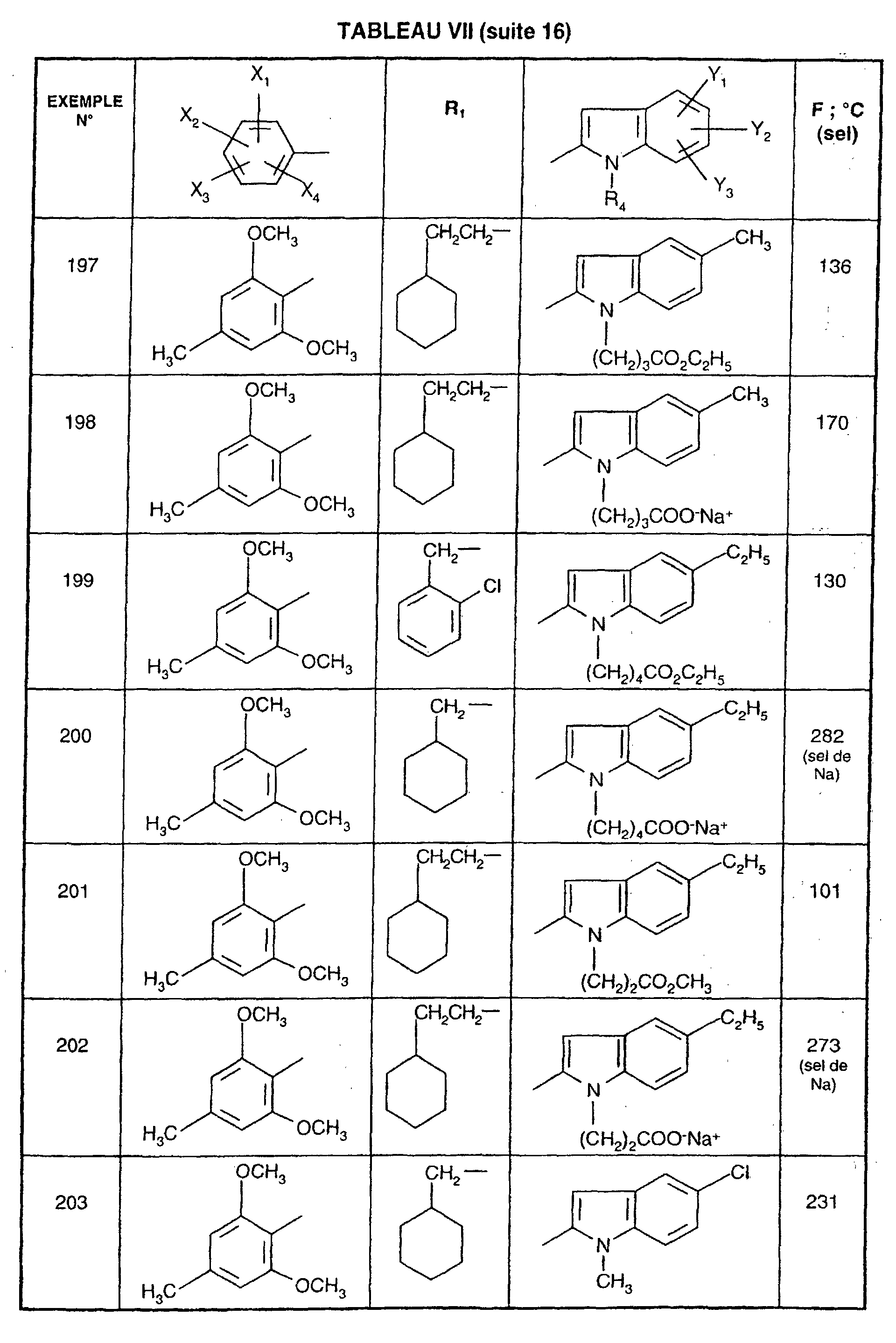

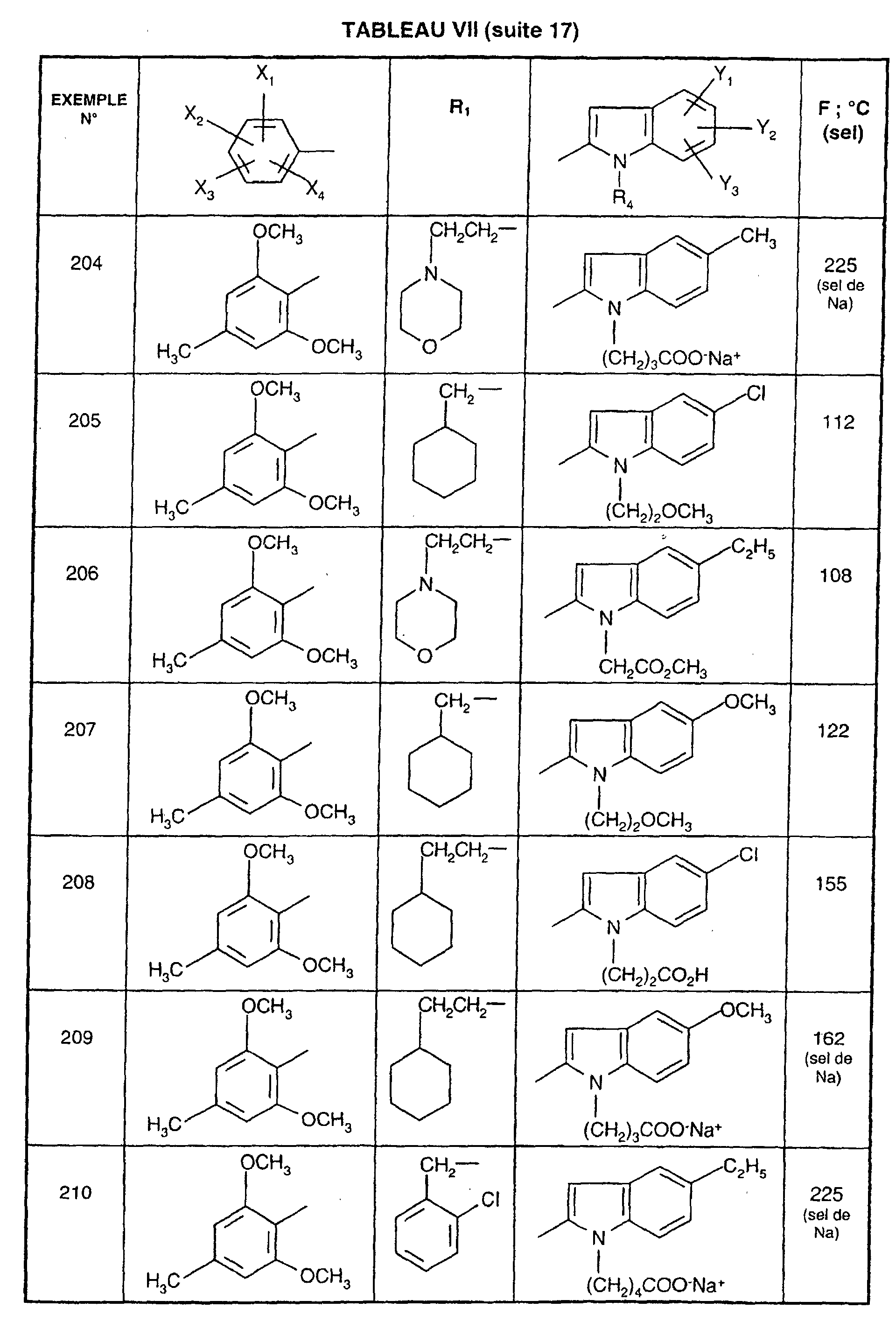

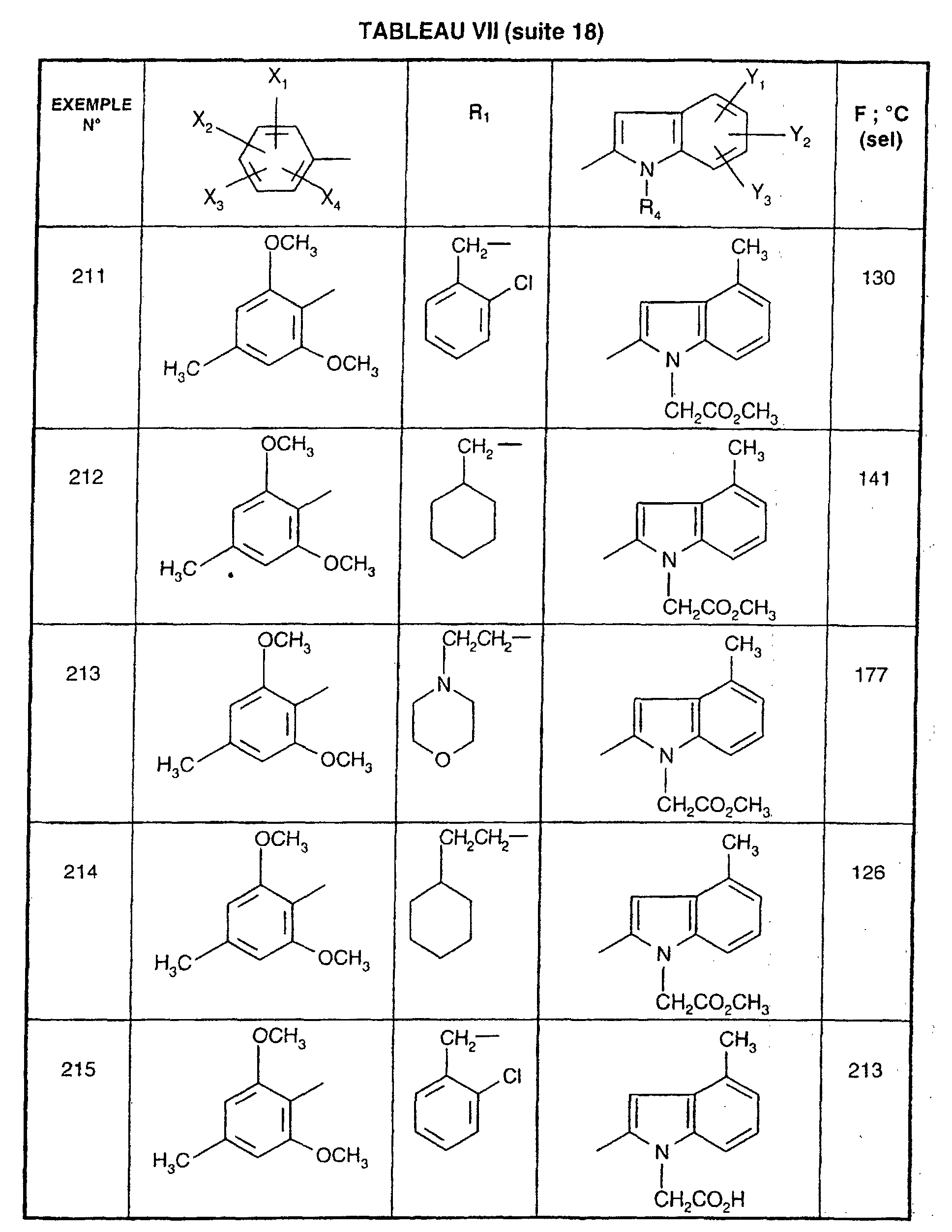

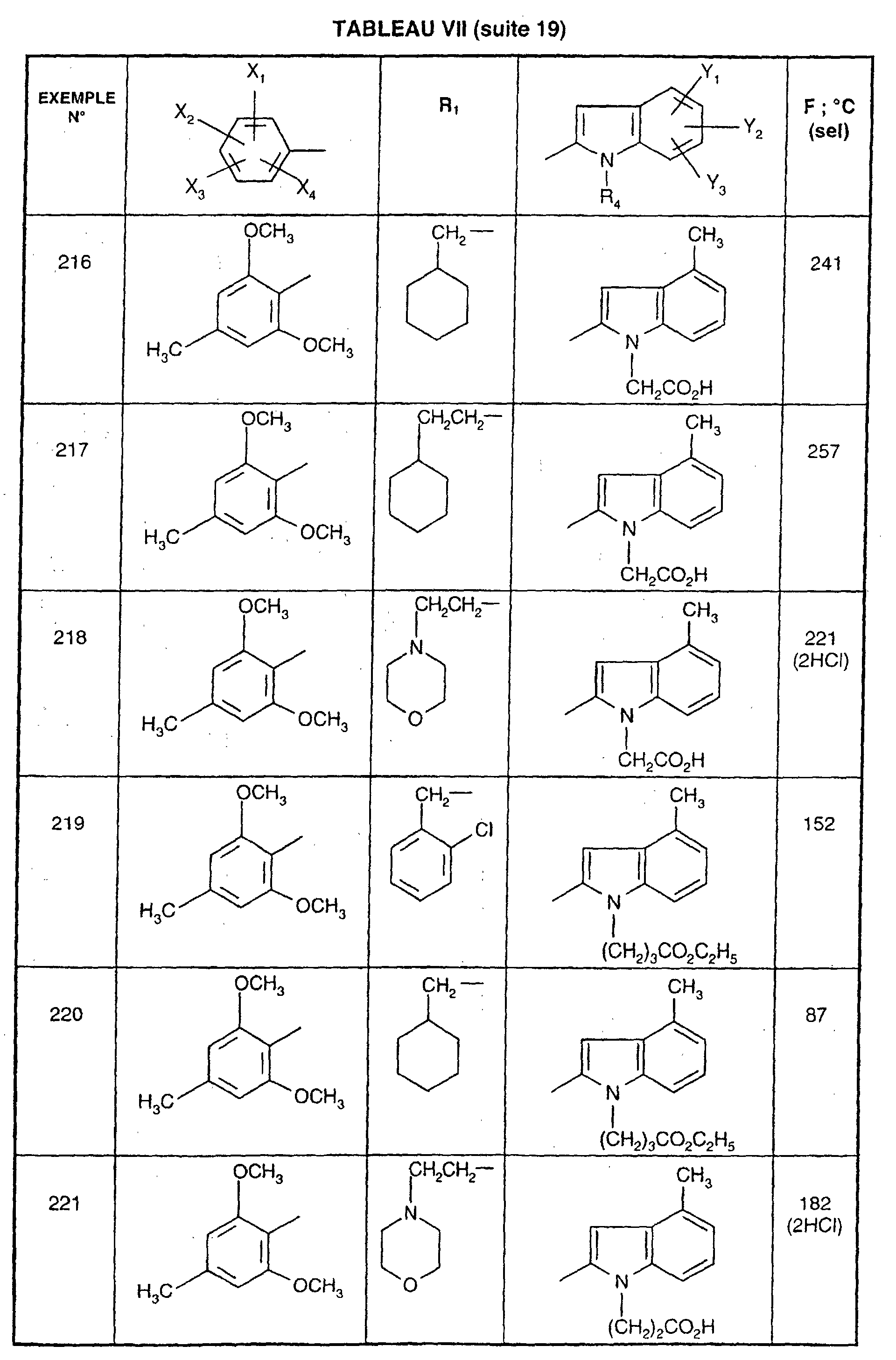

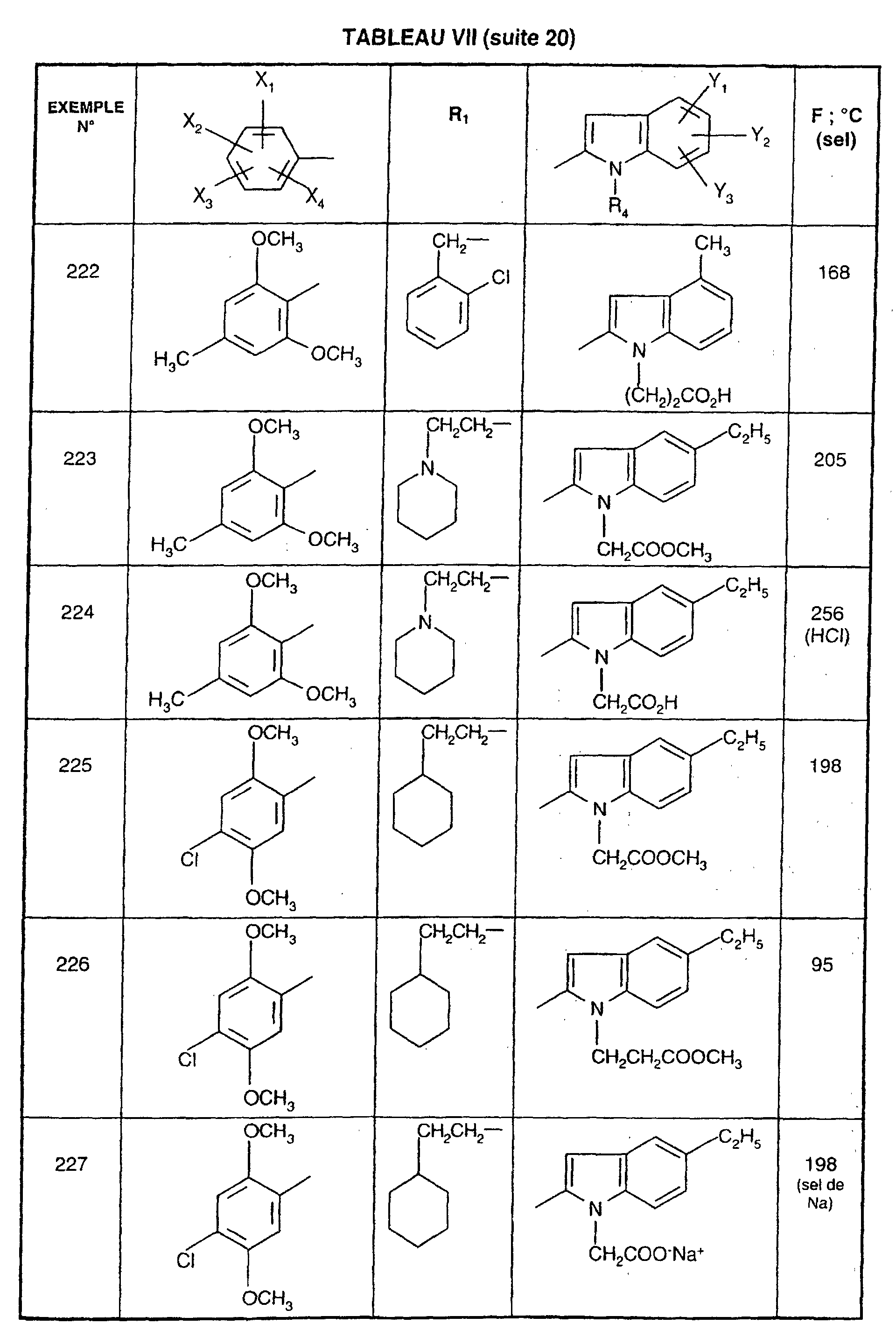

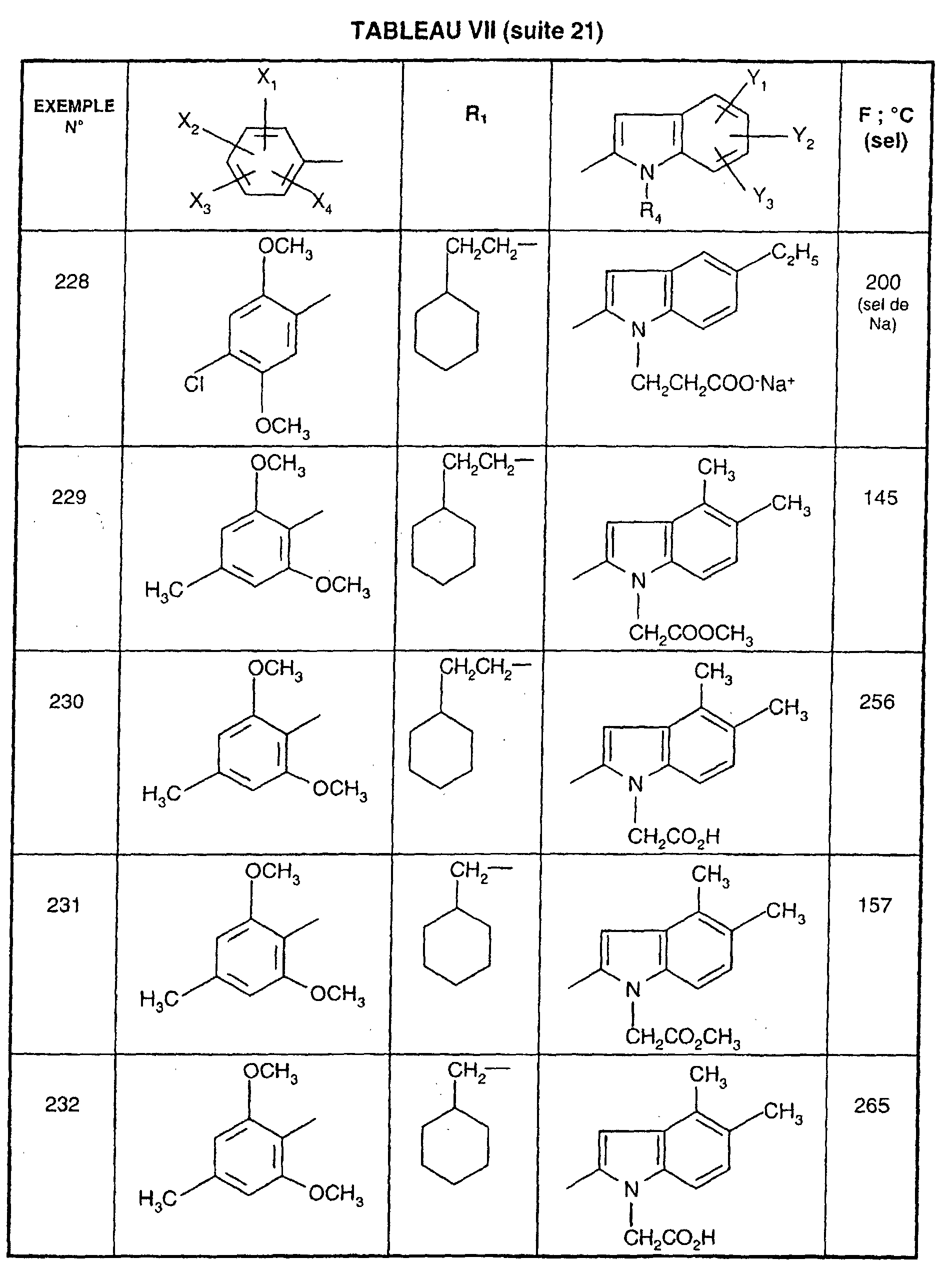

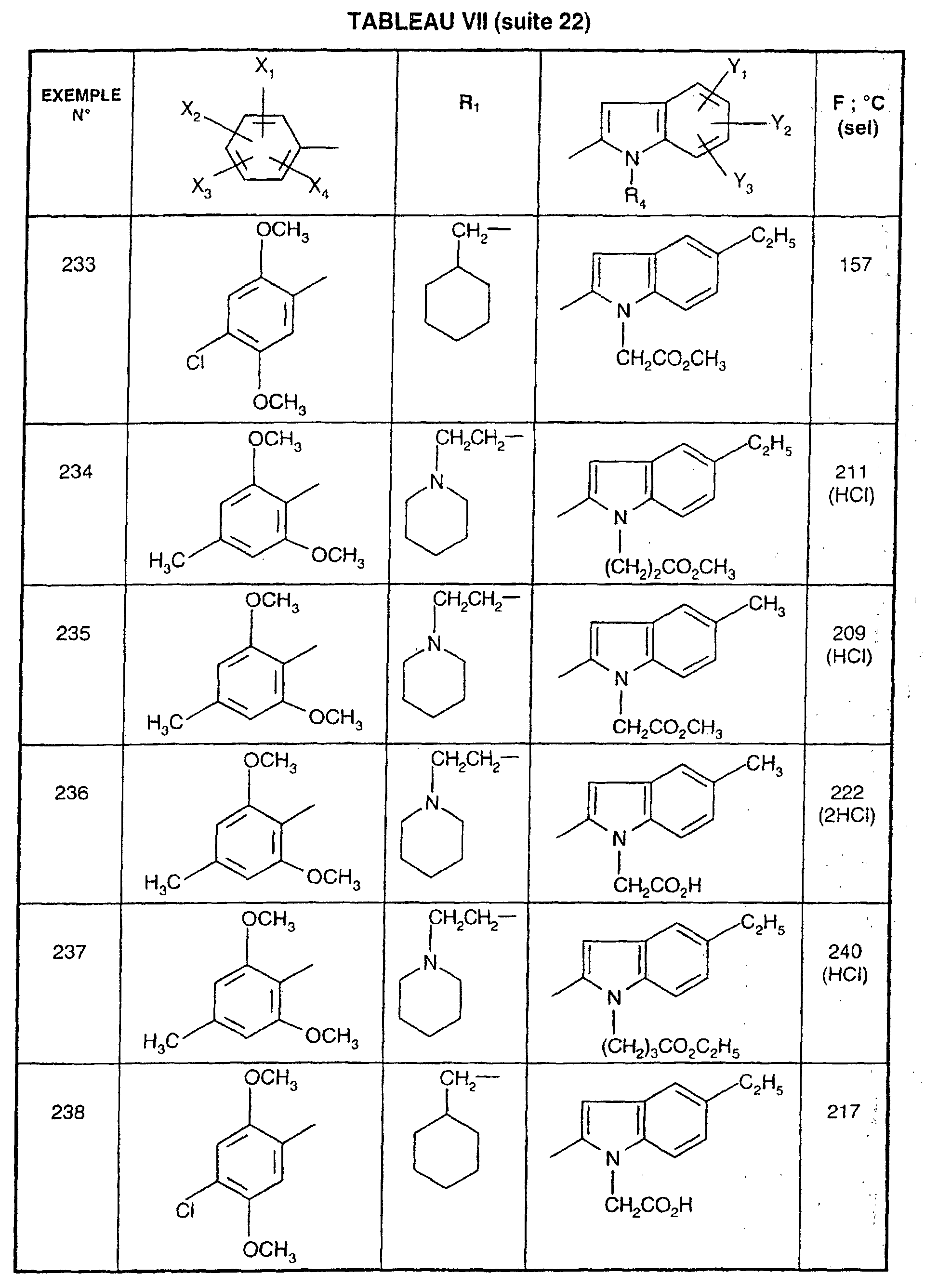

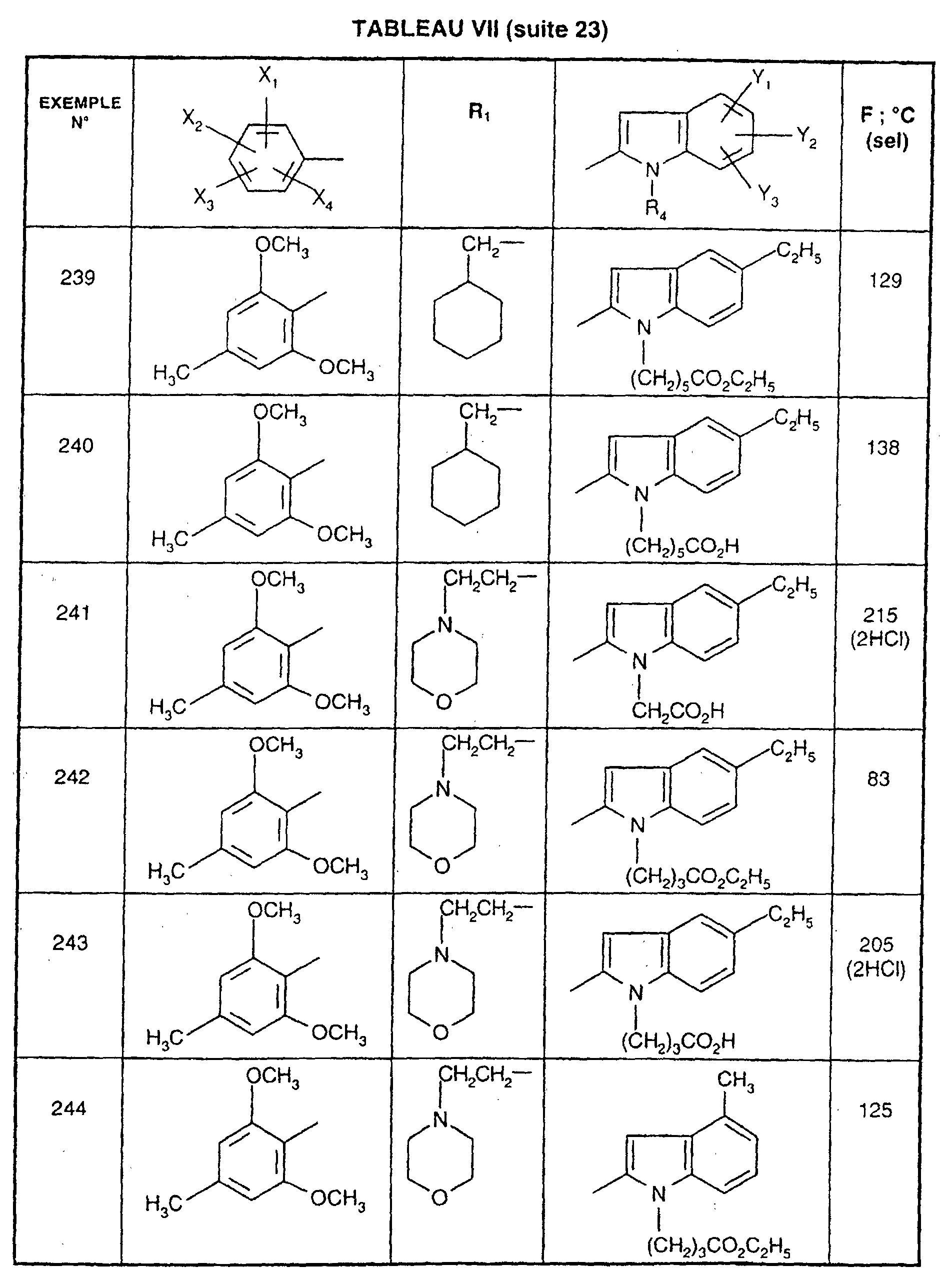

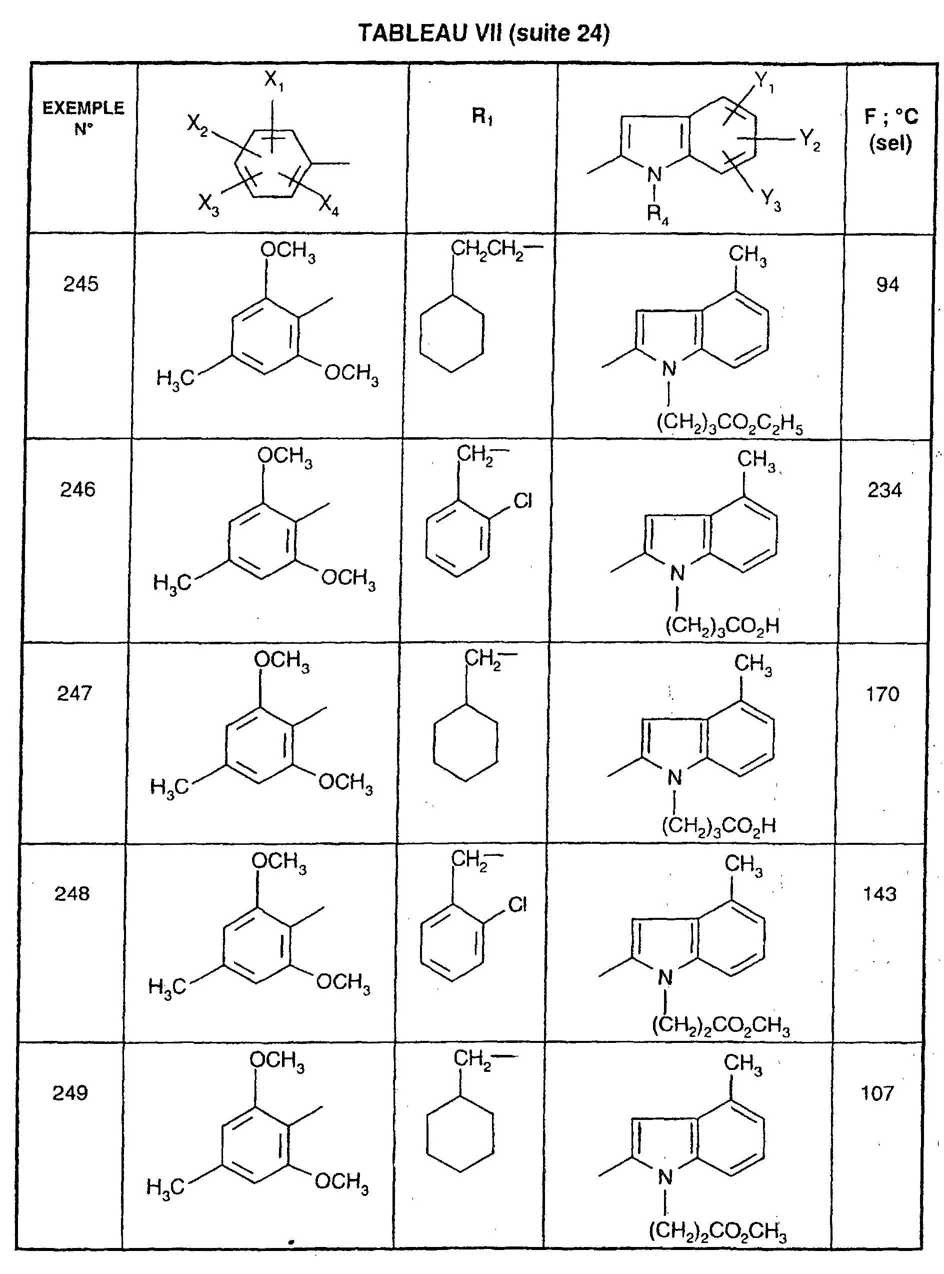

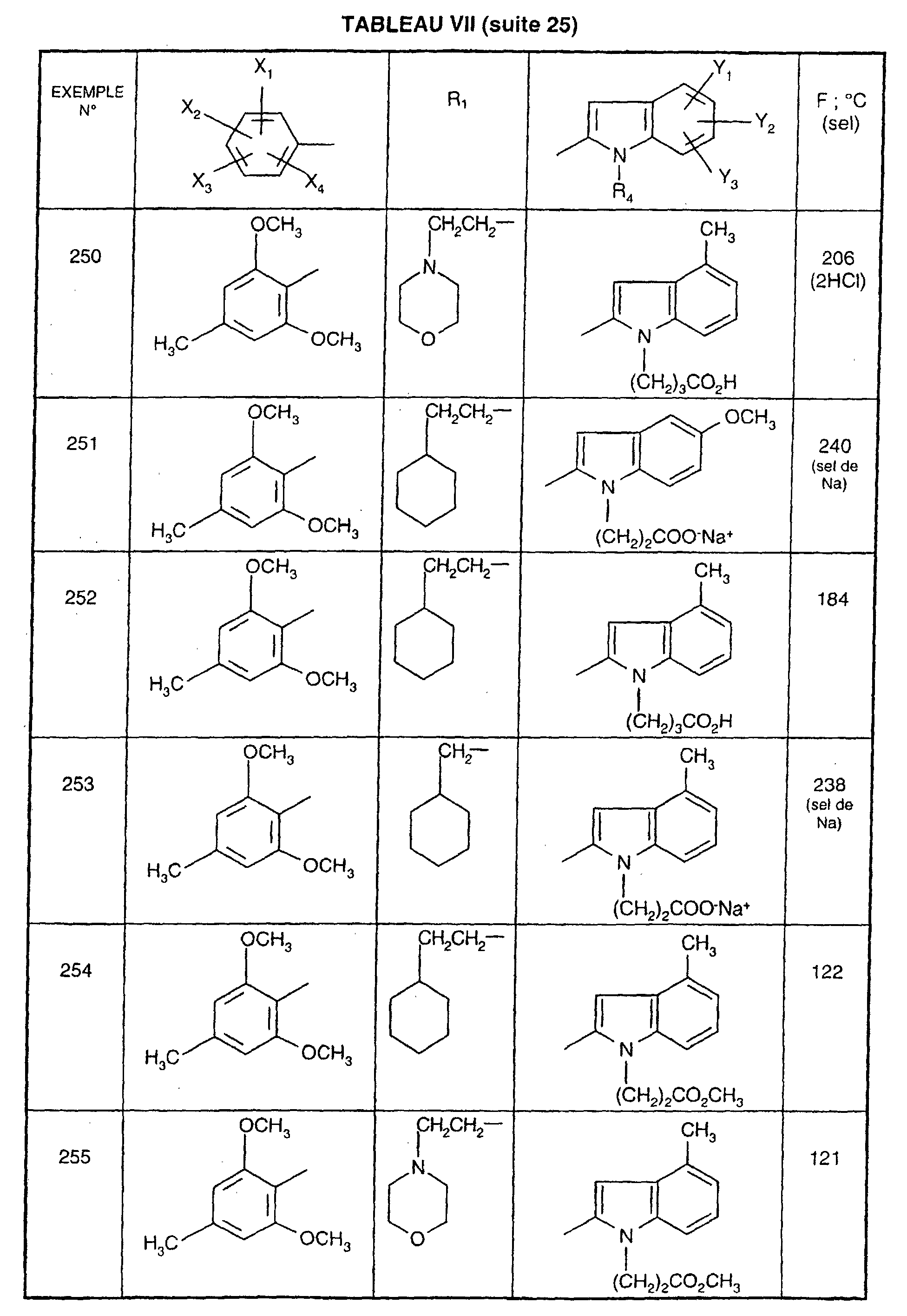

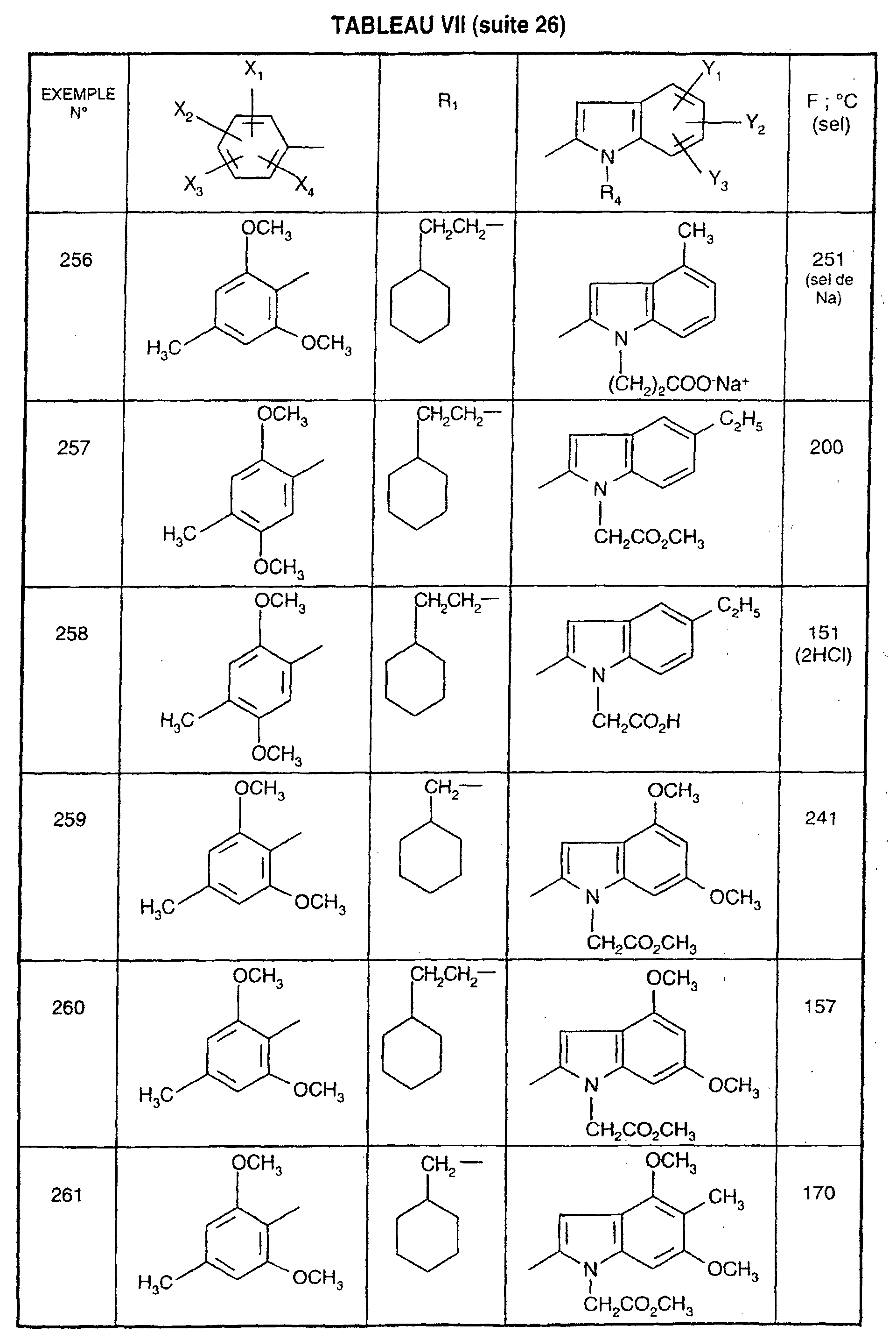

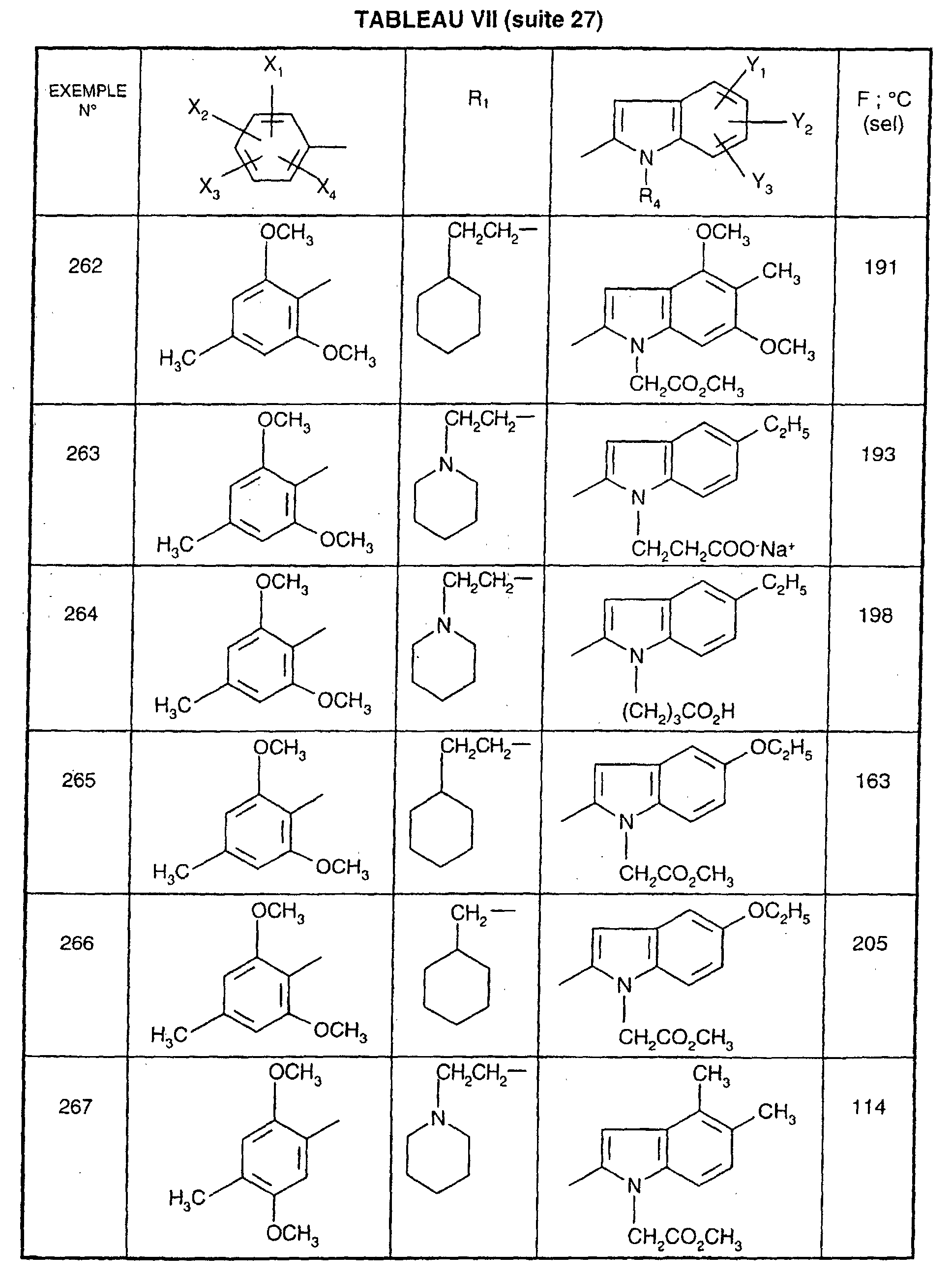

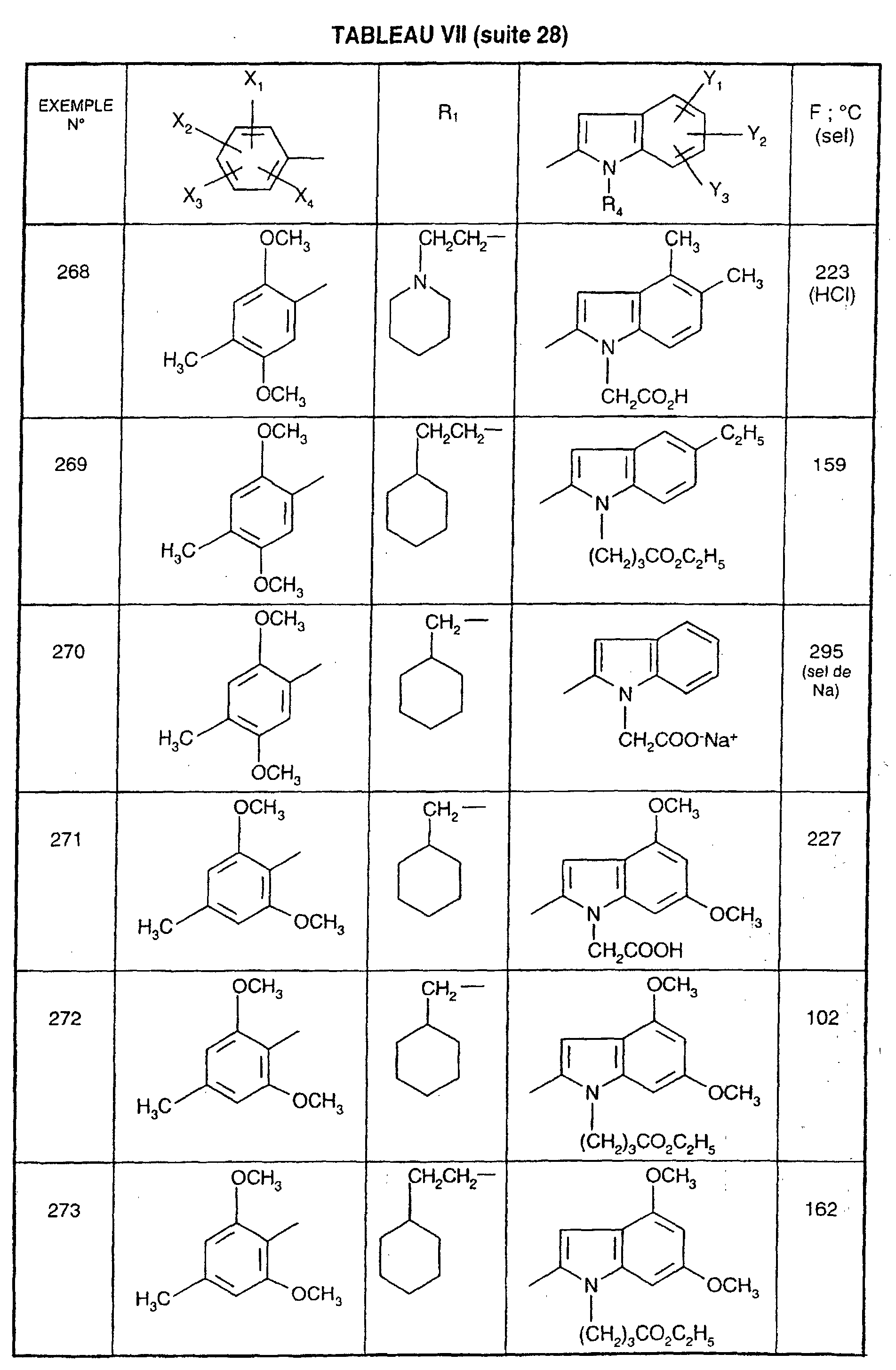

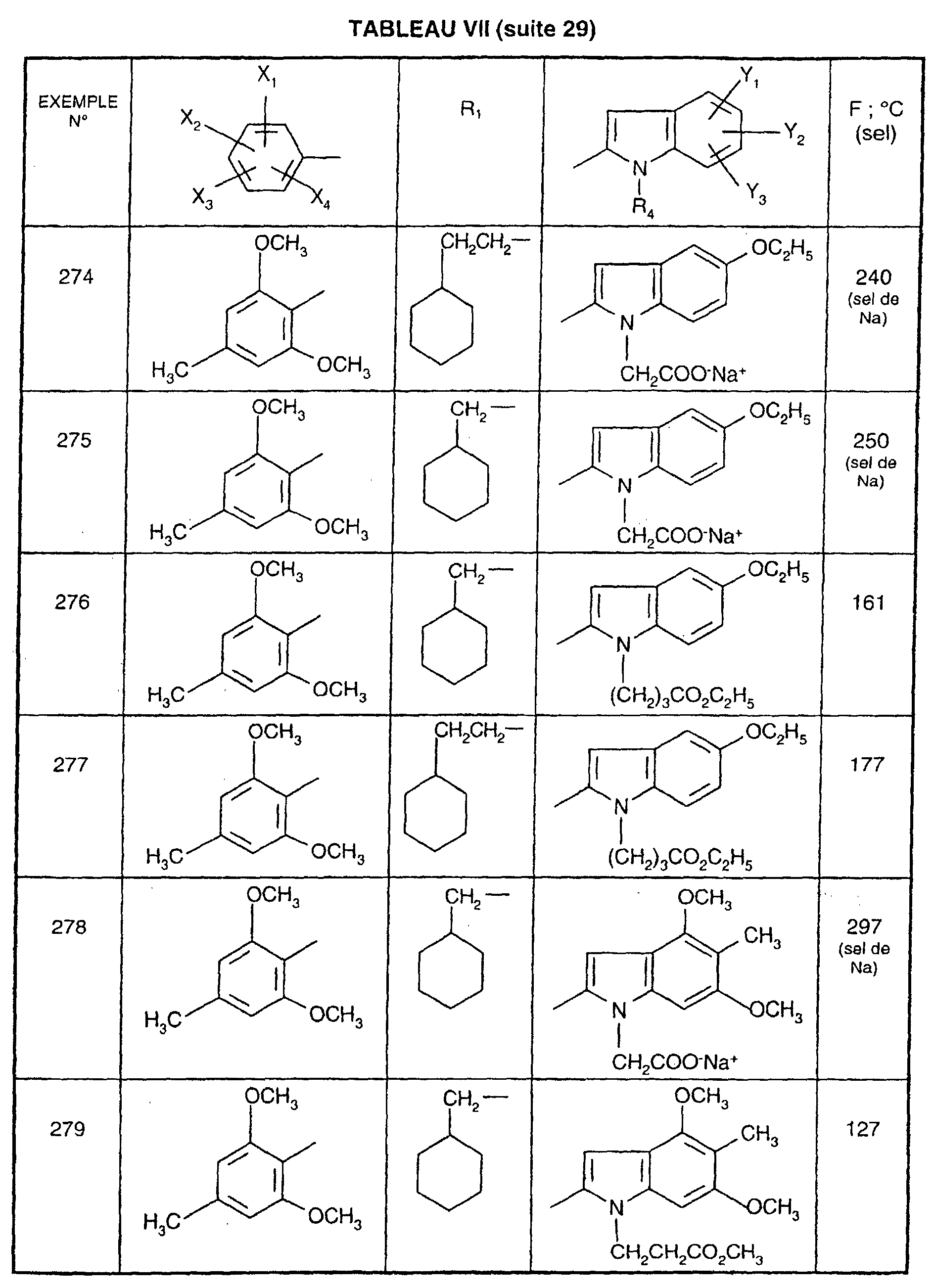

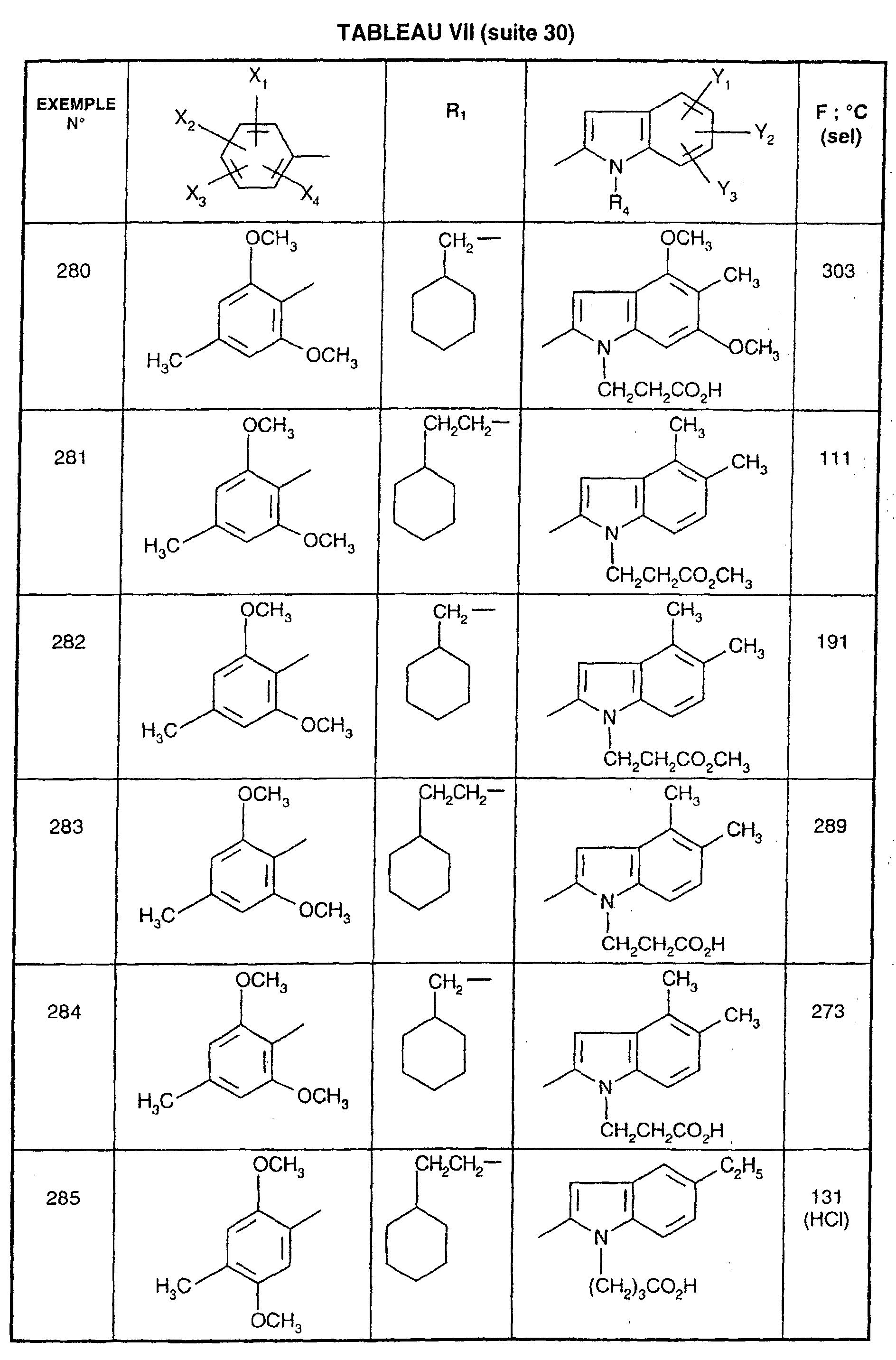

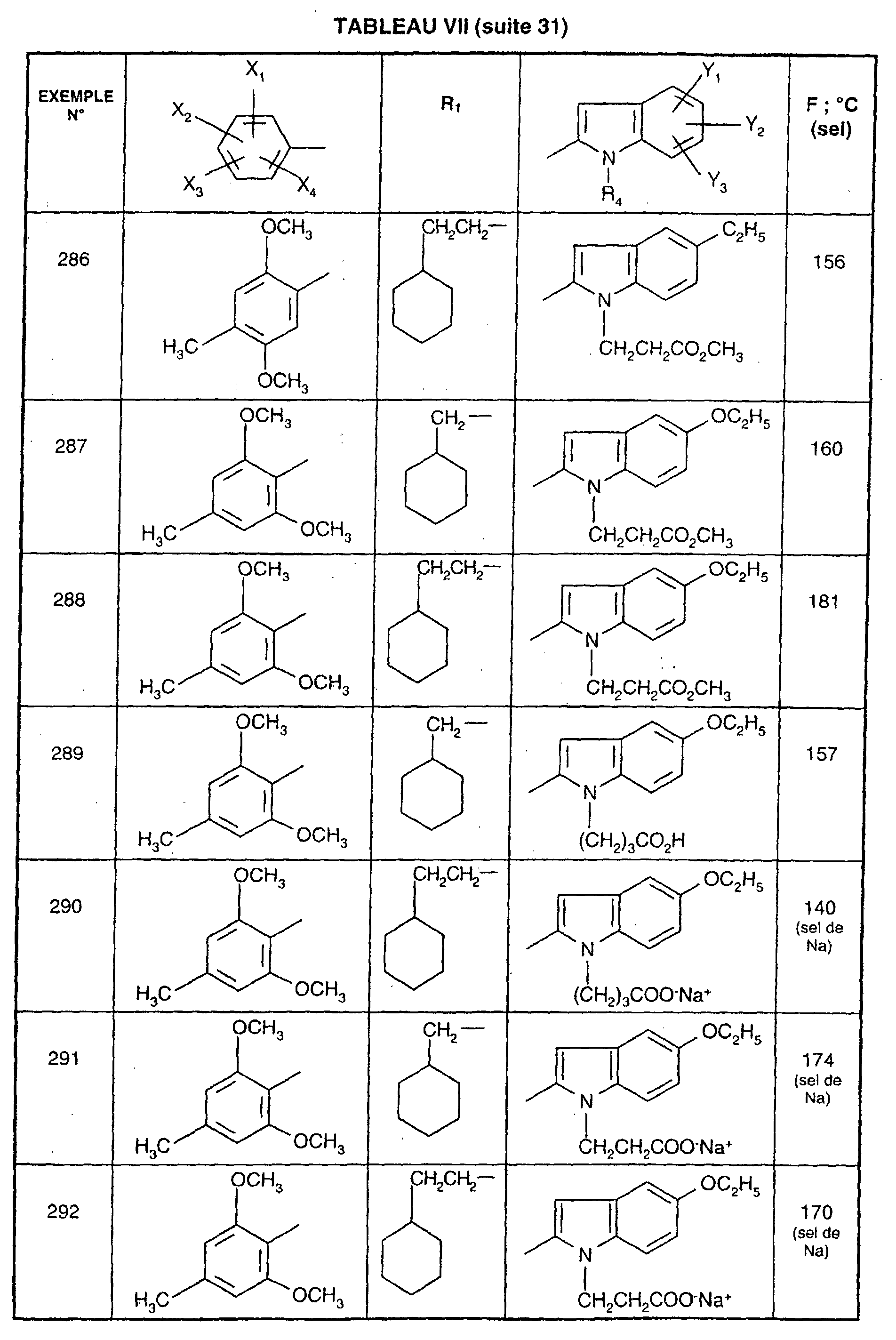

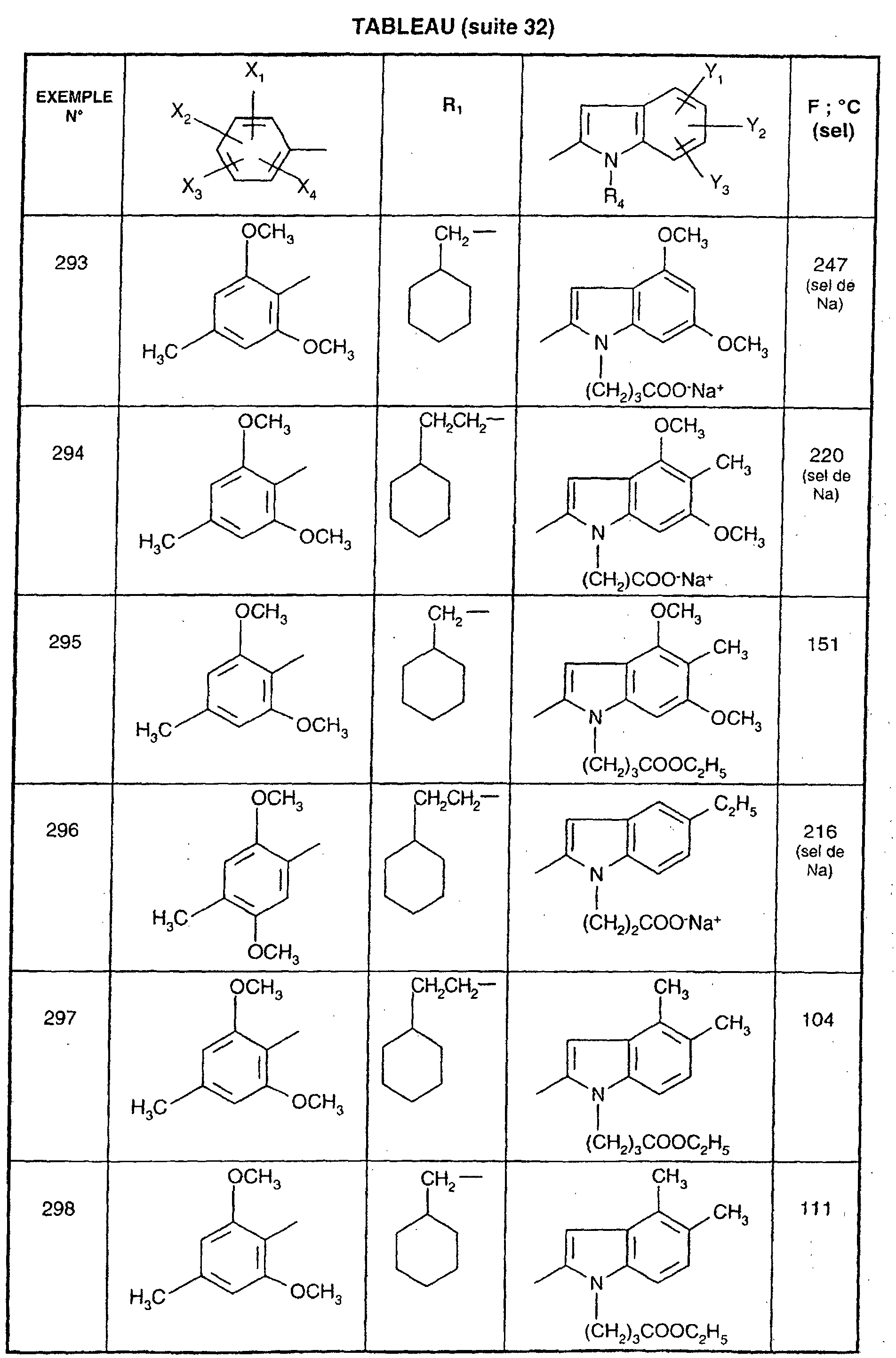

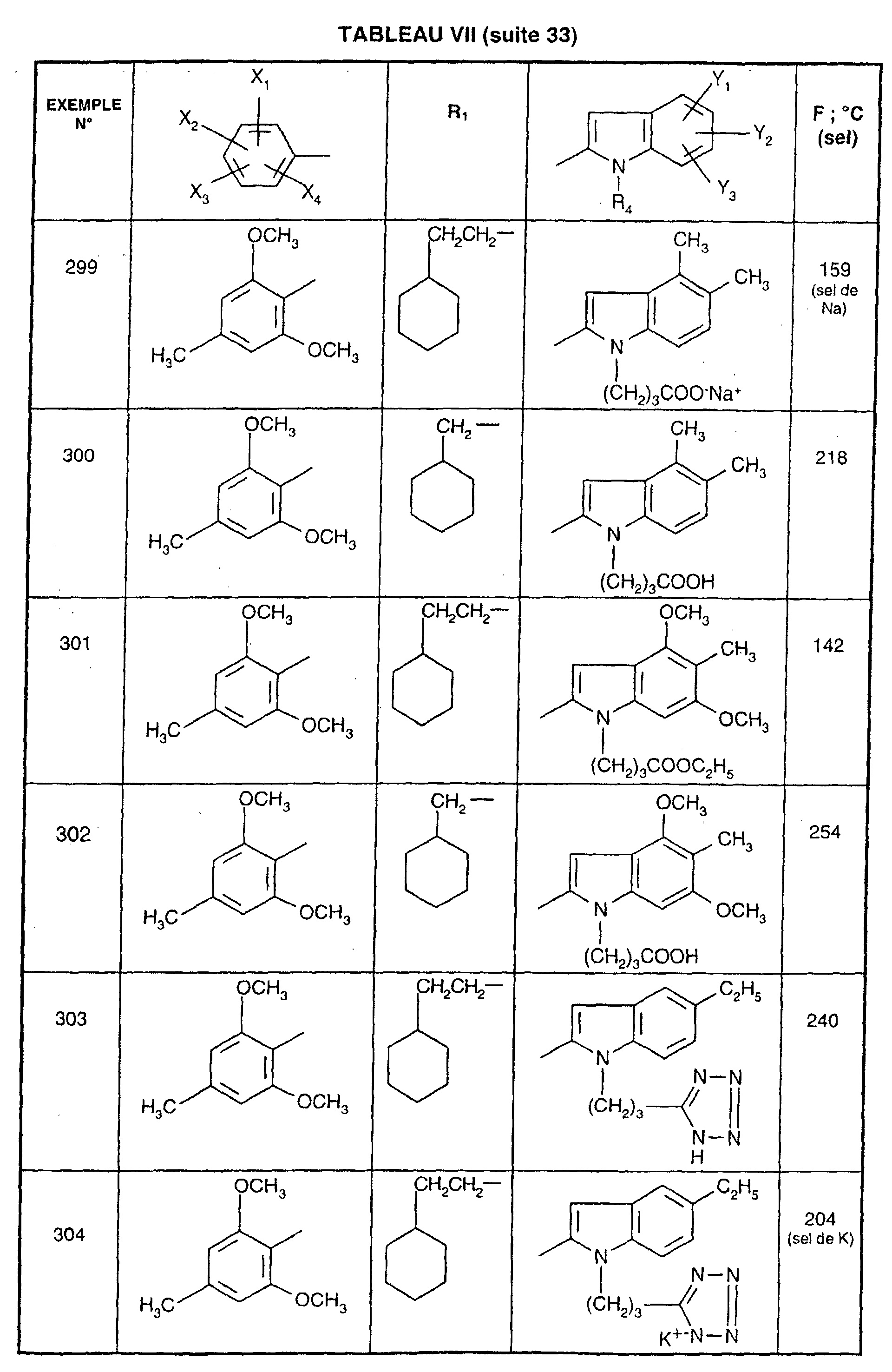

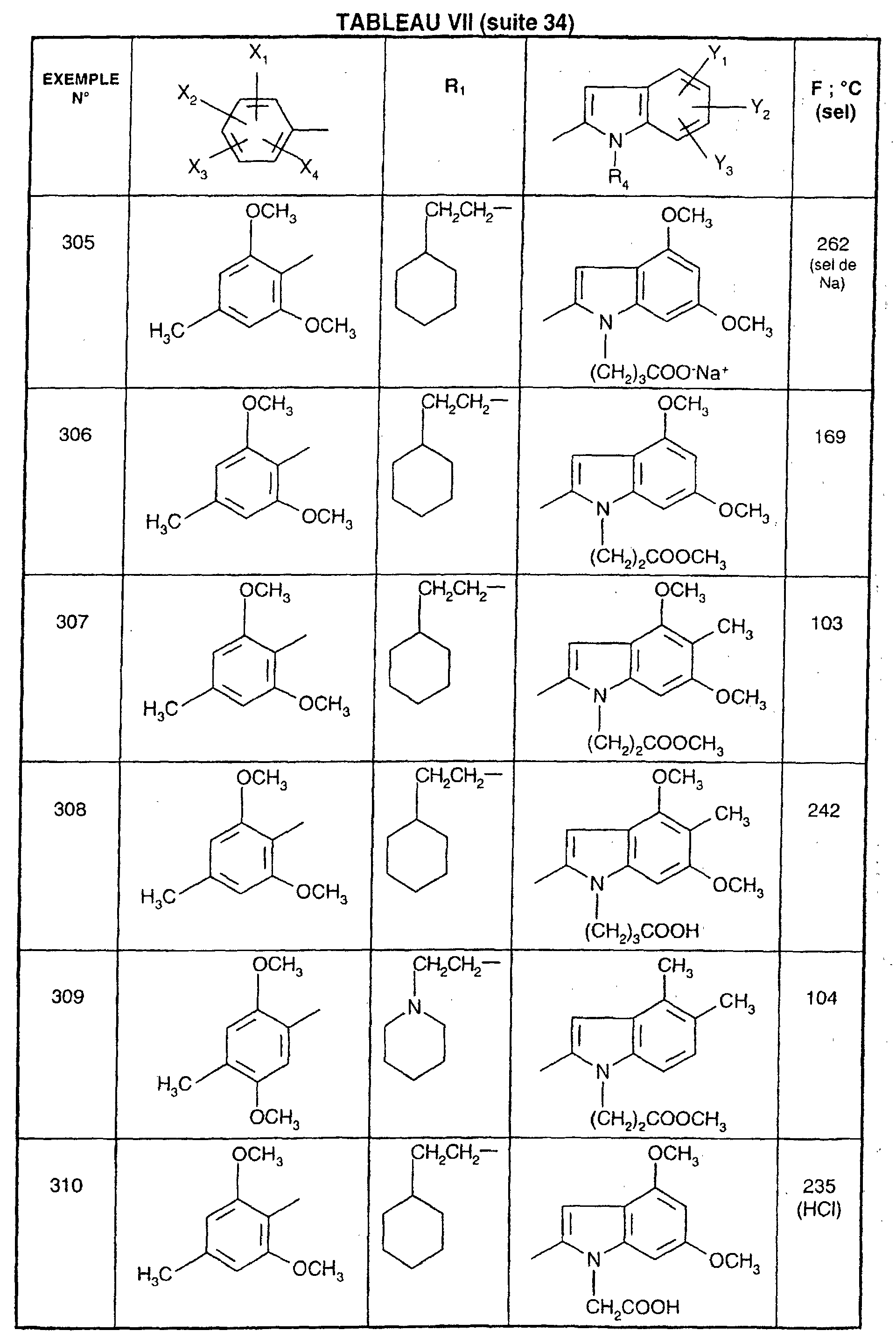

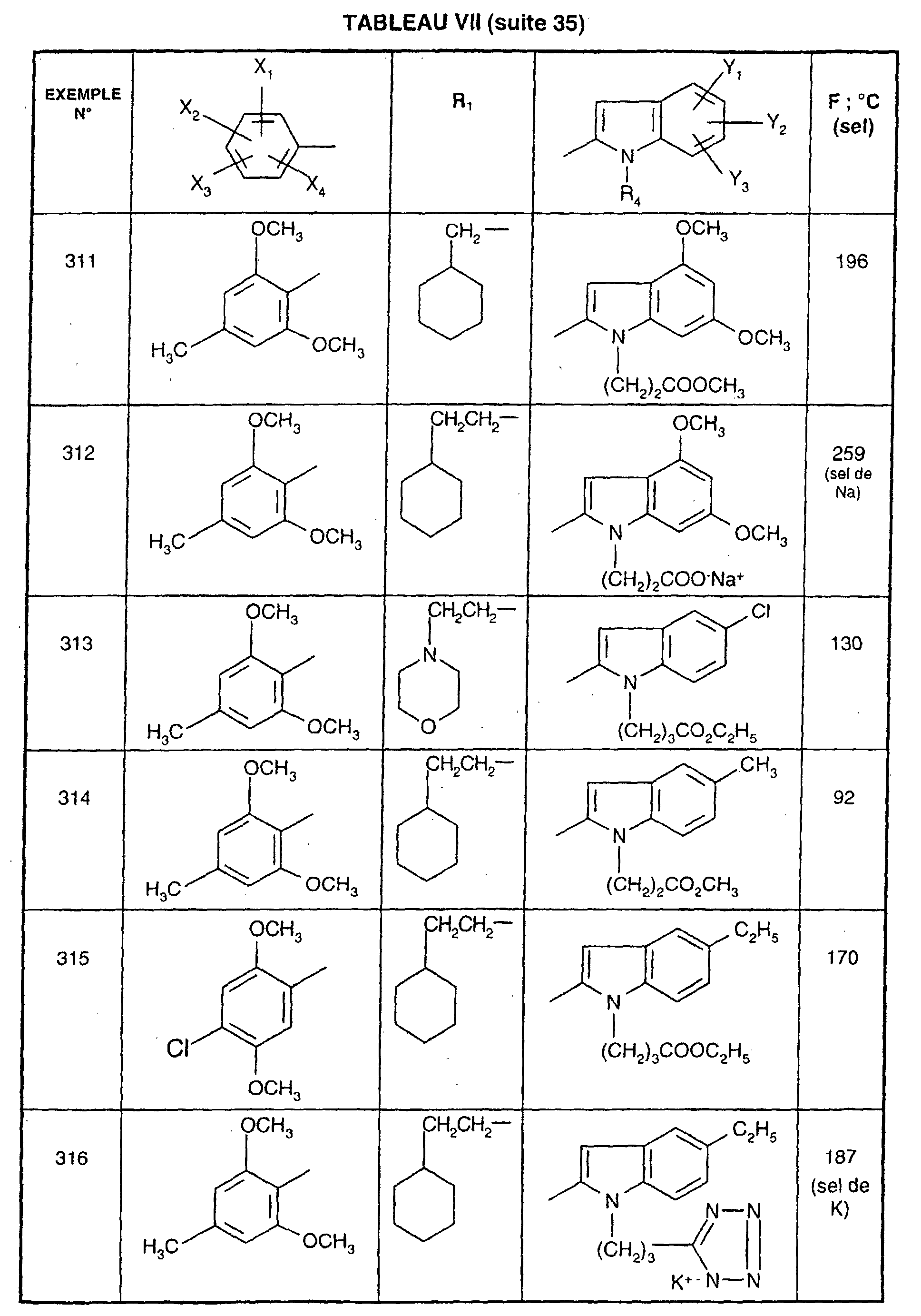

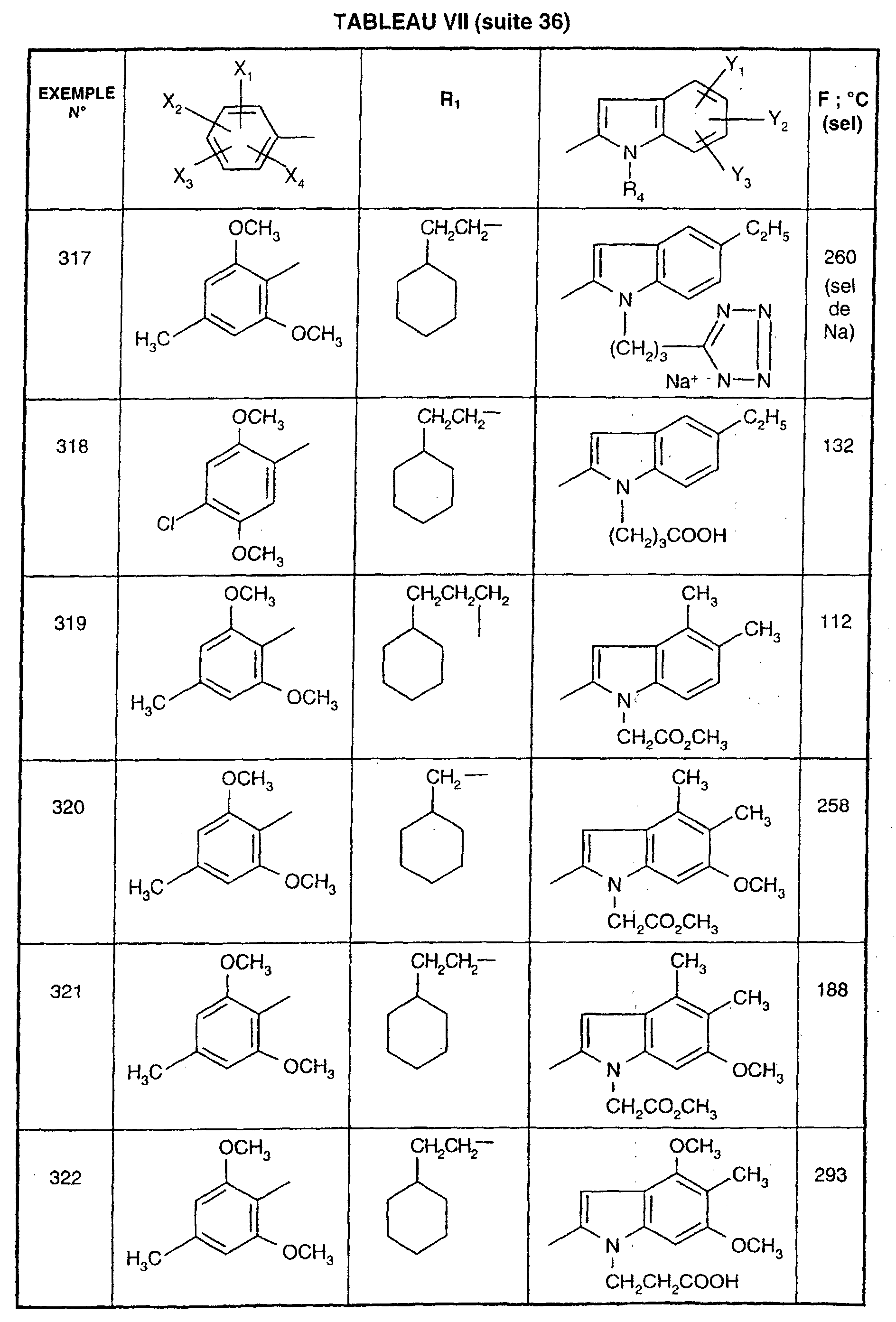

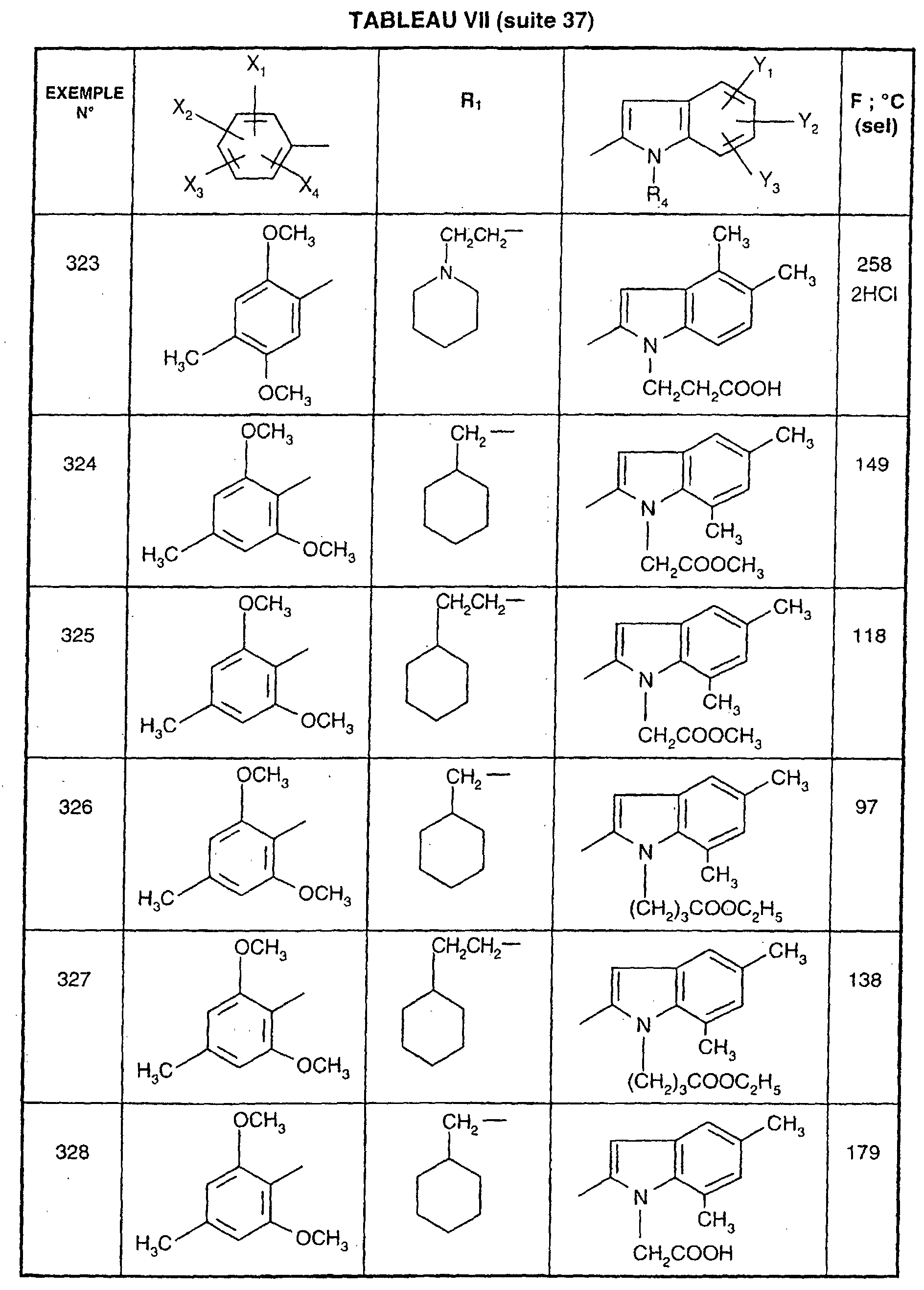

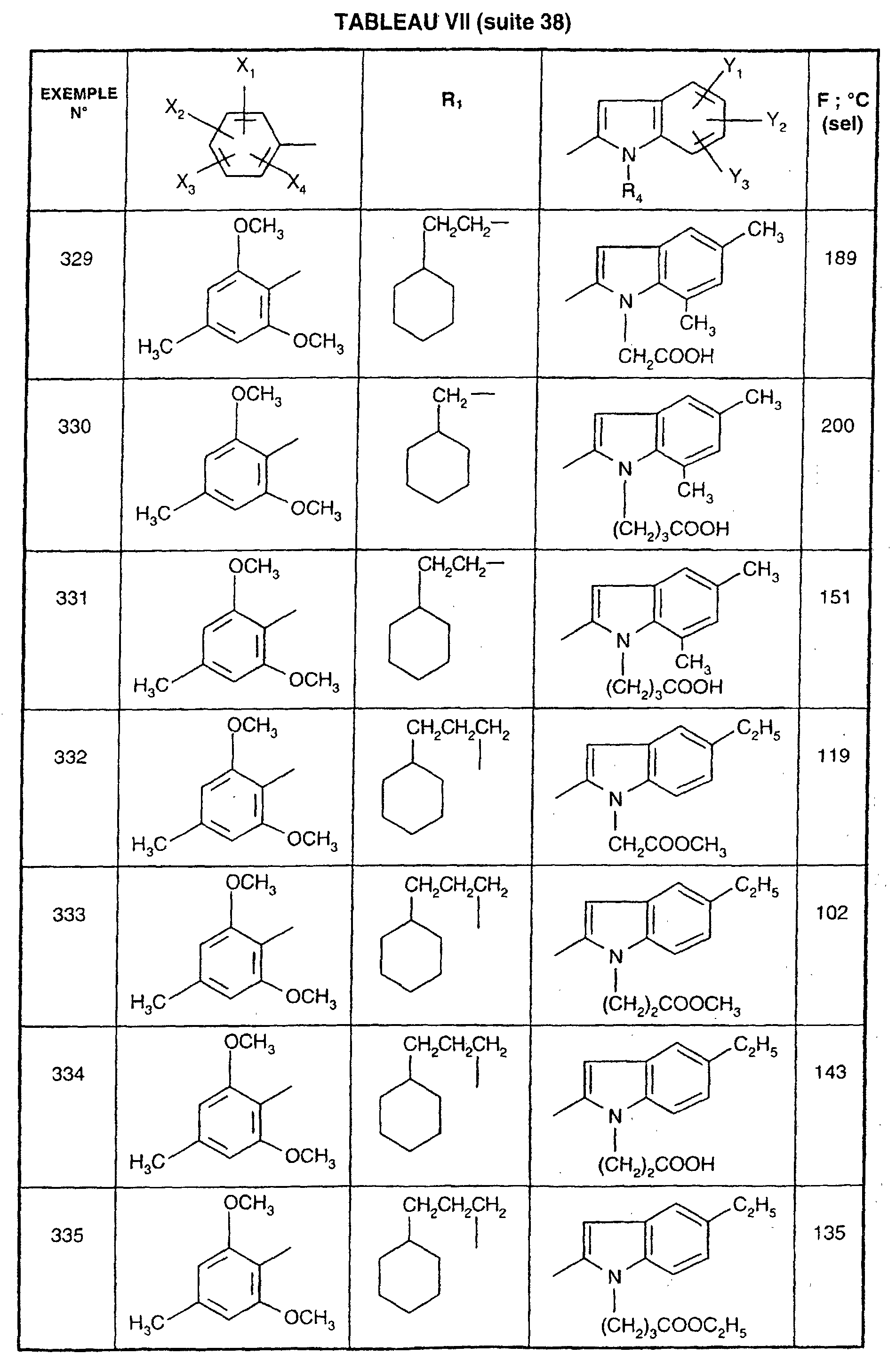

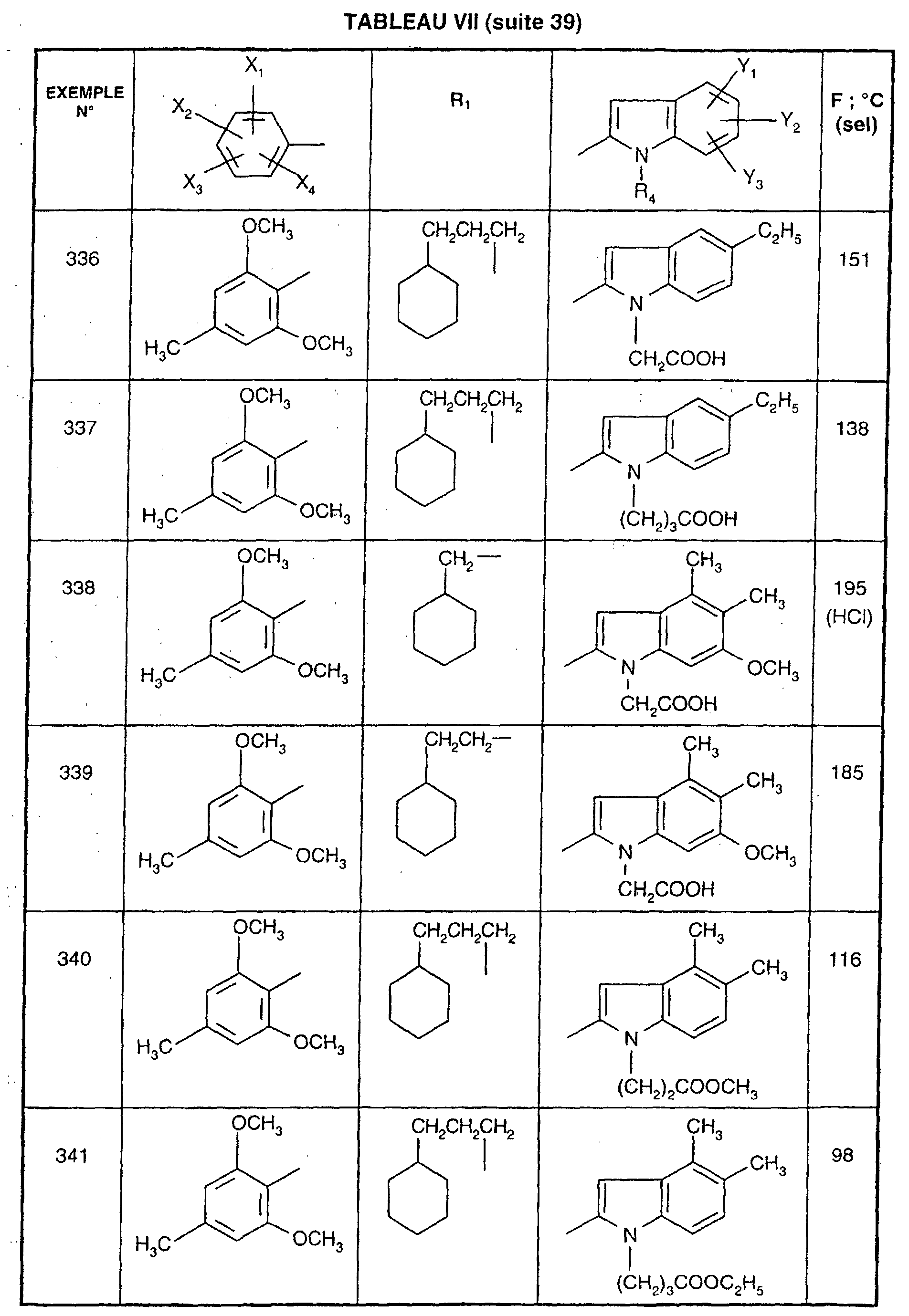

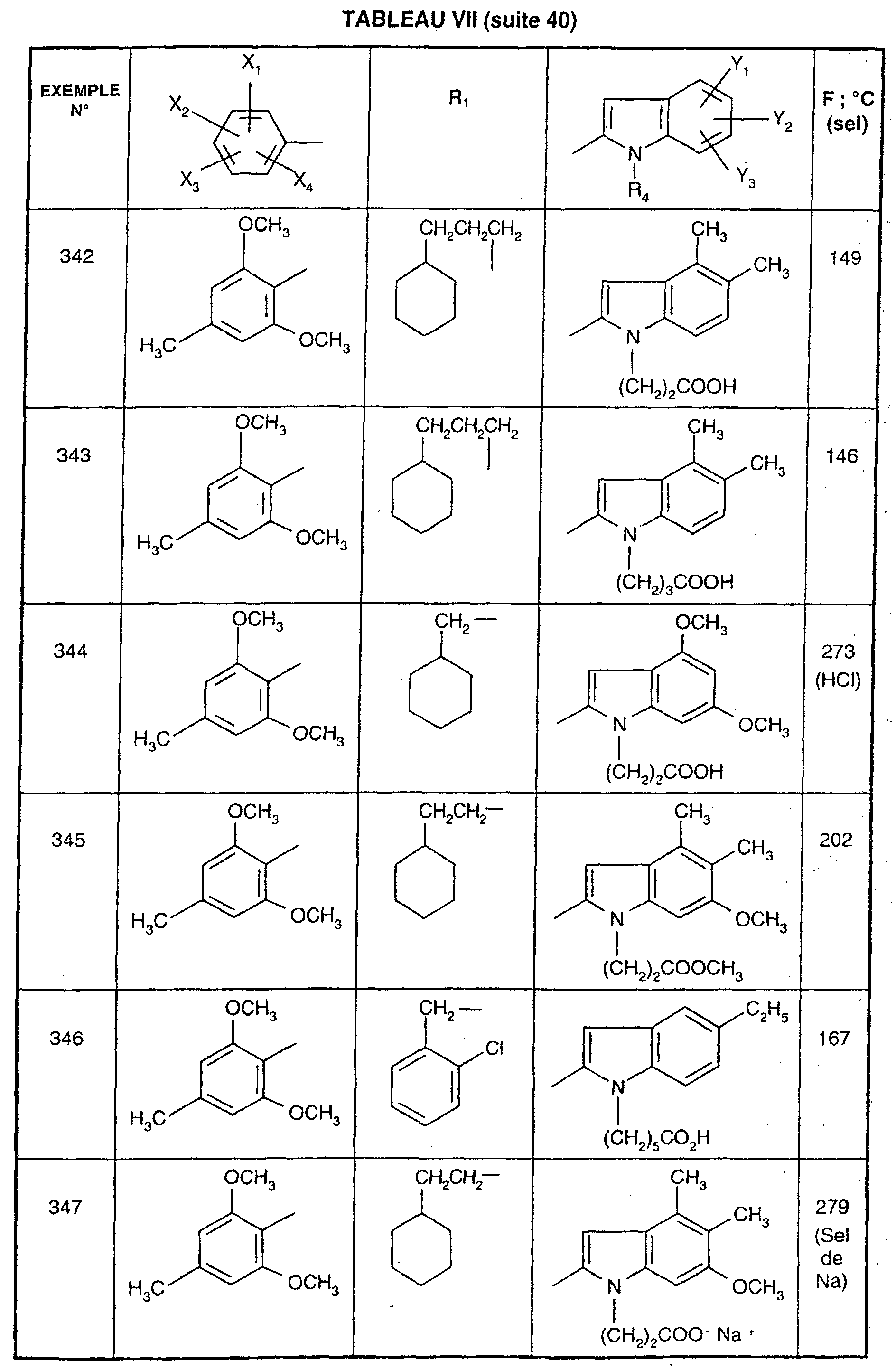

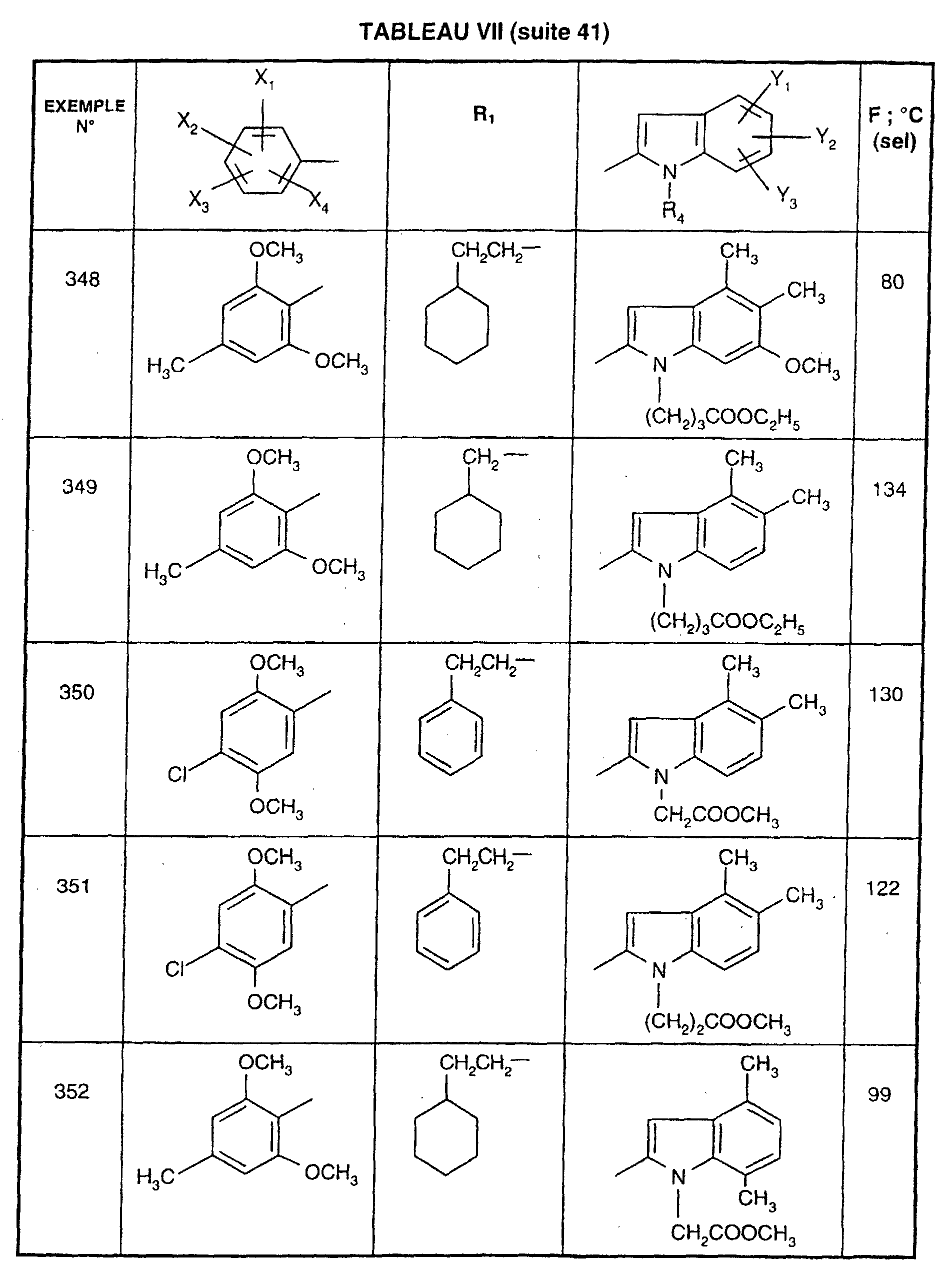

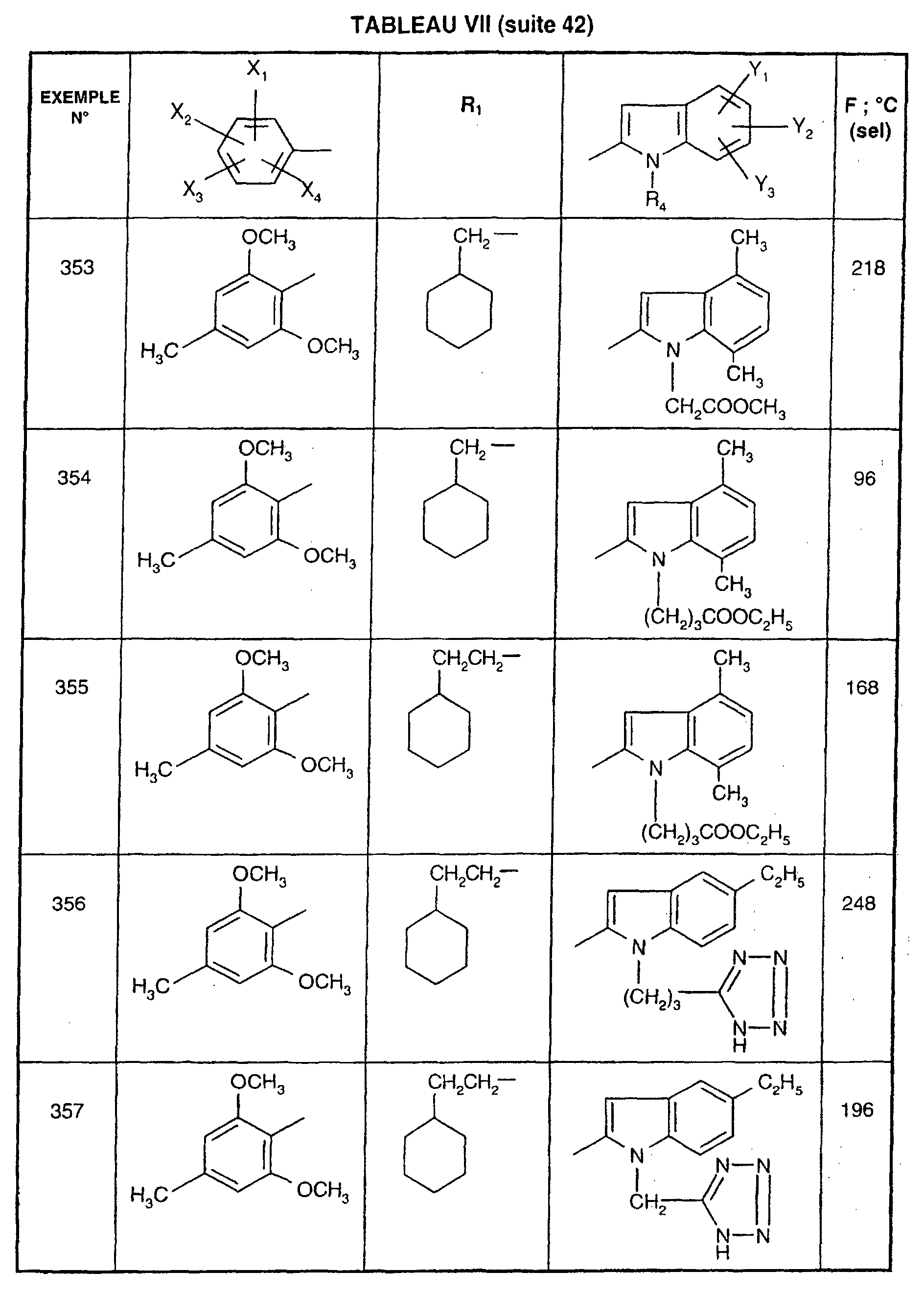

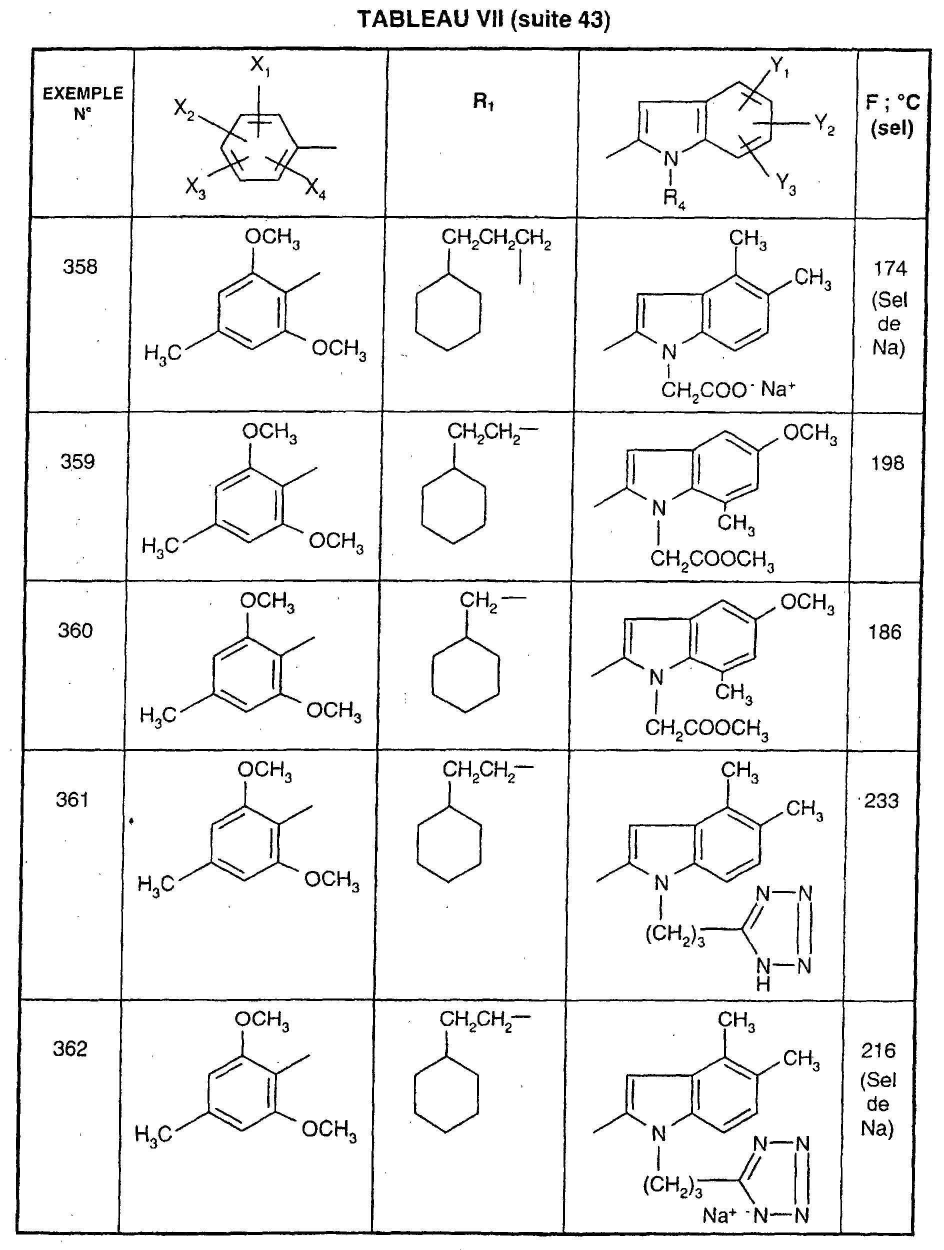

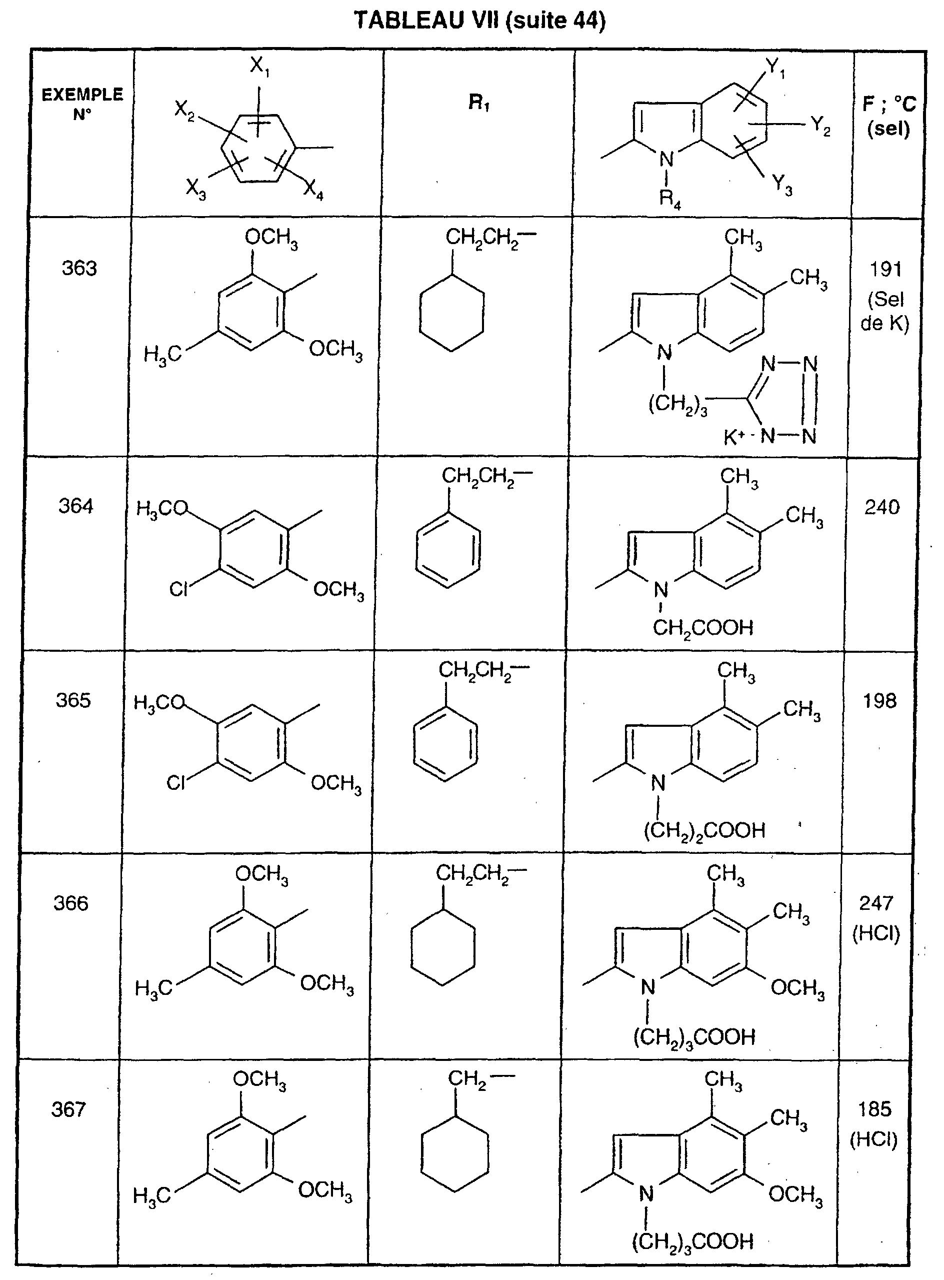

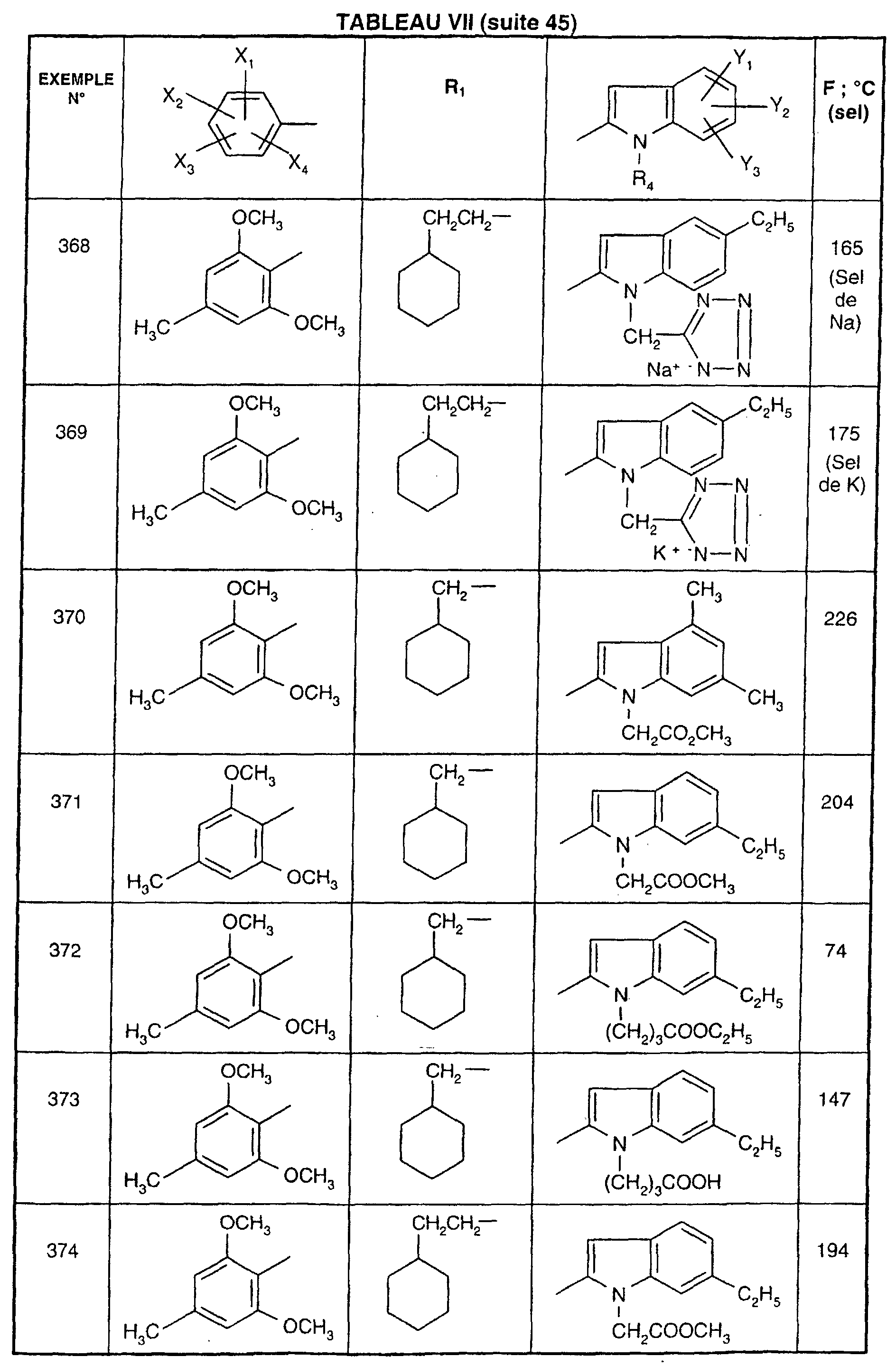

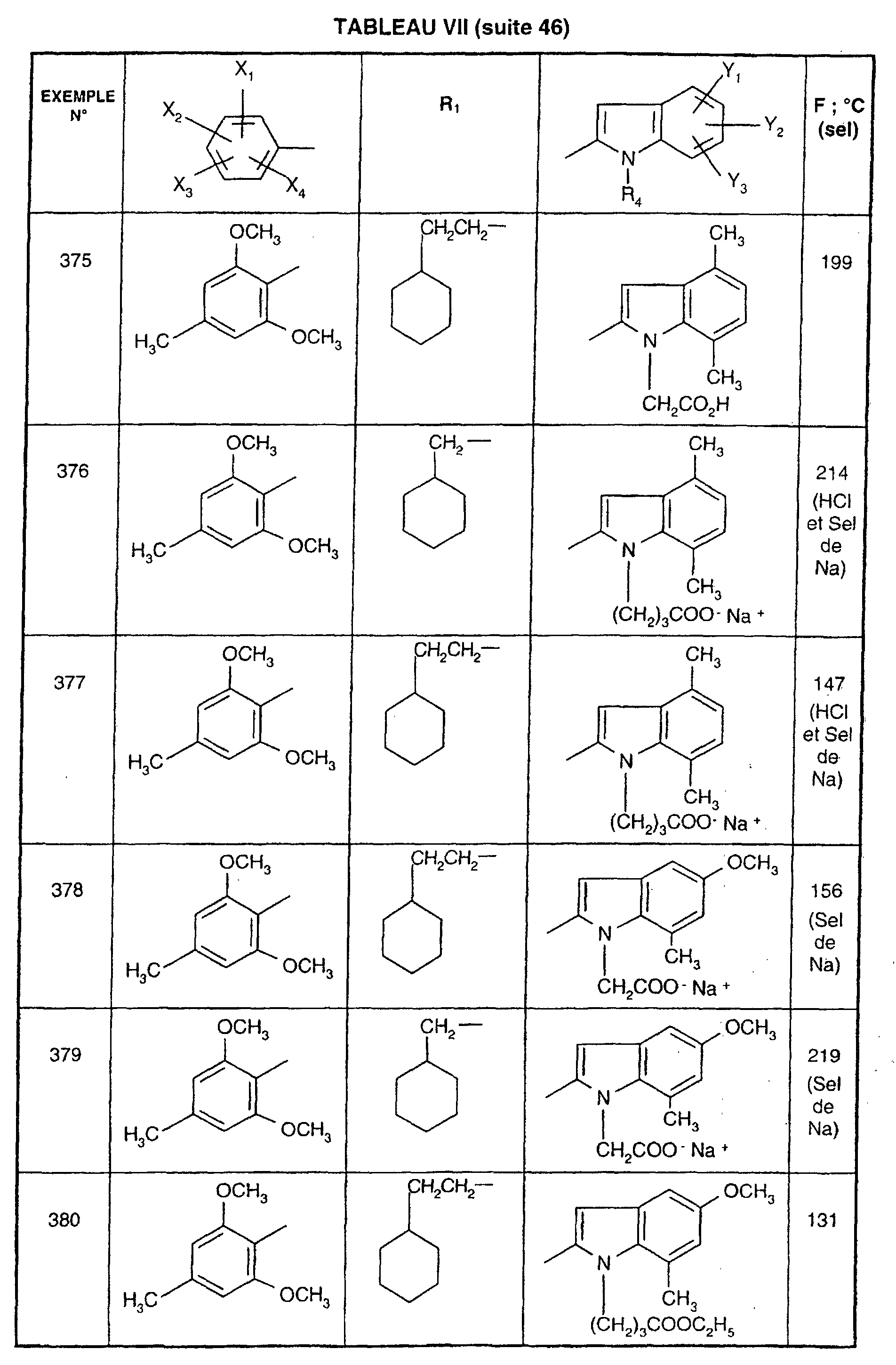

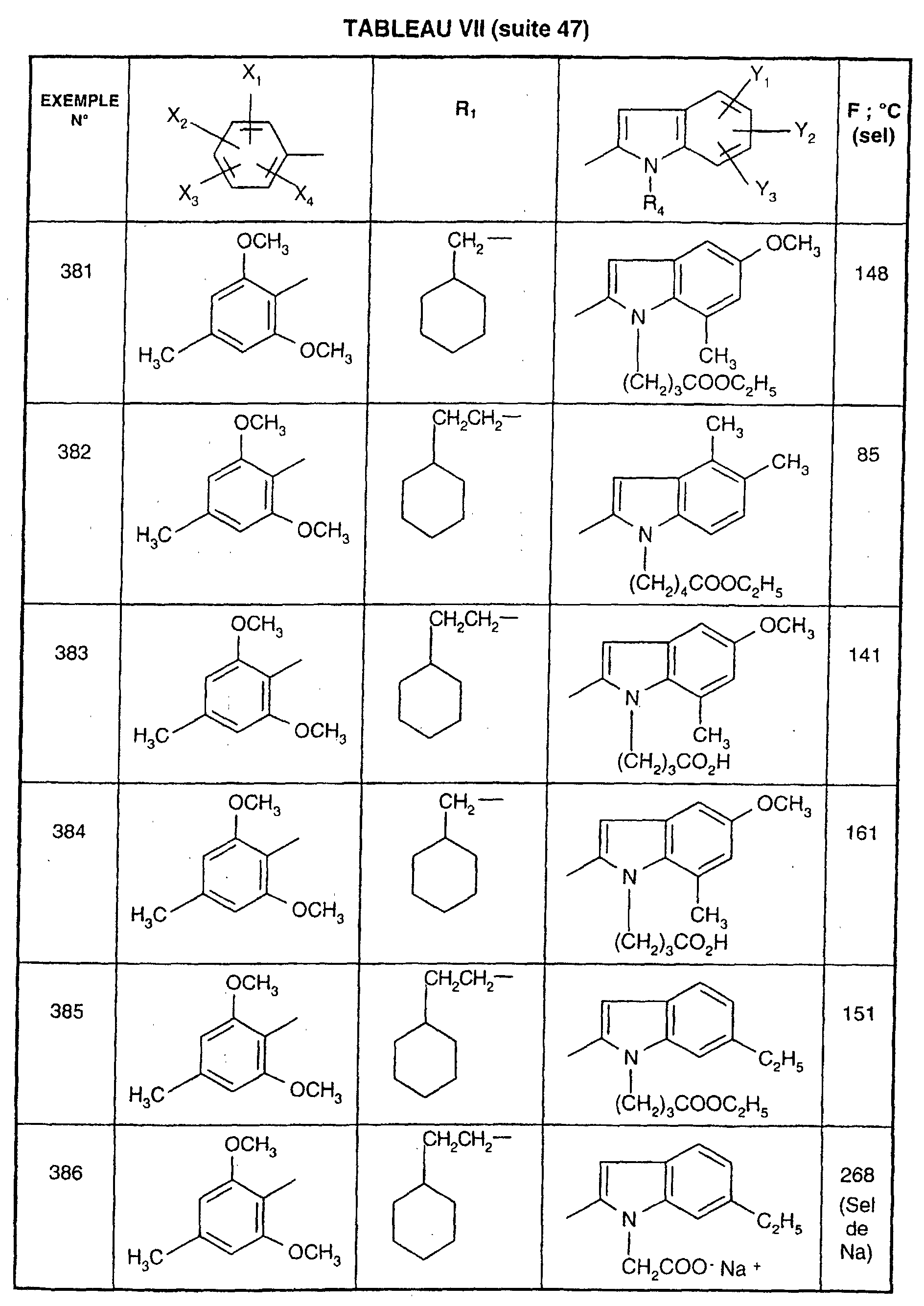

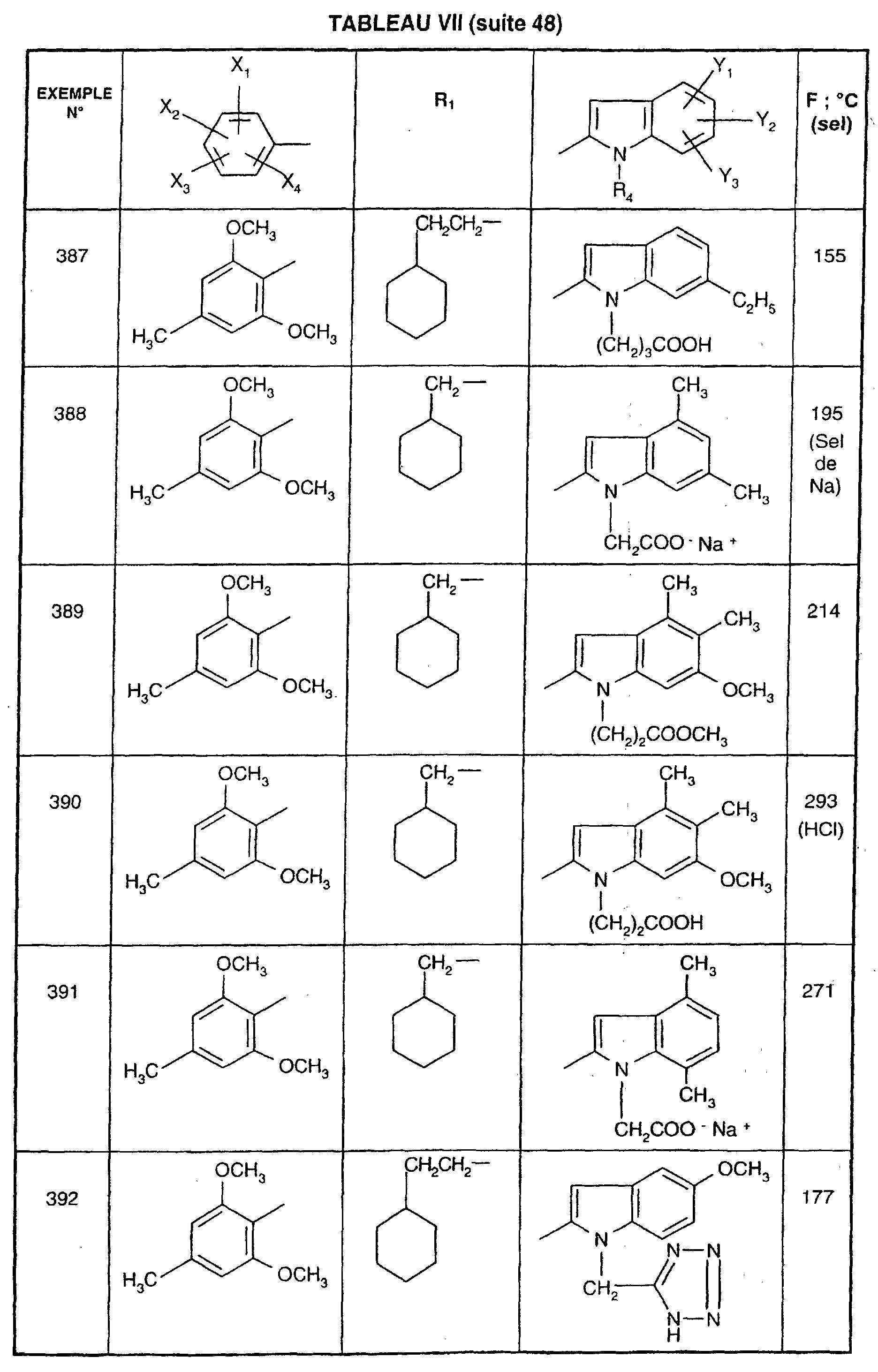

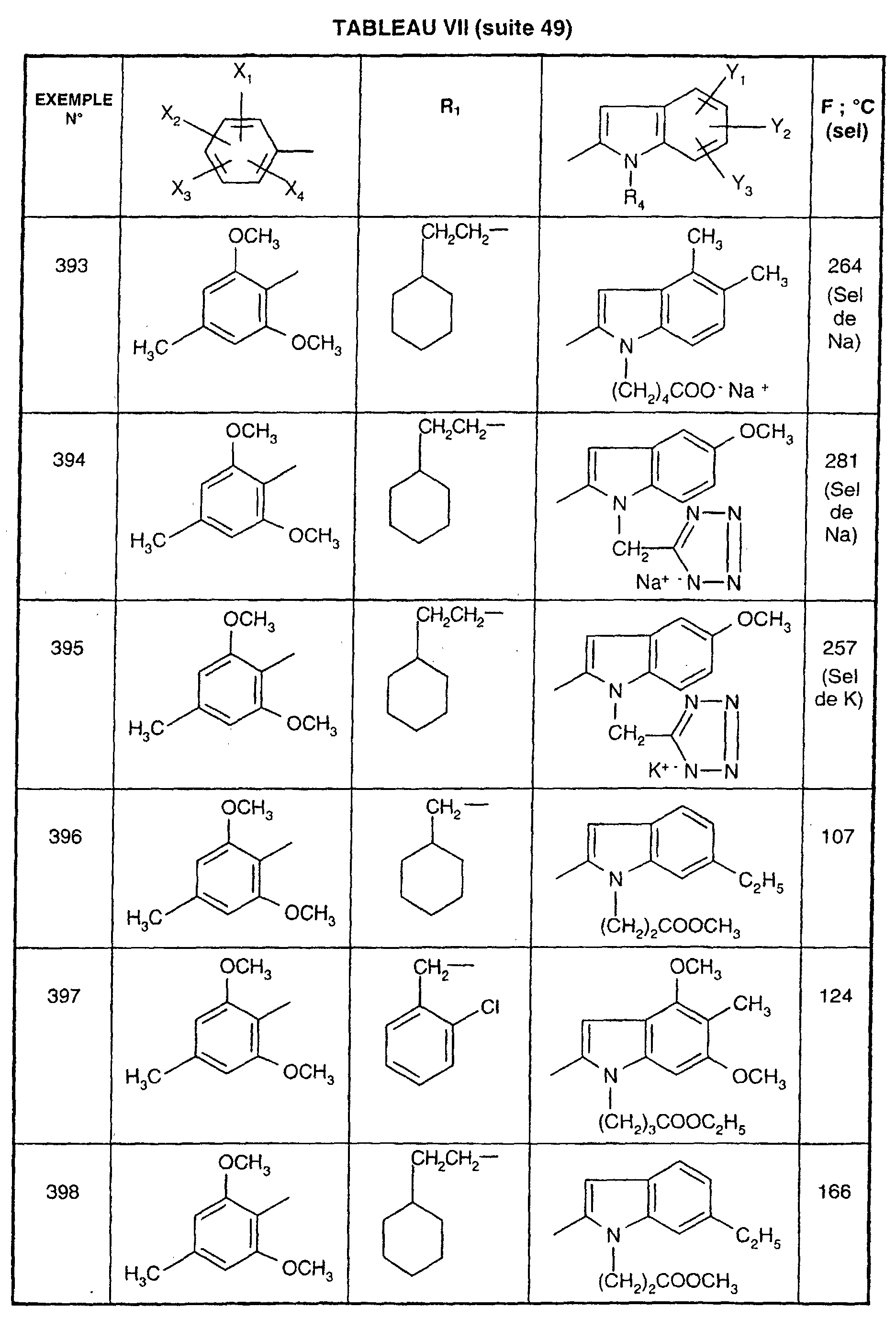

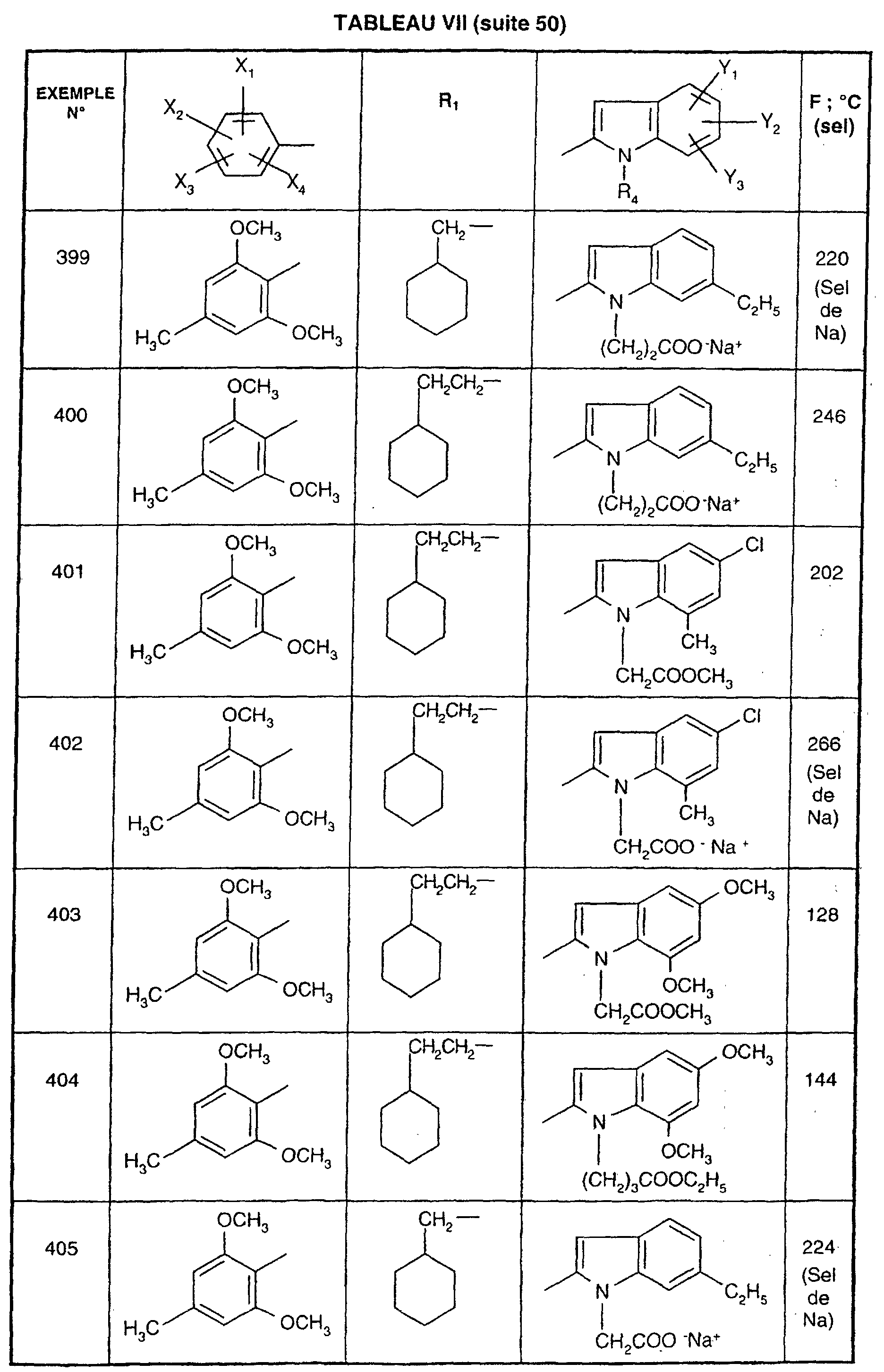

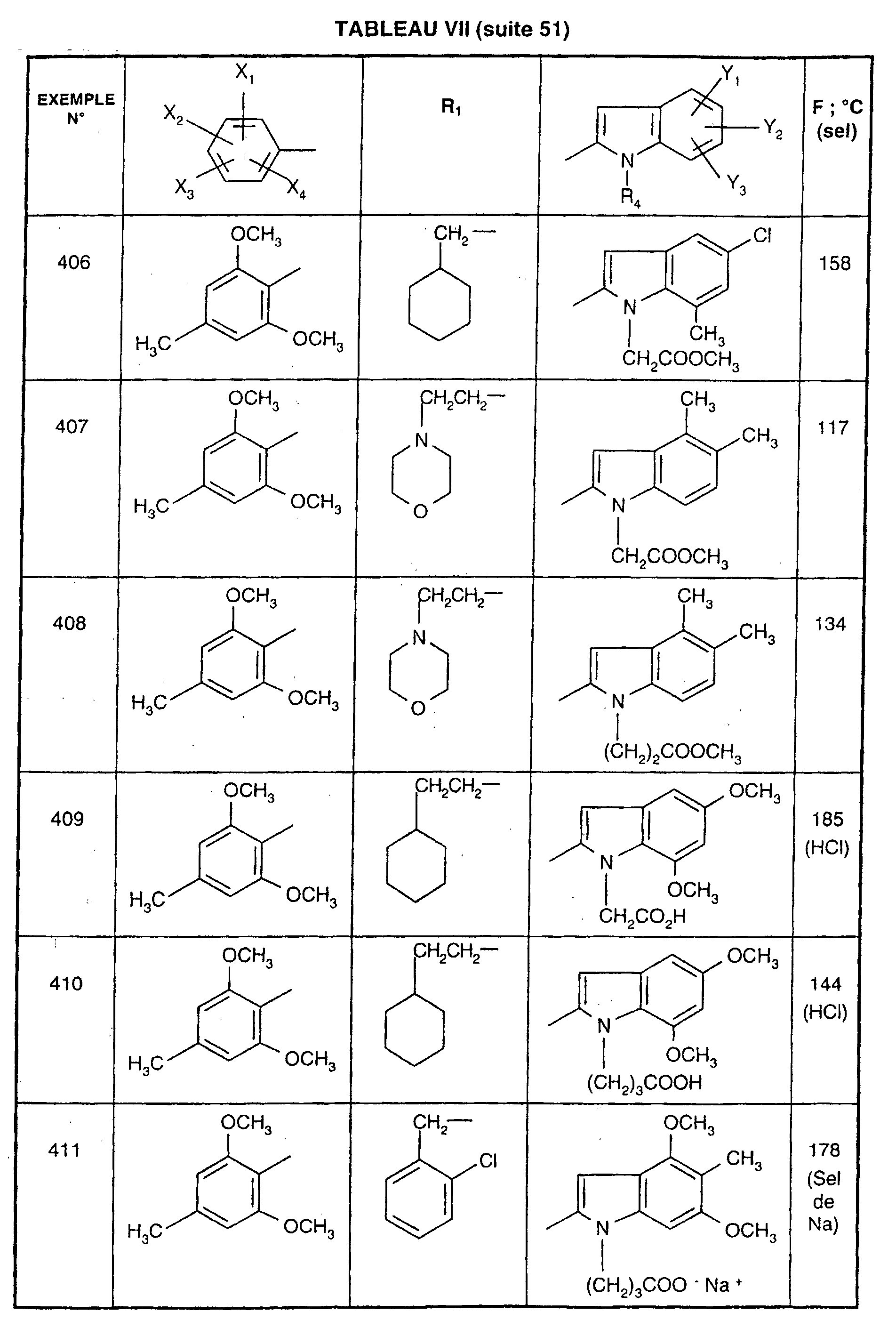

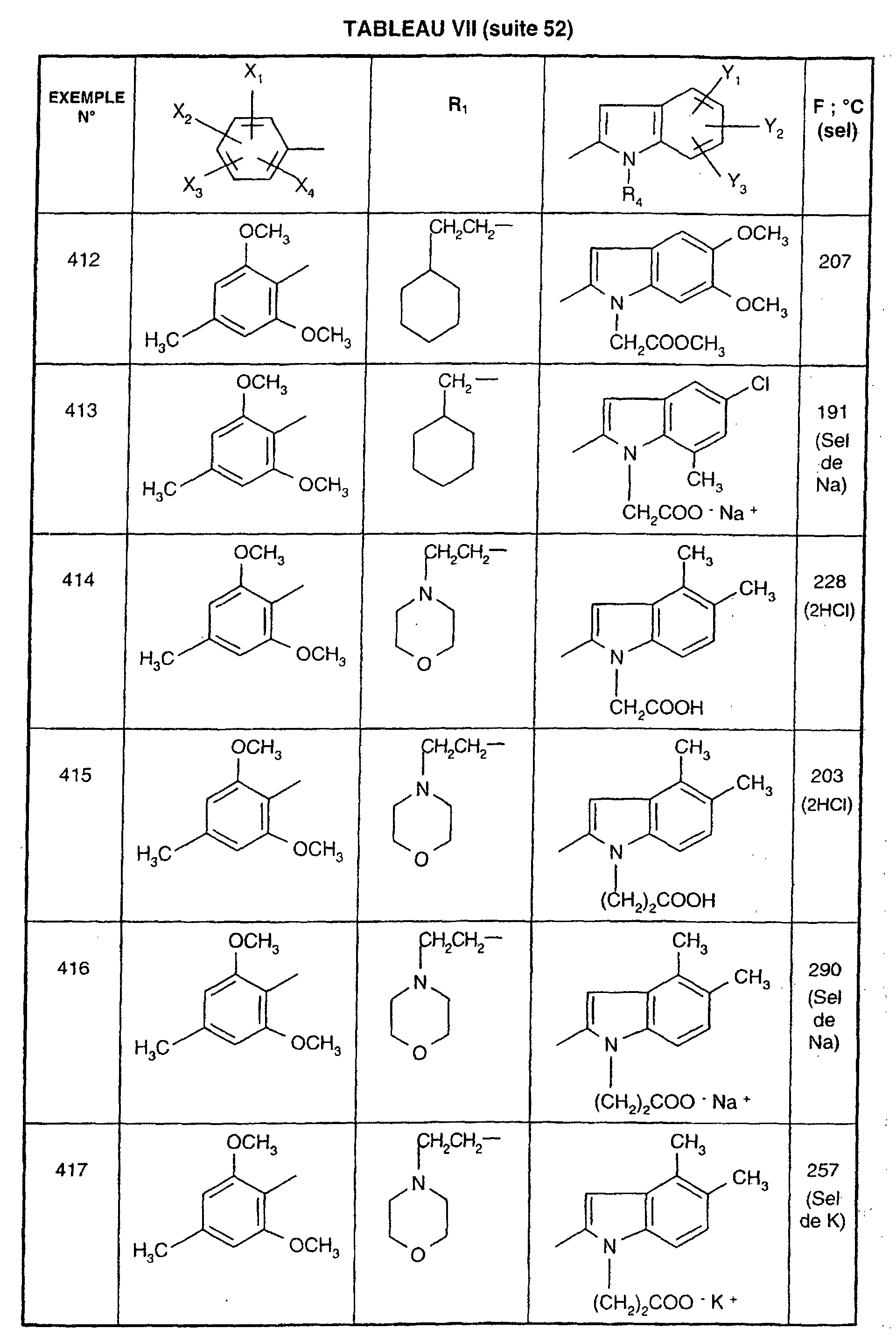

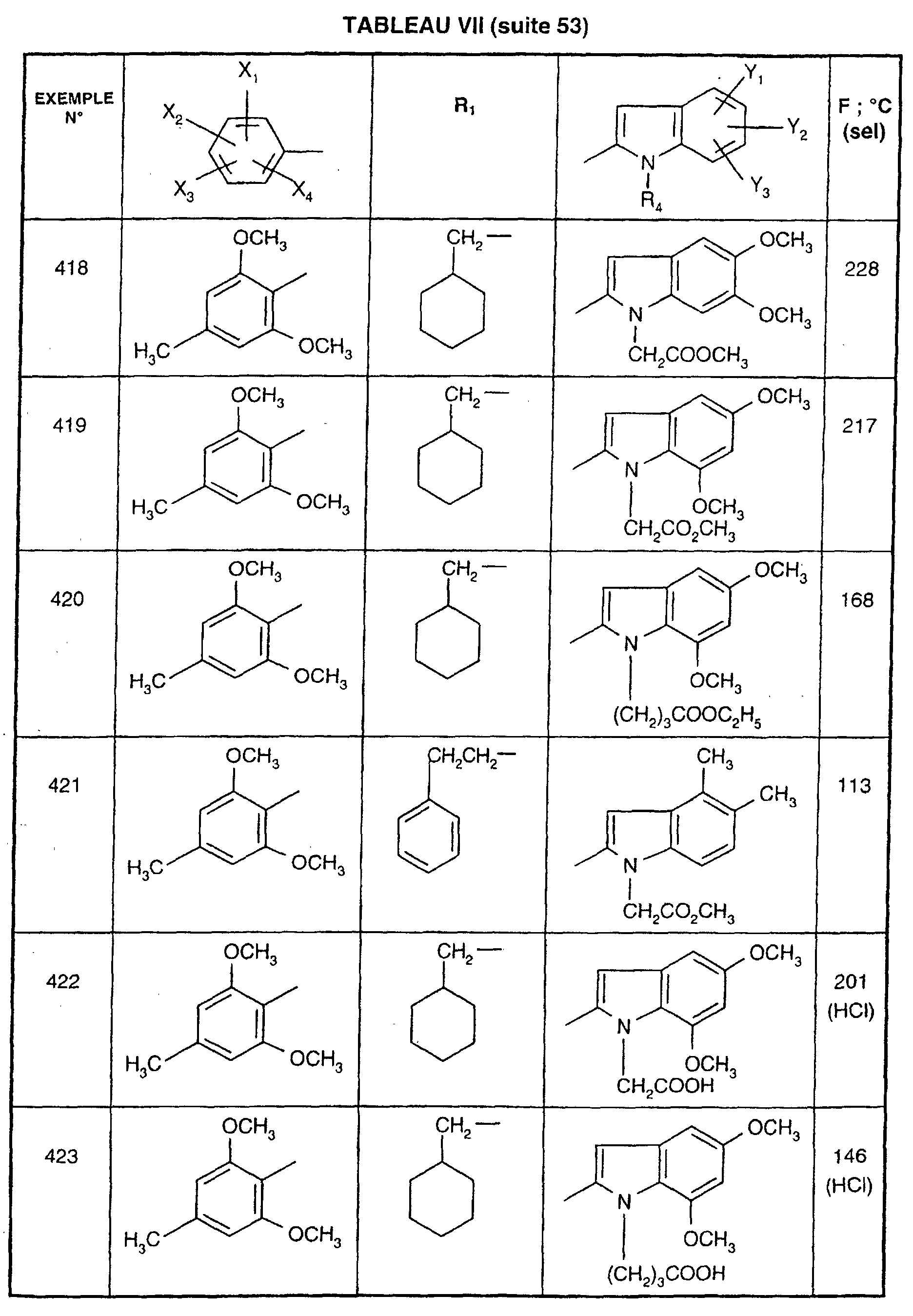

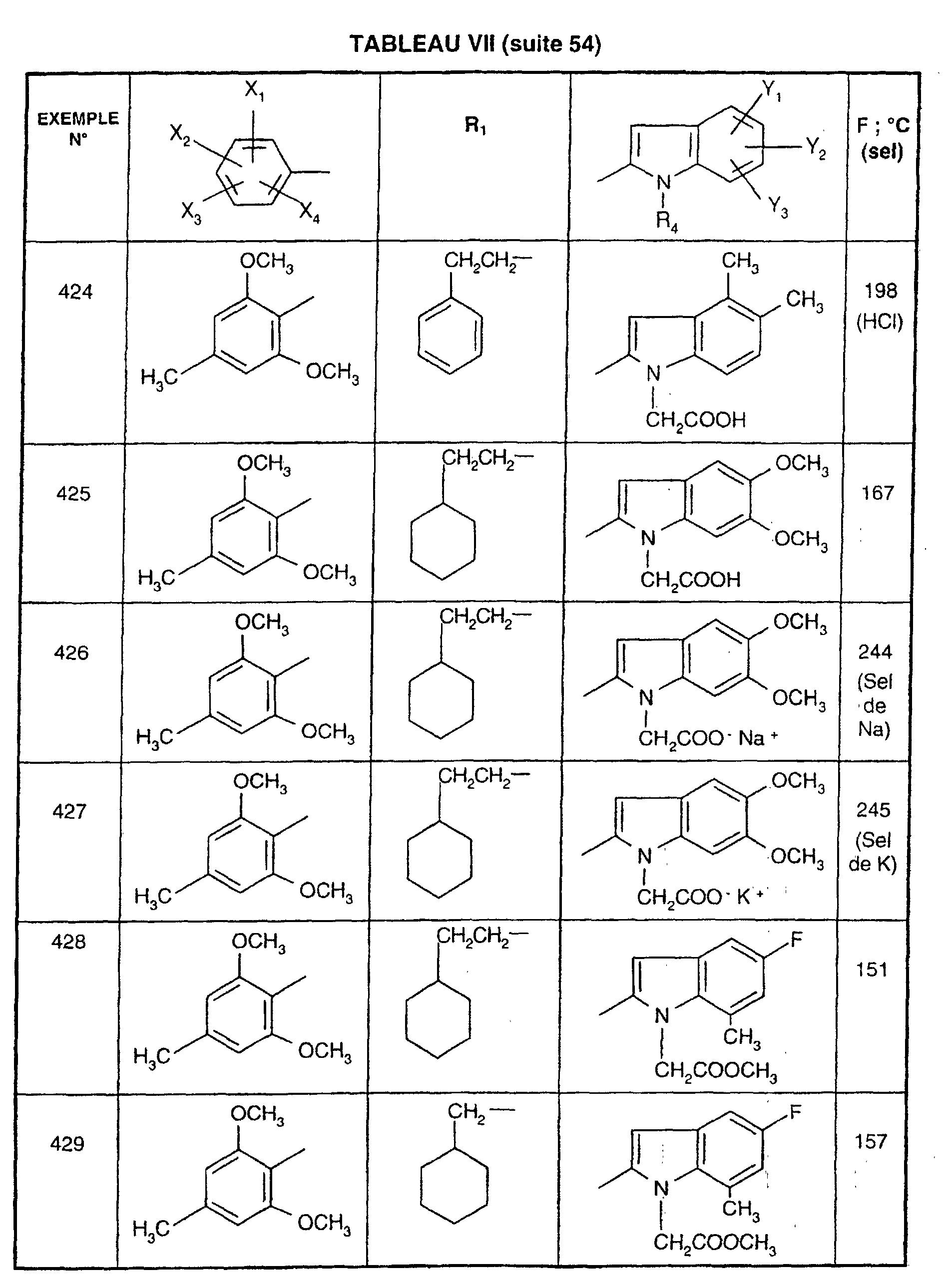

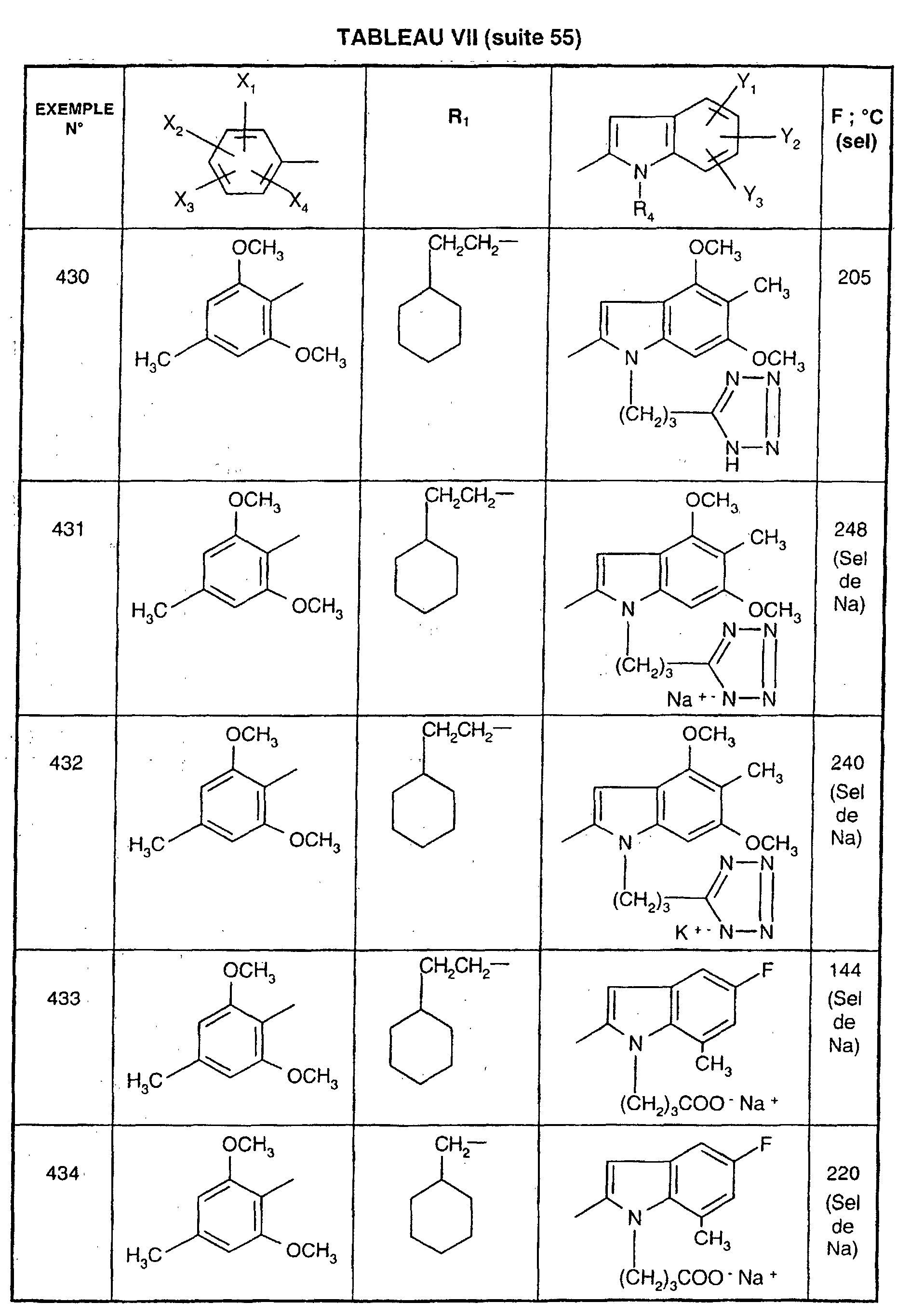

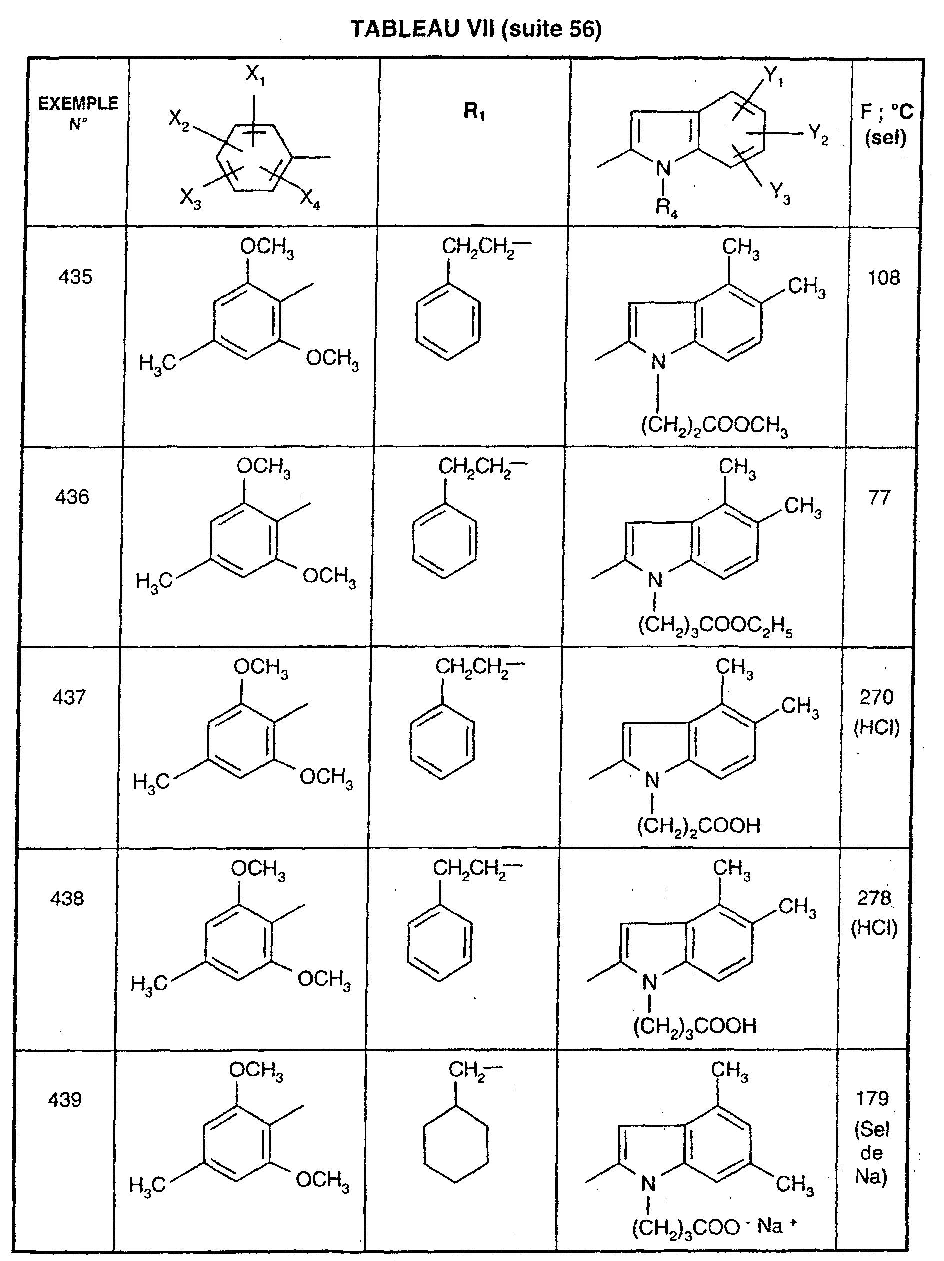

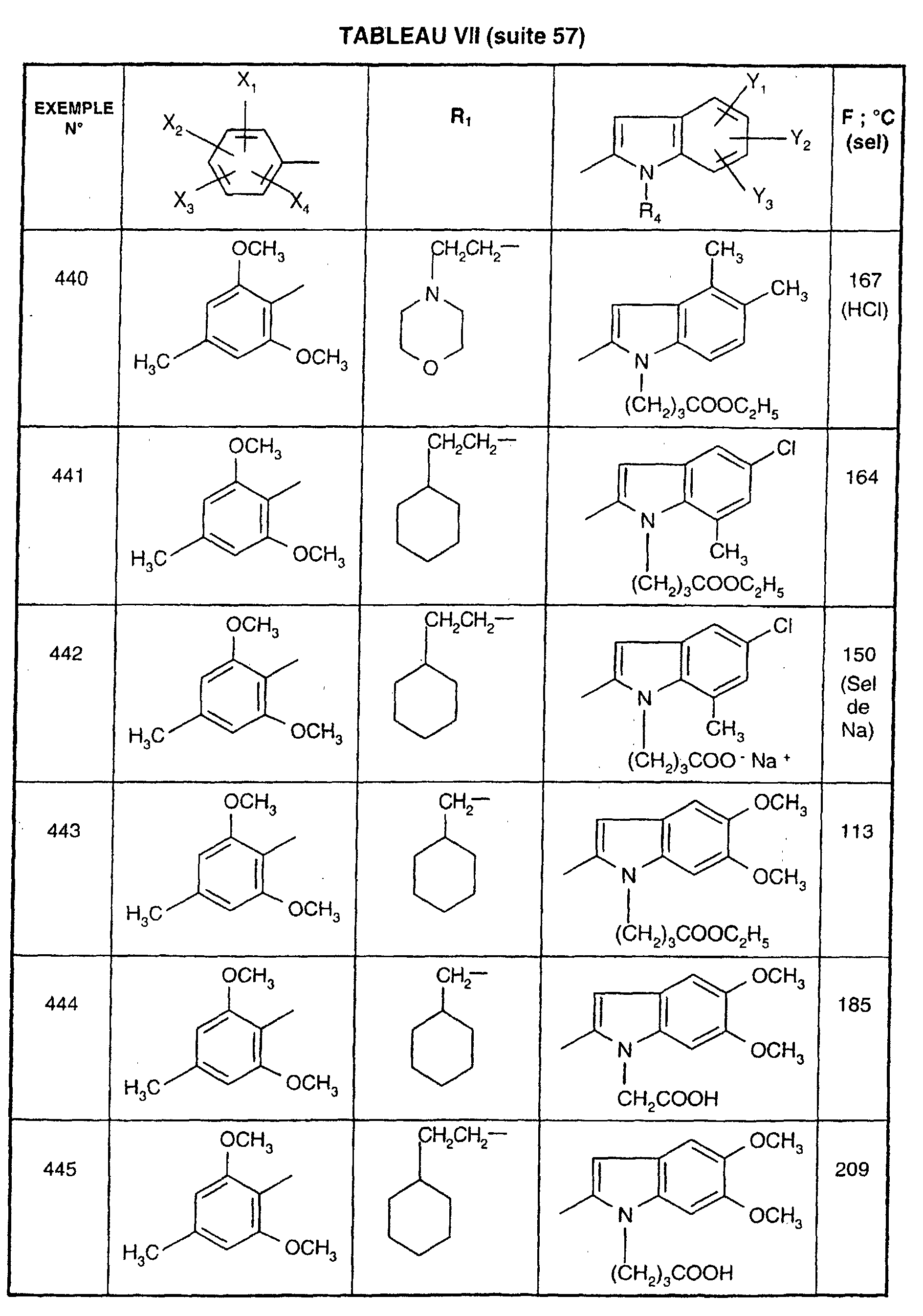

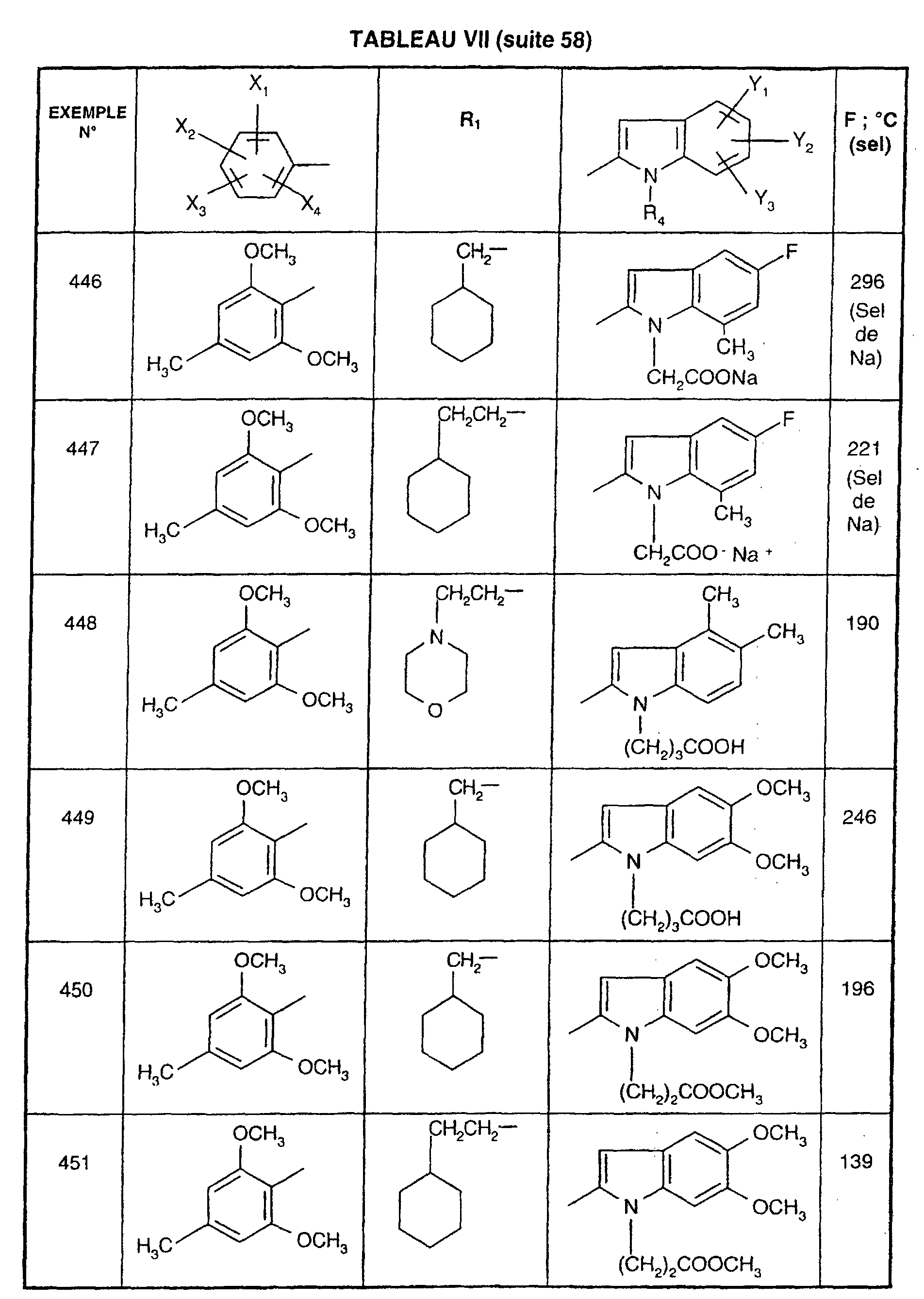

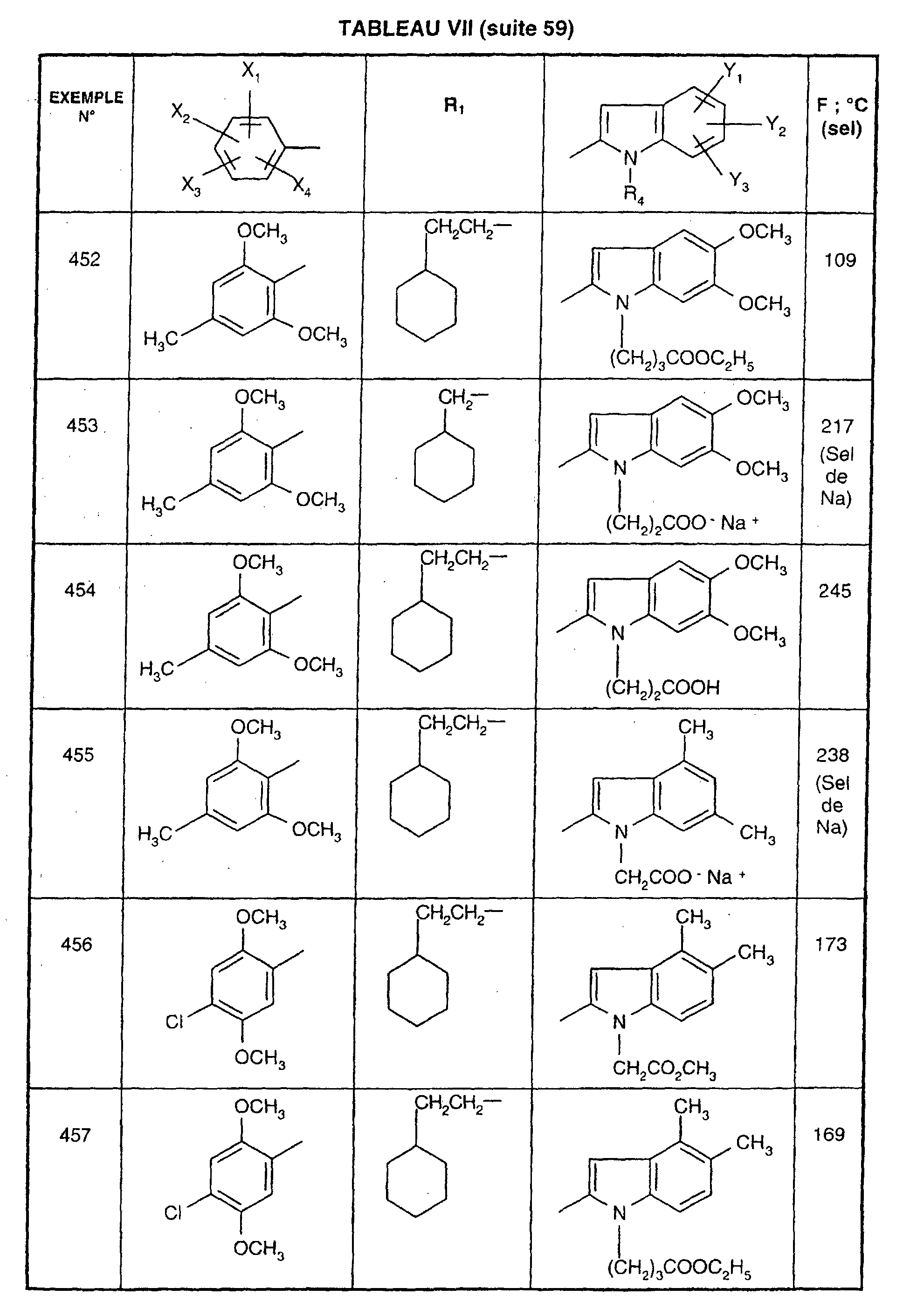

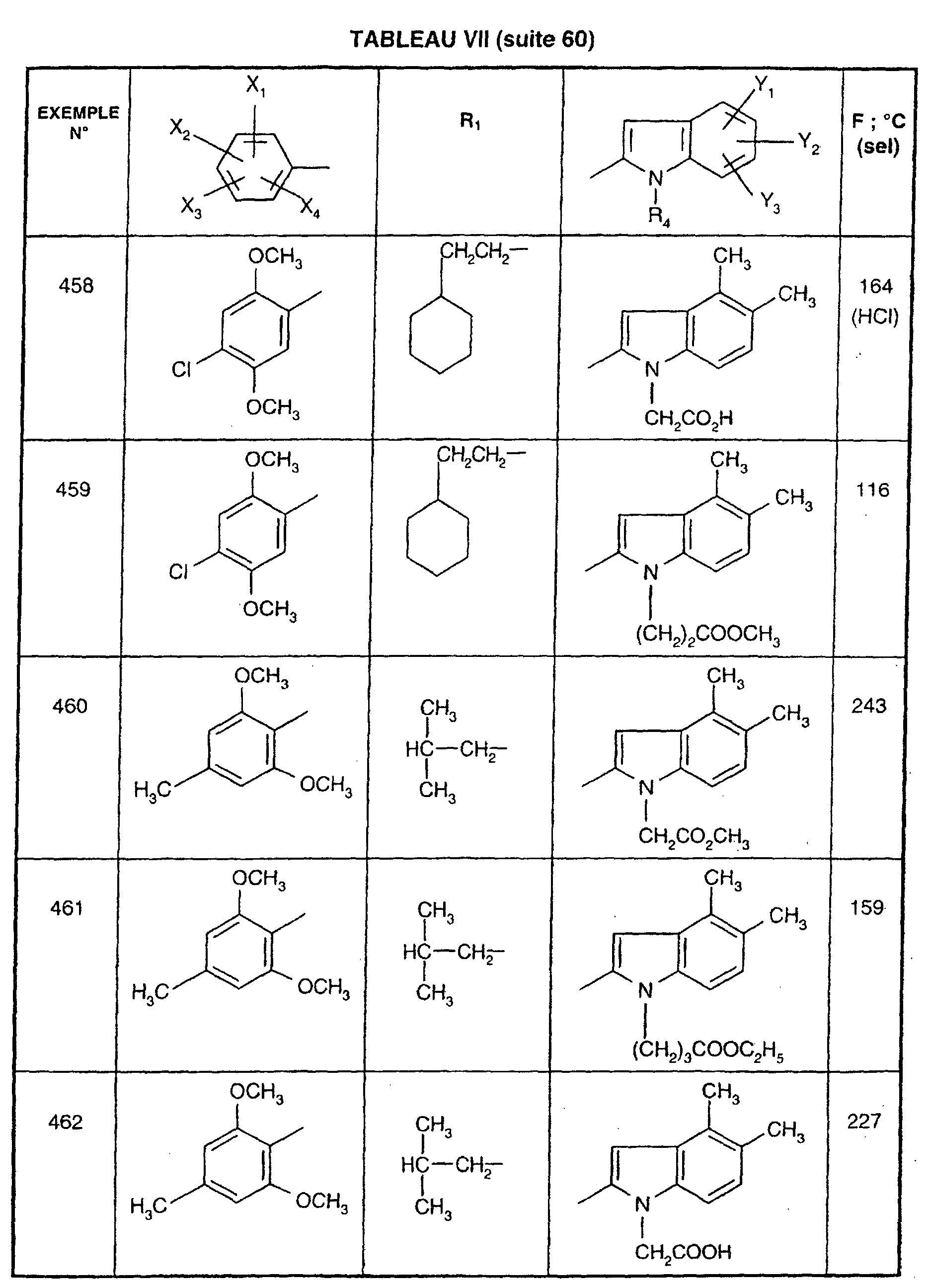

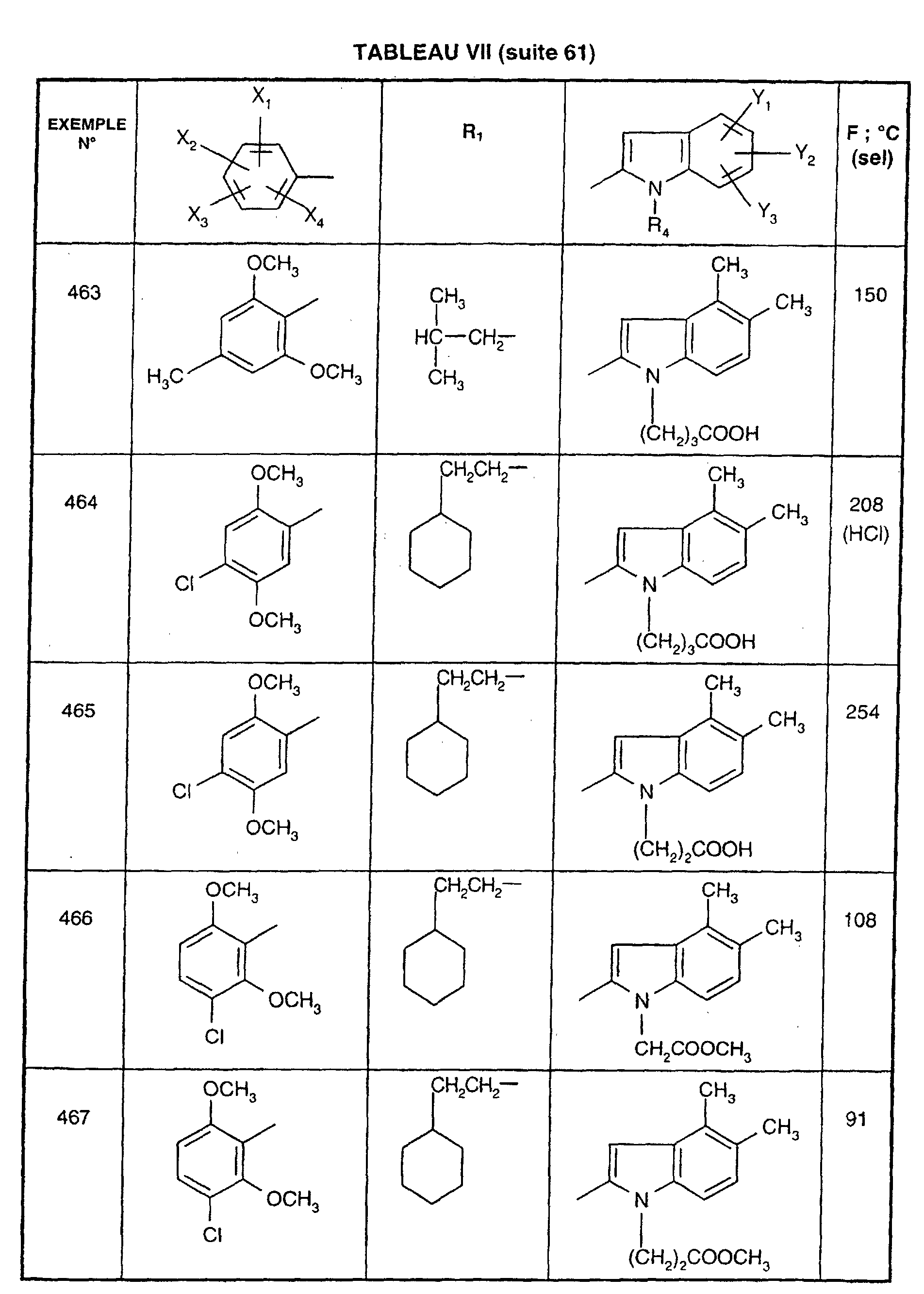

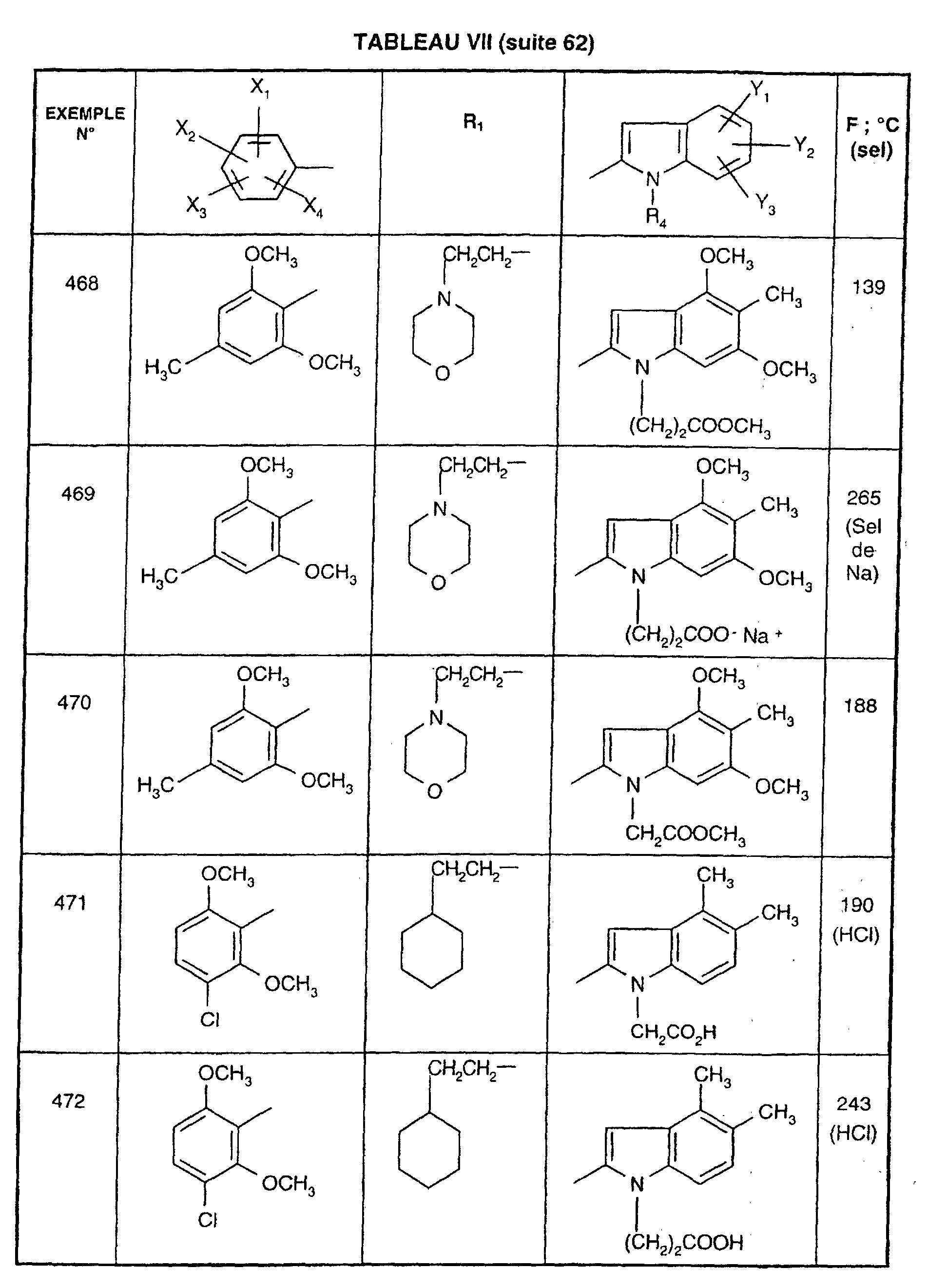

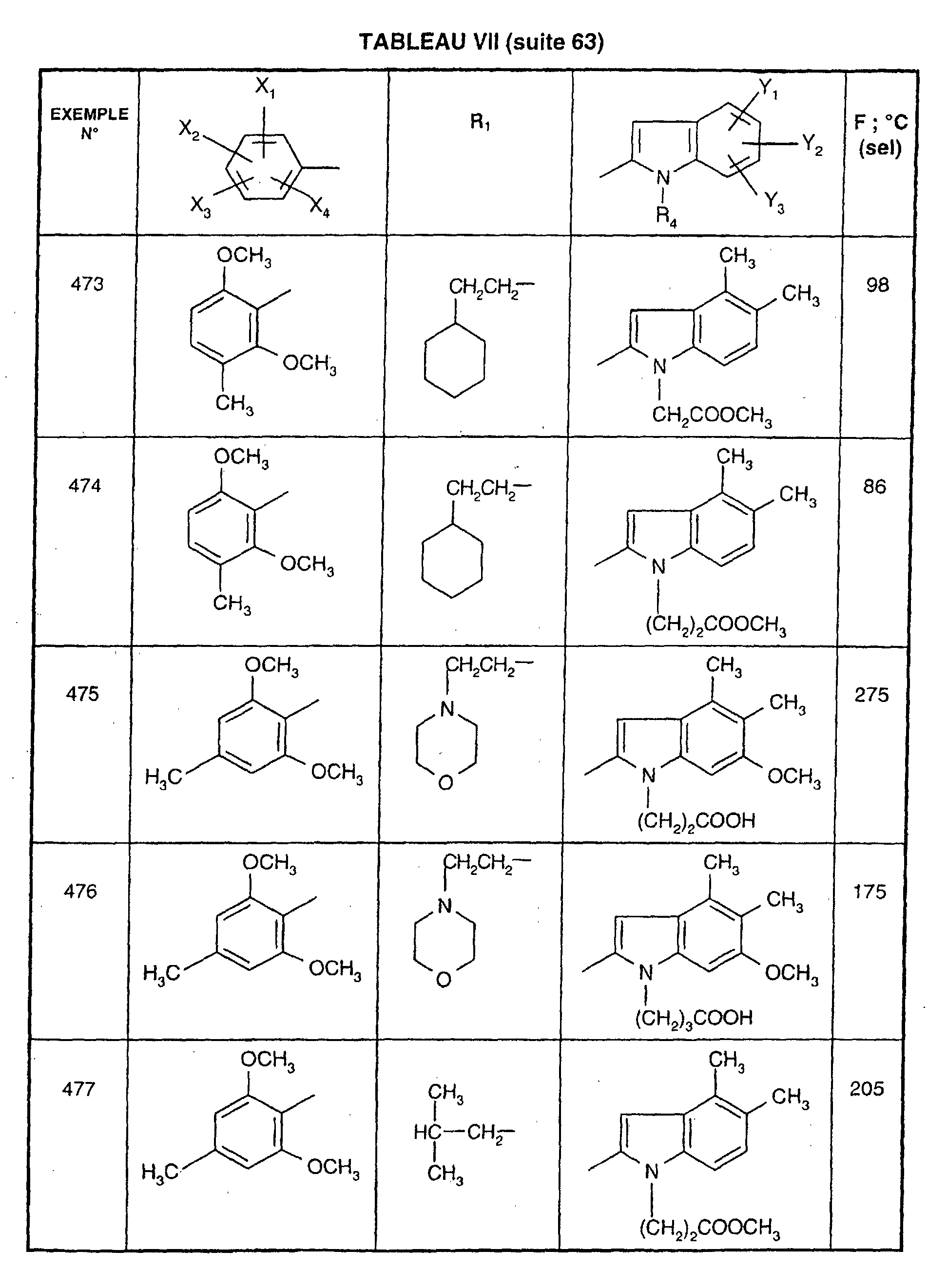

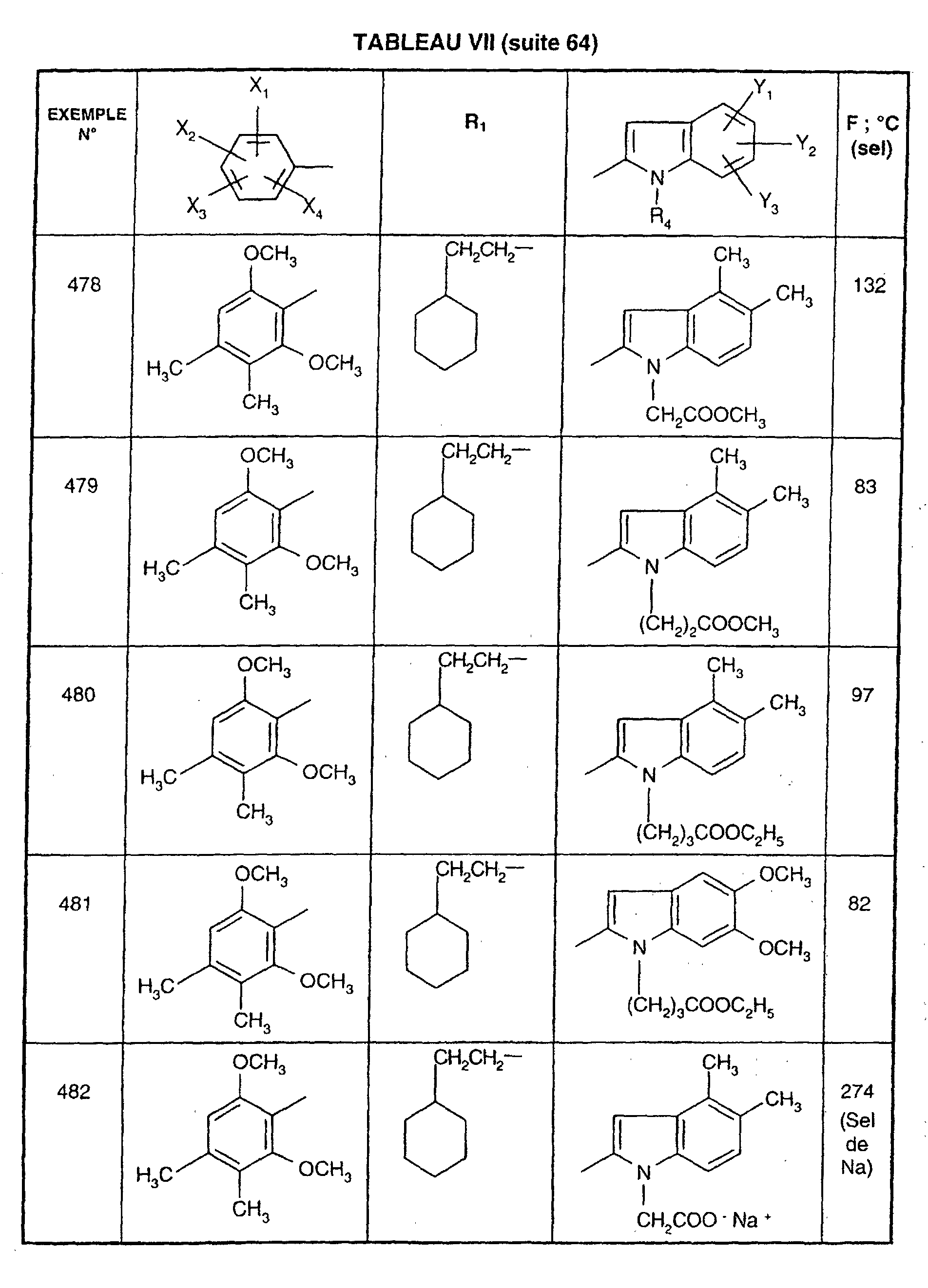

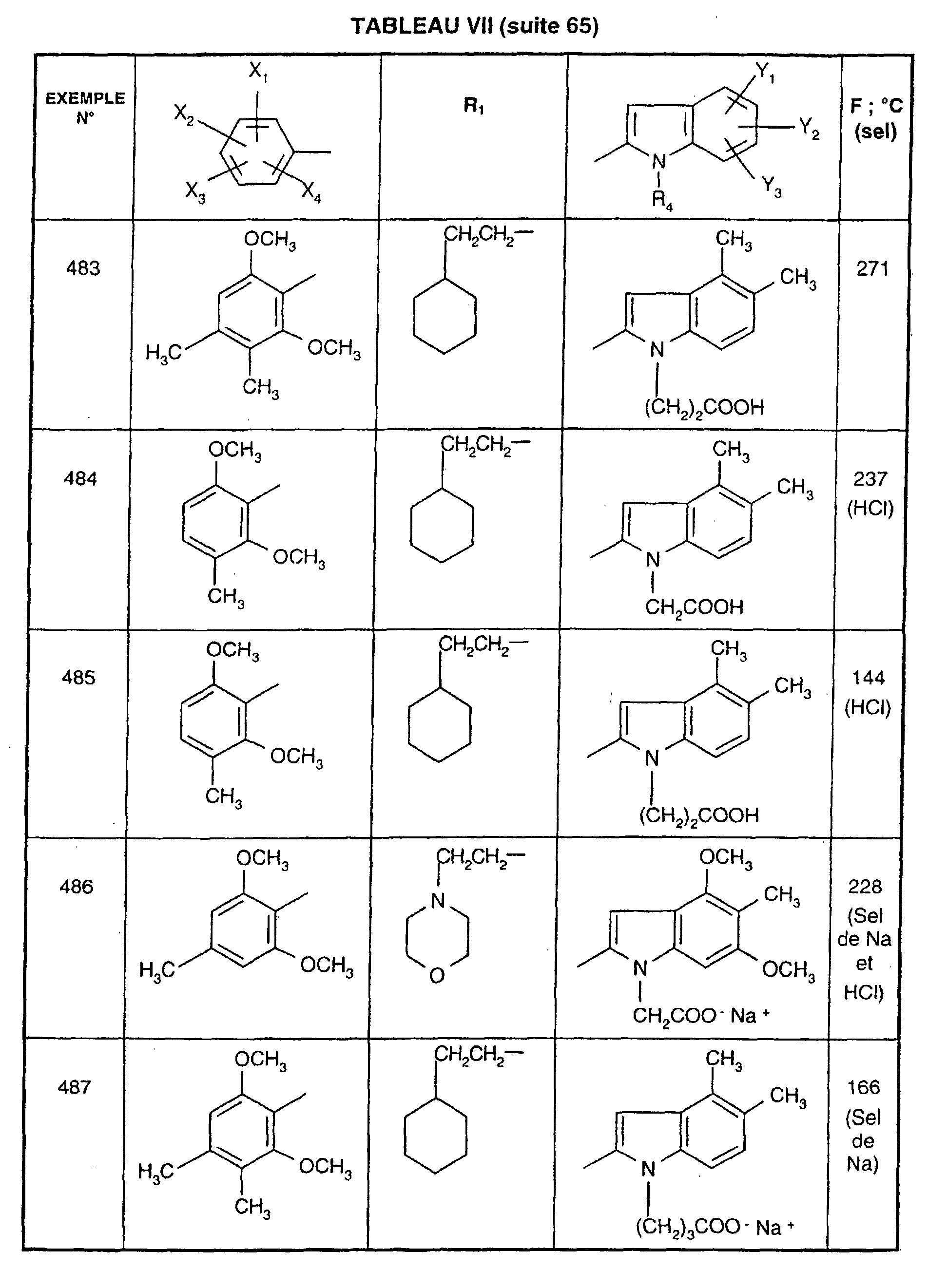

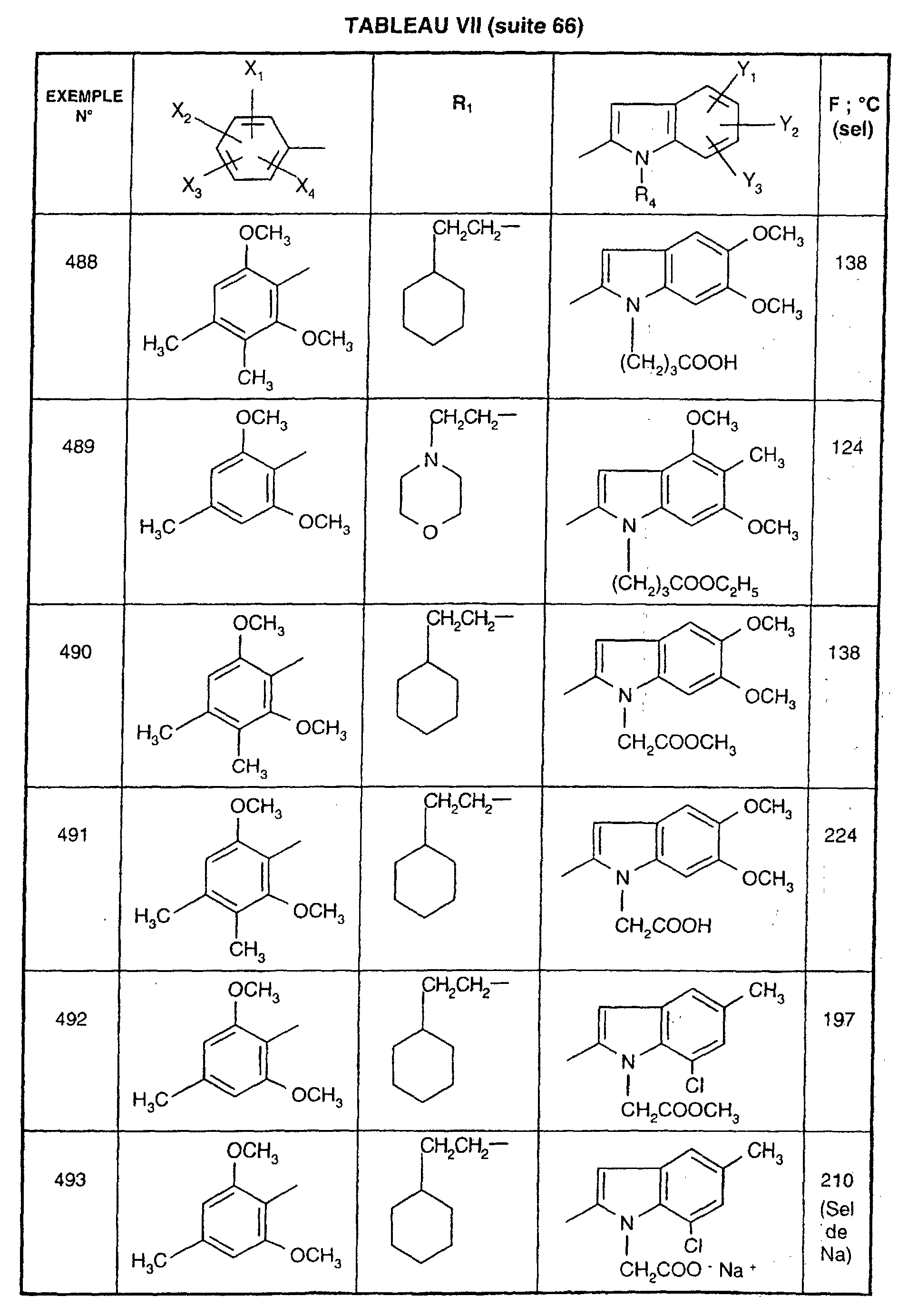

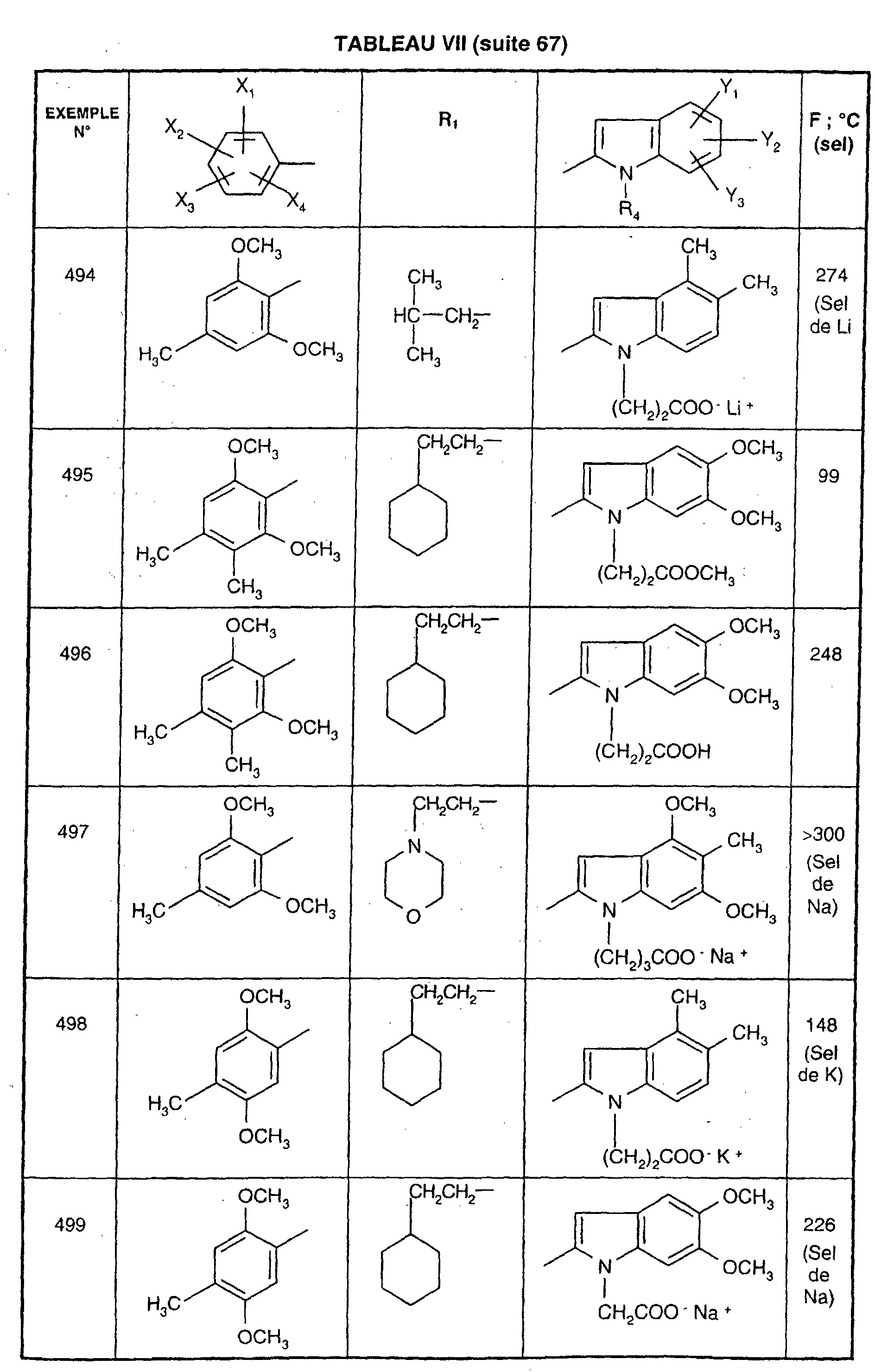

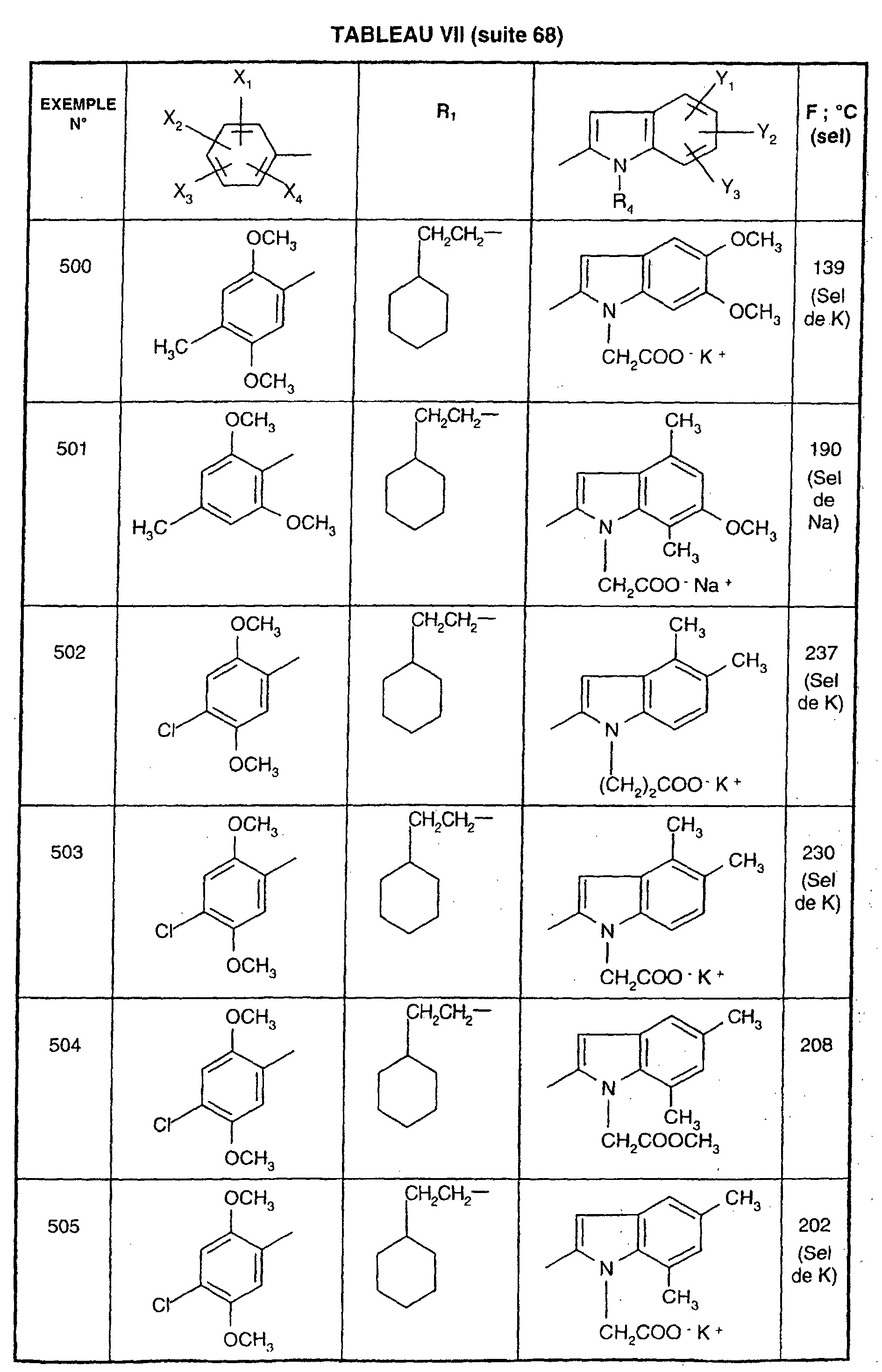

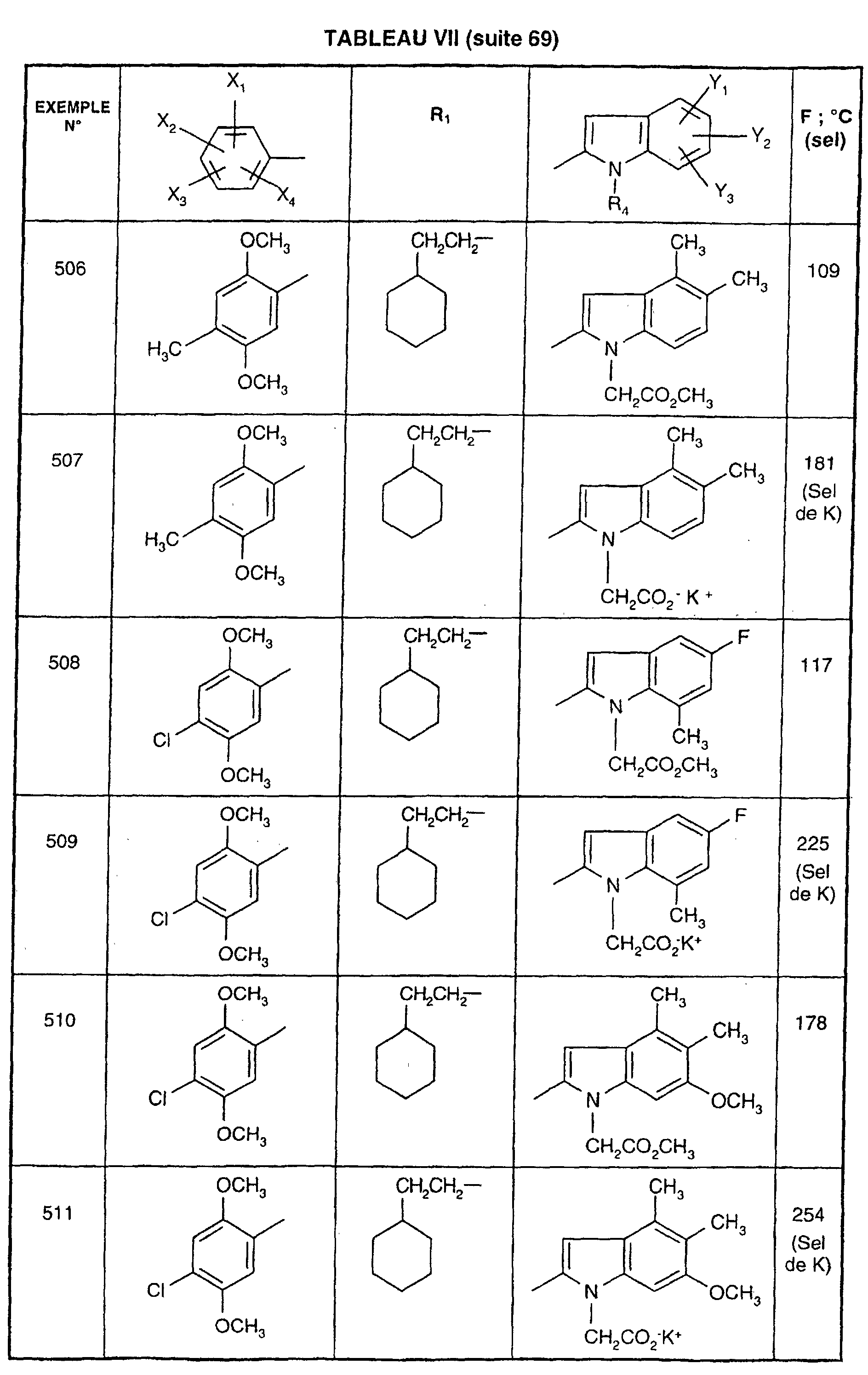

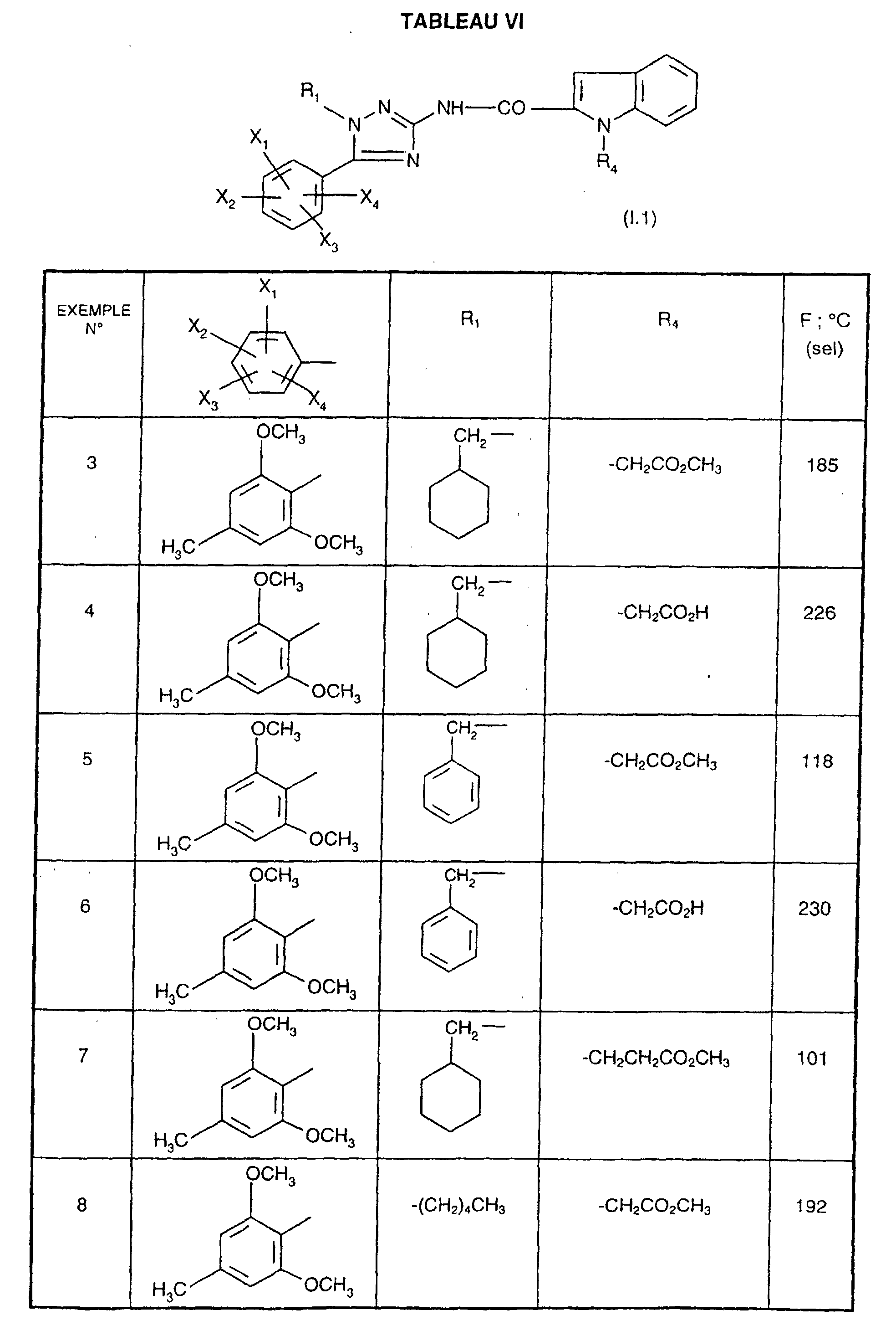

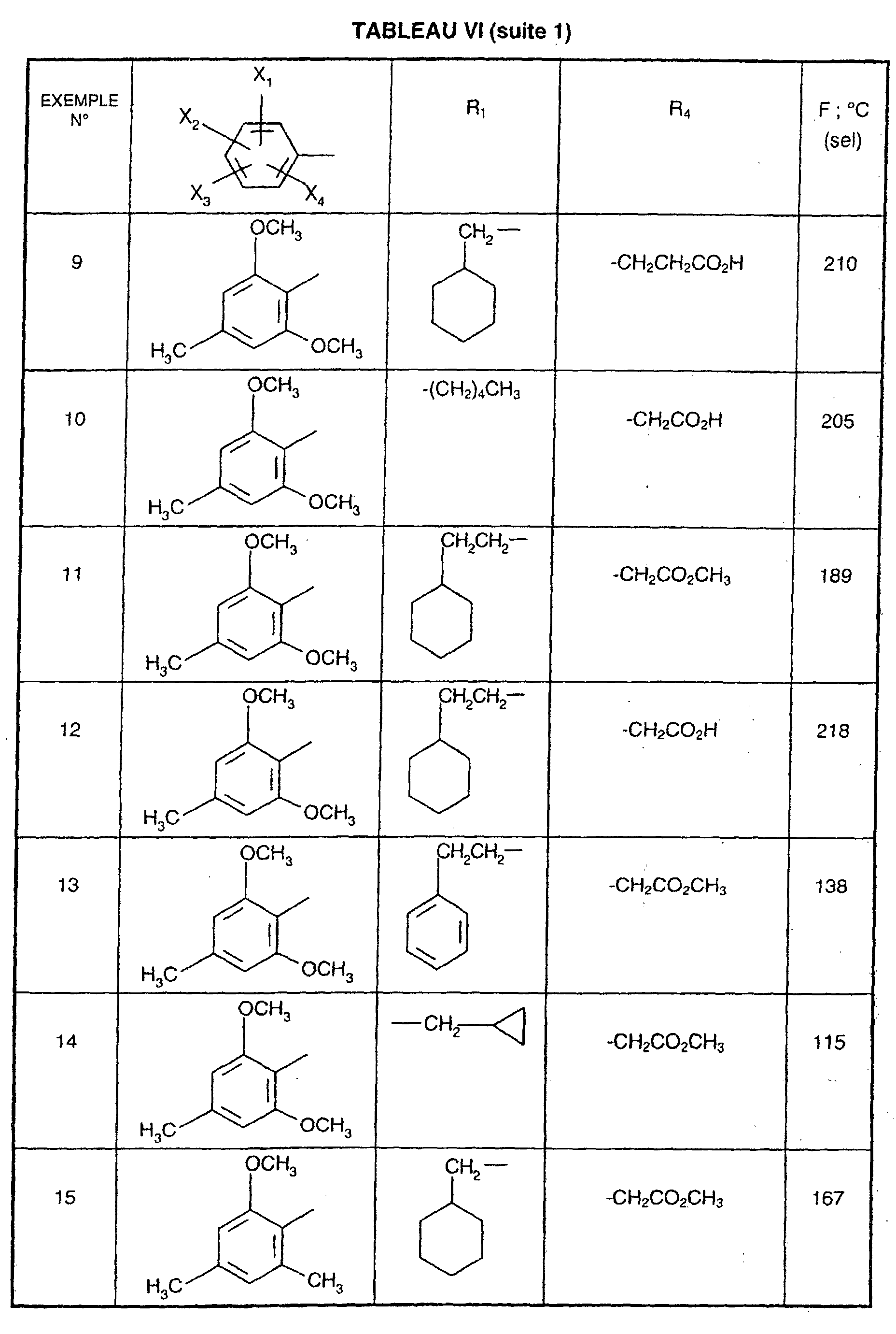

### EXEMPLE 512 : N2-[5-(4-chloro-2,5-diméthoxyphényl)-1-(2-cyclohéxyléthyl)-1H-1,2,4-triazol-3-yl]-4,5-diméthyl-1 -[3-(2H-1,2,3,4-tétraazol-5-yl)propyl]-1H-2-indolecarboxamide.

### Etape 1 : 4-[2-({[1-(2-Cyclohexyléthyl)-5-(2,5-diméthoxy-4-chlorophényl)-1H-1,2,4-triazol-3-yl]amino}carbonyl)-4,5-diméthyl-1H-1-indolyl]butyronitrile

A 15 ml de dichlorométhane, on additionne successivement 1 ml de pyridine (0,013 mole) et 0,21 ml (0,0029 mole) de chlorure de thionyle. Après 15 minutes à 0°C, on introduit 0,615 g de l'acide 4,5-diméthyl-1-(3-cyanopropyl)-*1H*-2-indolecarboxylique (0,0024 mole), puis 0,9 g de chlorhydrate de 1-(2-cyclohexyléthyl)-5-(2,5-diméthoxy-4-chlorophényl)-*1H*-1,2,4 -triazol-3-amine. On maintient le mélange réactionnel pendant 18 heures à température ambiante, puis on fait un lavage acide, puis un lavage basique. On sèche la phase organique sur sulfate de sodium anhydre et concentre sous pression réduite. Le résidu huileux est chromatographié sur une colonne de gel de silice, en éluant avec un mélange dichlorométhane/méthanol 98,5/1,5 (v/v) pour fournir une poudre blanche ; F=178°C ; Rendement = 87 %.

### Etape 2 : N2-[5-(4-chloro-2,5-diméthoxyphényl)-1-(2-cyclohéxyléthyl)-1H-1,2,4-triazol-3-yl]-4,5-diméthyl-1-[3-(2H-1,2,3,4-tétraazol-5-yl)propyl]-1H-2-indolecarboxamide.

A 0,720 g (0,0012 mole) de 4-[2-({[1-(2-cyclohexyléthyl)-5-(2,5-diméthoxy-4-chlorophényl)-1*H*-1,2,4-triazol-3-yl]amino}carbonyl)-4,5-diméthyl-*1H*-1-indolyl]butyronitrile en solution dans 15 ml de tétrahydrofurane, on additionne 0,5 ml d'azidotriméthylsilane, 0,030 g d'oxyde de dibutylétain et chauffe à reflux pendant 18 heures. On laisse revenir le mélange réactionnel à température ambiante, on élimine le tétrahydrofurane sous pression réduite et on chromatographie le résidu sur une colonne de gel de silice en éluant avec un mélange dichlorométhane/méthanol 95/5 (v/v). On obtient un solide blanc ; F = 233°C ; Rendement 78 %.

Ce mode opératoire décrit pour l'EXEMPLE 512 est également utilisé pour les EXEMPLES 303, 304, 316, 317, 356, 357, 361, 362, 363, 368, 369, 392, 394, 395, 430, 431, 432.

Les sels de potassium et de sodium de ces composés sont obtenus dans l'acétonitrile par addition d'un équivalent de base à température ambiante, suivie de l'évaporation sous pression réduite du solvant puis séchage.

## Revendications

1. Composé de formule : 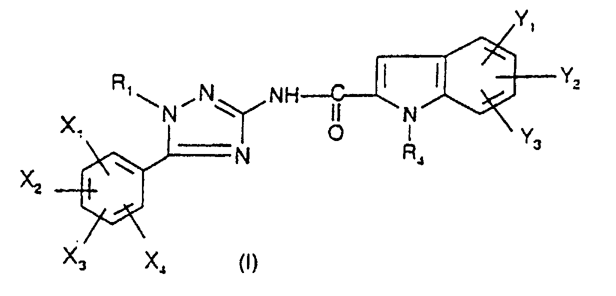 dans laquelle :
- R₁ représente un (C₂-C₆)alkyle ; un groupe -(CH₂)ₘ-G avec m variant de 0 à 5 et G représentant un groupe hydrocarboné mono- ou polycyclique non aromatique en C₃-C₁₃ éventuellement substitué par un ou plusieurs (C₁-C₃)alkyle; un phényl(C₁-C₃)alkyle dans lequel le groupe phényle est éventuellement substitué une ou plusieurs fois par un halogène, par un (C₁-C₃)alkyle ou par un (C₁-C₃)alcoxy ; un groupe -(CH₂)ₙNR₂R₃ dans lequel n représente un nombre entier de 1 à 6 et R₂ et R₃ identiques ou différents représentent un (C₁-C₃)alkyle ou constituent avec l'atome d'azote auquel ils sont liés un groupe morpholino, pipéridino, pyrolidinyle ou pipérazinyle ;
- X₁,X₂,X₃ ou X₄ représentent chacun indépendamment un atome d'hydrogène, d'halogène, un (C₁-C₆)alkyle, un (C₁-C₃)alcoxy ou un trifluorométhyle; étant entendu qu'un seul parmi X₁, X₂, X₃ et X₄ représente éventuellement un atome d'hydrogène ;
- R₄ représente l'hydrogène, un groupe -(CH₂)ₙCOOR₅ dans lequel n est tel que défini précédemment et R₅ représente un atome d'hydrogène, un (C₁-C₆)alkyle ou un (C₆-C₁₀)aryl-(C₁-C₆)alkyle ; un (C₁-C₆)alkyle ; un groupe -(CH₂)ₙOR₅ ou un groupe -(CH₂)ₙNR₂R₃ dans lesquels n, R₂, R₃ et R₅ sont tels que définis précédemment ; un groupe -(CH₂)-ₙ tétrazolyle dans lequel n est tel que défini précédemment, ou R₄ représente l'un de ces groupes sous la forme de sel d'un métal alcalin ou alcalino-terreux ;
- Y₁, Y₂ et Y₃, représentent indépendamment un hydrogène, un halogène, un (C₁-C₃)alkyle, un (C₁-C₃)alcoxy, un nitro, cyano, (C₁-C₆)acylamino, carbamoyle, trifluorométhyle, un groupe COOR₆ dans lequel R₆ représente l'hydrogène, ou (C₁-C₃)alkyle ;
ou un de ses sels ou solvates.

2. Composé de formule (I) selon la revendication 1, dans laquelle R₁, R₄, X₁, X₂, X₃ et X₄ sont tels que définis à la revendication 1 et Y₁, Y₂ et Y₃ représentent l'hydrogène; un de ses sets ou solvates.

3. Composé de formule (I) selon la revendication 1, dans laquelle R₁ et R₄ sont tels que définis à la revendication 1, Y₁, Y₂ et Y₃ représentent l'hydrogène ; et 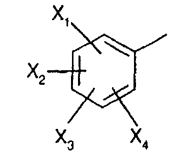 représente 2,6-diméthoxy-4-méthylphényle;
un de ses sels ou solvates.

4. Composé de formule (I) selon la revendication 1, dans laquelle R₁, R₄, Y₁, Y₂ et Y₃ sont tels que définis à la revendication 1 et 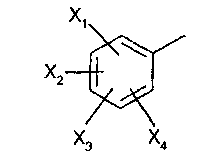 représente 2,6-diméthoxy-4-méthylphényle;
un de ses sels ou solvates.

5. Composé de formule (I) selon la revendication 1, dans laquelle R₁, R₄, Y₁, Y₂ et Y₃ sont tels que définis à la revendication 1 et 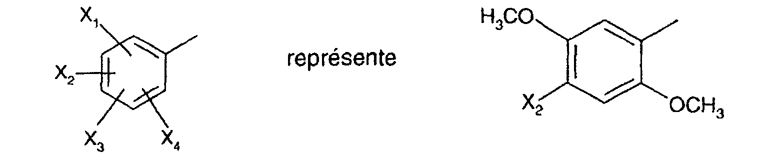 X₂ représentant un méthyle ou un atome de chlore ;
un de ses sels ou solvates.

6. Composé de formule : 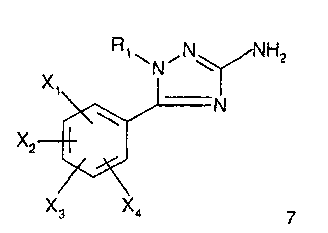 dans lequel R₁, X₁, X₂, X₃ et X₄ sont tels que définis pour (I) à la revendication 1.

7. Procédé de préparation d'un composé de formule (I) selon l'une quelconque des revendications 1 à 5 comprenant l'étape consistant à faire réagir un aminotriazole de formule : 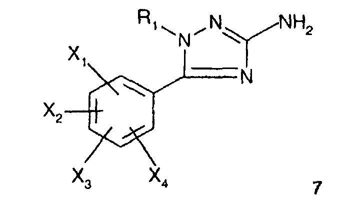 dans laquelle R₁, X₁, X₂, X₃ et X₄ sont tels que définis pour (I) à la revendication 1, avec un dérivé acide indole carboxylique de formule 8: 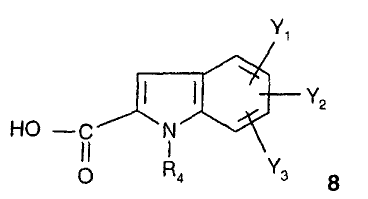 dans laquelle R₄, Y₁, Y₂ et Y₃ sont tels que définis pour (I) à la revendication 1, pour obtenir les composés de formule (I),
un de leurs sels ou solvates.

8. Procédé de préparation d'un composé de formule (I) selon l'une quelconque des revendications 1 à 5 comprenant la réaction d'un aminotriazole de formule : 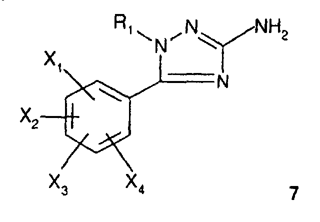 dans lequel R₁, X₁, X₂, X₃ et X₄ sont tels que définis pour (I)
• soit avec un dérivé acide indole carboxylique de formule 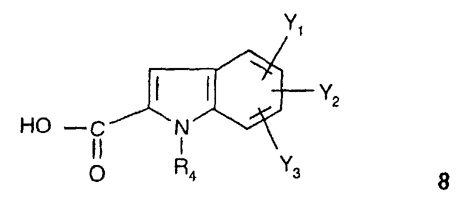 dans laquelle R₄, Y₁, Y₂ et Y₃ sont tels que définis ci-dessus pour (I) ;
• soit avec un dérivé acide indole carboxylique de formule : 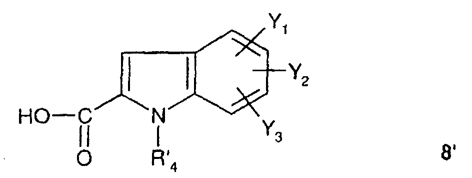 dans laquelle Y₁,Y₂,Y₃ sont tels que définis ci-dessus pour (I) et R'₄ est un groupe précurseur de R₄ pour former intermédiairement le composé de formule : 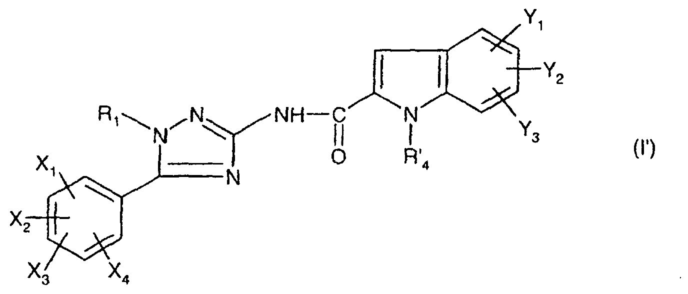 dans laquelle R₁, X₁, X₂, X₃, X₄, Y₁, Y₂ et Y₃ sont tels que définis pour (I) et R'₄ est un groupe précurseur de R₄, R₄ étant tel que défini pour (I)

9. Composition pharmaceutique contenant à titre de principe actif, un composé de formule (I) selon la revendication 1 ou un de ses sels pharmaceutiquement acceptables.

10. Composition pharmaceutique contenant, en tant que principe actif, un composé selon la revendication 2 ou un de ses sels pharmaceutiquement acceptables.

11. Composition pharmaceutique contenant, en tant que principe actif, un composé selon la revendication 3 ou un de ses sels pharmaceutiquement acceptables.

12. Composition pharmaceutique contenant, en tant que principe actif, un composé selon la revendication 4 ou un de ses sels pharmaceutiquement acceptables.

13. Composition pharmaceutique contenant, en tant que principe actif, un composé selon la revendication 5 ou un de ses sels pharmaceutiquement acceptables.

14. Utilisation d'un composé selon l'une quelconque des revendications 1 à 5 pour la préparation de médicaments destinés à traiter les désordres alimentaires, l'obésité et à réduire la prise de nourriture.

15. Utilisation d'un composé selon l'une quelconque des revendications 1 à 5 pour la préparation de médicaments destinés à traiter la dyskinésie tardive.

16. Utilisation d'un composé selon l'une quelconque des revendications 1 à 5 pour la préparation de médicaments destinés à traiter les troubles de la sphère gastrointestinale.

## Patentansprüche

1. Verbindung der Formel: 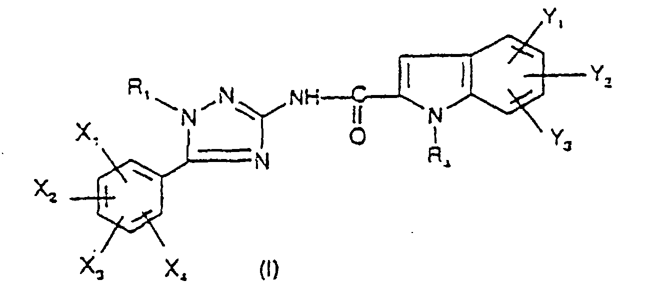 in der:
- R₁ (C₂-C₆)-Alkyl; eine Gruppe -(CH₂)ₘ-G, worin m einen Wert von 0 bis 5 aufweist und G eine mono- oder polycyclische, nichtaromatische C₃-C₁₃-Kohlenwasserstoffgruppe darstellt, die gegebenenfalls durch eine oder mehrere (C₁-C₃)-Alklygruppen substituiert ist; Phenyl-(C₁-C₃)-alkyl, worin die Phenylgruppe gegebenenfalls ein- oder mehrfach durch Halogen, (C₁-C₃)-Alkyl oder (C₁-C₃)-Alkoxy substituiert ist; eine Gruppe -(CH₂)ₙNR₂R₃, worin n eine ganze Zahl mit einem Wert von 1 bis 6 darstellt und R₂ und R₃, die gleichartig oder verschieden sind, (C₁-C₃)-Alkyl oder zusammen mit dem Stickstoffatom, an das sie gebunden sind, eine Morpholino-, Piperidino-, Pyrrolidinyl- oder Piperazinylgruppe darstellen, bedeutet;
- X₁, X₂, X₃ oder X₄ jeweils unabhängig voneinander Wasserstoffatome, Halogenatome, (C₁-C₆)-Alkyl, (C₁-C₃)-Alkoxy oder Trifluormethyl bedeuten; mit der Maßgabe, daß lediglich eine der Gruppen X₁, X₂, X₃ und X₄ gegebenenfalls ein Wasserstoffatom darstellt;
- R₄ Wasserstoff, eine Gruppe -(CH₂)ₙCOOR₅, in der n die oben angegebene Bedeutung besitzt und R₅ ein Wasserstoffatom, (C₁-C₆)-Alkyl oder (C₆-C₁₀)-Aryl-(C₁-C₆)-alkyl darstellt; (C₁-C₆)-Alkyl; eine Gruppe -(CH₂)ₙOR₅ oder eine Gruppe -(CH₂)nNR₂R₃, worin n, R₂, R₃ und R₅ die oben angegebenen Bedeutungen besitzen; eine -(CH₂)ₙ-Tetrazolylgruppe, worin n die oben angegebene Bedeutung besitzt; oder R₄ eine dieser Gruppen in Form des Alkalimetall- oder Erdalkalimetallsalzes bedeutet;
- Y₁, Y₂ und Y₃ unabhängig voneinander Wasserstoff, Halogen, (C₁-C₃)-Alkyl, (C₁-C₃)-Alkoxy, Nitro, Cyano, (C₁-C₆)-Acylamino, Carbamoyl, Trifluormethyl, eine Gruppe COOR₆, worin R₆ Wasserstoff oder (C₁-C₃)-Alkyl darstellt, bedeuten; oder eines ihrer Salze oder Solvate.

2. Verbindung der Formel (I) nach Anspruch 1, worin R₁, R₄, X₁, X₂, X₃ und X₄ die in Anspruch 1 angegebenen Bedeutungen besitzen und Y₁, Y₂ und Y₃ Wasserstoff bedeuten; oder ihre Salze oder Solvate.

3. Verbindung der Formel (I) nach Anspruch 1, worin R₁ und R₄ die in Anspruch 1 angegebenen Bedeutungen besitzen, Y₁, Y₂ und Y₃ Wasserstoff bedeuten; und 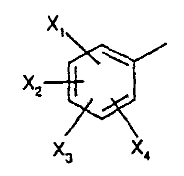 2,6-Dimethoxy-4-methylphenyl darstellt;
und deren Salze oder Solvate.

4. Verbindung der Formel (I) nach Anspruch 1, worin R₁, R₄, Y₁, Y₂ und Y₃ die in Anspruch 1 angegebenen Bedeutungen besitzen und 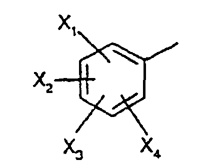 2,6-Dimethoxy-4-methylphenyl bedeutet;
und deren Salze oder Solvate.

5. Verbindung der Formel (I) nach Anspruch 1, worin R₁, R₄, Y₁, Y₂ und Y₃ die in Anspruch 1 angegebenen Bedeutungen besitzen und 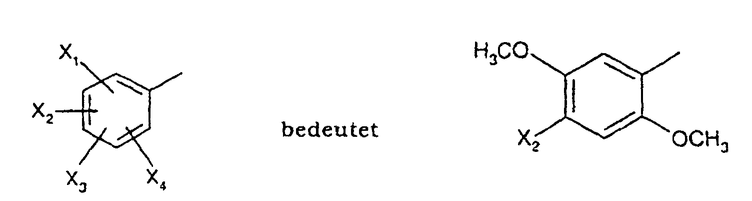 worin X₂ Methyl oder ein Chloratom darstellt;
deren Salze oder Solvate.

6. Verbindung der Formel: 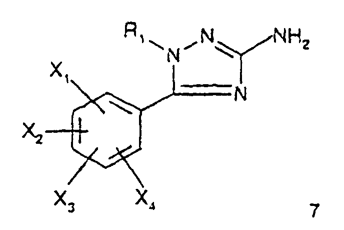 in der R₁, X₁, X₂, X₃ und X₄ die bezüglich (I) in Anspruch 1 angegebenen Bedeutungen besitzen.

7. Verfahren zur Herstellung einer Verbindung der Formel (I) nach irgendeinem der Ansprüche 1 bis 5 umfassend eine Stufe, die darin besteht, ein Aminotriazol der Formel: 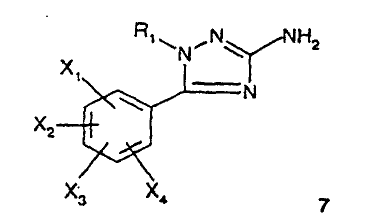 in der R₁, X₁, X₂, X₃ und X₄ die bezüglich (I) in Anspruch 1 angegebenen Bedeutungen besitzen, mit einem Indolcarbonsäurederivat der Formel 8: 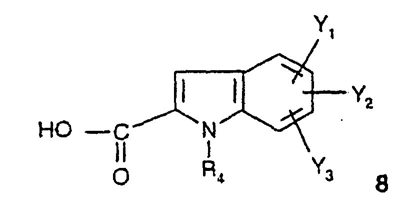 in der R₄, Y₁, Y₂ und Y₃ die bezüglich (I) in Anspruch 1 angegebenen Bedeutungen besitzen, umsetzt zur Bildung der Verbindungen der Formel (I)
oder deren Salze oder Solvate.

8. Verfahren zur Herstellung einer Verbindung der Formel (I) nach irgendeinem der Ansprüche 1 bis 5 umfassend die Umsetzung eines Aminotriazols der Formel: 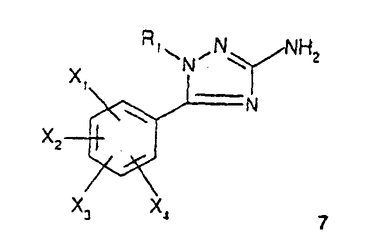 in der R₁, X₁, X₂, X₃ und X₄ die bezüglich (I) angegebenen Bedeutungen besitzen,
• entweder mit einem Indolcarbonsäurederivat der Formel 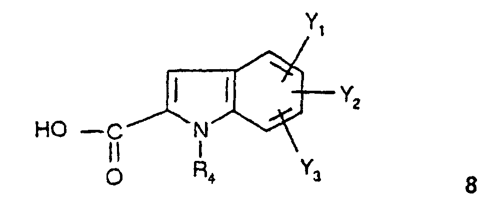 in der R₄, Y₁, Y₂ und Y₃ die oben bezüglich (I) angegebenen Bedeutungen besitzen:
• oder mit einem Indolcarbonsäurederivat der Formel: 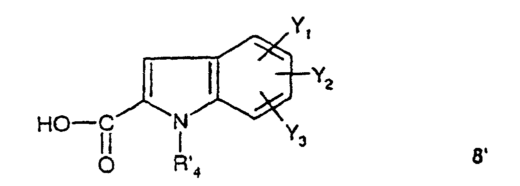 in der Y₁, Y₂ und Y₃ die oben bezüglich (I) angegebenen Bedeutungen besitzen und R'₄ eine Vorläufergruppe für R₄ darstellt, zur Bildung der Zwischenverbindung der Formel: 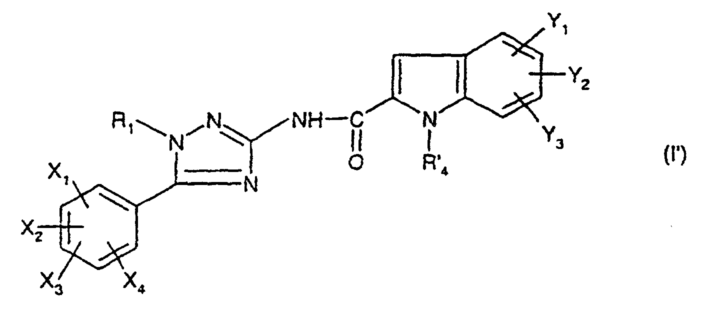 in der R₁, X₁, X₂, X₃, X₄, Y₁, Y₂ und Y₃ die bezüglich (I) angegebenen Bedeutungen besitzen und R'₄ eine Vorläufergruppe für R₄ darstellt, worin R₄ die bezüglich (I) angegebenen Bedeutungen besitzt.

9. Pharmazeutische Zubereitung enthaltend als Wirkstoff eine Verbindung der Formel (I) nach Anspruch 1 oder eines ihrer pharmazeutisch annehmbaren Salze.

10. Pharmazeutische Zubereitung enthaltend als Wirkstoff eine Verbindung nach Anspruch 2 oder eines ihrer pharmazeutisch annehmbaren Salze.

11. Pharmazeutische Zubereitung enthaltend als Wirkstoff eine Verbindung nach Anspruch 3 oder eines ihrer pharmazeutisch annehmbaren Salze.

12. Pharmazeutische Zubereitung enthaltend als Wirkstoff eine Verbindung nach Anspruch 4 oder eines ihrer pharmazeutisch annehmbaren Salze.

13. Pharmazeutische Zubereitung enthaltend als Wirkstoff eine Verbindung nach Anspruch 5 oder eines ihrer pharmazeutisch annehmbaren Salze.

14. Verwendung einer Verbindung nach irgendeinem der Ansprüche 1 bis 5 für die Herstellung von Arzneimitteln zur Behandlung von Ernährungsstörungen, der Fettsucht und zur Verringerung der Nahrungsaufnahme.

15. Verwendung einer Verbindung nach irgendeinem der Ansprüche 1 bis 5 für die Herstellung von Arzneimitteln zur Behandlung der tardiven Dyskinesie.

16. Verwendung einer Verbindung nach irgendeinem der Ansprüche 1 bis 5 für die Herstellung von Arzneimitteln zur Behandlung von Störungen des gastro-intestinalen Bereiches.

## Claims

1. Compound of formula: 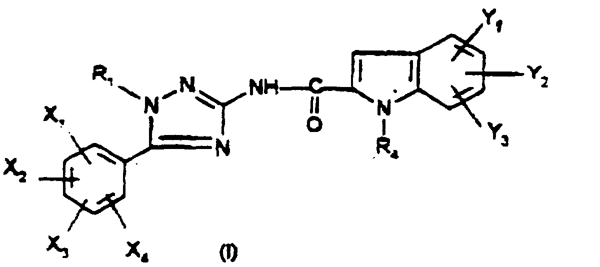 in which:
- R₁ represents a (C₂-C₆)alkyl; a group -(CH₂)ₘ-G with m ranging from 0 to 5 and G representing a non-aromatic C₃-C₁₃ mono- or polycyclic hydrocarbon group optionally substituted with one or more (C₁-C₃)alkyl; a phenyl(C₁-C₃)alkyl in which the phenyl group is optionally substituted one or more times with a halogen, with a (C₁-C₃)alkyl or with a (C₁-C₃)alkoxy; a group -(CH₂)ₙNR₂R₃ in which n represents an integer from 1 to 6 and R₂ and R₃, which may be identical or different, represent a (C₁-C₃)alkyl or constitute, with the nitrogen atom to which they are attached, a morpholino, piperidino, pyrrolidinyl or piperazinyl group;
- X₁, X₂, X₃ or X₄ each independently represents a hydrogen or halogen atom, a (C₁-C₆)alkyl, a (C₁-C₃)alkoxy or a trifluoromethyl; it being understood that only one from among X₁, X₂, X₃ and X₄ possibly represents a hydrogen atom;
- R₄ represents hydrogen, a group -(CH₂)ₙCOOR₅ in which n is as defined above and R₅ represents a hydrogen atom, a(C₁-C₆)alkyl or a (C₆-C₁₀)aryl-(C₁-C₆)alkyl; a (C₁-C₆)alkyl; a group -(CH₂)ₙOR₅ or a group -(CH₂)ₙNR₂R₃ in which n, R₂, R₃ and R₅ are as defined above; a group-(CH₂)ₙ-tetrazolyl in which n is as defined above, or R₄ represents one of these groups in the form of an alkali-metal or alkaline-earth metal salt;
- Y₁, Y₂ and Y₃ independently represent a hydrogen, a halogen, a (C₁-C₃)alkyl, a (C₁-C₃)alkoxy, a nitro, cyano, (C₁-C₆)acylamino, carbamoyl, trifluoromethyl, a group COOR₆ in which R₆ represents hydrogen, or (C₁-C₃) alkyl;
or one of the salts or solvates thereof.

2. Compound of formula (I) according to Claim 1, in which R₁, R₁, X₁, X₂, X₃ and X₄ are as defined in Claim 1 and Y₁, Y₂ and Y₃ represent hydrogen; a salt or solvate thereof.

3. Compound of formula (I) according to Claim 1, in which R₁ and R₄ are as defined in Claim 1, Y₁, Y₂ and Y₃ represent hydrogen; and 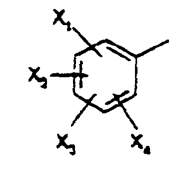 represents 2,6-dimethoxy-4-methylphenyl;
a salt or solvate thereof.

4. Compound of formula (I) according to Claim 1, in which R₁, R₄, Y₁, Y₂ and Y₃ are as defined in Claim 1, and 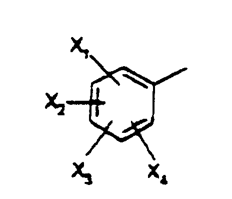 represents 2,6-dimethoxy-4-methylphenyl;
a salt or solvate thereof.

5. Compound of formula (I) according to Claim 1, in which R₁, R₄, Y₁, Y₂ and Y₃ are as defined in Claim 1, and 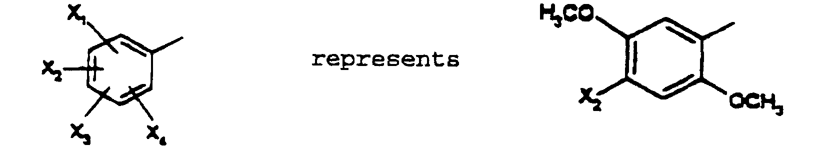 X₂ representing a methyl or a chlorine atom;
a salt or solvate thereof.

6. Compound of formula: 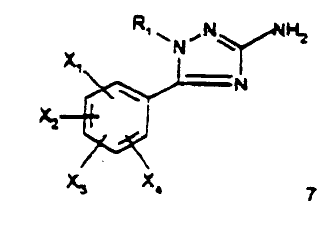 in which R₁, X₁, X₂, X₃ and X₄ are as defined for (I) in Claim 1.

7. Process for the preparation of a compound of formula (I) according to any one of Claims 1 to 5, comprising the step consisting in reacting an aminotriazole of formula: 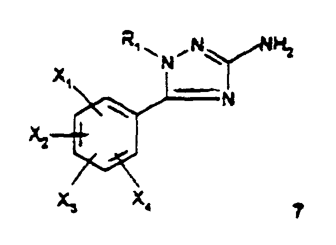 in which R₁, X₁, X₂, X₃ and X₄ are as defined for (I) in Claim 1, with an indolecarboxylic acid derivative of formula 8: 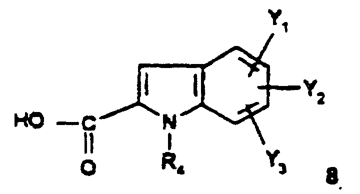 in which R₄, Y₁, Y₂ and Y₃ are as defined for (I) in Claim 1, in order to obtain the compounds of formula (I),
a salt or solvate thereof.

8. Process for the preparation of a compound of formula (I) according to any one of Claims 1 to 5, comprising the reaction of an aminotriazole of formula: 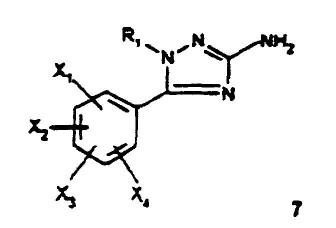 in which R₁, X₁, X₂, X₃ and X₄ are as defined for (I)
- either with an indolecarboxylic acid derivative of formula: 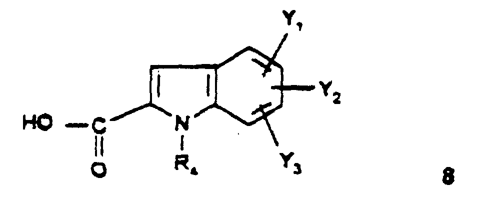 in which R₄, Y₁, Y₂ and Y₃ are as defined above for (I);
- or with an indolecarboxylic acid derivative of formula: 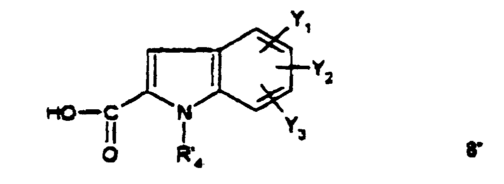 in which Y₁, Y₂ and Y₃ are as defined above for (I) and R'₄ is a precursor group of R₄, in order to form the compound of formula: 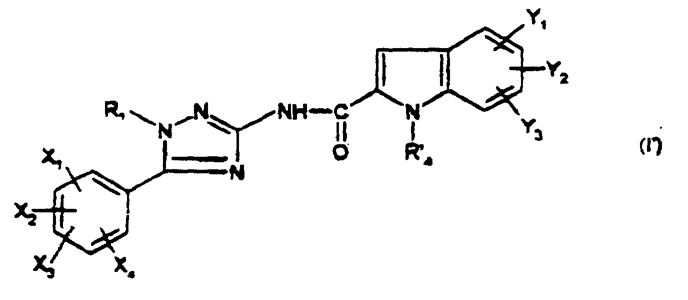 in which R₁, X₁, X₂, X₃, X₄, Y₁, Y₂ and Y₃ are as defined for (I) and R'₄ is a precursor group of R₄, R₄ being defined for (I), as an intermediate.

9. Pharmaceutical composition containing, as active principle, a compound of formula (I) according to Claim 1, or one of the pharmaceutically acceptable salts thereof.

10. Pharmaceutical composition containing, as active principle, a compound according to Claim 2, or one of the pharmaceutically acceptable salts thereof.

11. Pharmaceutical composition containing, as active principle, a compound according to Claim 3, or one of the pharmaceutically acceptable salts thereof.

12. Pharmaceutical composition containing, as active principle, a compound according to Claim 4, or one of the pharmaceutically acceptable salts thereof.

13. Pharmaceutical composition containing, as active principle, a compound according to Claim 5, or one of the pharmaceutically acceptable salts thereof.

14. Use of a compound according to any one of Claims 1 to 5 for the preparation of medicines intended to treat dietary disorders and obesity and to reduce the intake of food.

15. Use of a compound according to any one of Claims 1 to 5, for the preparation of medicines intended to treat tardive dyskinesia.

16. Use of a compound according to any one of Claims 1 to 5 for the preparation of medicines intended to treat disorders of the gastrointestinal sphere.
